# EUROPEAN PATENT SPECIFICATION

(11) **EP 4 518 968 B1**
(45) Date of publication and mention of the grant of the patent: **04.11.2026**
(21) Application number: 23728167.0
(22) Date of filing: 03.05.2023
(51) Int. Cl.: A61P 1/00, A61P 9/00, A61P 13/00, A61P 21/00, A61P 35/00, C07D 241/44, C07D 401/06, C07D 401/12, C07D 401/14, C07D 403/06, C07D 407/12, C07D 409/06, C07D 413/06, A61K 31/498, C07D 471/04, C07D 475/00, A61K 31/519, A61K 31/4985

(54) **BICYCLIC PIPERAZINONES AND THERAPEUTIC USE IN THE TREATMENT OF NEUROMUSCULAR DISORDERS**
BICYCLISCHE PIPERAZINONE UND IHRE THERAPEUTISCHE VERWENDUNG ZUR BEHANDLUNG VON NEUROMUSKULÄREN ERKRANKUNGEN
PIPÉRAZINONES BICYCLIQUES ET LEURS UTILISATIONS THÉRAPEUTIQUES POUR LE TRAITEMENT DES MALADIES NEUROMUSCULAIRES

(30) Priority: 04.05.2022 US 202263338375 P
(43) Date of publication of application: 12.03.2025
(73) Proprietor: Cytokinetics, Inc., South San Francisco CA 94080 (US)
(72) Inventor: EVANS, Chris, South San Francisco, California 94080 (US); MORGAN, Bradley P., South San Francisco, California 94080 (US); COLLIBEE, Scott, South San Francisco, California 94080 (US); MAKINO, Takuya, Tokyo, 103-8411 (JP); KUROSAKI, Toshio, Tokyo, 103-8411 (JP); HONJO, Eriko, Tokyo, 103-8411 (JP); KOIZUMI, Yuka, Tokyo, 103-8411 (JP)
(74) Representative: Carpmaels & Ransford LLP
(86) International application number: PCT/US2023/020814
(87) International publication number: WO 2023/215367

(56) References cited:
- WO-A1-2004/054584
- WO-A1-2014/040077
- WO-A2-2006/094210
- TEMPEST PAUL ET AL: "Two-step solution-phase synthesis of novel benzimidazoles utilizing a UDC (Ugi/de-Boc/cyclize) strategy", TETRAHEDRON LETTERS, vol. 42, no. 30, 2001, pages 4959 - 4962, XP085002661, ISSN: 0040-4039, DOI: 10.1016/S0040-4039(01)00919-4

## Description

### CROSS REFERENCE TO RELATED APPLICATIONS

### FIELD

Provided herein are bicyclic piperazinone compounds, pharmaceutical compositions comprising such compounds, and methods of using such compounds and pharmaceutical compositions for treating various diseases, disorders, and conditions responsive to the modulation of the contractility of the skeletal sarcomere.

### BACKGROUND

The cytoskeleton of skeletal and cardiac muscle cells is unique compared to that of all other cells. It consists of a nearly crystalline array of closely packed cytoskeletal proteins called the sarcomere. The sarcomere is elegantly organized as an interdigitating array of thin and thick filaments. The thick filaments are composed of myosin, the motor protein responsible for transducing the chemical energy of ATP hydrolysis into force and directed movement. The thin filaments are composed of actin monomers arranged in a helical array. There are four regulatory proteins bound to the actin filaments, which allows the contraction to be modulated by calcium ions. An influx of intracellular calcium initiates muscle contraction; thick and thin filaments slide past each other driven by repetitive interactions of the myosin motor domains with the thin actin filaments.

Of the thirteen distinct classes of myosin in human cells, the myosin-II class is responsible for contraction of skeletal, cardiac, and smooth muscle. This class of myosin is significantly different in amino acid composition and in overall structure from myosin in the other twelve distinct classes. Myosin-II forms homo-dimers resulting in two globular head domains linked together by a long alpha-helical coiled-coiled tail to form the core of the sarcomere's thick filament. The globular heads have a catalytic domain where the actin binding and ATPase functions of myosin take place. Once bound to an actin filament, the release of phosphate (cf. ADP-Pi to ADP) signals a change in structural conformation of the catalytic domain that in turn alters the orientation of the light-chain binding lever arm domain that extends from the globular head; this movement is termed the power stroke. This change in orientation of the myosin head in relation to actin causes the thick filament, of which it is a part, to move with respect to the thin actin filament, to which it is bound. Un-binding of the globular head from the actin filament (Ca²⁺ regulated), coupled with return of the catalytic domain and light chain to their starting conformation/orientation completes the catalytic cycle, is responsible for intracellular movement and muscle contraction.

Tropomyosin and troponin mediate the calcium effect on the interaction on actin and myosin. The troponin complex is comprised of three polypeptide chains: troponin C, which binds calcium ions; troponin I, which binds to actin; and troponin T, which binds to tropomyosin. The skeletal troponin-tropomyosin complex regulates the myosin-binding sites extending over several actin units at once.

Troponin, a complex of the three polypeptides described above, is an accessory protein that is closely associated with actin filaments in vertebrate muscle. The troponin complex acts in conjunction with the muscle form of tropomyosin to mediate the Ca²⁺ dependency of myosin ATPase activity and thereby regulate muscle contraction. The troponin polypeptides T, I, and C, are named for their tropomyosin binding, inhibitory, and calcium binding activities, respectively. Troponin T binds to tropomyosin and is believed to be responsible for positioning the troponin complex on the muscle thin filament. Troponin I binds to actin, and the complex formed by troponin I, troponin T, and tropomyosin inhibits the interaction of actin and myosin. Skeletal troponin C is capable of binding up to four calcium molecules. Studies suggest that when the level of calcium in the muscle is raised, troponin C exposes a binding site for troponin I, recruiting it away from actin. This causes the tropomyosin molecule to shift its position as well, thereby exposing the myosin binding sites on actin and stimulating myosin ATPase activity.

Human skeletal muscle is composed of different types of contractile fibers, classified by their myosin type and termed either slow or fast fibers. Table 1 summarizes the different proteins that make up these types of muscle.

**Table 1**

| | Muscle Fiber Type | |
|---|---|---|
| | Fast Skeletal | Slow Skeletal |
| Myosin Heavy Chain (MHC) | IIa, (IIb*), IIx/d | Cardiac β |
| Troponin I (TnI) | TnI fast Skeletal | TnI slow Skeletal |
| Troponin T (TnT) | TnT fast Skeletal | TnT slow Skeletal |
| Troponin C (TnC) | TnC fast Skeletal | TnC slow/cardiac |
| Tropomyosin (TM) | TM-β/ TM-α/TPM3** | TM-β/ TM-αs |

| | | |
|---|---|---|
| *MHC IIb is not expressed in human muscle but is present in rodents and other mammals. **TPM3 represents tropomyosin 3 | | |

In healthy humans, most skeletal muscles are composed of both fast and slow fibers, although the proportions of each vary with muscle type. Slow skeletal fibers, often called type I fibers, have more structural similarity with cardiac muscle and tend to be used more for fine and postural control. They usually have a greater oxidative capacity and are more resistant to fatigue with continued use. Fast skeletal muscle fibers, often called type II fibers, are classified into fast oxidative (IIa) and fast glycolytic (type IIx/d) fibers. While these muscle fibers have different myosin types, they share many components, including the troponin and tropomyosin regulatory proteins. Fast skeletal muscle fibers tend to exert greater force but fatigue faster than slow skeletal muscle fibers and are functionally useful for acute, large scale movements such as rising from a chair or correcting falls.

Muscle contraction and force generation is controlled through nervous stimulation by innervating motor neurons. Each motor neuron may innervate many (approximately 100 to 380) muscle fibers as a contractile whole, termed a motor unit. When a muscle is required to contract, motor neurons send stimuli as nerve impulses (action potentials) from the brain stem or spinal cord to each fiber within the motor unit. The contact region between nerve and muscle fibers is a specialized synapse called the neuromuscular junction (NMJ). Here, membrane depolarizing action potentials in the nerve are translated into an impulse in the muscle fiber through release of the neurotransmitter acetylcholine (ACh). ACh triggers a second action potential in the muscle that spreads rapidly along the fiber and into invaginations in the membrane, termed t-tubules. T-tubules are physically connected to Ca²⁺ stores within the sarcoplasmic reticulum (SR) of muscle via the dihydropyridine receptor (DHPR). Stimulation of the DHPR activates a second Ca²⁺ channel in the SR, the ryanodine receptor, to trigger the release of Ca²⁺ from stores in the SR to the muscle cytoplasm where it can interact with the troponin complex to initiate muscle contraction. If muscle stimulation stops, calcium is rapidly taken back up into the SR through the ATP dependent Ca²⁺ pump, sarco/endoplasmic reticulum Ca²⁺-ATPase (SERCA).

Currently, there is limited treatment or no cure for most neuromuscular diseases. Thus, there is a need for the development of new compounds that modulate skeletal muscle contractility. There remains a need for agents that exploit new mechanisms of action and which may have better outcomes in terms of relief of symptoms, safety, and patient mortality, both short-term and long-term and an improved therapeutic index.

### SUMMARY

The invention provides compounds that are expected to be used as an active ingredient in a pharmaceutical composition, and in particular, in a pharmaceutical composition for preventing or treating a disease or condition responsive to modulation of the contractility of the skeletal sarcomere. Modulation of the skeletal sarcomere may be modulation, for example, by modulation of the troponin complex of the fast skeletal muscle sarcomere through one or more of fast skeletal myosin, actin, tropomyosin, troponin C, troponin I, and troponin T, and fragments and isoforms thereof.

In one aspect, provided herein is a compound of formula (I): 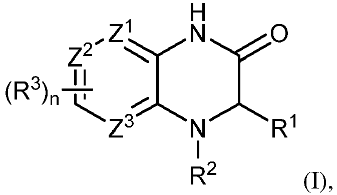 or a pharmaceutically acceptable salt thereof, wherein:
Z¹, Z², and Z³ are, independently of each other, CH, CR³, or N, provided that at least one of Z¹, Z², and Z³ is CH or CR³;
R¹ is unsubstituted C₃-C₄ alkyl;
R² is -C(=O)R⁴, -C(=O)-(CH₂)ₚ-R⁵, -C(=O)NR⁶R⁷, or -C(=O)OCH₃;
each R³ is independently halogen, C₁-C₆-alkyl, C₁-C₆-haloakyl, C₁-C₆-alkoxy, C₁-C₆-haloakoxy, C₃-C₁₂-cycloalkyl, or 3-12 membered heterocycloalkyl;
R⁴ is C₆-C₁₂-aryl optionally substituted with one to five R^{4A}, 5-12 membered heteroaryl optionally substituted with one to five R^{4B}, C₃-C₁₂ cycloalkyl optionally substituted with one to five R^{4C}, 4-12 membered heterocycloalkyl optionally substituted with one to five R^{4D}, or 4-12 membered heterocycloalkenyl optionally substituted with one to five R^{4E};
each R^{4A} is independently selected from the group consisting of halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), C₆-C₁₂-aryl, and 5-12 membered heteroaryl;
each R^{4B} is independently selected from the group consisting of halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), and 5-12 membered heteroaryl;
each R^{4B1} is independently selected from the group consisting of halo, C₁-C₆-alkyl, C₁-C₆-haloalkyl, OH, C₁-C₆-alkoxy, C₁-C₆-haloalkoxy, and C₁-C₆-alkylene-OH;
each R^{4C} is independently selected from the group consisting of halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH; each R^{4D} is independently selected from the group consisting of halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH; each R^{4E} is independently selected from the group consisting of oxo, halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
R⁵ is OH, C₁-C₆-alkoxy, C₁-C₆-haloalkoxy, C₃-C₁₂ cycloalkyl, 3-12 membered heterocycloalkyl, or C₆-C₁₂-aryl;
R⁶ and R⁷, independently of each other, are selected from the group consisting of H, C₁-C₆-alkyl, C₁-C₆-haloalkyl, C₃-C₁₂ cycloalkyl optionally substituted with one to five R⁸, 4-12 membered heterocycloalkyl optionally substituted with one to five R⁸, C₆-C₁₂-aryl optionally substituted with one to five R⁸, 5-12 membered heteroaryl optionally substituted with one to five R⁸, C₁-C₆-alkylene-OH, C₁-C₆-alkylene-(C₃-C₁₂ cycloalkyl), C₁-C₆-alkylene-(4-12 membered heterocycloalkyl), C₁-C₆-alkylene-(C₆-C₁₂-aryl), and C₁-C₆-alkylene-(5-12 membered heteroaryl);
each R⁸ is independently selected from the group consisting of halo, C₁-C₆-alkyl, C₁-C₆-haloalkyl, OH, C₁-C₆-alkoxy, C₁-C₆-alkylene-OH, and C₁-C₆-haloalkoxy;
n is 0, 1, 2, 3, or 4;
p is 1, 2, or 3; and
provided that the compound of Formula (I) is not a compound selected from the group consisting of:
   4-(3-methylbenzoyl)-3-propyl-3,4-dihydroquinoxalin-2(1H)-one,
   4-(2-chlorobenzoyl)-3-isobutyl-3,4-dihydroquinoxalin-2(1H)-one, and
   4-(4-(tert-butyl)benzoyl)-3-propyl-3,4-dihydroquinoxalin-2(1H)-one.

In another aspect provided herein is a compound of formula (II): 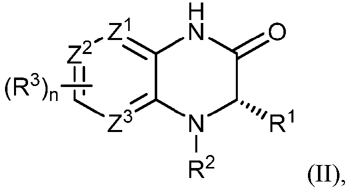 or a pharmaceutically acceptable salt thereof, wherein Z¹, Z², Z³, R¹, R², R³, and n are as defined for the compound of formula (I), or any variation or embodiment thereof.

In another aspect provided herein is a compound of formula (II-a): 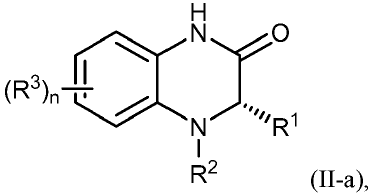 or a pharmaceutically acceptable salt thereof, wherein R¹, R², R³, and n are as defined for the compound of formula (I), or any variation or embodiment thereof.
WO2014/040077 relates to "alpha7betal integrin modulatory agents and methods of using such to treat conditions associated with decreased alpha7beta1 integrin expression or activity, including muscular dystrophy".
WO2006/094210 relates to "sirtuin-modulating compounds and methods of use thereof" and also "compositions comprising a sirtuin-modulating compound in combination with another therapeutic agent".

In another aspect, provided herein is a compound selected from Table 2, or a pharmaceutically acceptable salt thereof.
The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In another aspect, provided herein is a method for treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty, in a subject, comprising administering to the subject an effective amount of one or more of the compounds described herein, or a pharmaceutically acceptable salt thereof.

In another aspect, provided herein is a method for treating a disease or condition selected from the group consisting of amyotrophic lateral sclerosis (ALS), spinal muscular atrophy (SMA), myasthenia gravis, and muscular myopathies, in a subject, comprising administering to the subject an effective amount of one or more of the compounds described herein, or a pharmaceutically acceptable salt thereof.

In another aspect, provided herein is a method for treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction, in a subject, comprising administering to the subject an effective amount of one or more of the compounds described herein, or a pharmaceutically acceptable salt thereof.

In another aspect, provided herein is a use of one or more of the compounds described herein, or a pharmaceutically acceptable salt thereof, for the manufacture of a medicament for treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject.

In another aspect, provided herein is a use of one or more of the compounds described herein, or a pharmaceutically acceptable salt thereof, for the manufacture of a medicament for treating a disease or condition selected from the group consisting of amyotrophic lateral sclerosis (ALS), spinal muscular atrophy (SMA), myasthenia gravis, and muscular myopathies in a subject.

In another aspect, provided herein is a use of one or more of the compounds described herein, or a pharmaceutically acceptable salt thereof, for the manufacture of a medicament for treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

In another aspect, provided herein is one or more of the compounds described herein, or a pharmaceutically acceptable salt thereof, for use in treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject.

In another aspect, provided herein is one or more of the compounds described herein, or a pharmaceutically acceptable salt thereof, for use in treating a disease or condition selected from the group consisting of amyotrophic lateral sclerosis (ALS), spinal muscular atrophy (SMA), myasthenia gravis, and muscular myopathies in a subject.

In another aspect, provided herein is one or more of the compounds described herein, or a pharmaceutically acceptable salt thereof, for use in treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

Unless specifically described otherwise, when symbols in one formula in the specification are also used in other formulas, the same symbols denote the same meanings. When the same symbol is used more than once in a given formula, it is to be understood that each instance of that symbol in the formula represents an independently selected chemical moiety and that all instances of the symbol in the formula need not necessarily represent identical chemical moieties.

In a further aspect, the present invention relates to a pharmaceutical composition comprising one or more of the compounds described herein, or a pharmaceutically acceptable salt thereof, for preventing or treating various diseases, disorders, and conditions responsive to the modulation of the contractility of the skeletal sarcomere.

In some aspects, the present invention relates to methods of preventing or treating frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, using one or more of the compounds described herein, or a pharmaceutically acceptable salt thereof, or a pharmaceutical composition thereof.

Additional embodiments, features, and advantages of the present disclosure will be apparent from the following detailed description and through practice of the present disclosure.

### DETAILED DESCRIPTION

### Definitions

As used in the present specification, the following words and phrases are generally intended to have the meanings as set forth below, except to the extent that the context in which they are used indicates otherwise.

Throughout this application, unless the context indicates otherwise, references to a compound of formula (I) includes all subgroups of formula (I) defined herein, such as formula (I-a), (I-b), (I-c), (I-d), (I-e), (II), (II-a), (II-b), (II-c), (II-d), or (II-e), including all substructures, subgenera, preferences, embodiments, examples, and particular compounds defined and/or described herein. References to a compound of formula (I) and subgroups thereof, such as formula (I-a), (I-b), (I-c), (I-d), (I-e), (II), (II-a), (II-b), (II-c), (II-d), or (II-e), include ionic forms, polymorphs, pseudopolymorphs, amorphous forms, solvates, co-crystals, chelates, tautomers, and/or isotopes thereof. In some embodiments, references to a compound of formula (I) and subgroups thereof, such as formula (I-a), (I-b), (I-c), (I-d), (I-e), (II), (II-a), (II-b), (II-c), (II-d), or (II-e), include polymorphs, solvates, co-crystals, and/or tautomers thereof. In some embodiments, references to a compound of formula (I) and subgroups thereof, such as (I-a), (I-b), (I-c), (I-d), (I-e), (II), (II-a), (II-b), (II-c), (II-d), or (II-e), include polymorphs, solvates, and/or co-crystals thereof. In some embodiments, references to a compound of formula (I) and subgroups thereof, such as formula (I-a), (I-b), (I-c), (I-d), (I-e), (II), (II-a), (II-b), (II-c), (II-d), or (II-e), include isomers, tautomers and/or oxides thereof. In some embodiments, references to a compound of formula (I) and subgroups thereof, such as formula (I-a), (I-b), (I-c), (I-d), (I-e), (II), (II-a), (II-b), (II-c), (II-d), or (II-e), include solvates thereof. Similarly, the term "salts" includes solvates of salts of compounds.

"Alkyl" encompasses straight and branched carbon chains having the indicated number of carbon atoms, for example, from 1 to 20 carbon atoms, or 1 to 8 carbon atoms, or 1 to 6 carbon atoms. For example, C₁₋₆ alkyl encompasses both straight and branched chain alkyl of from 1 to 6 carbon atoms. When an alkyl residue having a specific number of carbons is named, all branched and straight chain versions having that number of carbons are intended to be encompassed; thus, for example, "propyl" includes n-propyl and isopropyl; and "butyl" includes n-butyl, sec-butyl, isobutyl and t-butyl. Examples of alkyl groups include, but are not limited to, methyl, ethyl, propyl, isopropyl, n-butyl, sec-butyl, tert-butyl, pentyl, 2-pentyl, 3-pentyl, isopentyl, neopentyl, hexyl, 2-hexyl, 3-hexyl, and 3-methylpentyl.

When a range of values is given (e.g., C₁₋₆ alkyl), each value within the range as well as all intervening ranges are included. For example, "C₁₋₆ alkyl" includes C₁, C₂, C₃, C₄, C₅, C₆, C₁₋₆, C₂₋₆, C₃₋₆, C₄₋₆, C₅₋₆, C₁₋₅, C₂₋₅, C₃₋₅, C₄₋₅, C₁₋₄, C₂₋₄, C₃₋₄, C₁₋₃, C₂₋₃, and C₁₋₂ alkyl.

"Alkenyl" refers to an unsaturated branched or straight-chain alkyl group having the indicated number of carbon atoms (e.g., 2 to 8, or 2 to 6 carbon atoms) and at least one carbon-carbon double bond. The group may be in either the cis or trans configuration (Z or E configuration) about the double bond(s). Alkenyl groups include, but are not limited to, ethenyl, propenyl (e.g., prop-1-en-1-yl, prop-1-en-2-yl, prop-2-en-1-yl (allyl), prop-2-en-2-yl), and butenyl (e.g., but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl).

"Alkynyl" refers to an unsaturated branched or straight-chain alkyl group having the indicated number of carbon atoms (e.g., 2 to 8 or 2 to 6 carbon atoms) and at least one carbon-carbon triple bond. Alkynyl groups include, but are not limited to, ethynyl, propynyl (e.g., prop-1-yn-1-yl, prop-2-yn-1-yl) and butynyl (e.g., but-1-yn-1-yl, but-1-yn-3-yl, but-3-yn-1-yl).

"Cycloalkyl" indicates a non-aromatic, fully saturated carbocyclic ring having the indicated number of carbon atoms, for example, 3 to 10, or 3 to 8, or 3 to 6 ring carbon atoms. Cycloalkyl groups may be monocyclic or polycyclic (e.g., bicyclic, tricyclic). Examples of cycloalkyl groups include cyclopropyl, cyclobutyl, cyclopentyl, and cyclohexyl, as well as bridged, caged, and spirocyclic ring groups (e.g., norbornane, bicyclo[2.2.2]octane, spiro[3.3]heptane). In addition, one ring of a polycyclic cycloalkyl group may be aromatic, provided the polycyclic cycloalkyl group is bound to the parent structure via a non-aromatic carbon. For example, a 1,2,3,4-tetrahydronaphthalen-1-yl group (wherein the moiety is bound to the parent structure via a non-aromatic carbon atom) is a cycloalkyl group, while 1,2,3,4-tetrahydronaphthalen-5-yl (wherein the moiety is bound to the parent structure via an aromatic carbon atom) is not considered a cycloalkyl group. Examples of polycyclic cycloalkyl groups consisting of a cycloalkyl group fused to an aromatic ring are described below.

"Cycloalkenyl" indicates a non-aromatic, partially unsaturated carbocyclic ring having the indicated number of carbon atoms, for example, 3 to 10, or 3 to 8, or 3 to 6 ring carbon atoms. Cycloalkenyl groups may be monocyclic or polycyclic (e.g., bicyclic, tricyclic). Examples of cycloalkyl groups include cyclopropenyl, cyclobutenyl, cyclopentenyl, and cyclohexenyl, as well as bridged, caged, and spirocyclic ring groups (e.g., norbornene, bicyclo[2.2.2]octene, spiro[3.3]heptene). In addition, one ring of a polycyclic cycloalkyl group may be aromatic, provided the polycyclic cycloalkyl group is bound to the parent structure via a non-aromatic carbon. For example, a 1,4-dihydronaphthalen-1-yl group (wherein the moiety is bound to the parent structure via a non-aromatic carbon atom) is a cycloalkyl group, while 1,4-dihydronaphthalen-5-yl (wherein the moiety is bound to the parent structure via an aromatic carbon atom) is not considered a cycloalkyl group.

"Aryl" indicates an aromatic carbocyclic ring having the indicated number of carbon atoms, for example, 6 to 12 or 6 to 10 carbon atoms. Aryl groups may be monocyclic or polycyclic (e.g., bicyclic, tricyclic). In some instances, both rings of a polycyclic aryl group are aromatic (e.g., naphthyl). In other instances, polycyclic aryl groups may include a non-aromatic ring fused to an aromatic ring, provided the polycyclic aryl group is bound to the parent structure via an atom in the aromatic ring. Thus, a 1,2,3,4-tetrahydronaphthalen-5-yl group (wherein the moiety is bound to the parent structure via an aromatic carbon atom) is considered an aryl group, while 1,2,3,4-tetrahydronaphthalen-1-yl (wherein the moiety is bound to the parent structure via a non-aromatic carbon atom) is not considered an aryl group. Similarly, a 1,2,3,4-tetrahydroquinolin-8-yl group (wherein the moiety is bound to the parent structure via an aromatic carbon atom) is considered an aryl group, while 1,2,3,4-tetrahydroquinolin-1-yl group (wherein the moiety is bound to the parent structure via a non-aromatic nitrogen atom) is not considered an aryl group. However, the term "aryl" does not encompass or overlap with "heteroaryl", as defined herein, regardless of the point of attachment (e.g., both quinolin-5-yl and quinolin-2-yl are heteroaryl groups). In some instances, aryl is phenyl or naphthyl. In certain instances, aryl is phenyl. Additional examples of aryl groups comprising an aromatic carbon ring fused to a non-aromatic ring are described below.

"Heteroaryl" indicates an aromatic ring containing the indicated number of atoms (e.g., 5 to 12, or 5 to 10 membered heteroaryl) made up of one or more heteroatoms (e.g., 1, 2, 3 or 4 heteroatoms) selected from N, O and S and with the remaining ring atoms being carbon. Heteroaryl groups do not contain adjacent S and O atoms. In some embodiments, the total number of S and O atoms in the heteroaryl group is not more than 2. In some embodiments, the total number of S and O atoms in the heteroaryl group is not more than 1. Unless otherwise indicated, heteroaryl groups may be bound to the parent structure by a carbon or nitrogen atom, as valency permits. For example, "pyridyl" includes 2-pyridyl, 3-pyridyl and 4-pyridyl groups, and "pyrrolyl" includes 1-pyrrolyl, 2-pyrrolyl and 3-pyrrolyl groups.

In some instances, a heteroaryl group is monocyclic. Examples include pyrrole, pyrazole, imidazole, triazole (e.g., 1,2,3-triazole, 1,2,4-triazole, 1,2,4-triazole), tetrazole, furan, isoxazole, oxazole, oxadiazole (e.g., 1,2,3-oxadiazole, 1,2,4-oxadiazole, 1,3,4-oxadiazole), thiophene, isothiazole, thiazole, thiadiazole (e.g., 1,2,3-thiadiazole, 1,2,4-thiadiazole, 1,3,4-thiadiazole), pyridine, pyridazine, pyrimidine, pyrazine, triazine (e.g., 1,2,4-triazine, 1,3,5-triazine) and tetrazine.

In some instances, both rings of a polycyclic heteroaryl group are aromatic. Examples include indole, isoindole, indazole, benzoimidazole, benzotriazole, benzofuran, benzoxazole, benzoisoxazole, benzoxadiazole, benzothiophene, benzothiazole, benzoisothiazole, benzothiadiazole, 1H-pyrrolo[2,3-b]pyridine, 1H-pyrazolo[3,4-b]pyridine, 3H-imidazo[4,5-b]pyridine, 3H-[1,2,3]triazolo[4,5-b]pyridine, 1H-pyrrolo[3,2-b]pyridine, 1H-pyrazolo[4,3-b]pyridine, 1H-imidazo[4,5-b]pyridine, 1H-[1,2,3]triazolo[4,5-b]pyridine, 1H-pyrrolo[2,3-c]pyridine, 1H-pyrazolo[3,4-c]pyridine, 3H-imidazo[4,5-c]pyridine, 3H-[1,2,3]triazolo[4,5-c]pyridine, 1H-pyrrolo[3,2-c]pyridine, 1H-pyrazolo[4,3-c]pyridine, 1H-imidazo[4,5-c]pyridine, 1H-[1,2,3]triazolo[4,5-c]pyridine, furo[2,3-b]pyridine, oxazolo[5,4-b]pyridine, isoxazolo[5,4-b]pyridine, [1,2,3]oxadiazolo[5,4-b]pyridine, furo[3,2-b]pyridine, oxazolo[4,5-b]pyridine, isoxazolo[4,5-b]pyridine, [1,2,3]oxadiazolo[4,5-b]pyridine, furo[2,3-c]pyridine, oxazolo[5,4-c]pyridine, isoxazolo[5,4-c]pyridine, [1,2,3]oxadiazolo[5,4-c]pyridine, furo[3,2-c]pyridine, oxazolo[4,5-c]pyridine, isoxazolo[4,5-c]pyridine, [1,2,3]oxadiazolo[4,5-c]pyridine, thieno[2,3-b]pyridine, thiazolo[5,4-b]pyridine, isothiazolo[5,4-b]pyridine, [1,2,3]thiadiazolo[5,4-b]pyridine, thieno[3,2-b]pyridine, thiazolo[4,5-b]pyridine, isothiazolo[4,5-b]pyridine, [1,2,3]thiadiazolo[4,5-b]pyridine, thieno[2,3-c]pyridine, thiazolo[5,4-c]pyridine, isothiazolo[5,4-c]pyridine, [1,2,3]thiadiazolo[5,4-c]pyridine, thieno[3,2-c]pyridine, thiazolo[4,5-c]pyridine, isothiazolo[4,5-c]pyridine, [1,2,3]thiadiazolo[4,5-c]pyridine, quinoline, isoquinoline, cinnoline, quinazoline, quinoxaline, phthalazine, naphthyridine (e.g., 1,8-naphthyridine, 1,7-naphthyridine, 1,6-naphthyridine, 1,5-naphthyridine, 2,7-naphthyridine, 2,6-naphthyridine), imidazo[1,2-a]pyridine, 1H-pyrazolo[3,4-d]thiazole, 1H-pyrazolo[4,3-d]thiazole and imidazo[2,1-b]thiazole.

In other instances, polycyclic heteroaryl groups may include a non-aromatic ring (e.g., cycloalkyl, cycloalkenyl, heterocycloalkyl, heterocycloalkenyl) fused to a heteroaryl ring, provided the polycyclic heteroaryl group is bound to the parent structure via an atom in the aromatic ring. For example, a 4,5,6,7-tetrahydrobenzo[d]thiazol-2-yl group (wherein the moiety is bound to the parent structure via an aromatic carbon atom) is considered a heteroaryl group, while 4,5,6,7-tetrahydrobenzo[d]thiazol-5-yl (wherein the moiety is bound to the parent structure via a non-aromatic carbon atom) is not considered a heteroaryl group. Examples of polycyclic heteroaryl groups consisting of a heteroaryl ring fused to a non-aromatic ring are described below.

"Heterocyclyl" includes heterocycloalkyl moieties and heterocycloalkenyl moieites, as defined below.

"Heterocycloalkyl" indicates a non-aromatic, fully saturated ring having the indicated number of atoms (e.g., 3 to 10, or 3 to 7, membered heterocycloalkyl) made up of one or more heteroatoms (e.g., 1, 2, 3 or 4 heteroatoms) selected from N, O and S and with the remaining ring atoms being carbon. Heterocycloalkyl groups may be monocyclic or polycyclic (e.g., bicyclic, tricyclic). Examples of heterocycloalkyl groups include oxiranyl, aziridinyl, azetidinyl, pyrrolidinyl, imidazolidinyl, pyrazolidinyl, piperidinyl, piperazinyl, morpholinyl and thiomorpholinyl. Examples include thiomorpholine S-oxide and thiomorpholine S,S-dioxide. Examples of spirocyclic heterocycloalkyl groups include azaspiro[3.3]heptane, diazaspiro[3.3]heptane, diazaspiro[3.4]octane, and diazaspiro[3.5]nonane. In addition, one ring of a polycyclic heterocycloalkyl group may be aromatic (e.g., aryl or heteroaryl), provided the polycyclic heterocycloalkyl group is bound to the parent structure via a non-aromatic carbon or nitrogen atom. For example, a 1,2,3,4-tetrahydroquinolin-1-yl group (wherein the moiety is bound to the parent structure via a non-aromatic nitrogen atom) is considered a heterocycloalkyl group, while 1,2,3,4-tetrahydroquinolin-8-yl group (wherein the moiety is bound to the parent structure via an aromatic carbon atom) is not considered a heterocycloalkyl group. Examples of polycyclic heterocycloalkyl groups consisting of a heterocycloalkyl group fused to an aromatic ring are described below.

"Heterocycloalkenyl" indicates a non-aromatic ring having the indicated number of atoms (e.g., 3 to 10, or 3 to 7, membered heterocycloalkyl) made up of one or more heteroatoms (e.g., 1, 2, 3 or 4 heteroatoms) selected from N, O and S and with the remaining ring atoms being carbon, and at least one double bond derived by the removal of one molecule of hydrogen from adjacent carbon atoms, adjacent nitrogen atoms, or adjacent carbon and nitrogen atoms of the corresponding heterocycloalkyl. Heterocycloalkenyl groups may be monocyclic or polycyclic (e.g., bicyclic, tricyclic). Examples of heterocycloalkenyl groups include dihydrofuranyl (e.g., 2,3-dihydrofuranyl, 2,5-dihydrofuranyl), dihydrothiophenyl (e.g., 2,3-dihydrothiophenyl, 2,5-dihydrothiophenyl), dihydropyrrolyl (e.g., 2,3-dihydro-1H-pyrrolyl, 2,5-dihydro-1H-pyrrolyl), dihydroimidazolyl (e.g., 2,3-dihydro-1H-imidazolyl, 4,5-dihydro-1H-imidazolyl), pyranyl, dihydropyranyl (e.g., 3,4-dihydro-2H-pyranyl, 3,6-dihydro-2H-pyranyl), tetrahydropyridinyl (e.g., 1,2,3,4-tetrahydropyridinyl, 1,2,3,6-tetrahydropyridinyl) and dihydropyridine (e.g., 1,2-dihydropyridine, 1,4-dihydropyridine). In addition, one ring of a polycyclic heterocycloalkenyl group may be aromatic (e.g., aryl or heteroaryl), provided the polycyclic heterocycloalkenyl group is bound to the parent structure via a non-aromatic carbon or nitrogen atom. For example, a 1,2-dihydroquinolin-1-yl group (wherein the moiety is bound to the parent structure via a non-aromatic nitrogen atom) is considered a heterocycloalkenyl group, while 1,2-dihydroquinolin-8-yl group (wherein the moiety is bound to the parent structure via an aromatic carbon atom) is not considered a heterocycloalkenyl group. Examples of polycyclic heterocycloalkenyl groups consisting of a heterocycloalkenyl group fused to an aromatic ring are described below.

Examples of polycyclic rings consisting of an aromatic ring (e.g., aryl or heteroaryl) fused to a non-aromatic ring (e.g., cycloalkyl, cycloalkenyl, heterocycloalkyl, heterocycloalkenyl) include indenyl, 2,3-dihydro-1H-indenyl, 1,2,3,4-tetrahydronaphthalenyl, benzo[1,3]dioxolyl, tetrahydroquinolinyl, 2,3-dihydrobenzo[1,4]dioxinyl, indolinyl, isoindolinyl, 2,3-dihydro-1H-indazolyl, 2,3-dihydro-1H-benzo[d]imidazolyl, 2,3-dihydrobenzofuranyl, 1,3-dihydroisobenzofuranyl, 1,3-dihydrobenzo[c]isoxazolyl, 2,3-dihydrobenzo[d]isoxazolyl, 2,3-dihydrobenzo[d]oxazolyl, 2,3-dihydrobenzo[b]thiophenyl, 1,3-dihydrobenzo[c]thiophenyl, 1,3-dihydrobenzo[c]isothiazolyl, 2,3-dihydrobenzo[d]isothiazolyl, 2,3-dihydrobenzo[d]thiazolyl, 5,6-dihydro-4H-cyclopenta[d]thiazolyl, 4,5,6,7-tetrahydrobenzo[d]thiazolyl, 5,6-dihydro-4H-pyrrolo[3,4-d]thiazolyl , 4,5,6,7-tetrahydrothiazolo[5,4-c]pyridinyl, indolin-2-one, indolin-3-one, isoindolin-1-one, 1,2-dihydroindazol-3-one, 1H-benzo[d]imidazol-2(3H)-one, benzofuran-2(3H)-one, benzofuran-3(2H)-one, isobenzofuran-1(3H)-one, benzo[c]isoxazol-3(1H)-one, benzo[d]isoxazol-3(2H)-one, benzo[d]oxazol-2(3H)-one, benzo[b]thiophen-2(3H)-one, benzo[b]thiophen-3(2H)-one, benzo[c]thiophen-1(3H)-one, benzo[c]isothiazol-3(1H)-one, benzo[d]isothiazol-3(2H)-one, benzo[d]thiazol-2(3H)-one, 4,5-dihydropyrrolo[3,4-d]thiazol-6-one, 1,2-dihydropyrazolo[3,4-d]thiazol-3-one, quinolin-4(3H)-one, quinazolin-4(3H)-one, quinazoline-2,4(1H,3H)-dione, quinoxalin-2(1H)-one, quinoxaline-2,3(1H,4H)-dione, cinnolin-4(3H)-one, pyridin-2(1H)-one, pyrimidin-2(1H)-one, pyrimidin-4(3H)-one, pyridazin-3(2H)-one, 1H-pyrrolo[3,2-b]pyridin-2(3H)-one, 1H-pyrrolo[3,2-c]pyridin-2(3H)-one, 1H-pyrrolo[2,3-c]pyridin-2(3H)-one, 1H-pyrrolo[2,3-b]pyridin-2(3H)-one, 1,2-dihydropyrazolo[3,4-d]thiazol-3-one and 4,5-dihydropyrrolo[3,4-d]thiazol-6-one. As discussed herein, whether each ring is considered an aryl, heteroaryl, cycloalkyl, cycloalkenyl, heterocycloalkyl or heterocycloalkenyl group is determined by the atom through which the moiety is bound to the parent structure.

"Halogen" or "halo" refers to fluorine, chlorine, bromine or iodine.

"Annular" refers to a moiety that is a member of a ring, including, but not limited to, a cycloalkyl ring, a cycloalkenyl ring, an aryl ring, a heteroaryl ring, or a heterocyclyl ring. For example, if a heteroaryl ring is described as "comprising two or more annular heteroatoms", two or more of the ring members of the heteroaryl ring will be heteroatoms.

Unless otherwise indicated, compounds disclosed and/or described herein include all possible enantiomers, diastereomers, meso isomers and other stereoisomeric forms, including racemic mixtures, optically pure forms and intermediate mixtures thereof. Enantiomers, diastereomers, meso isomers and other stereoisomeric forms can be prepared using chiral synthons or chiral reagents, or resolved using conventional techniques. Unless specified otherwise, when the compounds disclosed and/or described herein contain olefinic double bonds or other centers of geometric asymmetry, it is intended that the compounds include both E and Z isomers. When the compounds described herein contain moieties capable of tautomerization, and unless specified otherwise, it is intended that the compounds include all possible tautomers.

"Protecting group" has the meaning conventionally associated with it in organic synthesis, i.e., a group that selectively blocks one or more reactive sites in a multifunctional compound such that a chemical reaction can be carried out selectively on another unprotected reactive site, and such that the group can readily be removed after the selective reaction is complete. A variety of protecting groups are disclosed, for example, in T.H. Greene and P. G. M. Wuts, Protective Groups in Organic Synthesis, Third Edition, John Wiley & Sons, New York (1999). For example, a "hydroxy protected form" contains at least one hydroxy group protected with a hydroxy protecting group. Likewise, amines and other reactive groups may similarly be protected.

The term "pharmaceutically acceptable salt" refers to a salt of any of the compounds herein which are known to be non-toxic and are commonly used in the pharmaceutical literature. In some embodiments, the pharmaceutically acceptable salt of a compound retains the biological effectiveness of the compounds described herein and are not biologically or otherwise undesirable. Examples of pharmaceutically acceptable salts can be found in Berge et al., Pharmaceutical Salts, J. Pharmaceutical Sciences, January 1977, 66(1), 1-19. Pharmaceutically acceptable acid addition salts can be formed with inorganic acids and organic acids. Inorganic acids from which salts can be derived include, for example, hydrochloric acid, hydrobromic acid, sulfuric acid, nitric acid, and phosphoric acid. Organic acids from which salts can be derived include, for example, acetic acid, propionic acid, glycolic acid, pyruvic acid, lactic acid, oxalic acid, malic acid, maleic acid, malonic acid, succinic acid, fumaric acid, tartaric acid, citric acid, benzoic acid, cinnamic acid, mandelic acid, methanesulfonic acid, ethanesulfonic acid, 2-hydroxyethylsulfonic acid, p-toluenesulfonic acid, stearic acid and salicylic acid. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, and aluminum. Organic bases from which salts can be derived include, for example, primary, secondary, and tertiary amines; substituted amines including naturally occurring substituted amines; cyclic amines; and basic ion exchange resins. Examples of organic bases include isopropylamine, trimethylamine, diethylamine, triethylamine, tripropylamine, and ethanolamine. In some embodiments, the pharmaceutically acceptable base addition salt is selected from ammonium, potassium, sodium, calcium, and magnesium salts.

If the compound described herein is obtained as an acid addition salt, the free base can be obtained by basifying a solution of the acid salt. Conversely, if the compound is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds (see, e.g., Berge et al., Pharmaceutical Salts, J. Pharmaceutical Sciences, January 1977, 66(1), 1-19). Those skilled in the art will recognize various synthetic methodologies that may be used to prepare pharmaceutically acceptable addition salts.

A "solvate" is formed by the interaction of a solvent and a compound. Suitable solvents include, for example, water and alcohols (e.g., ethanol). Solvates include hydrates having any ratio of compound to water, such as monohydrates, dihydrates and hemi-hydrates.

The term "substituted" means that the specified group or moiety bears one or more substituents including, but not limited to, substituents such as alkoxy, acyl, acyloxy, carbonylalkoxy, acylamino, amino, aminoacyl, aminocarbonylamino, aminocarbonyloxy, cycloalkyl, cycloalkenyl, aryl, heteroaryl, aryloxy, cyano, azido, halo, hydroxyl, nitro, carboxyl, thiol, thioalkyl, cycloalkyl, cycloalkenyl, alkyl, alkenyl, alkynyl, heterocycloalkyl, heterocycloalkenyl, aralkyl, aminosulfonyl, sulfonylamino, sulfonyl, oxo, carbonylalkylenealkoxy and the like. The term "unsubstituted" means that the specified group bears no substituents. Where the term "substituted" is used to describe a structural system, the substitution is meant to occur at any valency-allowed position on the system. When a group or moiety bears more than one substituent, it is understood that the substituents may be the same or different from one another. In some embodiments, a substituted group or moiety bears from one to five substituents. In some embodiments, a substituted group or moiety bears one substituent. In some embodiments, a substituted group or moiety bears two substituents. In some embodiments, a substituted group or moiety bears three substituents. In some embodiments, a substituted group or moiety bears four substituents. In some embodiments, a substituted group or moiety bears five substituents.

By "optional" or "optionally", it is meant that the subsequently described event or circumstance may or may not occur, and that the description includes instances where the event or circumstance occurs and instances in which it does not. For example, "optionally substituted alkyl" encompasses both "alkyl" and "substituted alkyl" as defined herein. It will be understood by those skilled in the art, with respect to any group containing one or more substituents, that such groups are not intended to introduce any substitution or substitution patterns that are sterically impractical, synthetically non-feasible, and/or inherently unstable. It will also be understood that where a group or moiety is optionally substituted, the disclosure includes both embodiments in which the group or moiety is substituted and embodiments in which the group or moiety is unsubstituted.

The compounds disclosed and/or described herein can be enriched isotopic forms, e.g., enriched in the content of ²H, ³H, ¹¹C, ¹³C and/or ¹⁴C. In one embodiment, the compound contains at least one deuterium atom. Such deuterated forms can be made, for example, by the procedure described in U.S. Patent Nos. 5,846,514 and 6,334,997. Such deuterated compounds may improve the efficacy and increase the duration of action of compounds disclosed and/or described herein. Deuterium substituted compounds can be synthesized using various methods, such as those described in: Dean, D., Recent Advances in the Synthesis and Applications of Radiolabeled Compounds for Drug Discovery and Development, Curr. Pharm. Des., 2000; 6(10); Kabalka, G. et al., The Synthesis of Radiolabeled Compounds via Organometallic Intermediates, Tetrahedron, 1989, 45(21), 6601-21; and Evans, E., Synthesis of radiolabeled compounds, J. Radioanal. Chem., 1981, 64(1-2), 9-32.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in pharmaceutical compositions is contemplated. Supplementary active ingredients can also be incorporated into the pharmaceutical compositions.

The terms "patient," "individual," and "subject" refer to an animal, such as a mammal, bird, or fish. In some embodiments, the patient or subject is a mammal. Mammals include, for example, mice, rats, dogs, cats, pigs, sheep, horses, cows and humans. In some embodiments, the patient or subject is a human, for example a human that has been or will be the object of treatment, observation or experiment. The compounds, compositions and methods described herein can be useful in both human therapy and veterinary applications.

As used herein, the term "therapeutic" refers to the ability to treat a condition, disorder, or disease described herein, including, but not limited to, a condition, disorder, or disease responsive to the modulation of the contractility of the skeletal sarcomere. As used herein, "modulation" refers to a change in activity as a direct or indirect response to the presence of a chemical entity as described herein, relative to the activity of in the absence of the chemical entity. The change may be an increase in activity or a decrease in activity, and may be due to the direct interaction of the chemical entity with the a target or due to the interaction of the chemical entity with one or more other factors that in turn affect the target's activity. For example, the presence of the chemical entity may, for example, increase or decrease the target activity by directly binding to the target, by causing (directly or indirectly) another factor to increase or decrease the target activity, or by (directly or indirectly) increasing or decreasing the amount of target present in the cell or organism.

The term "therapeutically effective amount" or "effective amount" refers to that amount of a compound disclosed and/or described herein that is sufficient to affect treatment, as defined herein, when administered to a patient in need of such treatment. A therapeutically effective amount of a compound may be an amount sufficient to treat a disease, disorder, or condition described herein, including, but not limited to, a condition, disorder, or disease responsive to the modulation of the contractility of the skeletal sarcomere. The therapeutically effective amount will vary depending upon, for example, the subject and disease condition being treated, the weight and age of the subject, the severity of the disease condition, the particular compound, the dosing regimen to be followed, timing of administration, the manner of administration, all of which can readily be determined by one of ordinary skill in the art. The therapeutically effective amount may be ascertained experimentally, for example by assaying blood concentration of the chemical entity, or theoretically, by calculating bioavailability.

"Treatment" (and related terms, such as "treat", "treated", "treating") includes one or more of: inhibiting a disease or disorder; slowing or arresting the development of clinical symptoms of a disease or disorder; and/or relieving a disease or disorder (i.e., causing relief from or regression of clinical symptoms). The term covers both complete and partial reduction or prevention of the condition or disorder, and complete or partial reduction of clinical symptoms of a disease or disorder. Thus, compounds described and/or disclosed herein may prevent an existing disease or disorder from worsening, assist in the management of the disease or disorder, or reduce or eliminate the disease or disorder.

As used herein and in the appended claims, the singular forms "a", "an" and "the" include plural forms, unless the context clearly dictates otherwise.

As used herein, and unless otherwise specified, the terms "about" and "approximately," when used in connection with doses, amounts, or weight percent of ingredients of a composition or a dosage form, mean a dose, amount, or weight percent that is recognized by those of ordinary skill in the art to provide a pharmacological effect equivalent to that obtained from the specified dose, amount, or weight percent. Specifically, the terms "about" and "approximately," when used in this context, contemplate a dose, amount, or weight percent within 5% of the specified dose, amount, or weight percent.

### Compounds

Compounds and salts thereof (such as pharmaceutically acceptable salts) are detailed herein, including in the Brief Summary and in the appended claims. Also provided are the use of all of the compounds described herein, including any and all stereoisomers, including geometric isomers (cis/trans), E/Z isomers, enantiomers, diastereomers, and mixtures thereof in any ratio including racemic mixtures, salts and solvates of the compounds described herein, as well as methods of making such compounds. Any compound described herein may also be referred to as a drug.

In one aspect, provided herein is a compound of formula (I): 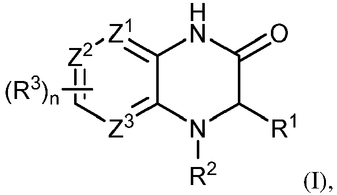 or a pharmaceutically acceptable salt thereof, wherein:
Z¹, Z², and Z³ are, independently of each other, CH, CR³, or N, provided that at least one of Z¹, Z², and Z³ is CH or CR³;
R¹ is unsubstituted C₃-C₄ alkyl;
R² is -C(=O)R⁴, -C(=O)-(CH₂)ₚ-R⁵, -C(=O)NR⁶R⁷, or -C(=O)OCH₃;
each R³ is independently halogen, C₁-C₆-alkyl, C₁-C₆-haloakyl, C₁-C₆-alkoxy, C₁-C₆-haloakoxy, C₃-C₁₂-cycloalkyl, or 3-12 membered heterocycloalkyl;
R⁴ is C₆-C₁₂-aryl optionally substituted with one to five R^{4A}, 5-12 membered heteroaryl optionally substituted with one to five R^{4B}, C₃-C₁₂ cycloalkyl optionally substituted with one to five R^{4C}, 4-12 membered heterocycloalkyl optionally substituted with one to five R^{4D}, or 4-12 membered heterocycloalkenyl optionally substituted with one to five R^{4E};
each R^{4A} is independently selected from the group consisting of halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), C₆-C₁₂-aryl, and 5-12 membered heteroaryl;
each R^{4B} is independently selected from the group consisting of halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), and 5-12 membered heteroaryl;
each R^{4B1} is independently selected from the group consisting of halo, C₁-C₆-alkyl, C₁-C₆-haloalkyl, OH, C₁-C₆-alkoxy, C₁-C₆-haloalkoxy, and C₁-C₆-alkylene-OH;
each R^{4C} is independently selected from the group consisting of halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
each R^{4D} is independently selected from the group consisting of halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
each R^{4E} is independently selected from the group consisting of oxo, halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
R⁵ is OH, C₁-C₆-alkoxy, C₁-C₆-haloalkoxy, C₃-C₁₂ cycloalkyl, 3-12 membered heterocycloalkyl, or C₆-C₁₂-aryl;
R⁶ and R⁷, independently of each other, are selected from the group consisting of H, C₁-C₆-alkyl, C₁-C₆-haloalkyl, C₃-C₁₂ cycloalkyl optionally substituted with one to five R⁸, 4-12 membered heterocycloalkyl optionally substituted with one to five R⁸, C₆-C₁₂-aryl optionally substituted with one to five R⁸, 5-12 membered heteroaryl optionally substituted with one to five R⁸, C₁-C₆-alkylene-OH, C₁-C₆-alkylene-(C₃-C₁₂ cycloalkyl), C₁-C₆-alkylene-(4-12 membered heterocycloalkyl), C₁-C₆-alkylene-(C₆-C₁₂-aryl), and C₁-C₆-alkylene-(5-12 membered heteroaryl);
each R⁸ is independently selected from the group consisting of halo, C₁-C₆-alkyl, C₁-C₆-haloalkyl, OH, C₁-C₆-alkoxy, C₁-C₆-alkylene-OH, and C₁-C₆-haloalkoxy;
n is 0, 1, 2, 3, or 4;
p is 1, 2, or 3; and
provided that the compound of Formula (I) is not a compound selected from the group consisting of:
   4-(3-methylbenzoyl)-3-propyl-3,4-dihydroquinoxalin-2(1H)-one,
   4-(2-chlorobenzoyl)-3-isobutyl-3,4-dihydroquinoxalin-2(1H)-one, and
   4-(4-(tert-butyl)benzoyl)-3-propyl-3,4-dihydroquinoxalin-2(1H)-one.

In some embodiments, provided herein is a compound of formula (II): 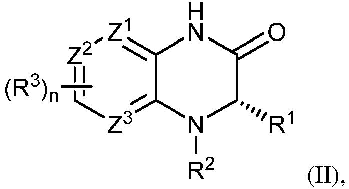 or a pharmaceutically acceptable salt thereof, wherein Z¹, Z², Z³, R¹, R², R³, and n are as defined for the compound of formula (I), or any variation or embodiment thereof.

In some embodiments of the compounds of formula (I) or (II), or a pharmaceutically acceptable salt thereof, one, two, or three of Z¹, Z², and Z³ is/are CH or CR³. In some embodiments, Z¹, Z², and Z³ are each independently CH or CR³. In some embodiments, Z¹ is N, and Z² and Z³ are each independently CH or CR³. In some embodiments, Z² is N, and Z¹ and Z³ are each independently CH or CR³. In some embodiments, Z¹ and Z² are each independently CH or CR³, and Z³ is N. In some embodiments, Z¹ is CH or CR³, and Z² and Z³ are each N.

In some embodiments, provided herein is a compound of formula (I), or a pharmaceutically acceptable salt thereof, wherein the compound is of formula (I-a), (I-b), (I-c), (I-d), or (I-e): 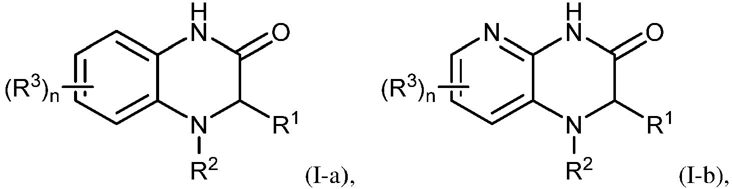 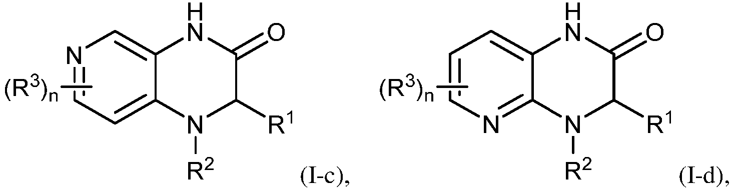 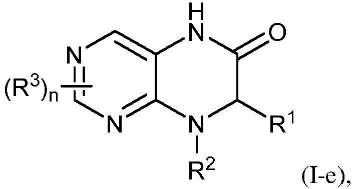 or a pharmaceutically acceptable salt thereof, wherein R¹, R², R³, and n are as defined for the compound of formula (I), or any variation or embodiment thereof.

In some embodiments, provided herein is a compound of formula (I-a): 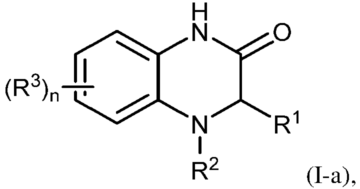 or a pharmaceutically acceptable salt thereof, wherein R¹, R², R³, and n are as defined for the compound of formula (I), or any variation or embodiment thereof.

In some embodiments, provided herein is a compound of formula (I-b): 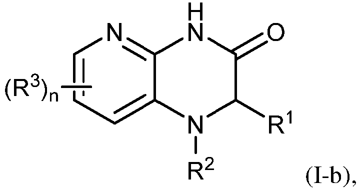 or a pharmaceutically acceptable salt thereof, wherein R¹, R², R³, and n are as defined for the compound of formula (I), or any variation or embodiment thereof.

In some embodiments, provided herein is a compound of formula (I-c): 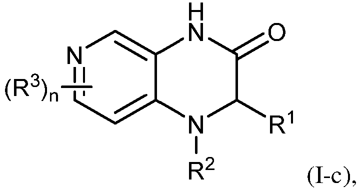 or a pharmaceutically acceptable salt thereof, wherein R¹, R², R³, and n are as defined for the compound of formula (I), or any variation or embodiment thereof.

In some embodiments, provided herein is a compound of formula (I-d): 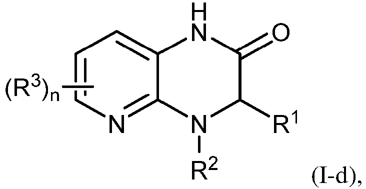 or a pharmaceutically acceptable salt thereof, wherein R¹, R², R³, and n are as defined for the compound of formula (I), or any variation or embodiment thereof.

In some embodiments, provided herein is a compound of formula (I-e): 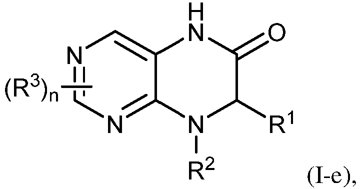 or a pharmaceutically acceptable salt thereof, wherein R¹, R², R³, and n are as defined for the compound of formula (I), or any variation or embodiment thereof.

In some embodiments, provided herein is a compound of formula (II), or a pharmaceutically acceptable salt thereof, wherein the compound is of formula (II-a), (II-b), (II-c), (II-d), or (II-e): 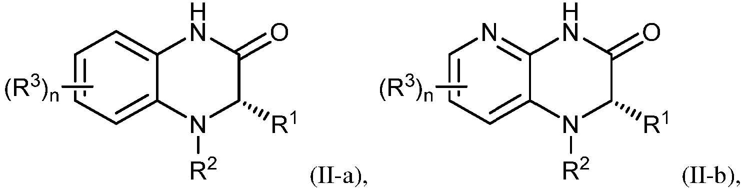 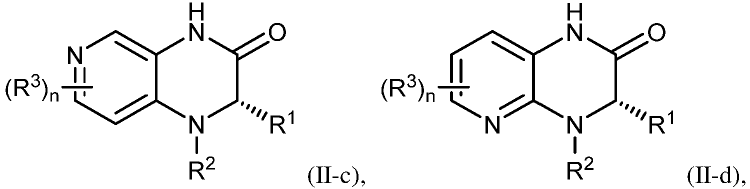 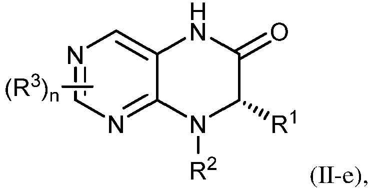 or a pharmaceutically acceptable salt thereof, wherein R¹, R², R³, and n are as defined for the compound of formula (I), or any variation or embodiment thereof.

In some embodiments, provided herein is a compound of formula (II-a): 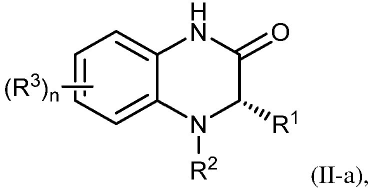 or a pharmaceutically acceptable salt thereof, wherein R¹, R², R³, and n are as defined for the compound of formula (I), or any variation or embodiment thereof.

In some embodiments, provided herein is a compound of formula (II-b): 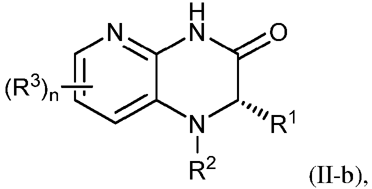 or a pharmaceutically acceptable salt thereof, wherein R¹, R², R³, and n are as defined for the compound of formula (I), or any variation or embodiment thereof.

In some embodiments, provided herein is a compound of formula (II-c): 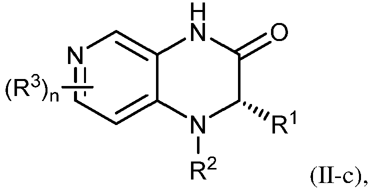 or a pharmaceutically acceptable salt thereof, wherein R¹, R², R³, and n are as defined for the compound of formula (I), or any variation or embodiment thereof.

In some embodiments, provided herein is a compound of formula (II-d): 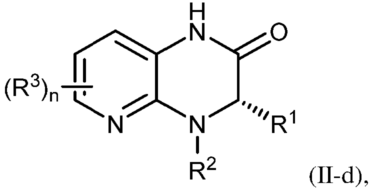 or a pharmaceutically acceptable salt thereof, wherein R¹, R², R³, and n are as defined for the compound of formula (I), or any variation or embodiment thereof.

In some embodiments, provided herein is a compound of formula (II-e): 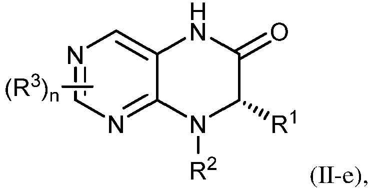 or a pharmaceutically acceptable salt thereof, wherein R¹, R², R³, and n are as defined for the compound of formula (I), or any variation or embodiment thereof.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, R¹ is propyl or butyl. In some embodiments, R¹ is prop-1-yl, prop-2-yl, but-1-yl, but-2-yl, or 2-methylprop-1-yl. In some embodiments, R¹ is prop-1-yl, prop-2-yl, but-2-yl, or 2-methylprop-1-yl. In some embodiments, R¹ is 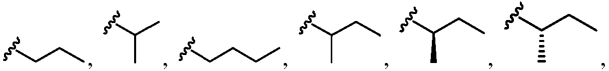 or 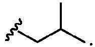 In some embodiments, R¹ is propyl. In some embodiments, R¹ is prop-1-yl or prop-2-yl. In some embodiments, R¹ is 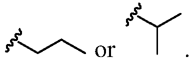 In some embodiments, R¹ is butyl. In some embodiments, R¹ is but-1-yl, but-2-yl, or 2-methylprop-1-yl. . In some embodiments, R¹ is 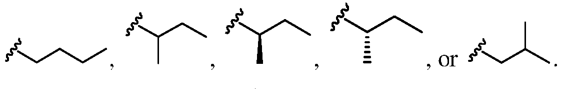 In some embodiments, R¹ is prop-1-yl. In some embodiments, R¹ is prop-2-yl. In some embodiments, R¹ is but-1-yl. In some embodiments, R¹ is but-2-yl. In some embodiments, R¹ is 2-methylprop-1-yl. In some embodiments, R¹ is 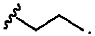 In some embodiments, R¹ is 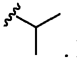 In some embodiments, R¹ is 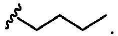 In some embodiments, R¹ is 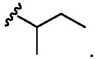 In some embodiments, R¹ is 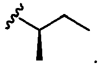 In some embodiments, R¹ is 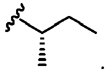 In some embodiments, R¹ is

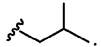

**In** some embodiments of the compounds of formula (I), (I-a), (I-b), (I-c), (I-d), (I-e), (II), (II-a), (II-b), (II-c), (II-d), or (II-e), or a pharmaceutically acceptable salt thereof, n is 0, 1, 2, 3, or 4. In some embodiments, n is 0 or 1. In some embodiments, n is 0. In some embodiments, n is 1. In some embodiments, n is 2. In some embodiments, n is 3. In some embodiments, n is 4.

**In** some embodiments of the compounds of formula (I), (I-a), (I-b), (I-c), (I-d), (I-e), (II), (II-a), (II-b), (II-c), (II-d), or (II-e), or a pharmaceutically acceptable salt thereof, each R³ is independently halogen, C₁-C₆-alkyl, C₁-C₆-haloakyl, C₁-C₆-alkoxy, C₁-C₆-haloakoxy, C₃-C₁₂-cycloalkyl, or 3-12 membered heterocycloalkyl. In other embodiments, each R³ is independently halogen or C₃-C₁₂-cycloalkyl. In other embodiments, each R³ is independently halogen or C₃-C₆-cycloalkyl. In some embodiments, each R³ is independently halogen. In some embodiments, each R³ is independently fluoro, chloro, or bromo. In certain embodiments, each R³ is fluoro. In other embodiments, each R³ is chloro. In another embodiment, each R³ is bromo. In some embodiments, each R³ is independently C₃-C₁₂-cycloalkyl. In some embodiments, each R³ is independently C₃-C₆-cycloalkyl. In one embodiment, each R³ is cyclopropyl. In another embodiment, each R³ is cyclobutyl. In one embodiment, each R³ is cyclopentyl. In one embodiment, each R³ is cyclohexyl.

**In** some embodiments of the compounds of formula (I), (I-a), (I-b), (I-c), (I-d), (I-e), (II), (II-a), (II-b), (II-c), (II-d), or (II-e), or a pharmaceutically acceptable salt thereof, n is 1 and R³ is halogen, C₁-C₆-alkyl, C₁-C₆-haloakyl, C₁-C₆-alkoxy, C₁-C₆-haloakoxy, C₃-C₁₂-cycloalkyl, or 3-12 membered heterocycloalkyl. In other embodiments, n is 1 and R³ is halogen or C₃-C₁₂-cycloalkyl. In other embodiments, n is 1 and R³ is halogen or C₃-C₆-cycloalkyl. In some embodiments, n is 1 and R³ is halogen. In some embodiments, n is 1 and R³ is fluoro, chloro, or bromo. In certain embodiments, n is 1 and R³ is fluoro. In other embodiments, n is 1 and R³ is chloro. In another embodiment, n is 1 and R³ is bromo. In some embodiments, n is 1 and R³ is C₃-C₁₂-cycloalkyl. In some embodiments, n is 1 and R³ is C₃-C₆-cycloalkyl. In one embodiment, n is 1 and R³ is cyclopropyl. In another embodiment, n is 1 and R³ is cyclobutyl. In one embodiment, n is 1 and R³ is cyclopentyl. In one embodiment, n is 1 and R³ is cyclohexyl.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, R² is - C(=O)R⁴, -C(=O)-(CH₂)ₚ-R⁵, -C(=O)NR⁶R⁷, or
-C(=O)OCH₃. In some embodiments, R² is -C(=O)R⁴. In some embodiments, R² is -C(=O)-(CH₂)ₚ-R⁵. In some embodiments, R² is -C(=O)NR⁶R⁷. In some embodiments, R² is -C(=O)OCH₃.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, R² is - C(=O)R⁴ and R⁴ is C₆-C₁₂-aryl optionally substituted with one to five R^{4A}, 5-12 membered heteroaryl optionally substituted with one to five R^{4B}, C₃-C₁₂ cycloalkyl optionally substituted with one to five R^{4C}, 4-12 membered heterocycloalkyl optionally substituted with one to five R^{4D}, or 4-12 membered heterocycloalkenyl optionally substituted with one to five R^{4E}. In some embodiments, R² is -C(=O)R⁴ and R⁴ is C₆-C₁₂-aryl optionally substituted with one to five R^{4A}, 5-12 membered heteroaryl optionally substituted with one to five R^{4B}, 4-12 membered heterocycloalkyl optionally substituted with one to five R^{4D}, or 4-12 membered heterocycloalkenyl optionally substituted with one to five R^{4E}.

In some embodiments, R² is -C(=O)R⁴ and R⁴ is unsubstituted C₆-C₁₂-aryl. In some embodiments, R⁴ is C₆-C₁₂-aryl substituted with one to five R^{4A}. In some embodiments, R⁴ is unsubstituted phenyl. In some embodiments, R⁴ is phenyl substituted with one to five R^{4A}. In some embodiments, R⁴ is 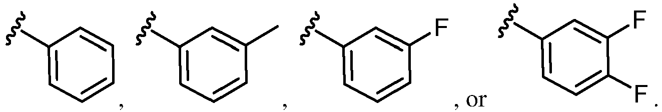 In some embodiments, R⁴ is 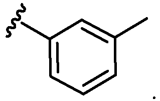 In some embodiments, R⁴ is 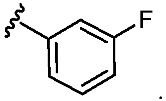 In some embodiments, R⁴ is 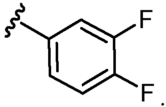 In some embodiments, each R^{4A} is independently halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), C₆-C₁₂-aryl, or 5-12 membered heteroaryl. In some embodiments, each R^{4A} is independently is halo or C₁-C₆-alkyl. In some embodiments, each R^{4A} is independently halo. In some embodiments, each R^{4A} is fluoro. In some embodiments, each R^{4A} is independently chloro. In some embodiments, each R^{4A} is independently bromo. In some embodiments, each R^{4A} is independently C₁-C₆-alkyl. In some embodiments, each R^{4A} is independently methyl. In some embodiments, each R^{4A} is independently ethyl. In some embodiments, each R^{4A} is independently propyl. In some embodiments, each R^{4A} is independently butyl. In some embodiments, each R^{4A} is independently pentyl. In some embodiments, each R^{4A} is independently hexyl.

In some embodiments, R² is -C(=O)R⁴ and R⁴ is unsubstituted 5-12 membered heteroaryl. In some embodiments, R⁴ is 5-12 membered heteroaryl substituted with one to five R^{4B}. In some embodiments, R⁴ is pyridinyl, pyrrolyl, pyrazolyl, isoxazolyl, thiazolyl, pyrrolopyridyl, imidazopyridyl, pyrrolopyrimidyl, pyrazolopyridyl, or pyrrolidinyl, each of which is optionally substituted with one to five R^{4B}. In some embodiments, R⁴ is 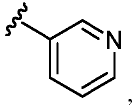 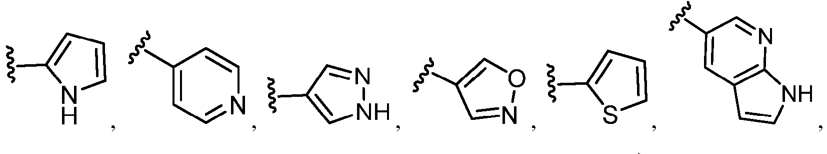 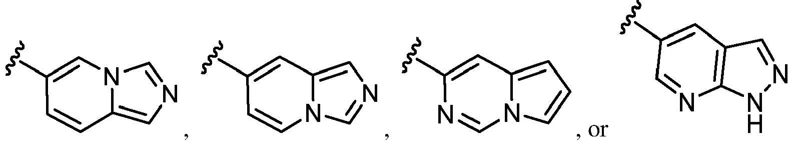 each of which is optionally substituted with one to five R^{4B}. In some embodiments, R⁴ is 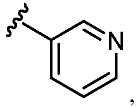 which is unsubstituted or substituted with one to four R^{4B}. In some embodiments, R⁴ is 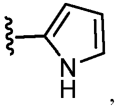 which is unsubstituted or substituted with one to four R^{4B}. In some embodiments, R⁴ is 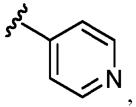 which is unsubstituted or substituted with one to four R^{4B}. In some embodiments, R⁴ is 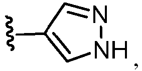 which is unsubstituted or substituted with one to three R^{4B}. In some embodiments, R⁴ is 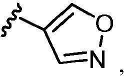 which is unsubstituted or substituted with one or two R^{4B}. In some embodiments, R⁴ is 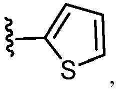 which is unsubstituted or substituted with one to three R^{4B}. In some embodiments, R⁴ is 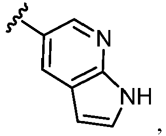 which is unsubstituted or substituted with one to five R^{4B}. In some embodiments, R⁴ is 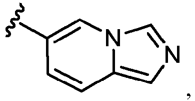 which is unsubstituted or substituted with one to five R^{4B}. In some embodiments, R⁴ is 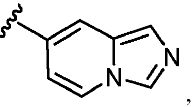 which is unsubstituted or substituted with one to five R^{4B}. In some embodiments, R⁴ is 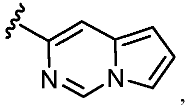 which is unsubstituted or substituted with one to five R^{4B}. In some embodiments, R⁴ is 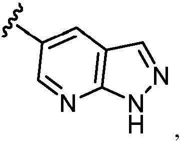 which is unsubstituted or substituted with one to four R^{4B}. In some embodiments, each R^{4B} is independently halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), or 5-12 membered heteroaryl. In some embodiments, each R^{4B} is independently halo, C₁-C₆-alkyl, C₁-C₆-haloalkoxy, NH₂, N(C₁-C₆-alkyl)₂, NH(C₁-C₆-haloalkyl), N(C₁-C₆-alkyl)(C₁-C₆-haloalkyl), NH(C₁-C₆-alkylene-(C₆-C₁₂-aryl)), N(C₁-C₆-alkyl)(C₁-C₆-alkylene-(C₆-C₁₂-aryl)), N(C₁-C₆-alkyl)(C₃-C₁₂ cycloalkyl), NHC(O)-C₁-C₆-alkyl, C₃-C₁₂ cycloalkyl, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), or 5-12 membered heteroaryl. In some embodiments, each R^{4B} is independently halo, C₁-C₆-alkyl, C₁-C₆-haloalkoxy, NH₂, N(C₁-C₆-alkyl)₂, NH(C₁-C₆-haloalkyl), N(C₁-C₆-alkyl)(C₁-C₆-haloalkyl), NH(C₁-C₆-alkylene-(C₆-C₁₂-aryl)), N(C₁-C₆-alkyl)(C₃-C₁₂ cycloalkyl), NHC(O)-C₁-C₆-alkyl, C₃-C₁₂ cycloalkyl, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), or 5-12 membered heteroaryl. In some embodiments, R^{4B} is halo. In some embodiments, R^{4B} is fluoro. In some embodiments, R^{4B} is chloro. In some embodiments, R^{4B} is C₁-C₆-alkyl. In some embodiments, R^{4B} is methyl. In some embodiments, R^{4B} is ethyl. In some embodiments, R^{4B} is propyl. In some embodiments, R^{4B} is C₁-C₆-haloalkoxy. In some embodiments, R^{4B} is 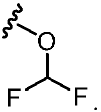 In some embodiments, R^{4B} is 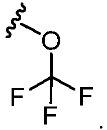 In some embodiments, R^{4B} is NH₂. In some embodiments, R^{4B} is N(C₁-C₆-alkyl)₂. In some embodiments, R^{4B} is 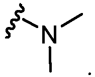 In some embodiments, R^{4B} is 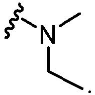 In some embodiments, R^{4B} is NH(C₁-C₆-haloalkyl). In some embodiments, R^{4B} is 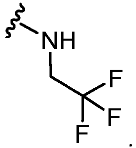 In some embodiments, R^{4B} is N(C₁-C₆-alkyl)(C₁-C₆-haloalkyl). In some embodiments, R^{4B} is 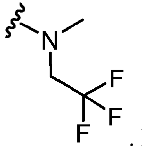 In some embodiments, R^{4B} is 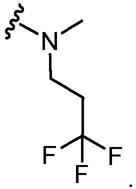 In some embodiments, R^{4B} is NH(C₁-C₆-alkylene-(C₆-C₁₂-aryl)). In some embodiments, R^{4B} is 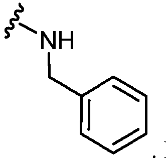 In some embodiments, R^{4B} is N(C₁-C₆-alkyl)(C₁-C₆-alkylene-(C₆-C₁₂-aryl)). In some embodiments, R^{4B} is 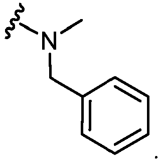 In some embodiments, R^{4B} is N(C₁-C₆-alkyl)(C₃-C₁₂ cycloalkyl). In some embodiments, R^{4B} is 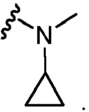 In some embodiments, R^{4B} is NHC(O)-C₁-C₆-alkyl. In some embodiments, R^{4B} is 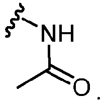 In some embodiments, R^{4B} is C₃-C₁₂ cycloalkyl. In some embodiments, R^{4B} is 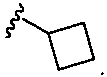 In some embodiments, R^{4B} is 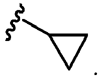 In some embodiments, R^{4B} is 4-12 membered heterocycloalkyl optionally substituted with one to five R^{4B1}. In some embodiments, R^{4B} is 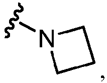 which is optionally substituted with one to five R^{4B1}. In some embodiments, R^{4B} is 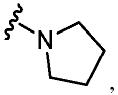 which is optionally substituted with one to five R^{4B1}. In some embodiments, R^{4B} is 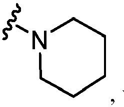 which is optionally substituted with one to five R^{4B1}. In some embodiments, R^{4B} is 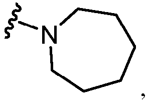 which is optionally substituted with one to five R^{4B1}. In some embodiments, R^{4B} is C₁-C₆-alkylene-OH. In some embodiments, R^{4B} is 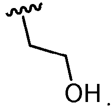 In some embodiments, R^{4B} is C₁-C₆-alkylene-CONH₂. In some embodiments, R^{4B} is 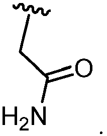 In some embodiments, R^{4B} is C₁-C₆-alkylene-(C₃-C₁₂ cycloalkyl). In some embodiments, R^{4B} is 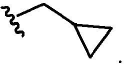 In some embodiments, R^{4B} is 5-12 membered heteroaryl. In some embodiments, R^{4B} is 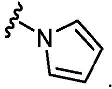 In some embodiments, R^{4B1} is halo, C₁-C₆-alkyl, C₁-C₆-haloalkyl, OH, C₁-C₆-alkoxy, C₁-C₆-haloalkoxy, and C₁-C₆-alkylene-OH. In some embodiments, R^{4B1} is halo or C₁-C₆-alkoxy. In some embodiments, R^{4B1} is halo. In some embodiments, R^{4B1} is fluoro. In some embodiments, R^{4B1} is C₁-C₆-alkoxy. In some embodiments, R^{4B1} is methoxy.

In some embodiments, R² is -C(=O)R⁴ and R⁴ is unsubstituted 4-12 membered heterocycloalkyl. In some embodiments, R⁴ is 4-12 membered heterocycloalkyl substituted with one to five R^{4D}. In some embodiments, R⁴ is indolinyl, azetidinyl, or piperidinyl, each of which is optionally substituted with one to five R^{4D}. In some embodiments, R⁴ is 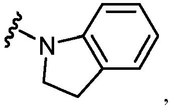 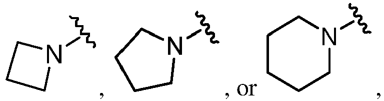 each of which is optionally substituted with one to five R^{4D}. In some embodiments, R⁴ is 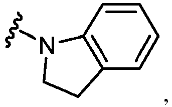 which is unsubstituted or substituted with one to five R^{4D}. In some embodiments, R⁴ is 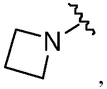 which is unsubstituted or substituted with one to five R^{4D}. In some embodiments, R⁴ is 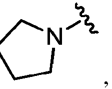 which is unsubstituted or substituted with one to five R^{4D}. In some embodiments, R⁴ is 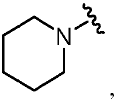 which is unsubstituted or substituted with one to five R^{4D}. In some embodiments, R^{4D} is C₁-C₆-alkyl, C₁-C₆-haloalkyl, OH, C₁-C₆-alkoxy, C₁-C₆-haloalkoxy, NHC(O)-C₁-C₆-alkyl, or C₁-C₆-alkylene-OH. In some embodiments, R^{4D} is halo, C₁-C₆-alkyl, OH, NHC(O)-C₁-C₆-alkyl, or C₁-C₆-alkylene-OH. In some embodiments, R^{4D} is halo. In some embodiments, R^{4D} is fluoro. In some embodiments, R^{4D} is C₁-C₆-alkyl. In some embodiments, R^{4D} is methyl. In some embodiments, R^{4D} is OH. In some embodiments, R^{4D} is NHC(O)-C₁-C₆-alkyl. In some embodiments, R^{4D} is NHC(O)Me. In some embodiments, R^{4D} is C₁-C₆-alkylene-OH. In some embodiments, R^{4D} is CH₂OH.

In some embodiments, R² is -C(=O)R⁴ and R⁴ is unsubstituted 4-12 membered heterocycloalkenyl. In some embodiments, R⁴ is 4-12 membered heterocycloalkenyl substituted with one to five R^{4E}. In some embodiments, R⁴ is pyridonyl. In some embodiments, R⁴ is 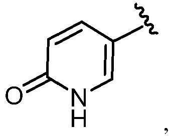 which is optionally substituted with one to four R^{4E}. In some embodiments, R^{4E} is oxo, halo, C₁-C₆-alkyl, C₁-C₆-haloalkyl, OH, C₁-C₆-alkoxy, C₁-C₆-haloalkoxy, NHC(O)-C₁-C₆-alkyl, or C₁-C₆-alkylene-OH. In some embodiments, R^{4E} is oxo or C₁-C₆-alkyl. In some embodiments, R^{4E} is oxo. In some embodiments, R^{4E} is C₁-C₆-alkyl. In some embodiments, R^{4E} is methyl.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, R² is - C(=O)-(CH₂)ₚ-R⁵ and R⁵ is OH, C₁-C₆-alkoxy, C₁-C₆-haloalkoxy, C₃-C₁₂ cycloalkyl, 3-12 membered heterocycloalkyl, or C₆-C₁₂-aryl. In some embodiments, R³ is OH or C₆-C₁₂-aryl. In some embodiments, R⁵ is OH. In some embodiments, R⁵ is C₆-C₁₂-aryl. In some embodiments, R⁵ is phenyl. In some embodiments, p is 1, 2, or 3. In some embodiments, p is 1 or 2. In some embodiments, p is 1. In some embodiments, p is 2. In some embodiments, p is 3.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, R² is - C(=O)NR⁶R⁷, and R⁶ and R⁷, independently of each other, are H, C₁-C₆-alkyl, C₁-C₆-haloalkyl, C₃-C₁₂ cycloalkyl optionally substituted with one to five R⁸, 4-12 membered heterocycloalkyl optionally substituted with one to five R⁸, C₆-C₁₂-aryl optionally substituted with one to five R⁸, 5-12 membered heteroaryl optionally substituted with one to five R⁸, C₁-C₆-alkylene-OH, C₁-C₆-alkylene-(C₃-C₁₂ cycloalkyl), C₁-C₆-alkylene-(4-12 membered heterocycloalkyl), C₁-C₆-alkylene-(C₆-C₁₂-aryl), or C₁-C₆-alkylene-(5-12 membered heteroaryl). In some embodiments, R⁶ and R⁷, independently of each other, are H, C₁-C₆-alkyl, C₁-C₆-haloalkyl, C₃-C₁₂ cycloalkyl, C₆-C₁₂-aryl optionally substituted with one to five R⁸, 5-12 membered heteroaryl optionally substituted with one to five R⁸, C₁-C₆-alkylene-OH, C₁-C₆-alkylene-(C₃-C₁₂ cycloalkyl), C₁-C₆-alkylene-(C₆-C₁₂-aryl), and C₁-C₆-alkylene-(5-12 membered heteroaryl). In some embodiments, R⁶ is H and R⁷ is H. In some embodiments, R⁶ is H and R⁷ is C₁-C₆-alkyl. In some embodiments, R⁶ is H and R⁷ is methyl. In some embodiments, R⁶ is H and R⁷ is ethyl. In some embodiments, R⁶ is H and R⁷ is C₁-C₆-haloalkyl. In some embodiments, R⁶ is H and R⁷ is and R⁷ is 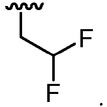 In some embodiments, R⁶ is C₁-C₆-alkyl and R⁷ is C₁-C₆-haloalkyl. In some embodiments, R⁶ is methyl and R⁷ is and R⁷ is

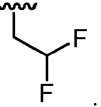

In some embodiments, R⁶ is H and R⁷ is C₃-C₁₂ cycloalkyl optionally substituted with one to five R⁸. In some embodiments, R⁶ is H and R⁷ is unsubstituted cyclohexyl. In some embodiments, R⁶ is H and R⁷ is cyclobutyl substituted with one R⁸. In some embodiments, R⁶ is H and R⁷ is 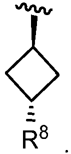 In some embodiments, R⁶ is H and R⁷ is 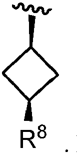 In some embodiments, R⁶ is H and R⁷ is 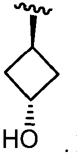 In some embodiments, R⁶ is H and R⁷ is 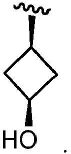 In some embodiments, R⁶ is H and R⁷ is C₆-C₁₂-aryl optionally substituted with one to five R⁸. In some embodiments, R⁶ is H and R⁷ is phenyl substituted with one R⁸. In some embodiments, R⁶ is H and R⁷ is 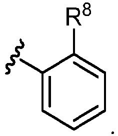 In some embodiments, R⁶ is H and R⁷ is 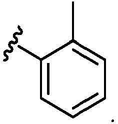 In some embodiments, R⁶ is H and R⁷ is 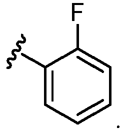 In some embodiments, R⁶ is H and R⁷ is 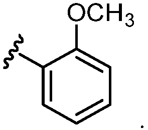 In some embodiments, R⁶ is H and R⁷ is 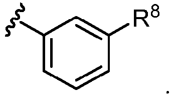 In some embodiments, R⁶ is H and R⁷ is 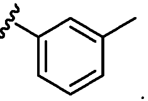 In some embodiments, R⁶ is H and R⁷ is 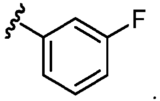 In some embodiments, R⁶ is H and R⁷ is 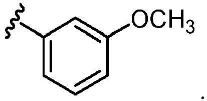 In some embodiments, R⁶ is H and R⁷ is 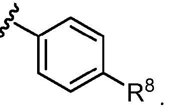 In some embodiments, R⁶ is H and R⁷ is 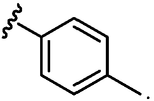 In some embodiments, R⁶ is H and R⁷ is 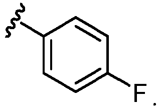 In some embodiments, R⁶ is H and R⁷ is 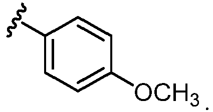 In some embodiments, R⁶ is H and R⁷ is 5-12 membered heteroaryl optionally substituted with one to five R⁸. In some embodiments, R⁶ is H and R⁷ is unsubstituted pyridin-3-yl. In some embodiments, R⁶ is H and R⁷ is unsubstituted pyridin-4-yl. In some embodiments, R⁶ is H and R⁷ is 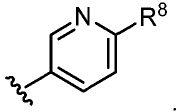 In some embodiments, R⁶ is H and R⁷ is 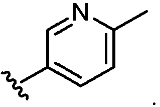 In some embodiments, R⁶ is H and R⁷ is 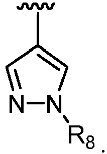 In some embodiments, R⁶ is H and R⁷ is 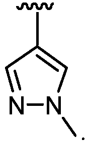 In some embodiments, R⁶ is H and R⁷ is C₁-C₆-alkylene-OH. In some embodiments, R⁶ is H and R⁷ is 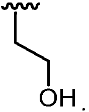 In some embodiments, R⁶ is H and R⁷ is 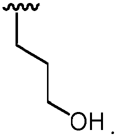 In some embodiments, R⁶ is C₁-C₆-alkyl and R⁷ is C₁-C₆-alkylene-OH. In some embodiments, R⁶ is methyl and R⁷ is 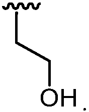 In some embodiments, R⁶ is methyl and R⁷ is 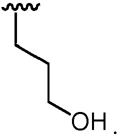 In some embodiments, R⁶ is H and R⁷ is 4-12 membered heterocycloalkyl optionally substituted with one to five R⁸. In some embodiments, R⁶ is H and R⁷ is 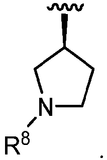 In some embodiments, R⁶ is H and R⁷ is 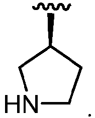 In some embodiments, R⁶ is H and R⁷ is 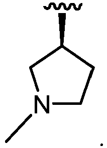 In some embodiments, R⁶ is H and R⁷ is 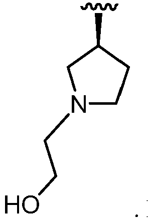 In some embodiments, R⁶ is H and R⁷ is 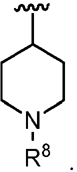 In some embodiments, R⁶ is H and R⁷ is 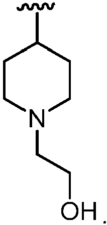 In some embodiments, R⁶ is H and R⁷ is 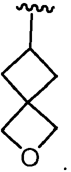 In some embodiments, R⁶ is H and R⁷ is 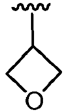 . In some embodiments, R⁶ is H and R⁷ is C₁-C₆-alkylene-(C₃-C₁₂ cycloalkyl). In some embodiments, R⁶ is H and R⁷ is 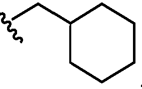 In some embodiments, R⁶ is H and R⁷ is C₁-C₆-alkylene-(C₆-C₁₂-aryl). In some embodiments, R⁶ is H and R⁷ is 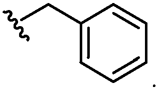 In some embodiments, R⁶ is H and R⁷ is 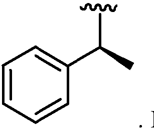 In some embodiments, R⁸ is halo, C₁-C₆-alkyl, C₁-C₆-haloalkyl, OH, C₁-C₆-alkoxy, C₁-C₆-alkylene-OH, or C₁-C₆-haloalkoxy. In some embodiments, R⁸ is halo, C₁-C₆-alkyl, C₁-C₆-alkylene-OH, or C₁-C₆-alkoxy. In some embodiments, R⁸ is halo. In some embodiments, R⁸ is fluoro. In some embodiments, R⁸ is C₁-C₆-alkyl. In some embodiments, R⁸ is methyl. In some embodiments, R⁸ is C₁-C₆-alkylene-OH. In some embodiments, R⁸ is 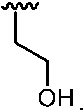 In some embodiments, R⁸ is C₁-C₆-alkoxy. In some embodiments, R⁸ is methoxy.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, R² is 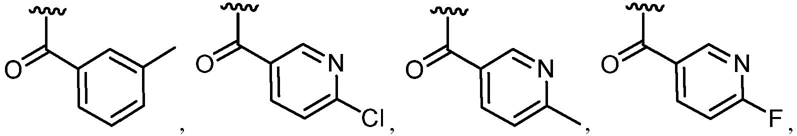 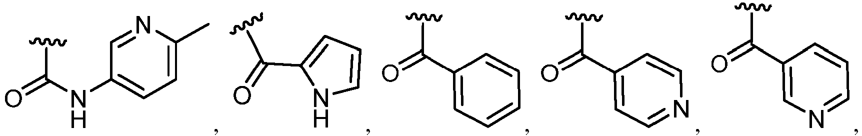 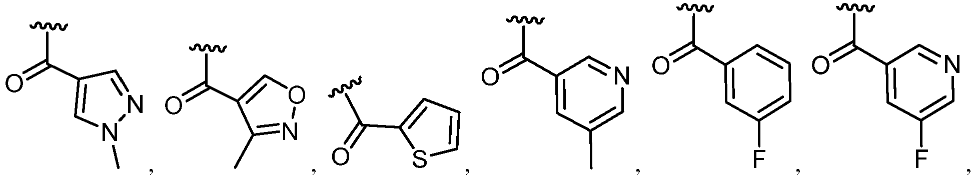 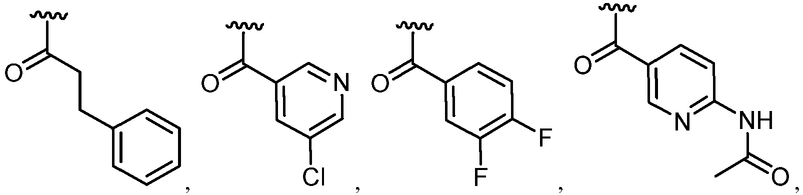 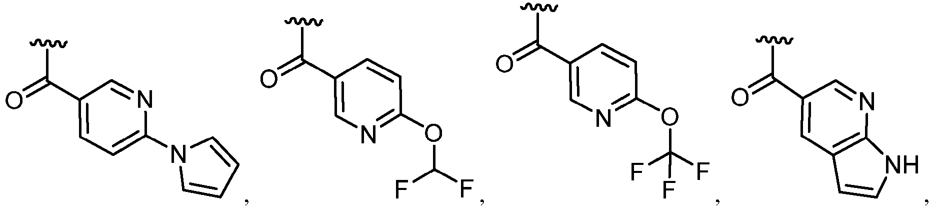 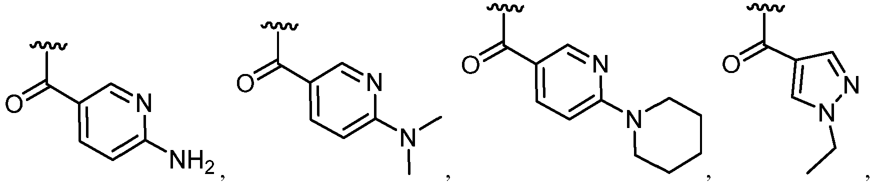 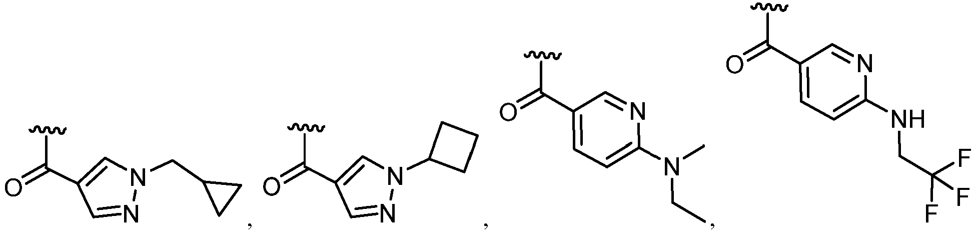 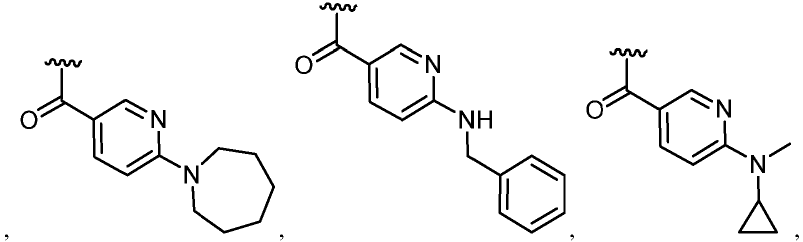 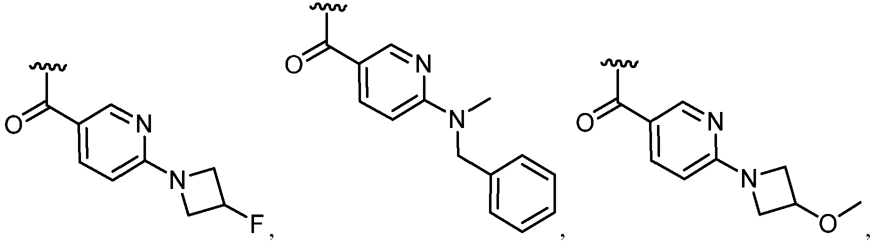 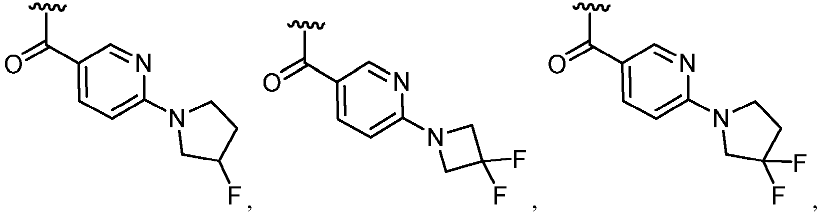 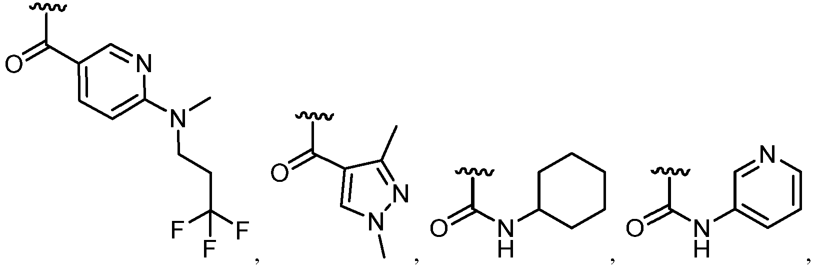 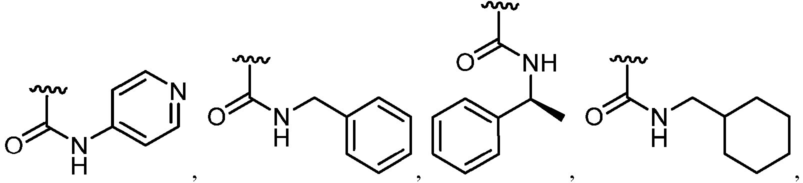 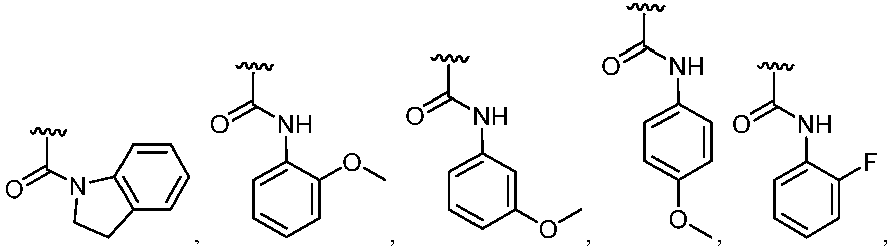 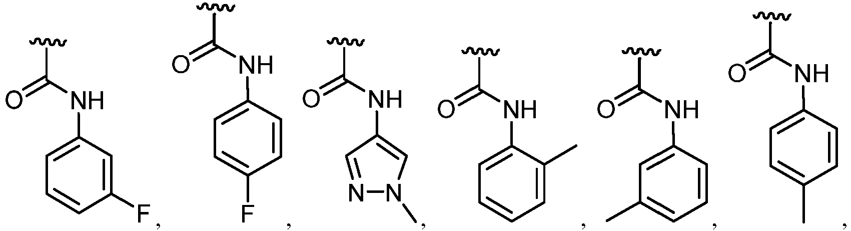 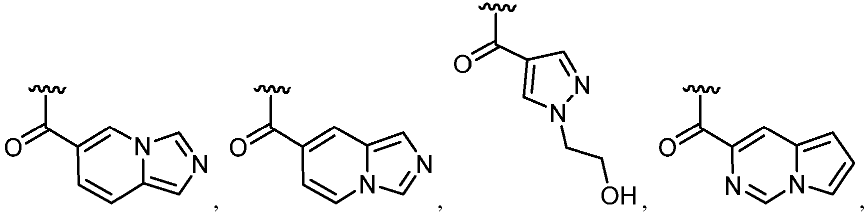 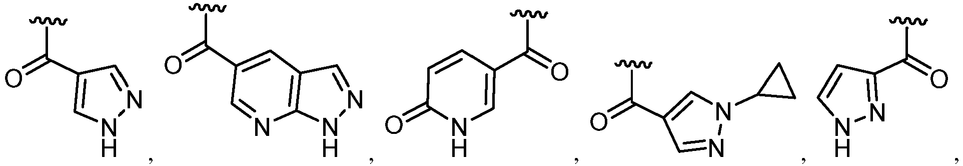 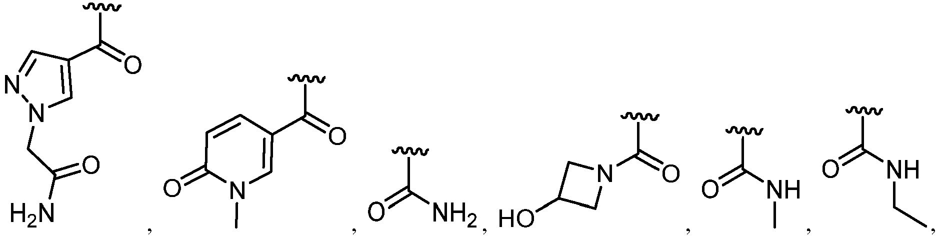 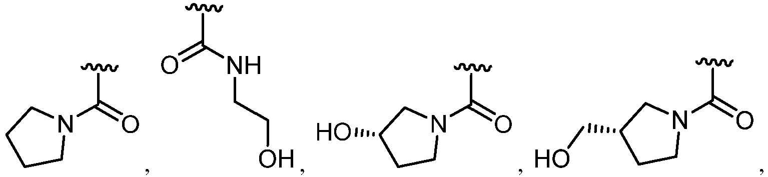 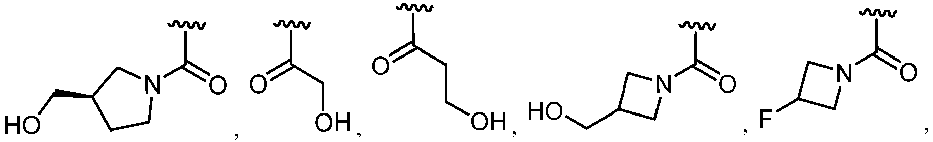 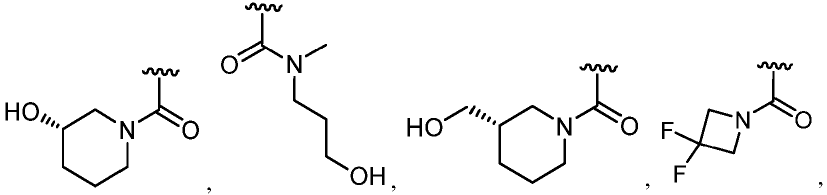 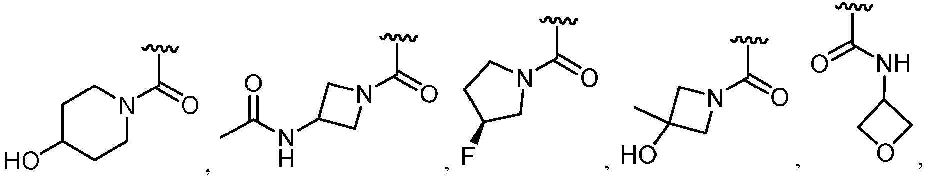 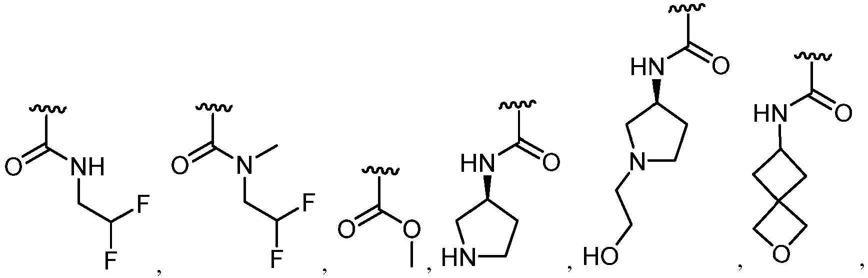 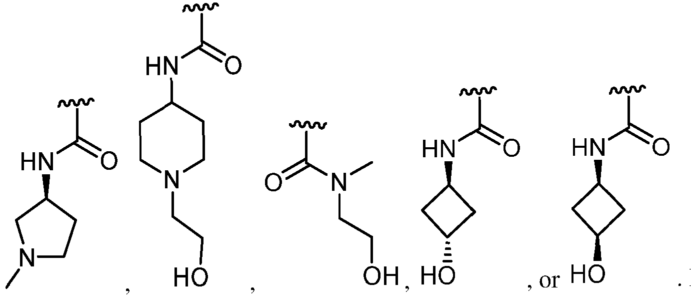 In some embodiments, R² is 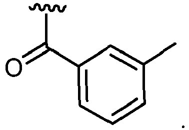 In some embodiments, R² is 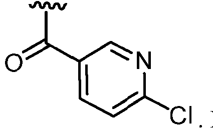 In some embodiments, R² is 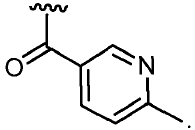 In some embodiments, R² is 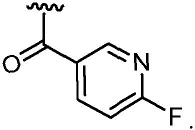 In some embodiments, R² is 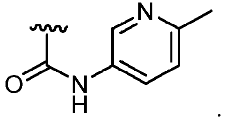 In some embodiments, R² is 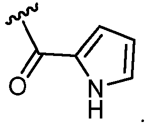 In some embodiments, R² is 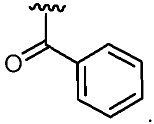 In some embodiments, R² is 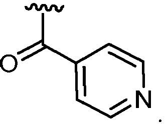 In some embodiments, R² is 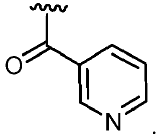 In some embodiments, R² is 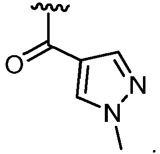 In some embodiments, R² is 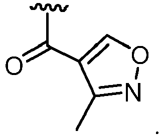 In some embodiments, R² is 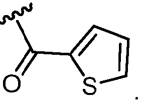 In some embodiments, R² is 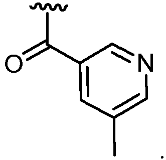 In some embodiments, R² is 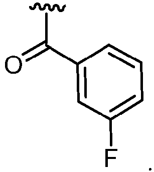 In some embodiments, R² is 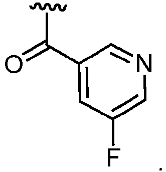 In some embodiments, R² is 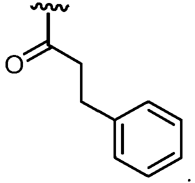 In some embodiments, R² is 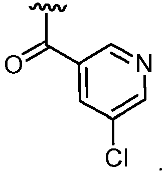 In some embodiments, R² is 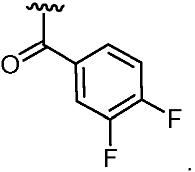 In some embodiments, R² is 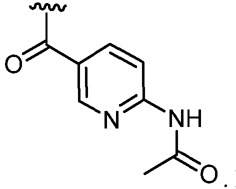 In some embodiments, R² is 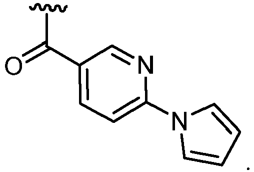 In some embodiments, R² is 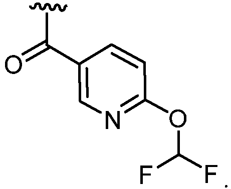 In some embodiments, R² is 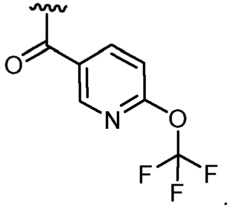 In some embodiments, R² is 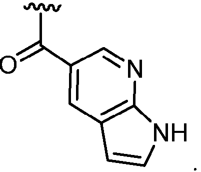 In some embodiments, R² is 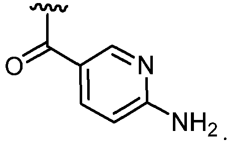 In some embodiments, R² is 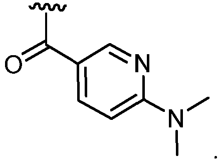 In some embodiments, R² is 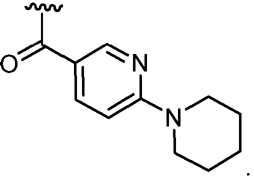 In some embodiments, R² is 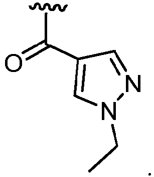 In some embodiments, R² is 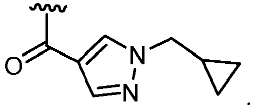 In some embodiments, R² is 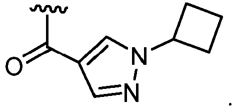 In some embodiments, R² is 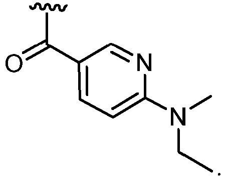 In some embodiments, R² is 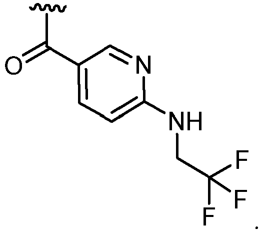 In some embodiments, R² is 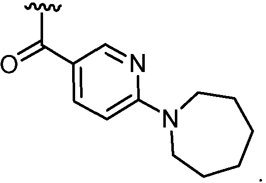 In some embodiments, R² is 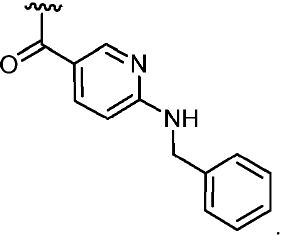 In some embodiments, R² is 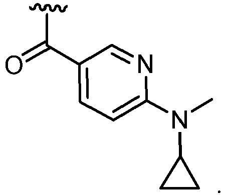 In some embodiments, R² is 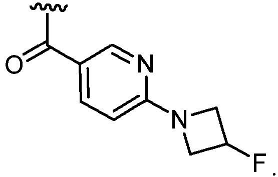 In some embodiments, R² is 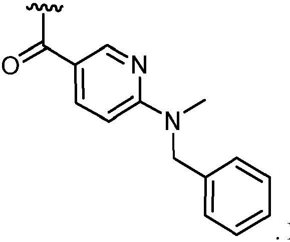 In some embodiments, R² is 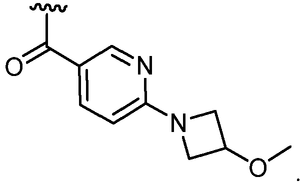 In some embodiments, R² is 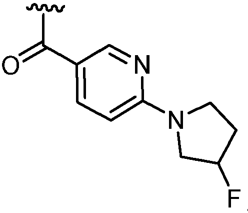 . In some embodiments, R² is 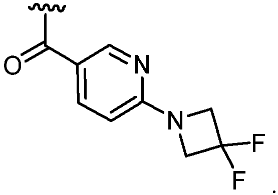 In some embodiments, R² is 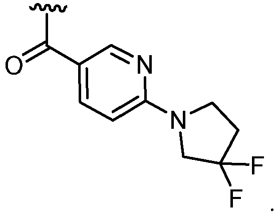 In some embodiments, R² is 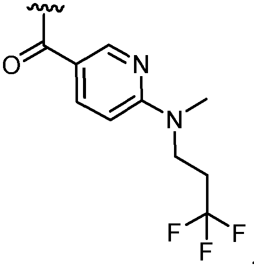 In some embodiments, R² is 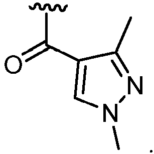 In some embodiments, R² is 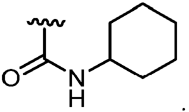 In some embodiments, R² is 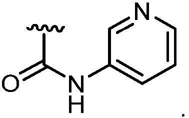 In some embodiments, R² is 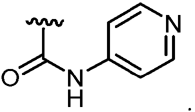 In some embodiments, R² is 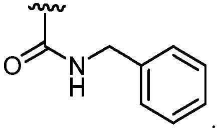 In some embodiments, R² is 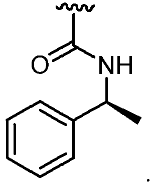 In some embodiments, R² is 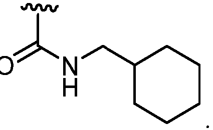 In some embodiments, R² is 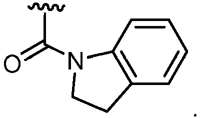 In some embodiments, R² is 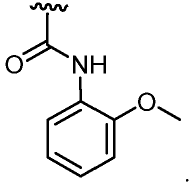 In some embodiments, R² is 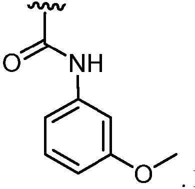 In some embodiments, R² is 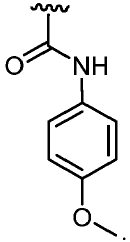 In some embodiments, R² is 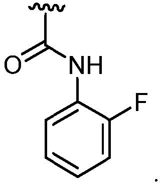 In some embodiments, R² is 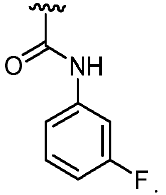 In some embodiments, R² is 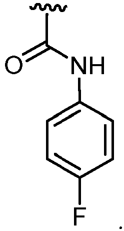 In some embodiments, R² is 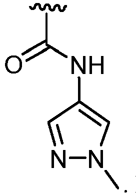 In some embodiments, R² is 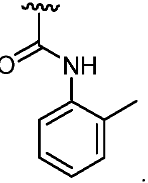 In some embodiments, R² is 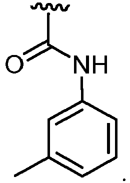 In some embodiments, R² is 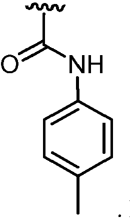 In some embodiments, R² is 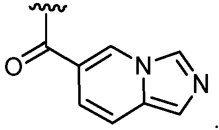 In some embodiments, R² is 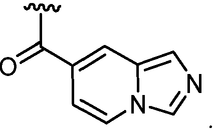 In some embodiments, R² is 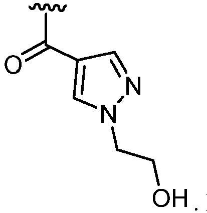 In some embodiments, R² is 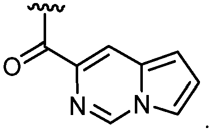 In some embodiments, R² is 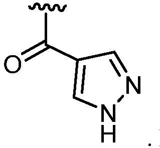 In some embodiments, R² is 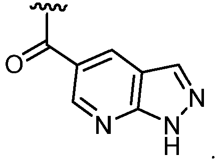 In some embodiments, R² is 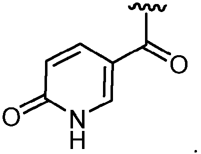 In some embodiments, R² is 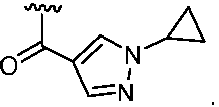 In some embodiments, R² is 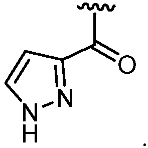 In some embodiments, R² is 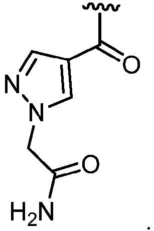 In some embodiments, R² is 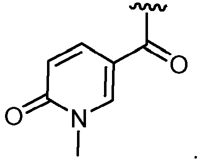 In some embodiments, R² is 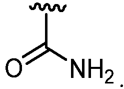 In some embodiments, R² is 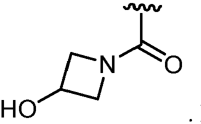 In some embodiments, R² is 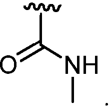 In some embodiments, R² is . In some embodiments, R² is . In some embodiments, R² is 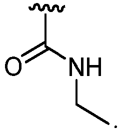 In some embodiments, R² is 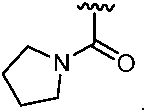 In some embodiments, R² is 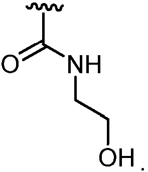 In some embodiments, R² is 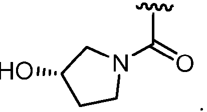 In some embodiments, R² is 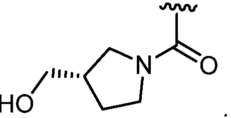 In some embodiments, R² is 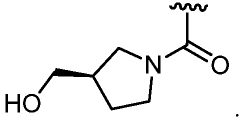 In some embodiments, R² is 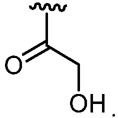 In some embodiments, R² is 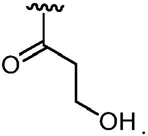 In some embodiments, R² is 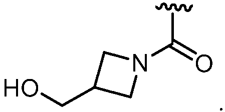 In some embodiments, R² is 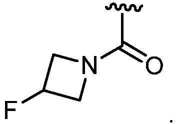 In some embodiments, R² is 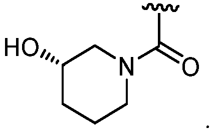 In some embodiments, R² is 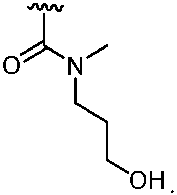 In some embodiments, R² is 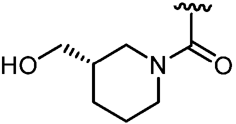 . In some embodiments, R² is 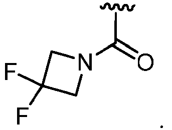 In some embodiments, R² is 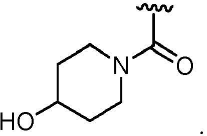 In some embodiments, R² is 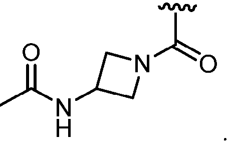 In some embodiments, R² is 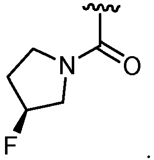 In some embodiments, R² is 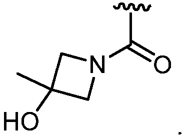 In some embodiments, R² is 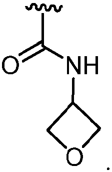 In some embodiments, R² is 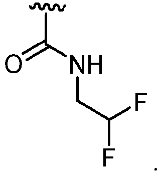 In some embodiments, R² is 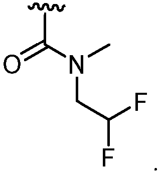 In some embodiments, R² is 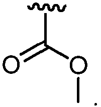 In some embodiments, R² is 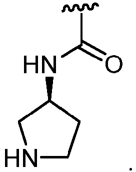 In some embodiments, R² is 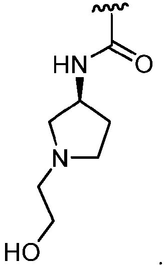 In some embodiments, R² is 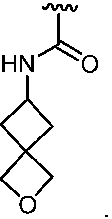 In some embodiments, R² is 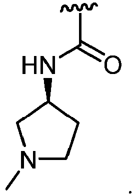 In some embodiments, R² is 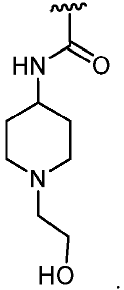 In some embodiments, R² is 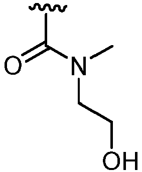 . In some embodiments, R² is 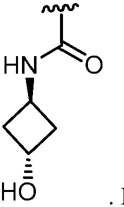 In some embodiments, R² is

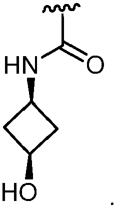

In one aspect, provided herein is a compound of the formula: 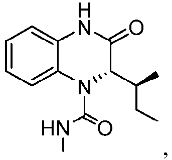 or a pharmaceutically acceptable salt thereof.

In another aspect, provided herein is a compound of the formula: 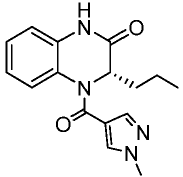 or a pharmaceutically acceptable salt thereof.

In another aspect, provided herein is a compound of the formula: 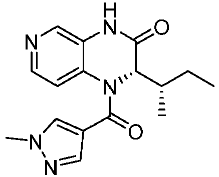 or a pharmaceutically acceptable salt thereof.

In some embodiments of the compounds of formula (I), (I-a), (I-b), (I-c), (I-d), (I-e), (II), (II-a), (II-b), (II-c), (II-d), or (II-e), or a pharmaceutically acceptable salt thereof, the compounds are not 4-(3-methylbenzoyl)-3-propyl-3,4-dihydroquinoxalin-2(1H)-one, 4-(2-chlorobenzoyl)-3-isobutyl-3,4-dihydroquinoxalin-2(1H)-one, or 4-(4-(tert-butyl)benzoyl)-3-propyl-3,4-dihydroquinoxalin-2(1H)-one.

In some embodiments, provided herein is a compound, or a pharmaceutically acceptable salt thereof, wherein the compound is selected from Table 2.

**Table 2**

| Compound No. | Compound Structure | Compound Name |
|---|---|---|
| 1 | 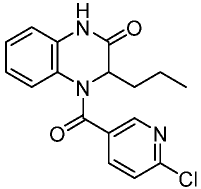 | 4-(6-chloronicotinoyl)-3-propyl-3,4-dihydroquinoxalin-2(1H)-one |
| 2 | 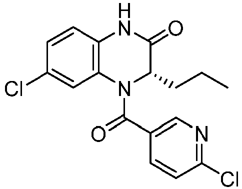 | (S)-6-chloro-4-(6-chloronicotinoyl)-3-propyl-3,4-dihydroquinoxalin-2(1H)-one |
| 3 | 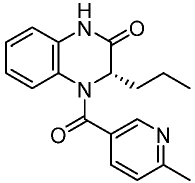 | (S)-4-(6-methylnicotinoyl)-3-propyl-3,4-dihydroquinoxalin-2(1H)-one |
| 4 | 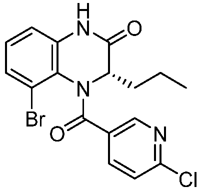 | (S)-5-bromo-4-(6-chloronicotinoyl)-3-propyl-3,4-dihydroquinoxalin-2(1H)-one |
| 5 | 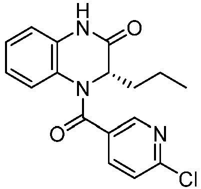 | (S)-4-(6-chloronicotinoyl)-3-propyl-3,4-dihydroquinoxalin-2(1H)-one |
| 6 | 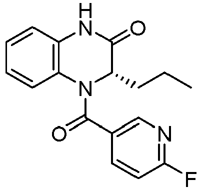 | (S)-4-(6-fluoronicotinoyl)-3-propyl-3,4-dihydroquinoxalin-2(1H)-one |
| 7 | 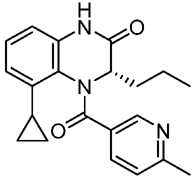 | (S)-5-cyclopropyl-4-(6-methylnicotinoyl)-3-propyl-3,4-dihydroquinoxalin-2(1H)-one |
| 8 | 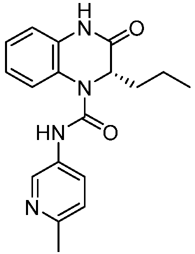 | (S)-N-(6-methylpyridin-3-yl)-3-oxo-2-propyl-3,4-dihydroquinoxaline- 1(2H)-carboxamide |
| 9 | 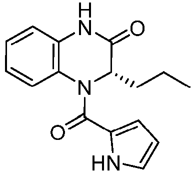 | (S)-3-propyl-4-(1H-pyrrole-2-carbonyl)-3,4-dihydroquinoxalin-2(1H)-one |
| 10 | 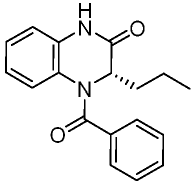 | (S)-4-benzoyl-3-propyl-3,4-dihydroquinoxalin-2(1H)-one |
| 11 | 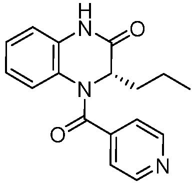 | (S)-4-isonicotinoyl-3-propyl-3,4-dihydroquinoxalin-2(1H)-one |
| 12 | 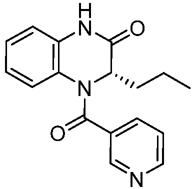 | (S)-4-nicotinoyl-3-propyl-3,4-dihydroquinoxalin-2(1H)-one |
| 13 | 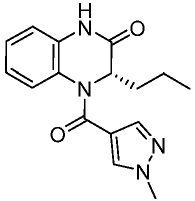 | (S)-4-(1-methyl-1H-pyrazole-4-carbonyl)-3-propyl-3,4-dihydroquinoxalin-2(1H)-one |
| 14 | 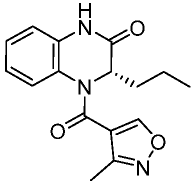 | (S)-4-(3-methylisoxazole-4-carbonyl)-3-propyl-3,4-dihydroquinoxalin-2(1H)-one |
| 15 | 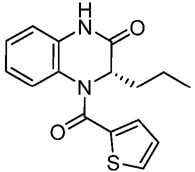 | (S)-3-propyl-4-(thiophene-2-carbonyl)-3,4-dihydroquinoxalin-2(1H)-one |
| 16 | 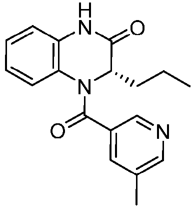 | (S)-4-(5-methylnicotinoyl)-3-propyl-3,4-dihydroquinoxalin-2(1H)-one |
| 17 | 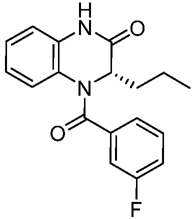 | (S)-4-(3-fluorobenzoyl)-3-propyl-3,4-dihydroquinoxalin-2(1H)-one |
| 18 | 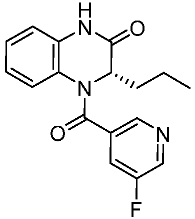 | (S)-4-(5-fluoronicotinoyl)-3-propyl-3,4-dihydroquinoxalin-2(1H)-one |
| 19 | 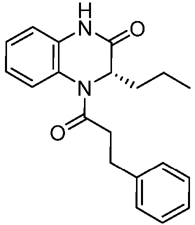 | (S)-4-(3-phenylpropanoyl)-3-propyl-3,4-dihydroquinoxalin-2(1H)-one |
| 20 | 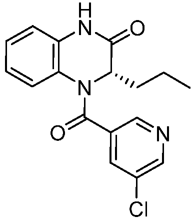 | (S)-4-(5-chloronicotinoyl)-3-propyl-3,4-dihydroquinoxalin-2(1H)-one |
| 21 | 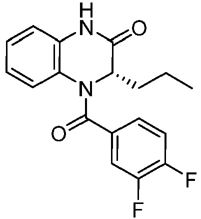 | (S)-4-(3,4-difluorobenzoyl)-3-propyl-3,4-dihydroquinoxalin-2(1H)-one |
| 22 | 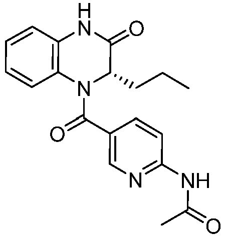 | (S)-N-(5-(3-oxo-2-propyl-1,2,3,4-tetrahydroquinoxaline-1-carbonyl)pyridin-2-yl)acetamide |
| 23 | 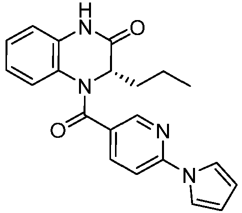 | (S)-4-(6-(1H-pyrrol-1-yl)nicotinoyl)-3-propyl-3,4-dihydroquinoxalin-2(1H)-one |
| 24 | 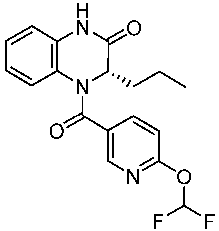 | (S)-4-(6-(difluoromethoxy)nicotinoyl)-3-propyl-3,4-dihydroquinoxalin-2(1H)-one |
| 25 | 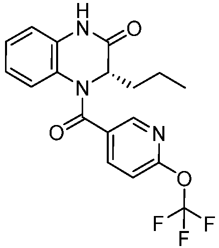 | (S)-3-propyl-4-(6-(trifluoromethoxy)nicotinoyl)-3,4-dihydroquinoxalin-2(1H)-one |
| 26 | 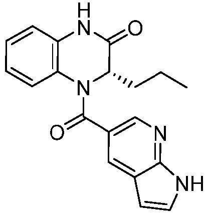 | (S)-3-propyl-4-(1H-pyrrolo[2,3-b]pyridine-5-carbonyl)-3,4-dihydroquinoxalin-2(1H)-one |
| 27 | 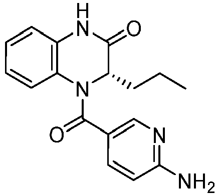 | (S)-4-(6-aminonicotinoyl)-3-propyl-3,4-dihydroquinoxalin-2(1H)-one |
| 28 | 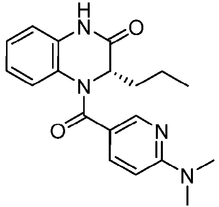 | (S)-4-(6-(dimethylamino)nicotinoyl)-3-propyl-3,4-dihydroquinoxalin-2(1H)-one |
| 29 | 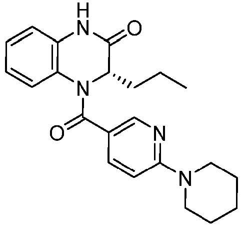 | (S)-4-(6-(piperidin-1-yl)nicotinoyl)-3-propyl-3,4-dihydroquinoxalin-2(1H)-one |
| 30 | 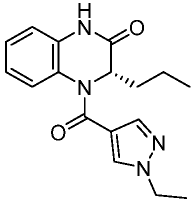 | (S)-4-(1-ethyl-1H-pyrazole-4-carbonyl)-3-propyl-3,4-dihydroquinoxalin-2(1H)-one |
| 31 | 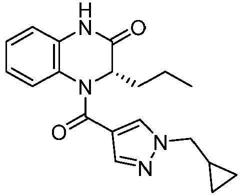 | (S)-4-(1-(cyclopropylmethyl)-1H-pyrazole-4-carbonyl)-3-propyl-3,4-dihydroquinoxalin-2(1H)-one |
| 32 | 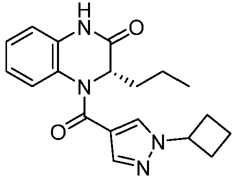 | (S)-4-(1-cyclobutyl-1H-pyrazole-4-carbonyl)-3-propyl-3,4-dihydroquinoxalin-2(1H)-one |
| 33 | 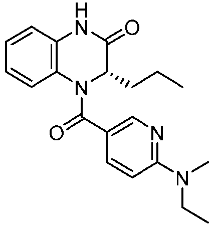 | (S)-4-(6-(ethyl(methyl)amino)nicotinoyl)-3-propyl-3,4-dihydroquinoxalin-2(1H)-one |
| 34 | 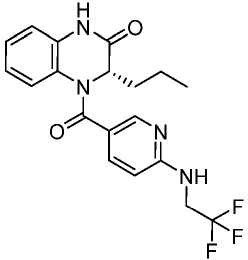 | (S)-3-propyl-4-(6-((2,2,2-trifluoroethyl)amino)nicotinoyl)-3,4-dihydroquinoxalin-2(1H)-one |
| 35 | 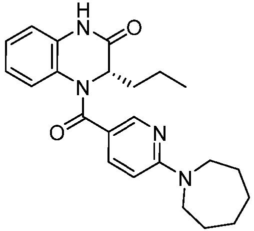 | (S)-4-(6-(azepan-1-yl)nicotinoyl)-3-propyl-3,4-dihydroquinoxalin-2(1H)-one |
| 36 | 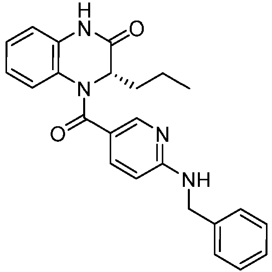 | (S)-4-(6-(benzylamino)nicotinoyl)-3-propyl-3,4-dihydroquinoxalin-2(1H)-one |
| 37 | 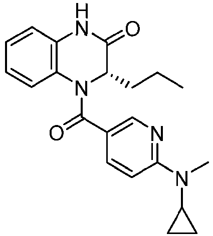 | (S)-4-(6-(cyclopropyl(methyl)amino)nicotinoyl)-3-propyl-3,4-dihydroquinoxalin-2(1H)-one |
| 38 | 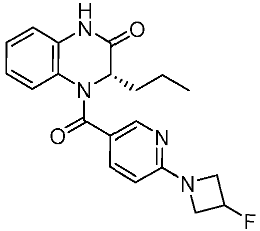 | (S)-4-(6-(3-fluoroazetidin-1-yl)nicotinoyl)-3-propyl-3,4-dihydroquinoxalin-2(1H)-one |
| 39 | 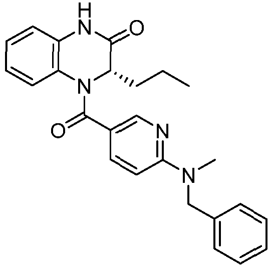 | (S)-4-(6-(benzyl(methyl)amino)nicotinoyl)-3-propyl-3,4-dihydroquinoxalin-2(1H)-one |
| 40 | 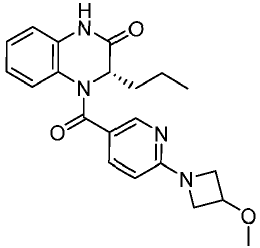 | (S)-4-(6-(3-methoxyazetidin-1-yl)nicotinoyl)-3-propyl-3,4-dihydroquinoxalin-2(1H)-one |
| 41 | 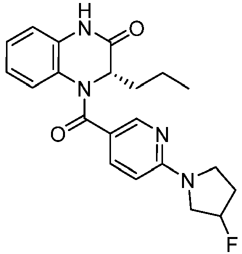 | (3S)-4-(6-(3-fluoropyrrolidin-1-yl)nicotinoyl)-3-propyl-3,4-dihydroquinoxalin-2(1H)-one |
| 42 | 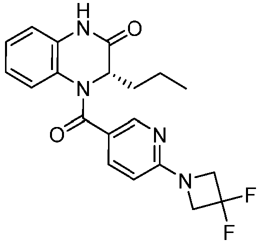 | (S)-4-(6-(3,3-difluoroazetidin-1-yl)nicotinoyl)-3-propyl-3,4-dihydroquinoxalin-2(1H)-one |
| 43 | 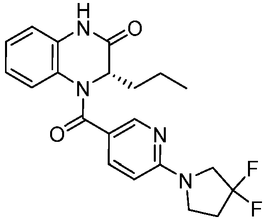 | (S)-4-(6-(3,3-difluoropyrrolidin-1-yl)nicotinoyl)-3-propyl-3,4-dihydroquinoxalin-2(1H)-one |
| 44 | 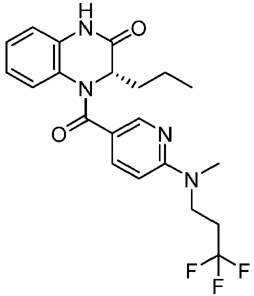 | (S)-4-(6-(methyl(3,3,3-trifluoropropyl)amino)nicotinoyl)-3-propyl-3,4-dihydroquinoxalin-2(1H)-one |
| 45 | 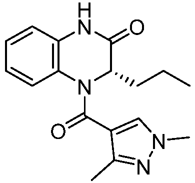 | (S)-4-(1,3-dimethyl- 1H-pyrazole-4-carbonyl)-3-propyl-3,4-dihydroquinoxalin-2(1H)-one |
| 46 | 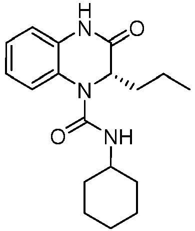 | (S)-N-cyclohexyl-3-oxo-2-propyl-3,4-dihydroquinoxaline- 1(2H)-carboxamide |
| 47 | 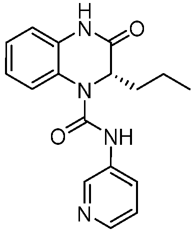 | (S)-3-oxo-2-propyl-N-(pyridin-3-yl)-3,4-dihydroquinoxaline- 1(2H)-carboxamide |
| 48 | 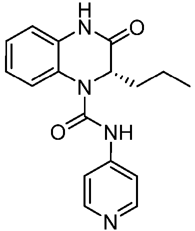 | (S)-3-oxo-2-propyl-N-(pyridin-4-yl)-3,4-dihydroquinoxaline- 1(2H)-carboxamide |
| 49 | 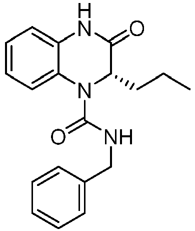 | (S)-N-benzyl-3-oxo-2-propyl-3,4-dihydroquinoxaline- 1(2H)-carboxamide |
| 50 | 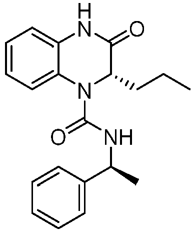 | (S)-3-oxo-N-((S)-1-phenylethyl)-2-propyl-3,4-dihydroquinoxaline- 1(2H)-carboxamide |
| 51 | 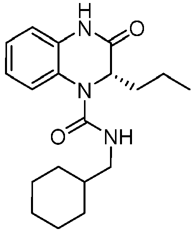 | (S)-N-(cyclohexylmethyl)-3-oxo-2-propyl-3,4-dihydroquinoxaline- 1(2H)-carboxamide |
| 52 | 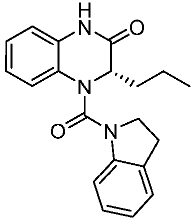 | (S)-4-(indoline-1-carbonyl)-3-propyl-3,4-dihydroquinoxalin-2(1H)-one |
| 53 | 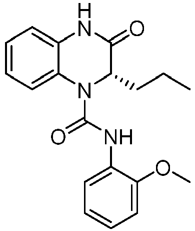 | (S)-N-(2-methoxyphenyl)-3-oxo-2-propyl-3,4-dihydroquinoxaline- 1(2H)-carboxamide |
| 54 | 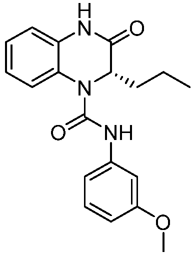 | (S)-N-(3-methoxyphenyl)-3-oxo-2-propyl-3,4-dihydroquinoxaline- 1(2H)-carboxamide |
| 55 | 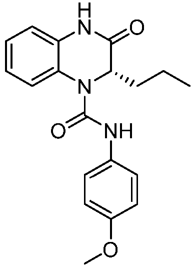 | (S)-N-(4-methoxyphenyl)-3-oxo-2-propyl-3,4-dihydroquinoxaline- 1(2H)-carboxamide |
| 56 | 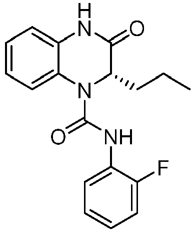 | (S)-N-(2-fluorophenyl)-3-oxo-2-propyl-3,4-dihydroquinoxaline- 1(2H)-carboxamide |
| 57 | 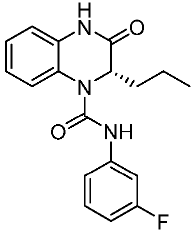 | (S)-N-(3-fluorophenyl)-3-oxo-2-propyl-3,4-dihydroquinoxaline- 1(2H)-carboxamide |
| 58 | 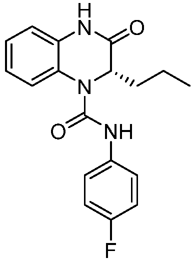 | (S)-N-(4-fluorophenyl)-3-oxo-2-propyl-3,4-dihydroquinoxaline- 1(2H)-carboxamide |
| 59 | 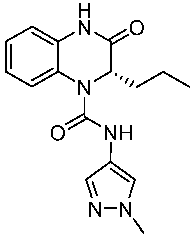 | (S)-N-(1-methyl-1H-pyrazol-4-yl)-3-oxo-2-propyl-3,4-dihydroquinoxaline-1(2H)-carboxamide |
| 60 | 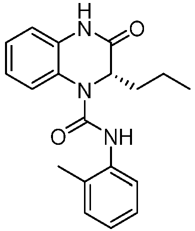 | (S)-3-oxo-2-propyl-N-(o-tolyl)-3,4-dihydroquinoxaline- 1(2H)-carboxamide |
| 61 | 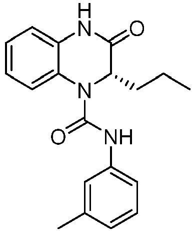 | (S)-3-oxo-2-propyl-N-(m-tolyl)-3,4-dihydroquinoxaline- 1(2H)-carboxamide |
| 62 | 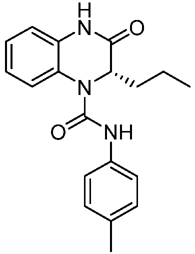 | (S)-3-oxo-2-propyl-N-(p-tolyl)-3,4-dihydroquinoxaline- 1(2H)-carboxamide |
| 63 | 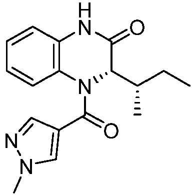 | (S)-3-((S)-sec-butyl)-4-(1-methyl-1H-pyrazole-4-carbonyl)-3,4-dihydroquinoxalin-2(1H)-one |
| 64 | 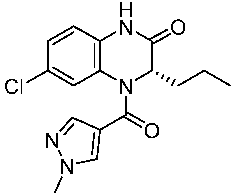 | (S)-6-chloro-4-(1-methyl-1H-pyrazole-4-carbonyl)-3-propy1-3,4-dihydroquinoxalin-2(1H)-one |
| 65 | 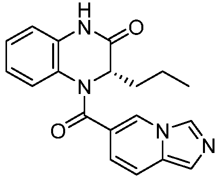 | (S)-4-(imidazo[1,5-a]pyridine-6-carbonyl)-3-propyl-3,4-dihydroquinoxalin-2(1H)-one |
| 66 | 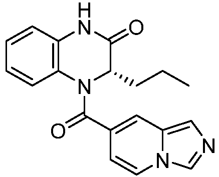 | (S)-4-(imidazo[1,5-a]pyridine-7-carbonyl)-3-propyl-3,4-dihydroquinoxalin-2(1H)-one |
| 67 | 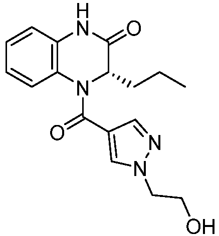 | (S)-4-(1-(2-hydroxyethyl)-1H-pyrazole-4-carbonyl)-3-propyl-3,4-dihydroquinoxalin-2(1H)-one |
| 68 | 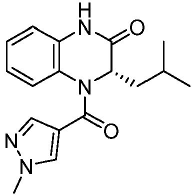 | (S)-3-isobutyl-4-(1-methyl-1H-pyrazole-4-carbonyl)-3,4-dihydroquinoxalin-2(1H)-one |
| 69 | 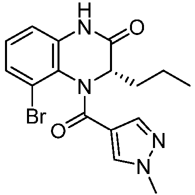 | (S)-5-bromo-4-(1-methyl-1H-pyrazole-4-carbonyl)-3-propyl-3,4-dihydroquinoxalin-2(1H)-one |
| 70 | 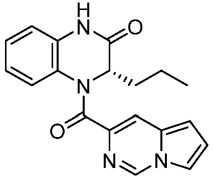 | (S)-3-propyl-4-(pyrrolo[1,2-c]pyrimidine-3-carbonyl)-3,4-dihydroquinoxalin-2(1H)-one |
| 71 | 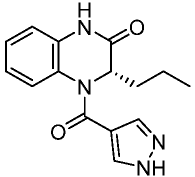 | (S)-3-propyl-4-(1H-pyrazole-4-carbonyl)-3,4-dihydroquinoxalin-2(1H)-one |
| 72 | 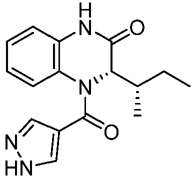 | (S)-3-((S)-sec-butyl)-4-(1H-pyrazole-4-carbonyl)-3,4-dihydroquinoxalin-2(1H)-one |
| 73 | 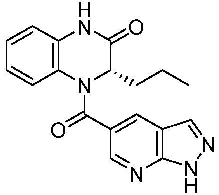 | (S)-3-propyl-4-(1H-pyrazolo[3,4-b]pyridine-5-carbonyl)-3,4-dihydroquinoxalin-2(1H)-one |
| 74 | 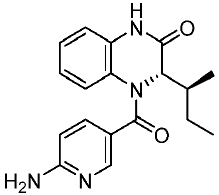 | (S)-4-(6-aminonicotinoyl)-3-((S)-sec-butyl)-3,4-dihydroquinoxalin-2(1H)-one |
| 75 | 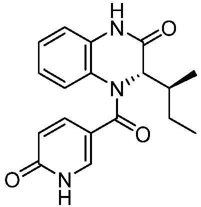 | (S)-3-((S)-sec-butyl)-4-(6-oxo-1,6-dihydropyridine-3-carbonyl)-3,4-dihydroquinoxalin-2(1H)-one |
| 76 | 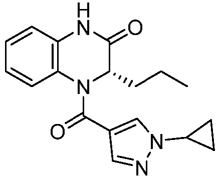 | (S)-4-(1-cyclopropyl-1H-pyrazole-4-carbonyl)-3-propyl-3,4-dihydroquinoxalin-2(1H)-one |
| 77 | 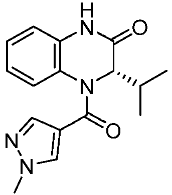 | (S)-3-isopropyl-4-(1-methyl-1H-pyrazole-4-carbonyl)-3,4-dihydroquinoxalin-2(1H)-one |
| 78 | 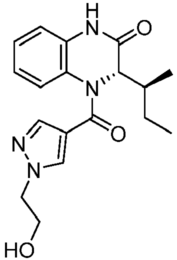 | (S)-3-((S)-sec-butyl)-4-(1-(2-hydroxyethyl)-1H-pyrazole-4-carbonyl)-3,4-dihydroquinoxalin-2(1H)-one |
| 79 | 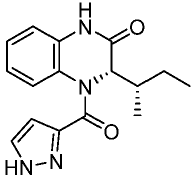 | (S)-3-((S)-sec-butyl)-4-(1H-pyrazole-3-carbonyl)-3,4-dihydroquinoxalin-2(1H)-one |
| 80 | 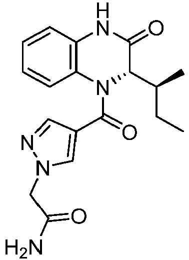 | 2-(4-((S)-2-((S)-sec-butyl)-3-oxo-1,2,3,4-tetrahydroquinoxaline-1-carbonyl)-1H-pyrazol-1-yl)acetamide |
| 81 | 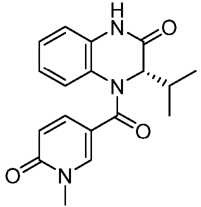 | (S)-3-isopropyl-4-(1-methyl-6-oxo-1,6-dihydropyridine-3-carbonyl)-3,4-dihydroquinoxalin-2(1H)-one |
| 82 | 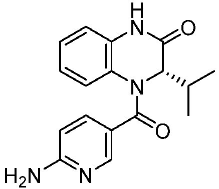 | (S)-4-(6-aminonicotinoyl)-3-isopropyl-3,4-dihydroquinoxalin-2(1H)-one |
| 83 | 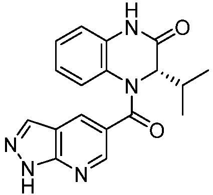 | (S)-3-isopropyl-4-(1H-pyrazolo[3,4-b]pyridine-5-carbonyl)-3,4-dihydroquinoxalin-2(1H)-one |
| 84 | 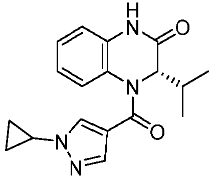 | (S)-4-(1-cyclopropyl-1H-pyrazole-4-carbonyl)-3-isopropyl-3,4-dihydroquinoxalin-2(1H)-one |
| 85 | 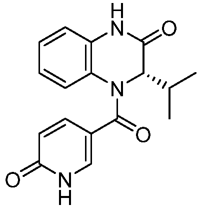 | (S)-3-isopropyl-4-(6-oxo-1,6-dihydropyridine-3-carbonyl)-3,4-dihydroquinoxalin-2(1H)-one |
| 86 | 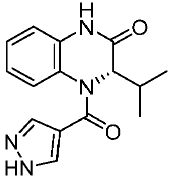 | (S)-3-isopropyl-4-(1H-pyrazole-4-carbonyl)-3,4-dihydroquinoxalin-2(1H)-one |
| 87 | 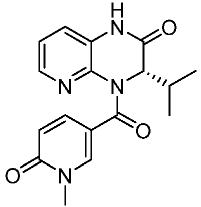 | (S)-3-isopropyl-4-(1-methyl-6-oxo-1,6-dihydropyridine-3-carbonyl)-3,4-dihydropyrido[2,3-b]pyrazin-2(1H)-one |
| 88 | 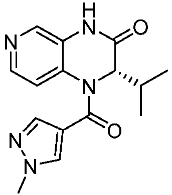 | (S)-2-isopropyl-1-(1-methyl-1H-pyrazole-4-carbonyl)-1,4-dihydropyrido[3,4-b]pyrazin-3(2H)-one |
| 89 | 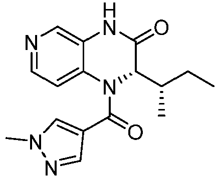 | (S)-2-((S)-sec-butyl)-1-(1-methyl-1H-pyrazole-4-carbonyl)-1,4-dihydropyrido[3,4-b]pyrazin-3(2H)-one |
| 90 | 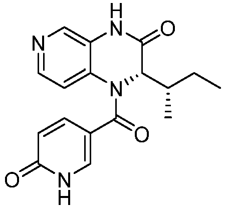 | (S)-2-((S)-sec-butyl)-1-(6-oxo-1,6-dihydropyridine-3-carbonyl)-1,4-dihydropyrido[3,4-b]pyrazin-3(2H)-one |
| 91 | 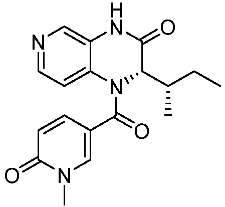 | (S)-2-((S)-sec-butyl)-1-(1-methyl-6-oxo-1,6-dihydropyridine-3-carbonyl)-1,4-dihydropyrido[3,4-b]pyrazin-3(2H)-one |
| 92 | 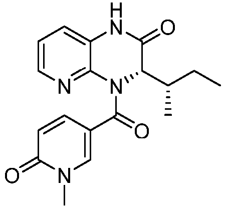 | (S)-3-((S)-sec-butyl)-4-(1-methyl-6-oxo-1,6-dihydropyridine-3-carbonyl)-3,4-dihydropyrido[2,3-b]pyrazin-2(1H)-one |
| 93 | 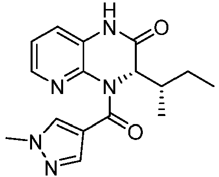 | (S)-3-((S)-sec-butyl)-4-(1-methyl-1H-pyrazole-4-carbonyl)-3,4-dihydropyrido[2,3-b]pyrazin-2(1H)-one |
| 94 | 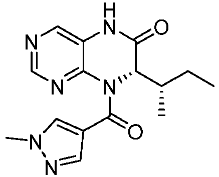 | (S)-7-((S)-sec-butyl)-8-(1-methyl-1H-pyrazole-4-carbonyl)-7,8-dihydropteridin-6(5H)-one |
| 95 | 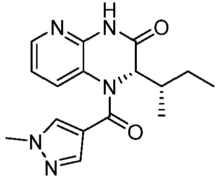 | (S)-2-((S)-sec-butyl)-1-(1-methyl-1H-pyrazole-4-carbonyl)-1,4-dihydropyrido[2,3-b]pyrazin-3(2H)-one |
| 96 | 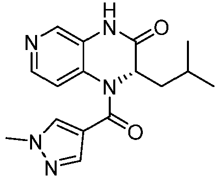 | (S)-2-isobutyl-1-(1-methyl-1H-pyrazole-4-carbonyl)-1,4-dihydropyrido[3,4-b]pyrazin-3(2H)-one |
| 97 | 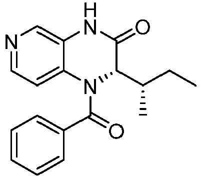 | (S)-1-benzoyl-2-((S)-sec-butyl)-1,4-dihydropyrido[3,4-b]pyrazin-3(2H)-one |
| 98 | 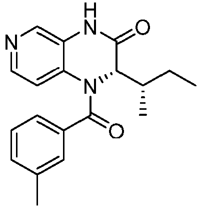 | (S)-2-((S)-sec-butyl)-1-(3-methylbenzoyl)-1,4-dihydropyrido[3,4-b]pyrazin-3(2H)-one |
| 99 | 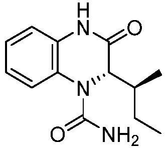 | (S)-2-((S)-sec-butyl)-3-oxo-3,4-dihydroquinoxaline- 1(2H)-carboxamide |
| 100 | 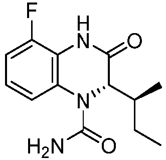 | (S)-2-((S)-sec-butyl)-5-fluoro-3-oxo-3,4-dihydroquinoxaline- 1(2H)-carboxamide |
| 101 | 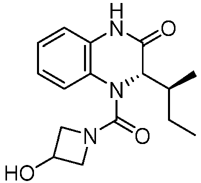 | (S)-3-((S)-sec-butyl)-4-(3-hydroxyazetidine-1-carbonyl)-3,4-dihydroquinoxalin-2(1H)-one |
| 102 | 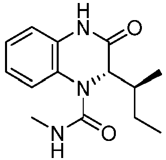 | (S)-2-((S)-sec-butyl)-N-methyl-3-oxo-3,4-dihydroquinoxaline- 1(2H)-carboxamide |
| 103 | 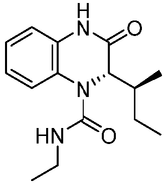 | (S)-2-((S)-sec-butyl)-N-ethyl-3-oxo-3,4-dihydroquinoxaline- 1(2H)-carboxamide |
| 104 | 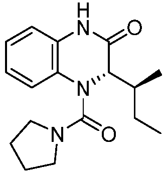 | (S)-3-((S)-sec-butyl)-4-(pyrrolidine- 1-carbonyl)-3,4-dihydroquinoxalin-2(1H)-one |
| 105 | 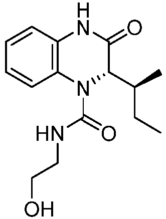 | (S)-2-((S)-sec-butyl)-N-(2-hydroxyethyl)-3-oxo-3,4-dihydroquinoxaline-1(2H)-carboxamide |
| 106 | 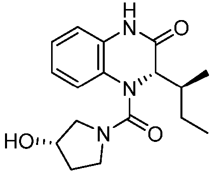 | (S)-3-((S)-sec-butyl)-4-((S)-3-hydroxypyrrolidine-1-carbonyl)-3,4-dihydroquinoxalin-2(1H)-one |
| 107 | 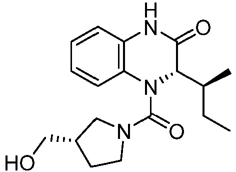 | (S)-3-((S)-sec-butyl)-4-((S)-3-(hydroxymethyl)pyrrolidine-1-carbonyl)-3,4-dihydroquinoxalin-2(1H)-one |
| 108 | 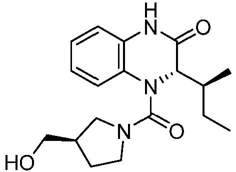 | (S)-3-((S)-sec-butyl)-4-((R)-3-(hydroxymethyl)pyrrolidine-1-carbonyl)-3,4-dihydroquinoxalin-2(1H)-one |
| 109 | 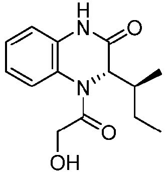 | (S)-3-((S)-sec-butyl)-4-(2-hydroxyacetyl)-3,4-dihydroquinoxalin-2(1H)-one |
| 110 | 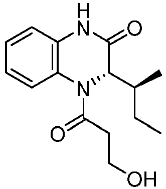 | (S)-3-((S)-sec-butyl)-4-(3-hydroxypropanoyl)-3,4-dihydroquinoxalin-2(1H)-one |
| 111 | 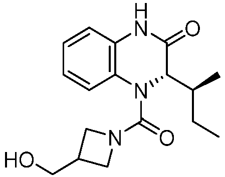 | (S)-3-((S)-sec-butyl)-4-(3-(hydroxymethyl)azetidine-1-carbonyl)-3,4-dihydroquinoxalin-2(1H)-one |
| 112 | 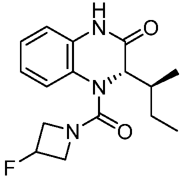 | (S)-3-((S)-sec-butyl)-4-(3-fluoroazetidine-1-carbonyl)-3,4-dihydroquinoxalin-2(1H)-one |
| 113 | 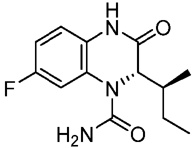 | (S)-2-((S)-sec-butyl)-7-fluoro-3-oxo-3,4-dihydroquinoxaline- 1(2H)-carboxamide |
| 114 | 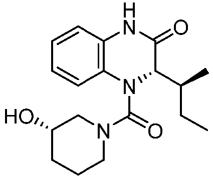 | (S)-3-((S)-sec-butyl)-4-((S)-3-hydroxypiperidine-1-carbonyl)-3,4-dihydroquinoxalin-2(1H)-one |
| 115 | 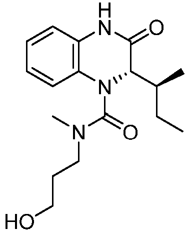 | (S)-2-((S)-sec-butyl)-N-(3-hydroxypropyl)-N-methyl-3-oxo-3,4-dihydroquinoxaline-1(2H)-carboxamide |
| 116 | 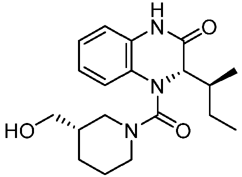 | (S)-3-((S)-sec-butyl)-4-((S)-3-(hydroxymethyl)piperidine-1-carbonyl)-3,4-dihydroquinoxalin-2(1H)-one |
| 117 | 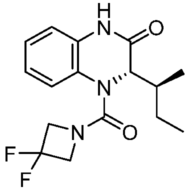 | (S)-3-((S)-sec-butyl)-4-(3,3-difluoroazetidine-1-carbonyl)-3,4-dihydroquinoxalin-2(1H)-one |
| 118 | 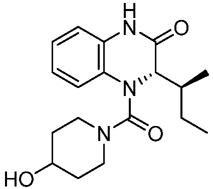 | (S)-3-((S)-sec-butyl)-4-(4-hydroxypiperidine-1-carbonyl)-3,4-dihydroquinoxalin-2(1H)-one |
| 119 | 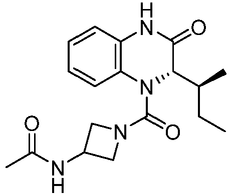 | N-(1-((S)-2-((S)-sec-butyl)-3-oxo-1,2,3,4-tetrahydroquinoxaline-1-carbonyl)azetidin-3-yl)acetamide |
| 120 | 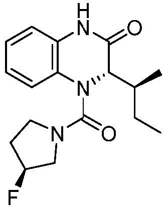 | (S)-3-((S)-sec-butyl)-4-((S)-3-fluoropyrrolidine-1-carbonyl)-3,4-dihydroquinoxalin-2(1H)-one |
| 121 | 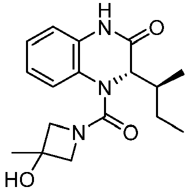 | (S)-3-((S)-sec-butyl)-4-(3-hydroxy-3-methylazetidine-1-carbonyl)-3,4-dihydroquinoxalin-2(1H)-one |
| 122 | 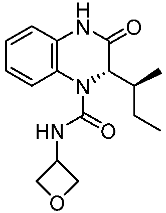 | (S)-2-((S)-sec-butyl)-N-(oxetan-3-yl)-3-oxo-3,4-dihydroquinoxaline- 1(2H)-carboxamide |
| 123 | 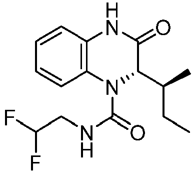 | (S)-2-((S)-sec-butyl)-N-(2,2-difluoroethyl)-3-oxo-3,4-dihydroquinoxaline-1(2H)-carboxamide |
| 124 | 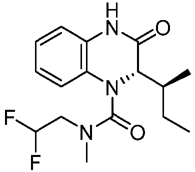 | (S)-2-((S)-sec-butyl)-N-(2,2-difluoroethyl)-N-methyl-3-oxo-3,4-dihydroquinoxaline-1(2H)-carboxamide |
| 125 | 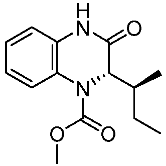 | methyl (S)-2-((S)-sec-butyl)-3-oxo-3,4-dihydroquinoxaline-1(2H)-carboxylate |
| 126 | 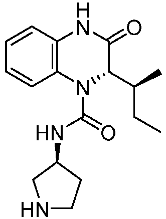 | (S)-2-((S)-sec-butyl)-3-oxo-N-((S)-pyrrolidin-3-yl)-3,4-dihydroquinoxaline-1(2H)-carboxamide |
| 127 | 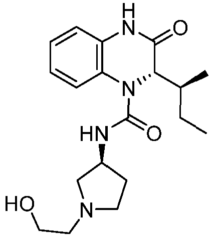 | (S)-2-((S)-sec-butyl)-N-((S)-1-(2-hydroxyethyl)pyrrolidin-3-yl)-3-oxo-3,4-dihydroquinoxaline-1(2H)-carboxamide |
| 128 | 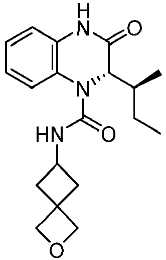 | (S)-2-((S)-sec-butyl)-3-oxo-N-(2-oxaspiro[3.3]heptan-6-yl)-3,4-dihydroquinoxaline-1(2H)-carboxamide |
| 129 | 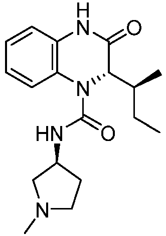 | (S)-2-((S)-sec-butyl)-N-((S)-1-methylpyrrolidin-3-yl)-3-oxo-3,4-dihydroquinoxaline-1(2H)-carboxamide |
| 130 | 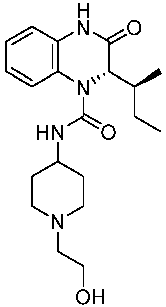 | (S)-2-((S)-sec-butyl)-N-(1-(2-hydroxyethyl)piperidin-4-yl)-3-oxo-3,4-dihydroquinoxaline- 1(2H)-carboxamide |
| 131 | 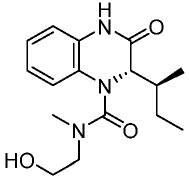 | (S)-2-((S)-sec-butyl)-N-(2-hydroxyethyl)-N-methyl-3-oxo-3,4-dihydroquinoxaline-1(2H)-carboxamide |
| 132 | 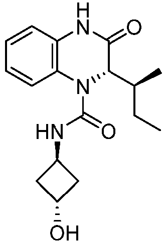 | (S)-2-((S)-sec-butyl)-N-((1r,3S)-3-hydroxycyclobutyl)-3-oxo-3,4-dihydroquinoxaline-1(2H)-carboxamide |
| 133 | 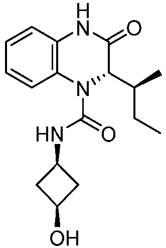 | (S)-2-((S)-sec-butyl)-N-((1s,3R)-3-hydroxycyclobutyl)-3-oxo-3,4-dihydroquinoxaline-1(2H)-carboxamide |

In some variations, any of the compounds described herein, such as a compound of formula (I), (I-a), (I-b), (I-c), (I-d), (I-e), (II), (II-a), (II-b), (II-c), (II-d), or (II-e) or any variation thereof, or a compound of Table 2, may be deuterated (e.g., a hydrogen atom is replaced by a deuterium atom). In some of these variations, the compound is deuterated at a single site. In other variations, the compound is deuterated at multiple sites. Deuterated compounds can be prepared from deuterated starting materials in a manner similar to the preparation of the corresponding non-deuterated compounds. Hydrogen atoms may also be replaced with deuterium atoms using other method known in the art.

In one aspect, provided herein is a compound of formula (I), or a pharmaceutically acceptable salt thereof, wherein the compound of formula (I), or a pharmaceutically acceptable salt thereof, is a compound selected from the compounds of Table 2, or a pharmaceutically acceptable salt thereof.

In one aspect, provided herein is a compound selected from the compounds of Table 2, or a pharmaceutically acceptable salt thereof.

In one aspect, provided herein is a compound of formula (I), or a pharmaceutically acceptable salt thereof, wherein the compound of formula (I), or a pharmaceutically acceptable salt thereof, is selected from the group consisting of compounds 1-133, or a pharmaceutically acceptable salt thereof.

In one aspect, provided herein is a compound selected from the group consisting of compounds 1-133, or a pharmaceutically acceptable salt thereof.

In one aspect, provided herein is a compound of formula (I), or a pharmaceutically acceptable salt thereof, wherein the compound of formula (I) is selected from the group consisting of:
4-(6-chloronicotinoyl)-3-propyl-3,4-dihydroquinoxalin-2(1H)-one;
6-chloro-4-(6-chloronicotinoyl)-3-propyl-3,4-dihydroquinoxalin-2(1H)-one;
4-(6-methylnicotinoyl)-3-propyl-3,4-dihydroquinoxalin-2(1H)-one;
5-bromo-4-(6-chloronicotinoyl)-3-propyl-3,4-dihydroquinoxalin-2(1H)-one;
4-(6-chloronicotinoyl)-3-propyl-3,4-dihydroquinoxalin-2(1H)-one;
4-(6-fluoronicotinoyl)-3-propyl-3,4-dihydroquinoxalin-2(1H)-one;
5-cyclopropyl-4-(6-methylnicotinoyl)-3-propyl-3,4-dihydroquinoxalin-2(1H)-one;
N-(6-methylpyridin-3-yl)-3-oxo-2-propyl-3,4-dihydroquinoxaline-1(2H)-carboxamide;
3-propyl-4-(1H-pyrrole-2-carbonyl)-3,4-dihydroquinoxalin-2(1H)-one;
4-benzoyl-3-propyl-3,4-dihydroquinoxalin-2(1H)-one;
4-isonicotinoyl-3-propyl-3,4-dihydroquinoxalin-2(1H)-one;
4-nicotinoyl-3-propyl-3,4-dihydroquinoxalin-2(1H)-one;
4-(1-methyl-1H-pyrazole-4-carbonyl)-3-propyl-3,4-dihydroquinoxalin-2(1H)-one;
4-(3-methylisoxazole-4-carbonyl)-3-propyl-3,4-dihydroquinoxalin-2(1H)-one;
3-propyl-4-(thiophene-2-carbonyl)-3,4-dihydroquinoxalin-2(1H)-one;
4-(5-methylnicotinoyl)-3-propyl-3,4-dihydroquinoxalin-2(1H)-one;
4-(3-fluorobenzoyl)-3-propyl-3,4-dihydroquinoxalin-2(1H)-one;
4-(5-fluoronicotinoyl)-3-propyl-3,4-dihydroquinoxalin-2(1H)-one;
4-(3-phenylpropanoyl)-3-propyl-3,4-dihydroquinoxalin-2(1H)-one;
4-(5-chloronicotinoyl)-3-propyl-3,4-dihydroquinoxalin-2(1H)-one;
4-(3,4-difluorobenzoyl)-3-propyl-3,4-dihydroquinoxalin-2(1H)-one;
N-(5-(3-oxo-2-propyl-1,2,3,4-tetrahydroquinoxaline-1-carbonyl)pyridin-2-yl)acetamide;
4-(6-(1H-pyrrol-1-yl)nicotinoyl)-3-propyl-3,4-dihydroquinoxalin-2(1H)-one;
4-(6-(difluoromethoxy)nicotinoyl)-3-propyl-3,4-dihydroquinoxalin-2(1H)-one;
3-propyl-4-(6-(trifluoromethoxy)nicotinoyl)-3,4-dihydroquinoxalin-2(1H)-one;
3-propyl-4-(1H-pyrrolo[2,3-b]pyridine-5-carbonyl)-3,4-dihydroquinoxalin-2(1H)-one;
4-(6-aminonicotinoyl)-3-propyl-3,4-dihydroquinoxalin-2(1H)-one;
4-(6-(dimethylamino)nicotinoyl)-3-propyl-3,4-dihydroquinoxalin-2(1H)-one;
4-(6-(piperidin-1-yl)nicotinoyl)-3-propyl-3,4-dihydroquinoxalin-2(1H)-one;
4-(1-ethyl-1H-pyrazole-4-carbonyl)-3-propyl-3,4-dihydroquinoxalin-2(1H)-one;
4-(1-(cyclopropylmethyl)-1H-pyrazole-4-carbonyl)-3-propyl-3,4-dihydroquinoxalin-2(1H)-one;
4-(1-cyclobutyl-1H-pyrazole-4-carbonyl)-3-propyl-3,4-dihydroquinoxalin-2(1H)-one;
4-(6-(ethyl(methyl)amino)nicotinoyl)-3-propyl-3,4-dihydroquinoxalin-2(1H)-one;
3-propyl-4-(6-((2,2,2-trifluoroethyl)amino)nicotinoyl)-3,4-dihydroquinoxalin-2(1H)-one;
4-(6-(azepan-1-yl)nicotinoyl)-3-propyl-3,4-dihydroquinoxalin-2(1H)-one;
4-(6-(benzylamino)nicotinoyl)-3-propyl-3,4-dihydroquinoxalin-2(1H)-one;
4-(6-(cyclopropyl(methyl)amino)nicotinoyl)-3-propyl-3,4-dihydroquinoxalin-2(1H)-one;
4-(6-(3-fluoroazetidin-1-yl)nicotinoyl)-3-propyl-3,4-dihydroquinoxalin-2(1H)-one;
4-(6-(benzyl(methyl)amino)nicotinoyl)-3-propyl-3,4-dihydroquinoxalin-2(1H)-one;
4-(6-(3-methoxyazetidin-1-yl)nicotinoyl)-3-propyl-3,4-dihydroquinoxalin-2(1H)-one;
4-(6-(3-fluoropyrrolidin-1-yl)nicotinoyl)-3-propyl-3,4-dihydroquinoxalin-2(1H)-one;
4-(6-(3,3-difluoroazetidin-1-yl)nicotinoyl)-3-propyl-3,4-dihydroquinoxalin-2(1H)-one;
4-(6-(3,3-difluoropyrrolidin-1-yl)nicotinoyl)-3-propyl-3,4-dihydroquinoxalin-2(1H)-one;
4-(6-(methyl(3,3,3-trifluoropropyl)amino)nicotinoyl)-3-propyl-3,4-dihydroquinoxalin-2(1H)-one;
4-(1,3-dimethyl-1H-pyrazole-4-carbonyl)-3-propyl-3,4-dihydroquinoxalin-2(1H)-one;
N-cyclohexyl-3-oxo-2-propyl-3,4-dihydroquinoxaline-1(2H)-carboxamide;
3-oxo-2-propyl-N-(pyridin-3-yl)-3,4-dihydroquinoxaline-1(2H)-carboxamide;
3-oxo-2-propyl-N-(pyridin-4-yl)-3,4-dihydroquinoxaline-1(2H)-carboxamide;
N-benzyl-3-oxo-2-propy1-3,4-dihydroquinoxaline-1(2H)-carboxamide;
3-oxo-N-(1-phenylethyl)-2-propyl-3,4-dihydroquinoxaline-1(2H)-carboxamide;
N-(cyclohexylmethyl)-3-oxo-2-propyl-3,4-dihydroquinoxaline-1(2H)-carboxamide;
4-(indoline-1-carbonyl)-3-propyl-3,4-dihydroquinoxalin-2(1H)-one;
N-(2-methoxyphenyl)-3-oxo-2-propyl-3,4-dihydroquinoxaline-1(2H)-carboxamide;
N-(3-methoxyphenyl)-3-oxo-2-propyl-3,4-dihydroquinoxaline-1(2H)-carboxamide;
N-(4-methoxyphenyl)-3-oxo-2-propyl-3,4-dihydroquinoxaline-1(2H)-carboxamide;
N-(2-fluorophenyl)-3-oxo-2-propyl-3,4-dihydroquinoxaline-1(2H)-carboxamide;
N-(3-fluorophenyl)-3-oxo-2-propyl-3,4-dihydroquinoxaline-1(2H)-carboxamide;
N-(4-fluorophenyl)-3-oxo-2-propyl-3,4-dihydroquinoxaline-1(2H)-carboxamide;
N-(1-methyl-1H-pyrazol-4-yl)-3-oxo-2-propyl-3,4-dihydroquinoxaline-1(2H)-carboxamide;
3-oxo-2-propyl-N-(o-tolyl)-3,4-dihydroquinoxaline-1(2H)-carboxamide;
3-oxo-2-propyl-N-(m-tolyl)-3,4-dihydroquinoxaline-1(2H)-carboxamide;
3-oxo-2-propyl-N-(p-tolyl)-3,4-dihydroquinoxaline-1(2H)-carboxamide;
3-(sec-butyl)-4-(1-methyl-1H-pyrazole-4-carbonyl)-3,4-dihydroquinoxalin-2(1H)-one;
6-chloro-4-(1-methyl-1H-pyrazole-4-carbonyl)-3-propyl-3,4-dihydroquinoxalin-2(1H)-one;
4-(imidazo[1,5-a]pyridine-6-carbonyl)-3-propyl-3,4-dihydroquinoxalin-2(1H)-one;
4-(imidazo[1,5-a]pyridine-7-carbonyl)-3-propyl-3,4-dihydroquinoxalin-2(1H)-one;
4-(1-(2-hydroxyethyl)-1H-pyrazole-4-carbonyl)-3-propyl-3,4-dihydroquinoxalin-2(1H)-one;
3-isobutyl-4-(1-methyl-1H-pyrazole-4-carbonyl)-3,4-dihydroquinoxalin-2(1H)-one;
5-bromo-4-(1-methyl-1H-pyrazole-4-carbonyl)-3-propyl-3,4-dihydroquinoxalin-2(1H)-one;
3-propyl-4-(pyrrolo[1,2-c]pyrimidine-3-carbonyl)-3,4-dihydroquinoxalin-2(1H)-one;
3-propyl-4-(1H-pyrazole-4-carbonyl)-3,4-dihydroquinoxalin-2(1H)-one;
3-(sec-butyl)-4-(1H-pyrazole-4-carbonyl)-3,4-dihydroquinoxalin-2(1H)-one;
3-propyl-4-(1H-pyrazolo[3,4-b]pyridine-5-carbonyl)-3,4-dihydroquinoxalin-2(1H)-one;
4-(6-aminonicotinoyl)-3-(sec-butyl)-3,4-dihydroquinoxalin-2(1H)-one;
3-(sec-butyl)-4-(6-oxo-1,6-dihydropyridine-3-carbonyl)-3,4-dihydroquinoxalin-2(1H)-one;
4-(1-cyclopropyl-1H-pyrazole-4-carbonyl)-3-propyl-3,4-dihydroquinoxalin-2(1H)-one;
3-isopropyl-4-(1-methyl-1H-pyrazole-4-carbonyl)-3,4-dihydroquinoxalin-2(1H)-one;
3-(sec-butyl)-4-(1-(2-hydroxyethyl)-1H-pyrazole-4-carbonyl)-3,4-dihydroquinoxalin-2(1H)-one;
3-(sec-butyl)-4-(1H-pyrazole-3-carbonyl)-3,4-dihydroquinoxalin-2(1H)-one;
2-(4-(2-(sec-butyl)-3-oxo-1,2,3,4-tetrahydroquinoxaline-1-carbonyl)-1H-pyrazol-1-yl)acetamide;
3-isopropyl-4-(1-methyl-6-oxo-1,6-dihydropyridine-3-carbonyl)-3,4-dihydroquinoxalin-2(1H)-one;
4-(6-aminonicotinoyl)-3-isopropyl-3,4-dihydroquinoxalin-2(1H)-one;
3-isopropyl-4-(1H-pyrazolo[3,4-b]pyridine-5-carbonyl)-3,4-dihydroquinoxalin-2(1H)-one;
4-(1-cyclopropyl-1H-pyrazole-4-carbonyl)-3-isopropyl-3,4-dihydroquinoxalin-2(1H)-one;
3-isopropyl-4-(6-oxo-1,6-dihydropyridine-3-carbonyl)-3,4-dihydroquinoxalin-2(1H)-one;
3-isopropyl-4-(1H-pyrazole-4-carbonyl)-3,4-dihydroquinoxalin-2(1H)-one;
3-isopropyl-4-(1-methyl-6-oxo-1,6-dihydropyridine-3-carbonyl)-3,4-dihydropyrido[2,3-b]pyrazin-2(1H)-one;
2-isopropyl-1-(1-methyl-1H-pyrazole-4-carbonyl)-1,4-dihydropyrido[3,4-b]pyrazin-3(2H)-one;
2-(sec-butyl)-1-(1-methyl-1H-pyrazole-4-carbonyl)-1,4-dihydropyrido[3,4-b]pyrazin-3(2H)-one;
2-(sec-butyl)-1-(6-oxo-1,6-dihydropyridine-3-carbonyl)-1,4-dihydropyrido[3,4-b]pyrazin-3(2H)-one;
2-(sec-butyl)-1-(1-methyl-6-oxo-1,6-dihydropyridine-3-carbonyl)-1,4-dihydropyrido[3,4-b]pyrazin-3(2H)-one;
3-(sec-butyl)-4-(1-methyl-6-oxo-1,6-dihydropyridine-3-carbonyl)-3,4-dihydropyrido[2,3-b]pyrazin-2(1H)-one;
3-(sec-butyl)-4-(1-methyl-1H-pyrazole-4-carbonyl)-3,4-dihydropyrido[2,3-b]pyrazin-2(1H)-one;
7-(sec-butyl)-8-(1-methyl-1H-pyrazole-4-carbonyl)-7,8-dihydropteridin-6(5H)-one;
2-(sec-butyl)-1-(1-methyl-1H-pyrazole-4-carbonyl)-1,4-dihydropyrido[2,3-b]pyrazin-3(2H)-one;
2-isobutyl-1-(1-methyl-1H-pyrazole-4-carbonyl)-1,4-dihydropyrido[3,4-b]pyrazin-3(2H)-one;
1-benzoyl-2-(sec-butyl)-1,4-dihydropyrido[3,4-b]pyrazin-3(2H)-one;
2-(sec-butyl)-1-(3-methylbenzoyl)-1,4-dihydropyrido[3,4-b]pyrazin-3(2H)-one;
2-(sec-butyl)-3-oxo-3,4-dihydroquinoxaline-1(2H)-carboxamide;
2-(sec-butyl)-5-fluoro-3-oxo-3,4-dihydroquinoxaline-1(2H)-carboxamide;
3-(sec-butyl)-4-(3-hydroxyazetidine-1-carbonyl)-3,4-dihydroquinoxalin-2(1H)-one;
2-(sec-butyl)-N-methyl-3-oxo-3,4-dihydroquinoxaline-1(2H)-carboxamide;
2-(sec-butyl)-N-ethyl-3-oxo-3,4-dihydroquinoxaline-1(2H)-carboxamide;
3-(sec-butyl)-4-(pyrrolidine-1-carbonyl)-3,4-dihydroquinoxalin-2(1H)-one;
2-(sec-butyl)-N-(2-hydroxyethyl)-3-oxo-3,4-dihydroquinoxaline-1(2H)-carboxamide;
3-(sec-butyl)-4-(3-hydroxypyrrolidine-1-carbonyl)-3,4-dihydroquinoxalin-2(1H)-one;
3-(sec-butyl)-4-(3-(hydroxymethyl)pyrrolidine-1-carbonyl)-3,4-dihydroquinoxalin-2(1H)-one;
3-(sec-butyl)-4-(3-(hydroxymethyl)pyrrolidine-1-carbonyl)-3,4-dihydroquinoxalin-2(1H)-one;
3-(sec-butyl)-4-(2-hydroxyacetyl)-3,4-dihydroquinoxalin-2(1H)-one;
3-(sec-butyl)-4-(3-hydroxypropanoyl)-3,4-dihydroquinoxalin-2(1H)-one;
3-(sec-butyl)-4-(3-(hydroxymethyl)azetidine-1-carbonyl)-3,4-dihydroquinoxalin-2(1H)-one;
3-(sec-butyl)-4-(3-fluoroazetidine-1-carbonyl)-3,4-dihydroquinoxalin-2(1H)-one;
2-(sec-butyl)-7-fluoro-3-oxo-3,4-dihydroquinoxaline-1(2H)-carboxamide;
3-(sec-butyl)-4-(3-hydroxypiperidine-1-carbonyl)-3,4-dihydroquinoxalin-2(1H)-one;
2-(sec-butyl)-N-(3-hydroxypropyl)-N-methyl-3-oxo-3,4-dihydroquinoxaline-1(2H)-carboxamide;
3-(sec-butyl)-4-(3-(hydroxymethyl)piperidine-1-carbonyl)-3,4-dihydroquinoxalin-2(1H)-one;
3-(sec-butyl)-4-(3,3-difluoroazetidine-1-carbonyl)-3,4-dihydroquinoxalin-2(1H)-one;
3-(sec-butyl)-4-(4-hydroxypiperidine-1-carbonyl)-3,4-dihydroquinoxalin-2(1H) -one;
N-(1-(2-(sec-butyl)-3-oxo-1,2,3,4-tetrahydroquinoxaline-1-carbonyl)azetidin-3-yl)acetamide;
3-(sec-butyl)-4-(3-fluoropyrrolidine-1-carbonyl)-3,4-dihydroquinoxalin-2(1H)-one;
3-(sec-butyl)-4-(3-hydroxy-3-methylazetidine-1-carbonyl)-3,4-dihydroquinoxalin-2(1H)-one;
2-(sec-butyl)-N-(oxetan-3-yl)-3-oxo-3,4-dihydroquinoxaline-1(2H)-carboxamide;
2-(sec-butyl)-N-(2,2-difluoroethyl)-3-oxo-3,4-dihydroquinoxaline-1(2H)-carboxamide;
2-(sec-butyl)-N-(2,2-difluoroethyl)-N-methyl-3-oxo-3,4-dihydroquinoxaline-1(2H)-carboxamide;
methyl 2-(sec-butyl)-3-oxo-3,4-dihydroquinoxaline-1(2H)-carboxylate;
2-(sec-butyl)-3-oxo-N-(pyrrolidin-3-yl)-3,4-dihydroquinoxaline-1(2H)-carboxamide;
2-(sec-butyl)-N-(1-(2-hydroxyethyl)pyrrolidin-3-yl)-3-oxo-3,4-dihydroquinoxaline-1(2H)-carboxamide;
2-(sec-butyl)-3-oxo-N-(2-oxaspiro[3.3]heptan-6-yl)-3,4-dihydroquinoxaline-1(2H)-carboxamide;
2-(sec-butyl)-N-(1-methylpyrrolidin-3-yl)-3-oxo-3,4-dihydroquinoxaline-1(2H)-carboxamide;
2-(sec-butyl)-N-(1-(2-hydroxyethyl)piperidin-4-yl)-3-oxo-3,4-dihydroquinoxaline-1(2H)-carboxamide;
2-(sec-butyl)-N-(2-hydroxyethyl)-N-methyl-3-oxo-3,4-dihydroquinoxaline-1(2H)-carboxamide;
2-(sec-butyl)-N-(3-hydroxycyclobutyl)-3-oxo-3,4-dihydroquinoxaline-1(2H)-carboxamide; and
2-(sec-butyl)-N-(3-hydroxycyclobutyl)-3-oxo-3,4-dihydroquinoxaline-1(2H)-carboxamide;
or a pharmaceutically acceptable salt thereof. Also provided herein are, where applicable, any and all stereoisomers of the compounds depicted herein, including geometric isomers (e.g., cis/trans isomers or E/Z isomers), enantiomers, diastereomers, or mixtures thereof in any ratio, including racemic mixtures.

Any formula given herein, such as formula (I), (I-a), (I-b), (I-c), (I-d), (I-e), (II), (II-a), (II-b), (II-c), (II-d), or (II-e), is intended to represent compounds having structures depicted by the structural formula as well as certain variations or forms. In particular, compounds of any formula given herein may have asymmetric centers and therefore exist in different enantiomeric or diastereomeric forms. All optical isomers and stereoisomers of the compounds of the general formula, and mixtures thereof in any ratio, are considered within the scope of the formula. Thus, any formula given herein is intended to represent a racemate, one or more enantiomeric forms, one or more diastereomeric forms, one or more atropisomeric forms, and mixtures thereof in any ratio. Where a compound of Table 2 is depicted with a particular stereochemical configuration, also provided herein is any alternative stereochemical configuration of the compound, as well as a mixture of stereoisomers of the compound in any ratio. For example, where a compound of Table 2 has a stereocenter that is in an "S" stereochemical configuration, also provided herein is enantiomer of the compound wherein that stereocenter is in an "R" stereochemical configuration. Likewise, when a compound of Table 2 has a stereocenter that is in an "R" configuration, also provided herein is enantiomer of the compound in an "S" stereochemical configuration. Also provided are mixtures of the compound with both the "S" and the "R" stereochemical configuration. Additionally, if a compound of Table 2 has two or more stereocenters, also provided are any enantiomer or diastereomer of the compound. For example, if a compound of Table 2 contains a first stereocenter and a second stereocenter with "R" and "R" stereochemical configurations, respectively, also provided are stereoisomers of the compound having first and second stereocenters with "S" and "S" stereochemical configurations, respectively, "S" and "R" stereochemical configurations, respectively, and "R" and "S" stereochemical configurations, respectively. If a compound of Table 2 contains a first stereocenter and a second stereocenter with "S" and "S" stereochemical configurations, respectively, also provided are stereoisomers of the compound having first and second stereocenters with "R" and "R" stereochemical configurations, respectively, "S" and "R" stereochemical configurations, respectively, and "R" and "S" stereochemical configurations, respectively. If a compound of Table 2 contains a first stereocenter and a second stereocenter with "S" and "R" stereochemical configurations, respectively, also provided are stereoisomers of the compound having first and second stereocenters with "R" and "S" stereochemical configurations, respectively, "R" and "R" stereochemical configurations, respectively, and "S" and "S" stereochemical configurations, respectively. Similarly, if a compound of Table 2 contains a first stereocenter and a second stereocenter with "R" and "S" stereochemical configurations, respectively, also provided are stereoisomers of the compound having first and second stereocenters with "S" and "R" stereochemical configurations, respectively, "R" and "R" stereochemical configurations, respectively, and "S" and "S" stereochemical configurations, respectively. Furthermore, certain structures may exist as geometric isomers (i.e., *cis* and *trans* isomers), as tautomers, or as atropisomers. Additionally, any formula given herein is intended to refer also to any one of hydrates, solvates, and amorphous and crystalline forms of such compounds, and mixtures thereof, even if such forms are not listed explicitly. In some embodiments, the solvent is water and the solvates are hydrates.

Representative examples of compounds detailed herein, including intermediates and final compounds, are depicted in the tables and elsewhere herein. It is understood that in one aspect, any of the compounds may be used in the methods detailed herein, including, where applicable, intermediate compounds that may be isolated and administered to an individual or subject.

The compounds depicted herein may be present as salts even if salts are not depicted, and it is understood that the compositions and methods provided herein embrace all salts and solvates of the compounds depicted here, as well as the non-salt and non-solvate form of the compound, as is well understood by the skilled artisan. In some embodiments, the salts of the compounds provided herein are pharmaceutically acceptable salts.

In one variation, the compounds herein are synthetic compounds prepared for administration to an individual or subject. In another variation, compositions are provided containing a compound in substantially pure form. In another variation, provided are pharmaceutical compositions comprising a compound detailed herein and a pharmaceutically acceptable carrier. In another variation, methods of administering a compound are provided. The purified forms, pharmaceutical compositions and methods of administering the compounds are suitable for any compound or form thereof detailed herein.

Any variation or embodiment of Z¹, Z², Z³, R¹, R², R³, R⁴, R⁵, R⁶, R⁷, R⁸, R^{4A}, R^{4B}, R^{4C}, R^{4D}, R^{4E}, R^{4B1}, n and p provided herein can be combined with every other variation or embodiment of Z¹, Z², Z³, R¹, R², R³, R⁴, R⁵, R⁶, R⁷, R⁸, R^{4A}, R^{4B}, R^{4c}, R^{4D}, R^{4E}, R^{4B1}, n and p, the same as if each and every combination had been individually and specifically described.

Other embodiments will be apparent to those skilled in the art from the following detailed description.

As used herein, when any variable occurs more than one time in a chemical formula, its definition on each occurrence is independent of its definition at every other occurrence.

Formula (I) includes all subformulas thereof. For example, formula (I) includes compounds of formula (I-a), (I-b), (I-c), (I-d), (I-e), (II), (II-a), (II-b), (II-c), (II-d), or (II-e), or a pharmaceutically acceptable salt thereof.

The names for compounds 1-133 provided herein, as shown in Table 2 and Examples 1-12, are provided by ChemDraw Professional 18.2.0.48.. One of skilled in the art would understand that the compounds may be named or identified using various commonly recognized nomenclature systems and symbols. By way of example, the compounds may be named or identified with common names, systematic or non-systematic names. The nomenclature systems and symbols that are commonly recognized in the art of chemistry include, for example, Chemical Abstract Service (CAS), ChemBioDraw Ultra, and International Union of Pure and Applied Chemistry (IUPAC).

### Compositions

Also provided are compositions, such as pharmaceutical compositions, that include a compound disclosed and/or described herein and one or more additional medicinal agents, pharmaceutical agents, adjuvants, carriers, excipients, and the like. Suitable medicinal and pharmaceutical agents include those described herein. In some embodiments, the pharmaceutical composition includes a pharmaceutically acceptable excipient or adjuvant and at least one chemical entity as described herein. Examples of pharmaceutically acceptable excipients include, but are not limited to, mannitol, lactose, starch, magnesium stearate, sodium saccharine, talcum, cellulose, sodium crosscarmellose, glucose, gelatin, sucrose, and magnesium carbonate. In some embodiments, provided are compositions, such as pharmaceutical compositions that contain one or more compounds described herein, or a pharmaceutically acceptable salt thereof.

In some embodiments, provided is a pharmaceutically acceptable composition comprising a compound of formula (I), (I-a), (I-b), (I-c), (I-d), (I-e), (II), (II-a), (II-b), (II-c), (II-d), or (II-e), or a compound of Table 2, or a pharmaceutically acceptable salt thereof. In some aspects, a composition may contain a synthetic intermediate that may be used in the preparation of a compound described herein. The compositions described herein may contain any other suitable active or inactive agents.

Any of the compositions described herein may be sterile or contain components that are sterile. Sterilization can be achieved by methods known in the art. Any of the compositions described herein may contain one or more compounds or conjugates that are substantially pure.

Also provided are packaged pharmaceutical compositions, comprising a pharmaceutical composition as described herein and instructions for using the composition to treat a patient suffering from a disease or condition described herein.

### Methods of Use

The compounds and compositions detailed herein, such as a pharmaceutical composition comprising a compound of any formula provided herein, or a pharmaceutically acceptable salt thereof, and a pharmaceutically acceptable carrier or excipient, may be used in methods of administration and treatment as provided herein.

Further, the invention relates to a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, and a pharmaceutically acceptable excipient. Furthermore, the invention relates to a pharmaceutical composition for preventing or treating a disease or condition responsive to modulation of the contractility of the skeletal sarcomere, for example, modulation of the troponin complex of the fast skeletal muscle sarcomere through one or more of fast skeletal myosin, actin, tropomyosin, troponin C, troponin I, and troponin T, and fragments and isoforms thereof, in a subject, comprising a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof. Furthermore, the invention relates to an agent for preventing or treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof, comprising a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

Furthermore, the invention relates to a pharmaceutical composition for treating a disease or condition responsive to modulation of the contractility of the skeletal sarcomere, for example, modulation of the troponin complex of the fast skeletal muscle sarcomere through one or more of fast skeletal myosin, actin, tropomyosin, troponin C, troponin I, and troponin T, and fragments and isoforms thereof, in a subject, comprising a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof. Furthermore, the invention relates to an agent for treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof, comprising a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

Moreover, the invention relates to use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for preventing or treating a disease or condition responsive to modulation of the contractility of the skeletal sarcomere, for example, modulation of the troponin complex of the fast skeletal muscle sarcomere through one or more of fast skeletal myosin, actin, tropomyosin, troponin C, troponin I, and troponin T, and fragments and isoforms thereof in a subject.

Moreover, the invention relates to use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof.

In one aspect, provided herein is the use of the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for preventing or treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof.

In one aspect, provided herein is the use of the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof.

In one aspect, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in preventing or treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof.

In one aspect, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof.

In one aspect, provided herein is a method for preventing or treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof, comprising administering to the subject an effective amount of the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof. Further, the "subject" is a human or a non-human animal in need of the prevention or treatment, and in one embodiment, a human in need of the prevention or treatment.

In one aspect, provided herein is a method for treating a disease or condition responsive to modulation of the contractility of the skeletal sarcomere in a subject, for example, modulation of the troponin complex of the fast skeletal muscle sarcomere through one or more of fast skeletal myosin, actin, tropomyosin, troponin C, troponin I, and troponin T, and fragments and isoforms thereof, comprising administering to the subject an effective amount of the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof. Further, the "subject" is a human or a non-human animal in need of the prevention or treatment, and in one embodiment, a human in need of the prevention or treatment.

In one aspect, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in medical therapy. Such medical therapy may be relating to a disease or condition responsive to modulation of the contractility of the skeletal sarcomere, for example, modulation of the troponin complex of the fast skeletal muscle sarcomere through one or more of fast skeletal myosin, actin, tropomyosin, troponin C, troponin I, and troponin T, and fragments and isoforms thereof.

In one aspect, a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, modulates the contractility of the skeletal sarcomere. Specifically, the compounds modulate the troponin complex of the fast skeletal muscle sarcomere through one or more of fast skeletal myosin, actin, tropomyosin, troponin C, troponin I, and troponin T, and fragments and isoforms thereof. As used in this context, "modulate" means either increasing or decreasing activity. In some instances, a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, potentiates (i.e., increases activity) of one or more of fast skeletal myosin, actin, tropomyosin, troponin C, troponin I, and troponin T, and fragments and isoforms thereof.

In another aspect, provided herein is a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, inhibits (i.e., decreases activity) of one or more of fast skeletal myosin, actin, tropomyosin, troponin C, troponin I, and troponin T, and fragments and isoforms thereof. As used in this context, "activation of the fast skeletal muscle fiber such as myofibril" means to amplify the response of fast skeletal muscle fiber (such as myofibril) to stimulation/Ca²⁺.

In some aspects, provided herein is a method of preventing or treating: frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or an effective amount of a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method of treating: frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or an effective amount of a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method for preventing or treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method for treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method for preventing or treating a disease or condition selected from the group consisting of amyotrophic lateral sclerosis (ALS), spinal muscular atrophy (SMA), myasthenia gravis, and muscular myopathies, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method for treating a disease or condition selected from the group consisting of amyotrophic lateral sclerosis (ALS), spinal muscular atrophy (SMA), myasthenia gravis, and muscular myopathies, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method for preventing or treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is a method for treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction, in a subject, comprising administering to the subject an effective amount of a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition comprising a compound of formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for preventing or treating: frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for treating: frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for preventing or treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for preventing or treating a disease or condition selected from the group consisting of amyotrophic lateral sclerosis (ALS), spinal muscular atrophy (SMA), myasthenia gravis, and muscular myopathies in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for treating a disease or condition selected from the group consisting of amyotrophic lateral sclerosis (ALS), spinal muscular atrophy (SMA), myasthenia gravis, and muscular myopathies in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for preventing or treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

In some aspects, provided herein is the use of a compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, for the manufacture of a pharmaceutical composition for treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or pharmaceutically acceptable salt thereof, or a pharmaceutical composition thereof, for use in preventing or treating: frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in treating: frailty associated with old age (termed sarcopenia); cachexia syndromes associated with diseases such as cancer, heart failure, chronic obstructive pulmonary disease (COPD), renal disease, and chronic kidney disease/dialysis; diseases and disorders of the central nervous system (CNS); neuromuscular diseases, such as amyotrophic lateral sclerosis (ALS), spinal muscular atrophy (SMA), and myasthenia gravis, peripheral neuropathies, Charcot-Marie-Tooth disease, Parkinson's disease, stroke, spinal cord injury, and motor units disorders; muscular myopathies, including body myositis myopathy, muscular dystrophies (limb girdle, facioscapulohumeral, oculopharyngeal), steroid myopathy, and mitochondrial myopathies; rehabilitation-related deficits: recovery from surgery (e.g., post-surgical muscle weakness), prolonged bed rest, immobilization/disuse atrophy, post-hip fracture recovery, ICU neuromyopathy, post trauma, stroke rehabilitation; Peripheral Vascular Disease (PVD) or Peripheral Arterial Disease (PAD) (e.g., claudication), metabolic syndrome, chronic fatigue syndrome, obesity, and frailty due to aging; post-anesthesia recovery or reversal of neuromuscular blockade; obstructive sleep apnea; chronic fatigue syndrome; metabolic syndrome, metabolic/ischemic disorders, or claudication; obesity; dysfunctions of pelvic floor and urethral/anal sphincter muscles (e.g., urinary incontinence such as stress urinary incontinence (SUI) and mixed urinary incontinence (MUI), and fecal incontinence); post-spinal cord injury (SCI) muscle dysfunction; ventilator-induced muscle weakness; or spinocerebral ataxias or demyelinating diseases, including multiple sclerosis, post-polio syndrome, or any combination of the foregoing, in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in preventing or treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in treating a disease or condition selected from the group consisting of peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in preventing or treating a disease or condition selected from the group consisting of amyotrophic lateral sclerosis (ALS), spinal muscular atrophy (SMA), myasthenia gravis, and muscular myopathies in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in treating a disease or condition selected from the group consisting of amyotrophic lateral sclerosis (ALS), spinal muscular atrophy (SMA), myasthenia gravis, and muscular myopathies in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in preventing or treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

In some aspects, provided herein is the compound of the formula (I), such as a compound of formula (I-a), (I-b), (I-c), (I-d), (I-e), (II), (II-a), (II-b), (II-c), (II-d), or (II-e), or a compound of Table 2, or a pharmaceutically acceptable salt thereof, or a pharmaceutical composition thereof, for use in treating a disease or condition selected from the group consisting of stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

### Dosages

The compounds and compositions disclosed and/or described herein are administered at a therapeutically effective dosage, e.g., a dosage sufficient to provide treatment for the disease state. While human dosage levels have yet to be optimized for the chemical entities described herein, generally, a daily dose ranges from about 0.01 to 100 mg/kg of body weight; in some embodiments, from about 0.05 to 10.0 mg/kg of body weight, and in some embodiments, from about 0.10 to 1.4 mg/kg of body weight. Thus, for administration to a 70 kg person, in some embodiments, the dosage range would be about from 0.7 to 7000 mg per day; in some embodiments, about from 3.5 to 700.0 mg per day, and in some embodiments, about from 7 to 100.0 mg per day. The amount of the chemical entity administered will be dependent, for example, on the subject and disease state being treated, the severity of the affliction, the manner and schedule of administration and the judgment of the prescribing physician. For example, an exemplary dosage range for oral administration is from about 5 mg to about 500 mg per day, and an exemplary intravenous administration dosage is from about 5 mg to about 500 mg per day, each depending upon the compound pharmacokinetics.

A daily dose is the total amount administered in a day. A daily dose may be, but is not limited to be, administered each day, every other day, each week, every 2 weeks, every month, or at a varied interval. In some embodiments, the daily dose is administered for a period ranging from a single day to the life of the subject. In some embodiments, the daily dose is administered once a day. In some embodiments, the daily dose is administered in multiple divided doses, such as in 2, 3, or 4 divided doses. In some embodiments, the daily dose is administered in 2 divided doses.

Administration of the compounds and compositions disclosed and/or described herein can be via any accepted mode of administration for therapeutic agents including, but not limited to, oral, sublingual, subcutaneous, parenteral, intravenous, intranasal, topical, transdermal, intraperitoneal, intramuscular, intrapulmonary, vaginal, rectal, or intraocular administration. In some embodiments, the compound or composition is administered orally or intravenously. In some embodiments, the compound or composition disclosed and/or described herein is administered orally.

Pharmaceutically acceptable compositions include solid, semi-solid, liquid and aerosol dosage forms, such as tablet, capsule, powder, liquid, suspension, suppository, and aerosol forms. The compounds disclosed and/or described herein can also be administered in sustained or controlled release dosage forms (e.g., controlled/sustained release pill, depot injection, osmotic pump, or transdermal (including electrotransport) patch forms) for prolonged timed, and/or pulsed administration at a predetermined rate. In some embodiments, the compositions are provided in unit dosage forms suitable for single administration of a precise dose.

The compounds disclosed and/or described herein can be administered either alone or in combination with one or more conventional pharmaceutical carriers or excipients (e.g., mannitol, lactose, starch, magnesium stearate, sodium saccharine, talcum, cellulose, sodium crosscarmellose, glucose, gelatin, sucrose, magnesium carbonate). If desired, the pharmaceutical composition can also contain minor amounts of nontoxic auxiliary substances such as wetting agents, emulsifying agents, solubilizing agents, pH buffering agents and the like (e.g., sodium acetate, sodium citrate, cyclodextrine derivatives, sorbitan monolaurate, triethanolamine acetate, triethanolamine oleate). Generally, depending on the intended mode of administration, the pharmaceutical composition will contain about 0.005% to 95%, or about 0.5% to 50%, by weight of a compound disclosed and/or described herein. Actual methods of preparing such dosage forms are known, or will be apparent, to those skilled in this art; for example, see Remington's Pharmaceutical Sciences, Mack Publishing Company, Easton, Pennsylvania.

In some embodiments, the compositions will take the form of a pill or tablet and thus the composition may contain, along with a compounds disclosed and/or described herein, one or more of a diluent (e.g., lactose, sucrose, dicalcium phosphate), a lubricant (e.g., magnesium stearate), and/or a binder (e.g., starch, gum acacia, polyvinylpyrrolidine, gelatin, cellulose, cellulose derivatives). Other solid dosage forms include a powder, marume, solution or suspension (e.g., in propylene carbonate, vegetable oils or triglycerides) encapsulated in a gelatin capsule.

Liquid pharmaceutically administrable compositions can, for example, be prepared by dissolving, dispersing or suspending etc. a compound disclosed and/or described herein and optional pharmaceutical additives in a carrier (e.g., water, saline, aqueous dextrose, glycerol, glycols, ethanol or the like) to form a solution or suspension. Injectables can be prepared in conventional forms, either as liquid solutions or suspensions, as emulsions, or in solid forms suitable for dissolution or suspension in liquid prior to injection. The percentage of the compound contained in such parenteral compositions depends, for example, on the physical nature of the compound, the activity of the compound and the needs of the subject. However, percentages of active ingredient of 0.01% to 10% in solution are employable, and may be higher if the composition is a solid which will be subsequently diluted to another concentration. In some embodiments, the composition will comprise from about 0.2 to 2% of a compound disclosed and/or described herein in solution.

Pharmaceutical compositions of the compounds disclosed and/or described herein may also be administered to the respiratory tract as an aerosol or solution for a nebulizer, or as a microfine powder for insufflation, alone or in combination with an inert carrier such as lactose. In such a case, the particles of the pharmaceutical composition may have diameters of less than 50 microns, or in some embodiments, less than 10 microns.

In addition, pharmaceutical compositions can include a compound disclosed and/or described herein and one or more additional medicinal agents, pharmaceutical agents, adjuvants, and the like. Suitable medicinal and pharmaceutical agents include those described herein.

### Kits

Also provided are articles of manufacture and kits containing any of the compounds or pharmaceutical compositions provided herein. The article of manufacture may comprise a container with a label. Suitable containers include, for example, bottles, vials, and test tubes. The containers may be formed from a variety of materials such as glass or plastic. The container may hold a pharmaceutical composition provided herein. The label on the container may indicate that the pharmaceutical composition is used for preventing, treating or suppressing a condition described herein, and may also indicate directions for either *in vivo* or *in vitro* use.

In one aspect, provided herein are kits containing a compound or composition described herein and instructions for use. The kits may contain instructions for use in the treatment of a heart disease in an individual or subject in need thereof. A kit may additionally contain any materials or equipment that may be used in the administration of the compound or composition, such as vials, syringes, or IV bags. A kit may also contain sterile packaging.

### Combinations

The compounds and compositions described and/or disclosed herein may be administered alone or in combination with other therapies and/or therapeutic agents useful in the treatment of the aforementioned disorders, diseases, or conditions.

### General Synthetic Methods

Compounds of formula (I), (I-a), (I-b), (I-c), (I-d), (I-e), (II), (II-a), (II-b), (II-c), (II-d), or (II-e) will now be described by reference to illustrative synthetic schemes for their general preparation below and the specific examples that follow. Artisans will recognize that, to obtain the various compounds herein, starting materials may be suitably selected so that the ultimately desired substituents will be carried through the reaction scheme, with or without protection, as appropriate, to yield the desired product. Alternatively, it may be necessary or desirable to employ, in the place of the ultimately desired substituent, a suitable group that may be carried through the reaction scheme and replaced as appropriate with the desired substituent. In addition, one of skill in the art will recognize that protecting groups may be used to protect certain functional groups (for example: amino, carboxy, or side chain groups) from reaction conditions, and that such groups are removed under standard conditions when appropriate. It is also to be understood that any of the steps shown in any of the following general schemes may be used in any combination and in any order that is chemically feasible to achieve a desired intermediate or disclosed compound. Unless otherwise specified, the variables are as defined above in reference to formula (I), (I-a), (I-b), (I-c), (I-d), (I-e), (II), (II-a), (II-b), (II-c), (II-d), or (II-e).

Where it is desired to obtain a particular enantiomer of a compound, this may be accomplished from a corresponding mixture of enantiomers using any suitable conventional procedure for separating or resolving enantiomers. Thus, for example, diastereomeric derivatives may be produced by reaction of a mixture of enantiomers, e.g. a racemate, and an appropriate chiral compound. The diastereomers may then be separated by any convenient means, for example by crystallization and the desired enantiomer recovered. In another resolution process, a racemate may be separated using chiral High Performance Liquid Chromatography. Alternatively, if desired a particular enantiomer may be obtained by using an appropriate chiral intermediate in one of the processes described.

Chromatography, recrystallization, and other conventional separation procedures may also be used with intermediates or final products where it is desired to obtain a particular isomer of a compound or to otherwise purify a product of a reaction.

General methods of preparing compounds described herein are depicted in exemplified methods below. Variable groups in the schemes provided herein are defined as for formula (I), (I-a), (I-b), (I-c), (I-d), (I-e), (II), (II-a), (II-b), (II-c), (II-d), or (II-e), or any variation thereof. Other compounds described herein may be prepared by similar methods.

In some embodiments, compounds provided herein may be synthesized according to Scheme I-A: 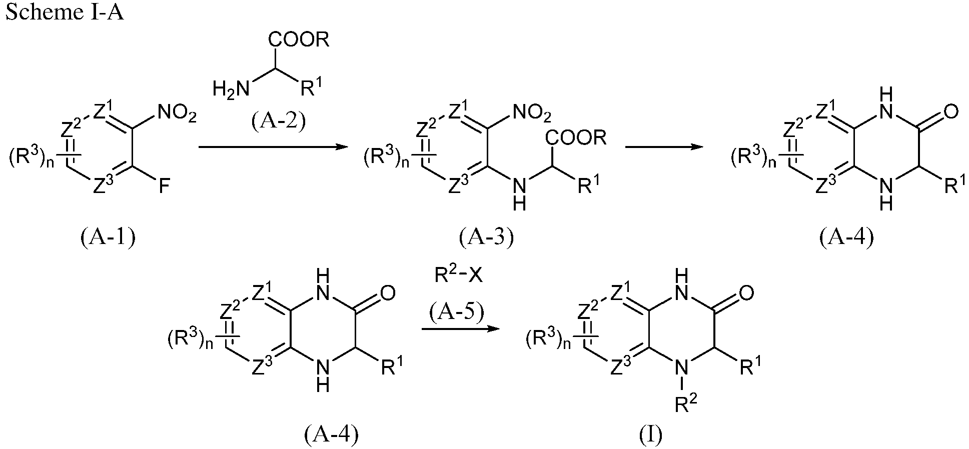 wherein Z¹, Z², Z³, R¹, R², R³, and n are as defined for the compound of formula (I), or any variation or embodiment thereof.

In such embodiments, provided is a method of preparing a compound of formula (I), or a pharmaceutically acceptable salt thereof, comprising reacting a compound of formula (A-4), or a salt thereof, with a compound of formula (A-5), or a salt thereof, wherein Z¹, Z², Z³, R¹, R², R³, and n are as defined for the compound of formula (I), and wherein X is OH or halogen. In some embodiments, X is OH and the step of reacting the compound of formula (A-4), or a salt thereof, with the compound of formula (A-5), or a salt thereof, is performed in the presence of a coupling agent. In some embodiments, the coupling agent is 1-chloro-*N*,*N*,2-trimethylprop-1-en-1-amine. In some embodiments, X is halogen. In some embodiments, X is chloro. In some embodiments, reaction between (A-4) and (A-5) is conducted in the presence of a base. In some embodiments, the base is DIPEA.

In some embodiments, the method further comprises preparing the compound of formula (A-5), or a salt thereof, wherein X is chloro, by reacting a compound of formula R²-OH with a chlorinating agent. In some embodiments, the chlorinating agent is 1-chloro-N,N,2-trimethylprop-1-en-1-amine. In some embodiments, the chlorinating agent is thionyl chloride.

In some embodiments, the method further comprises preparing the compound of formula (A-4), or a salt thereof, by converting a compound of formula (A-3), or a salt thereof, wherein R is C₁-C₆-alkyl, into the compound of formula (A-4), or a salt thereof, wherein Z¹, Z², Z³, R¹, R², R³, and n are as defined for the compound of formula (I). In some embodiments, the step of converting a compound of formula (A-3), or a salt thereof, into the compound of formula (A-4), or a salt thereof, is performed in the presence of iron and an acid. In some embodiments, the acid is ammonium chloride or acetic acid. In some embodiments, the acid is ammonium chloride. In some embodiments, the acid is acetic acid. In some embodiments, the step of converting a compound of formula (A-3), or a salt thereof, into the compound of formula (A-4), or a salt thereof, is performed in the presence of a palladium catalyst. In some embodiments, the palladium catalyst is palladium on carbon or palladium hydroxide on carbon (Pearlman's catalyst) and the step of converting a compound of formula (A-3), or a salt thereof, into the compound of formula (A-4), or a salt thereof, is performed in the presence of H₂. In some embodiments, the palladium catalyst is palladium on carbon and the step of converting a compound of formula (A-3), or a salt thereof, into the compound of formula (A-4), or a salt thereof, is performed in the presence of H₂. In some embodiments, the palladium catalyst is palladium hydroxide on carbon (Pearlman's catalyst) and the step of converting a compound of formula (A-3), or a salt thereof, into the compound of formula (A-4), or a salt thereof, is performed in the presence of H₂. In some embodiments, R is methyl or ethyl. In some embodiments, R is methyl. In some embodiments, R is ethyl.

In some embodiments, the method further comprises preparing the compound of formula (A-3), or a salt thereof, wherein R is C₁-C₆-alkyl, by reacting a compound of formula (A-1) with a compound of formula (A-2), or a salt thereof, wherein Z¹, Z², Z³, R¹, R², R³, and n are as defined for the compound of formula (I). In some embodiments, R is methyl or ethyl. In some embodiments, R is methyl. In some embodiments, R is ethyl.

In some embodiments, the method further comprises preparing the compound of formula (A-3), or a salt thereof, by reacting a compound of formula (A-1) with a hydrochloride salt of a compound of formula (A-2) in the presence of a base, wherein Z¹, Z², Z³, R¹, R², R³, and n are as defined for the compound of formula (I). In some embodiments, the base is diisopropylamine or lutidine. In some embodiments, the base is diisopropylamine. In some embodiments, the base is lutidine. In some embodiments, R is methyl or ethyl. In some embodiments, R is methyl. In some embodiments, R is ethyl.

In some embodiments, R⁴ is -C(=O)NR⁶R⁷ and the compound of formula (I), or a pharmaceutically acceptable salt thereof, is a compound of formula (A-8), or a pharmaceutically acceptable salt thereof, wherein Z¹, Z², Z³, R¹, R⁵, R⁶, and n are as defined for the compound of formula (I). The compound of formula (A-8), or a pharmaceutically acceptable salt thereof, can be prepared according to Scheme I-B: 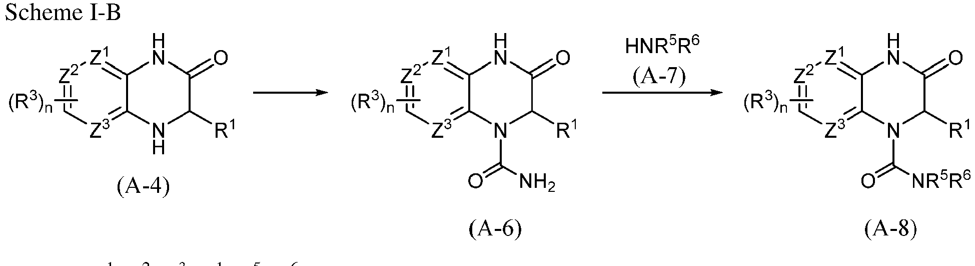 wherein Z¹, Z², Z³, R¹, R⁵, R⁶, and n are as defined for the compound of formula (I), or any variation or embodiment thereof.

In such embodiments, provided is a method of preparing the compound of formula (A-8), or a pharmaceutically acceptable salt thereof, by reacting a compound of formula (A-6), or a salt thereof, with a compound of formula (A-7), or a salt thereof, wherein Z¹, Z², Z³, R¹, R⁵, R⁶, and n are as defined for the compound of formula (I), or any variation or embodiment thereof.

In such embodiments, the method further comprises preparing the compound of formula (A-6), or a salt thereof, by reacting a compound of formula (A-4) with triphosgene and NH₃/MeOH, wherein Z¹, Z², Z³, R¹, and n are as defined for the compound of formula (I), or any variation or embodiment thereof.

In some embodiments, compounds of formula (II) provided herein may be synthesized according to Scheme II-A: 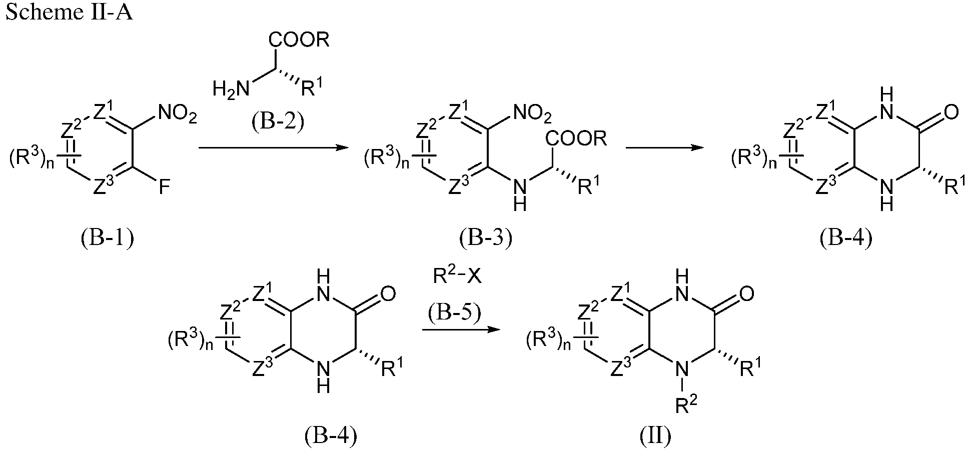 wherein Z¹, Z², Z³, R¹, R², R³, and n are as defined for the compound of formula (II), or any variation or embodiment thereof.

In such embodiments, provided is a method of preparing a compound of formula (II), or a pharmaceutically acceptable salt thereof, comprising reacting a compound of formula (B-4), or a salt thereof, with a compound of formula (B-5), or a salt thereof, wherein Z¹, Z², Z³, R¹, R², R³, and n are as defined for the compound of formula (II), and wherein X is OH or halogen. In some embodiments, X is OH and the step of reacting the compound of formula (B-4), or a salt thereof, with the compound of formula (B-5), or a salt thereof, is performed in the presence of a coupling agent. In some embodiments, the coupling agent is 1-chloro-*N*,*N*,2-trimethylprop-1-en-1-amine. In some embodiments, X is halogen. In some embodiments, X is chloro. In some embodiments, reaction between (B-4) and (B-5) is conducted in the presence of a base. In some embodiments, the base is DIPEA.

In some embodiments, the method further comprises preparing the compound of formula (B-5), or a salt thereof, wherein X is chloro, by reacting a compound of formula R²-OH with a chlorinating agent. In some embodiments, the chlorinating agent is 1-chloro-N,N,2-trimethylprop-1-en-1-amine. In some embodiments, the chlorinating agent is thionyl chloride.

In some embodiments, the method further comprises preparing the compound of formula (B-4), or a salt thereof, by converting a compound of formula (B-3), or a salt thereof, wherein R is C₁-C₆-alkyl, into the compound of formula (B-4), or a salt thereof, wherein Z¹, Z², Z³, R¹, R², R³, and n are as defined for the compound of formula (II). In some embodiments, the step of converting a compound of formula (B-3), or a salt thereof, into the compound of formula (B-4), or a salt thereof, is performed in the presence of iron and an acid. In some embodiments, the acid is ammonium chloride or acetic acid. In some embodiments, the acid is ammonium chloride. In some embodiments, the acid is acetic acid. In some embodiments, the step of converting a compound of formula (B-3), or a salt thereof, into the compound of formula (B-4), or a salt thereof, is performed in the presence of a palladium catalyst. In some embodiments, the palladium catalyst is palladium on carbon or palladium hydroxide on carbon (Pearlman's catalyst) and the step of converting a compound of formula (B-3), or a salt thereof, into the compound of formula (B-4), or a salt thereof, is performed in the presence of H₂. In some embodiments, the palladium catalyst is palladium on carbon and the step of converting a compound of formula (B-3), or a salt thereof, into the compound of formula (B-4), or a salt thereof, is performed in the presence of H₂. In some embodiments, the palladium catalyst is palladium hydroxide on carbon (Pearlman's catalyst) and the step of converting a compound of formula (B-3), or a salt thereof, into the compound of formula (B-4), or a salt thereof, is performed in the presence of H₂. In some embodiments, R is methyl or ethyl. In some embodiments, R is methyl. In some embodiments, R is ethyl.

In some embodiments, the method further comprises preparing the compound of formula (B-3), or a salt thereof, wherein R is C₁-C₆-alkyl, by reacting a compound of formula (B-1) with a compound of formula (B-2), or a salt thereof, wherein Z¹, Z², Z³, R¹, R², R³, and n are as defined for the compound of formula (II). In some embodiments, R is methyl or ethyl. In some embodiments, R is methyl. In some embodiments, R is ethyl.

In some embodiments, the method further comprises preparing the compound of formula (B-3), or a salt thereof, by reacting a compound of formula (B-1) with a hydrochloride salt of a compound of formula (B-2) in the presence of a base, wherein Z¹, Z², Z³, R¹, R², R³, and n are as defined for the compound of formula (II). In some embodiments, the base is diisopropylamine or lutidine. In some embodiments, the base is diisopropylamine. In some embodiments, the base is lutidine. In some embodiments, R is methyl or ethyl. In some embodiments, R is methyl. In some embodiments, R is ethyl.

In some embodiments, R⁴ is -C(=O)NR⁶R⁷ and the compound of formula (II), or a pharmaceutically acceptable salt thereof, is a compound of formula (B-8), or a pharmaceutically acceptable salt thereof, wherein Z¹, Z², Z³, R¹, R⁵, R⁶, and n are as defined for the compound of formula (II). The compound of formula (B-8), or a pharmaceutically acceptable salt thereof, can be prepared according to Scheme II-B: 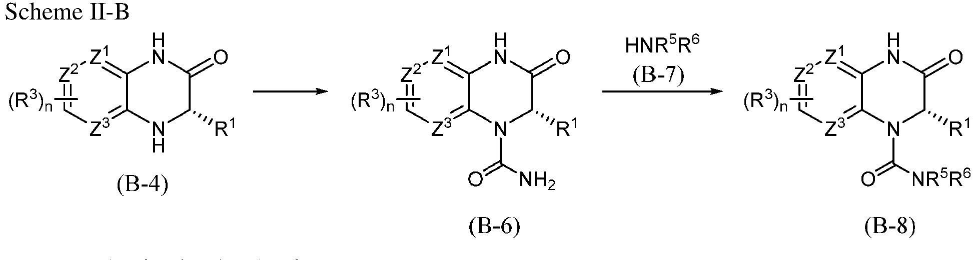 wherein Z¹, Z², Z³, R¹, R⁵, R⁶, and n are as defined for the compound of formula (II), or any variation or embodiment thereof.

In such embodiments, provided is a method of preparing the compound of formula (B-8), or a pharmaceutically acceptable salt thereof, by reacting a compound of formula (B-6), or a salt thereof, with a compound of formula (B-7), or a salt thereof, wherein Z¹, Z², Z³, R¹, R⁵, R⁶, and n are as defined for the compound of formula (II), or any variation or embodiment thereof.

In such embodiments, the method further comprises preparing the compound of formula (B-6), or a salt thereof, by reacting a compound of formula (B-4) with triphosgene and NH₃/MeOH, wherein Z¹, Z², Z³, R¹, and n are as defined for the compound of formula (II), or any variation or embodiment thereof.

In some embodiments, compounds of formula (II-a) provided herein may be synthesized according to Scheme III-A: 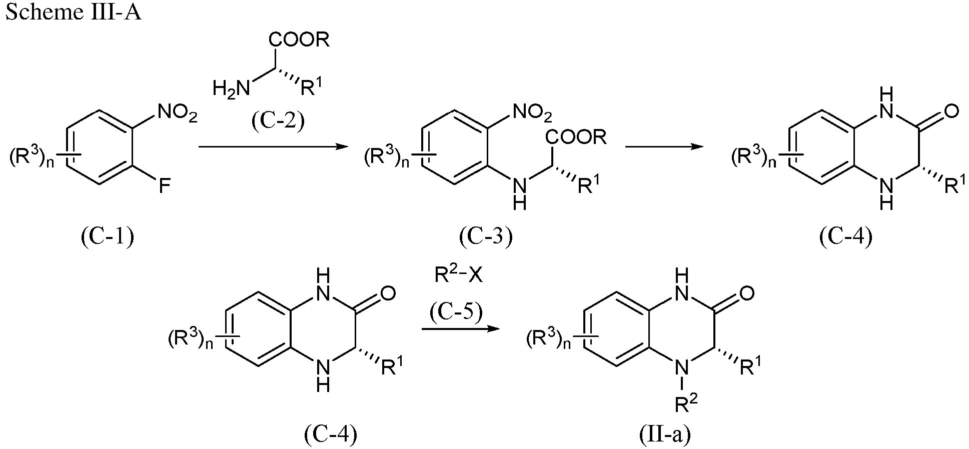 wherein R¹, R², R³, and n are as defined for the compound of formula (II-a), or any variation or embodiment thereof.

In such embodiments, provided is a method of preparing a compound of formula (II-a), or a pharmaceutically acceptable salt thereof, comprising reacting a compound of formula (C-4), or a salt thereof, with a compound of formula (C-5), or a salt thereof, wherein R¹, R², R³, and n are as defined for the compound of formula (II-a), and wherein X is OH or halogen. In some embodiments, X is OH and the step of reacting the compound of formula (C-4), or a salt thereof, with the compound of formula (C-5), or a salt thereof, is performed in the presence of a coupling agent. In some embodiments, the coupling agent is 1-chloro-*N*,*N*,2-trimethylprop-1-en-1-amine. In some embodiments, X is halogen. In some embodiments, X is chloro. In some embodiments, reaction between (C-4) and (C-5) is conducted in the presence of a base. In some embodiments, the base is DIPEA.

In some embodiments, the method further comprises preparing the compound of formula (C-5), or a salt thereof, wherein X is chloro, by reacting a compound of formula R²-OH with a chlorinating agent. In some embodiments, the chlorinating agent is 1-chloro-N,N,2-trimethylprop-1-en-1-amine. In some embodiments, the chlorinating agent is thionyl chloride.

In some embodiments, the method further comprises preparing the compound of formula (C-4), or a salt thereof, by converting a compound of formula (C-3), or a salt thereof, wherein R is C₁-C₆-alkyl, into the compound of formula (C-4), or a salt thereof, wherein R¹, R², R³, and n are as defined for the compound of formula (II-a). In some embodiments, the step of converting a compound of formula (C-3), or a salt thereof, into the compound of formula (C-4), or a salt thereof, is performed in the presence of iron and an acid. In some embodiments, the acid is ammonium chloride or acetic acid. In some embodiments, the acid is ammonium chloride. In some embodiments, the acid is acetic acid. In some embodiments, the step of converting a compound of formula (C-3), or a salt thereof, into the compound of formula (C-4), or a salt thereof, is performed in the presence of a palladium catalyst. In some embodiments, the palladium catalyst is palladium on carbon or palladium hydroxide on carbon (Pearlman's catalyst) and the step of converting a compound of formula (C-3), or a salt thereof, into the compound of formula (C-4), or a salt thereof, is performed in the presence of H₂. In some embodiments, the palladium catalyst is palladium on carbon and the step of converting a compound of formula (C-3), or a salt thereof, into the compound of formula (C-4), or a salt thereof, is performed in the presence of H₂. In some embodiments, the palladium catalyst is palladium hydroxide on carbon (Pearlman's catalyst) and the step of converting a compound of formula (C-3), or a salt thereof, into the compound of formula (C-4), or a salt thereof, is performed in the presence of H₂. In some embodiments, R is methyl or ethyl. In some embodiments, R is methyl. In some embodiments, R is ethyl.

In some embodiments, the method further comprises preparing the compound of formula (C-3), or a salt thereof, wherein R is C₁-C₆-alkyl, by reacting a compound of formula (C-1) with a compound of formula (C-2), or a salt thereof, wherein R¹, R², R³, and n are as defined for the compound of formula (II-a). In some embodiments, R is methyl or ethyl. In some embodiments, R is methyl. In some embodiments, R is ethyl.

In some embodiments, the method further comprises preparing the compound of formula (C-3), or a salt thereof, by reacting a compound of formula (C-1) with a hydrochloride salt of a compound of formula (C-2) in the presence of a base, wherein R¹, R², R³, and n are as defined for the compound of formula (II-a). In some embodiments, the base is diisopropylamine or lutidine. In some embodiments, the base is diisopropylamine. In some embodiments, the base is lutidine. In some embodiments, R is methyl or ethyl. In some embodiments, R is methyl. In some embodiments, R is ethyl.

In some embodiments, R⁴ is -C(=O)NR⁶R⁷ and the compound of formula (II-a), or a pharmaceutically acceptable salt thereof, is a compound of formula (C-8), or a pharmaceutically acceptable salt thereof, wherein R¹, R⁵, R⁶, and n are as defined for the compound of formula (II-a). The compound of formula (C-8), or a pharmaceutically acceptable salt thereof, can be prepared according to Scheme III-B: 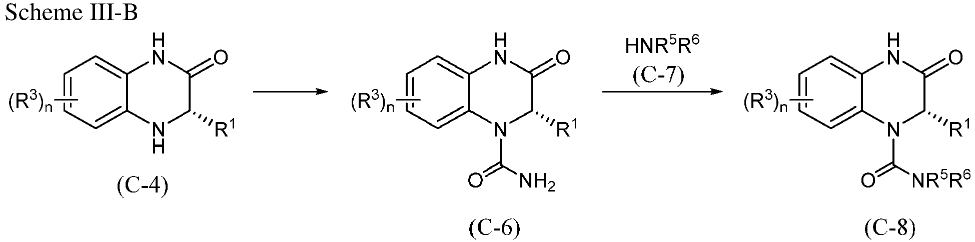 wherein R¹, R⁵, R⁶, and n are as defined for the compound of formula (II-a), or any variation or embodiment thereof.

In such embodiments, provided is a method of preparing the compound of formula (C-8), or a pharmaceutically acceptable salt thereof, by reacting a compound of formula (C-6), or a salt thereof, with a compound of formula (C-7), or a salt thereof, wherein R¹, R⁵, R⁶, and n are as defined for the compound of formula (II-a), or any variation or embodiment thereof.

In such embodiments, the method further comprises preparing the compound of formula (C-6), or a salt thereof, by reacting a compound of formula (C-4) with triphosgene and NH₃/MeOH, wherein Z¹, Z², Z³, R¹, and n are as defined for the compound of formula (II-a), or any variation or embodiment thereof.

Also provided herein are intermediate compounds, or a salt thereof, to make the compounds of Formula (I) or any sub-formula thereof. In some embodiments, the intermediate compounds are the intermediate compounds shown in Generic Synthetic Schemes above. In some embodiments, the intermediate compounds are the intermediate compounds shown in the Example section below.

### EXAMPLES

The following examples are offered to illustrate, but not to limit, the compounds, compositions, uses, and methods provided herein. In some examples, the compounds and intermediates are prepared using the general methods described above.

The following abbreviations are used throughout the Examples: DIPEA (N,N-diisopropylethylamine), LRMS (low-resolution mass spectrometry), Ac (acetyl), Et (ethyl), Me (methyl), tBu (tert-butyl), APCI (atmospheric pressure chemical ionization), THF (tetrahydrofuran), DMAP (4-dimethylaminopyridine), DMF (dimethylformamide), DMSO (dimethylsulfoxide), HPLC (high-performance liquid chromatography), TEA (triethylamine), and ESI (electrospray ionization).

### Example 1: Synthesis of Compound 64

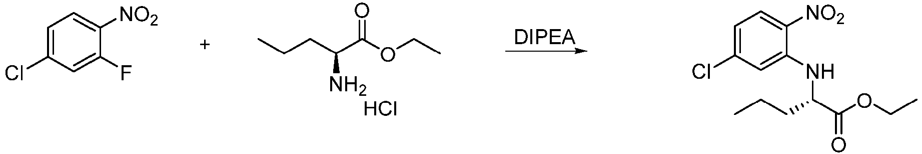

**Step 1: Ethyl (S)-2-((5-chloro-2-nitrophenyl)amino)pentanoate.** To a mixture of 4-chloro-2-fluoro-1-nitrobenzene (1.00 g, 5.7 mmol) in DMF (10 mL) was added ethyl (S)-2-aminopentanoate hydrochloride (1.14 g, 6.3 mmol) and DIPEA (2.4 mL, 14 mmol) at room temperature. The reaction mixture was heated to 70 °C and stirred overnight. The reaction mixture was then quenched with saturated ammonium chloride, extracted with 50% EtOAc/hexane, dried over MgSO4, and concentrated under reduced pressure to give ethyl (S)-2-((5-chloro-2-nitrophenyl)amino)pentanoate (1.75 g, quantitative) as an orange oil.

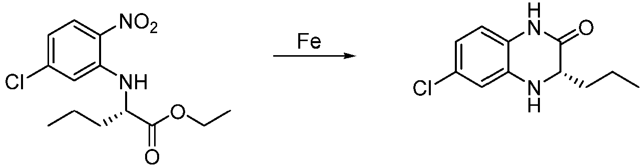

**Step 2: (*S*)-6-Chloro-3-propyl-3,4-dihydroquinoxalin-2(1*H*)-one.** To a mixture of ethyl (*S*)-2-((5-chloro-2-nitrophenyl)amino)pentanoate (1.74 g, 5.8 mmol) in MeOH (20 mL) and water (10 mL) was added Fe powder (2.26 g, 40.5 mmol) and NH₄Cl (371 mg, 6.9 mmol) at room temperature. The reaction mixture was heated to 70 °C and stirred for 4 h, diluted with water and CHCl₃, and filtered through a Celite pad. The filtrate was extracted with CHCl₃, dried over MgSO₄, concentrated, and purified using amino silica gel chromatography (0-50% EtOAc/hexane) to give (S)-6-chloro-3-propyl-3,4-dihydroquinoxalin-2(1*H*)-one (1.15 g, 88%) as a yellow solid.

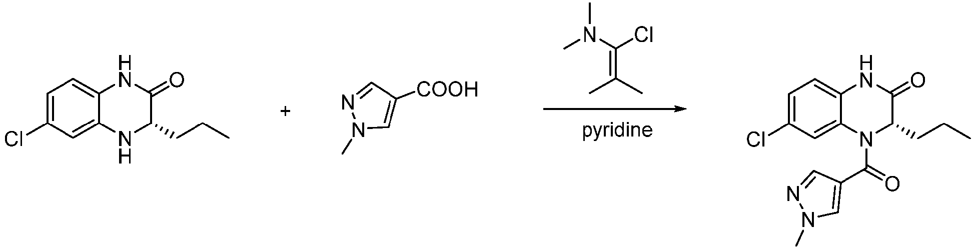

**Step 3: (*S*)-6-Chloro-4-(1-methyl-1*H*-pyrazole-4-carbonyl)-3-propyl-3,4-dihydroquinoxalin-2(1*H*)-one.** To a solution of 1-methyl-1*H*-pyrazole-4-carboxylic acid (130 mg, 1.0 mmol) in CH₂Cl₂ (2 mL) was added 1-chloro-*N*,*N*,2-trimethylprop-1-en-1-amine (145 µL, 1.1 mmol). After stirring at room temperature for 2 h, (*S*)-6-chloro-3-propyl-3,4-dihydroquinoxalin-2(1*H*)-one (150 mg, 668 µmol) and pyridine (160 µL, 2.0 mmol) were added. The reaction mixture was stirred at room temperature overnight, then quenched with water, and then diluted with CHCl₃. The organic layer was separated, concentrated under reduced pressure, and purified using amino silica gel chromatography (0-5% MeOH/CHCl₃) to give (*S*)-6-chloro-4-(1-methyl-1H-pyrazole-4-carbonyl)-3-propyl-3,4-dihydroquinoxalin-2(1H)-one (104 mg, 47%) as a colorless solid. 1H NMR (500 MHz, DMSO-d6) δ 10.87 (s, 1H), 7.90 (s, 1H), 7.26 (dd, J = 2.3, 8.6 Hz, 1H), 7.19 (s, 1H), 7.15 (d, J = 2.1 Hz, 1H), 7.06 (d, J = 8.6 Hz, 1H), 4.89 (dd, J = 5.9, 8.9 Hz, 1H), 3.81 (s, 3H), 1.51 - 1.43 (m, 1H), 1.40 - 1.22 (m, 3H), 0.90-0.84 (m, 3H). LRMS (ESI) m/z 333.2 [M + H]⁺.

### Example 2: Synthesis of Compound 100

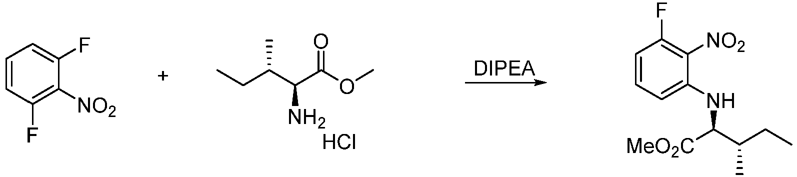

**Step 1: Methyl (3-fluoro-2-nitrophenyl)-*L*-isoleucinate.** To a mixture of 1,3-difluoro-2-nitrobenzene (1.00 g, 6.3 mmol) in DMF (5 mL) was added methyl *L*-isoleucinate hydrochloride (1.37 g, 7.5 mmol) and DIPEA (2.7 mL, 16.0 mmol). The reaction mixture was heated to 70 °C and stirred overnight. The reaction mixture was cooled to room temperature and water was added. The mixture was stirred for 30 min and then extracted with 20% EtOAc/hexane. The organic layer was washed with 5% NaCl, dried over MgSO₄, and concentrated under reduced pressure to give methyl (3-fluoro-2-nitrophenyl)-L-isoleucinate (1.70 g, 95%) as a brown oil.

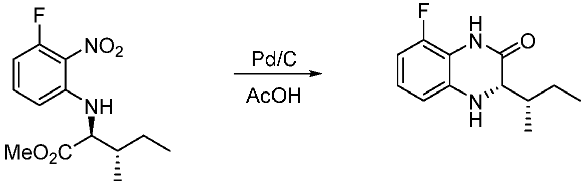

**Step 2: (*S*)-3-((*S*)-*sec*-Butyl)-8-fluoro-3,4-dihydroquinoxalin-2(1*H*)-one.** To a mixture of methyl (3-fluoro-2-nitrophenyl)-*L*-isoleucinate (1.7 g, 5.98 mmol) in ethanol (20 mL) was added 5% Pd/C (340 mg). The reaction was stirred under a hydrogen atmosphere for 2.5 d and then filtered through a celite pad. The celite pad was washed with ethanol (10 mL), and the combined organic filtrates were heated to 80 °C and stirred for 1 day. A solution of ammonium chloride (384 mg, 7.18 mmol) in H₂O (8.0 mL) was added, and the mixture was heated to 80 °C and stirred for 1 day. HCl (1 M, 7.0 mL) was added to the mixture, and the mixture was stirred at 80 °C for 2 d. The reaction mixture was then concentrated, diluted with chloroform, and neutralized with saturated sodium bicarbonate. The organic layer was separated, concentrated, and purified using silica gel chromatography (5-40% EtOAc/Hexane) followed by amino silica gel chromatography (5-50% EtOAc/hexanes) to give (S)-3-((S)-sec-butyl)-8-fluoro-3,4-dihydroquinoxalin-2(1*H*)-one (713 mg, 54%) as a pale yellow solid.

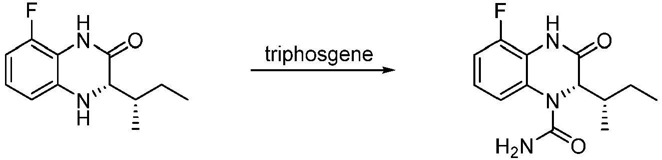

**Step 3: (*S*)-2-((*S*)-*sec*-Butyl)-5-fluoro-3-oxo-3,4-dihydroquinoxaline-1(2*H*)-carboxamide.** To an ice-cooled solution of (*S*)-3-((*S*)-*sec*-butyl)-8-fluoro-3,4-dihydroquinoxalin-2(1*H*)-one (200 mg, 0.9 mmol) and DIPEA (462 µL, 2.7 mmol) in THF (2.0 mL) was added triphosgene (107 mg, 361 µmol). After stirring for 25 min, NH₃/MeOH (7 M, 770 µL, 5.4 mmol) was added to the reaction mixture. The reaction mixture was warmed to room temperature and stirred for 1 h. The reaction mixture was then concentrated and diluted with CHCl₃ and H₂O. The organic layer was separated, concentrated under reduced pressure, purified using silica gel chromatography (0-5% MeOH/CHCl₃), and triturated with isopropylether to give (*S*)-2-((*S*)*-sec-*butyl)-5-fluoro-3-oxo-3,4-dihydroquinoxaline-1(2*H*)-carboxamide (138 mg, 58%) as a colorless solid. 1H NMR (500 MHz, DMSO-d6) δ 10.72 (s, 1H), 7.28 - 7.24 (m, 1H), 7.05 - 7.01 (m, 2H), 6.49 (s, 2H), 4.50 (d, J = 10.4 Hz, 1H), 1.44 - 1.35 (m, 1H), 1.24 - 1.15 (m, 1H), 1.10 - 1.00 (m, 1H), 0.86 (d, J = 6.7 Hz, 3H), 0.73 (t, J = 7.4 Hz, 3H). LRMS (ESI) m/z 266.3 [M + H]⁺.

The following compound was prepared by methods analogous to the method described for Compound 100:

| Compound No. | LRMS *m*/*z* [M+H] | ¹H NMR |
|---|---|---|
| 99 | 248.4 (ESI) | ¹H NMR (500 MHz, DMSO-d6) δ 10.56 (s, 1H), 7.40 (dd, J = 1.2, 8.0 Hz, 1H), 7.10 (dt, J = 1.4, 7.7 Hz, 1H), 7.01 (dt, J = 1.5, 7.7 Hz, 1H), 6.94 (dd, J = 1.4, 7.8 Hz, 1H), 6.35 (s, 2H), 4.50 (d, J = 10.1 Hz, 1H), 1.44 - 1.36 (m, 1H), 1.24 - 1.15 (m, 1H), 1.09 - 1.00 (m, 1H), 0.85 (d, J = 6.7 Hz, 3H), 0.73 (t, J = 7.5 Hz, 3H) |
| 113 | 266.4 (ESI) | ¹H NMR (500 MHz, DMSO-d6) δ 10.59 (s, 1H), 7.27 (dd, J = 2.4, 10.2 Hz, 1H), 6.97-6.91 (m, 2H), 6.55 (s, 2H), 4.46 (d, J = 10.0 Hz, 1H), 1.45 - 1.36 (m, 1H), 1.26 - 1.18 (m, 1H), 1.09 - 1.00 (m, 1H), 0.85 (d, J = 6.7 Hz, 3H), 0.74 (t, J = 7.4 Hz, 3H) |

### Example 3: Synthesis of Compound 7

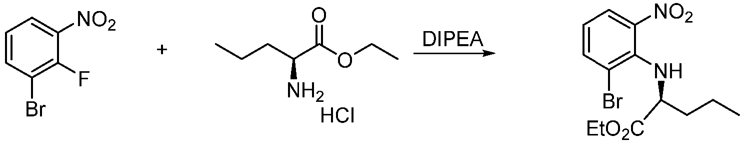

**Step 1: Ethyl (*S*)-2-((2-bromo-6-nitrophenyl)amino)pentanoate.** To a mixture of 1-bromo-2-fluoro-3-nitrobenzene (1.00 g, 4.6 mmol) in DMF (10 mL) was added ethyl (*S*)-2-aminopentanoate hydrochloride (0.91 g, 5.0 mmol) and DIPEA (2.0 mL, 12.0 mmol). The reaction mixture was heated to 70 °C and stirred overnight. The reaction mixture was cooled to room temperature and quenched with saturated ammonium chloride, extracted with 50% EtOAc/hexane, dried over MgSO₄, and concentrated under reduced pressure to give ethyl (*S*)-2-((2-bromo-6-nitrophenyl)amino)pentanoate (1.65 g, quantitative) as a brown oil.

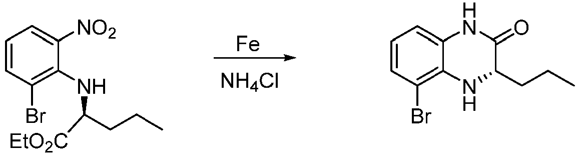

**Step 2: (*S*)-5-Bromo-3-propyl-3,4-dihydroquinoxalin-2(1*H*)-one.** To a mixture of ethyl (*S*)-2-((2-bromo-6-nitrophenyl)amino)pentanoate (1.65 g, 4.8 mmol) in MeOH (20 mL) and water (10 mL) was added Fe powder (1.87 g, 33.5 mmol) and NH₄Cl (310 mg, 5.8 mmol) at room temperature. The reaction mixture was heated to 70 °C and stirred for 4 h and then heated to 80 °C and stirred overnight. The reaction was cooled to room temperature, diluted with water and CHCl₃ and filtered through a Celite pad. The filtrate was extracted with CHCl₃, dried over MgSO₄, concentrated, and purified using amino silica gel chromatography (0-50% EtOAc/hexane) to give (S)-5-bromo-3-propyl-3,4-dihydroquinoxalin-2(1*H*)-one (1.04 g, 81%) as a yellow solid.

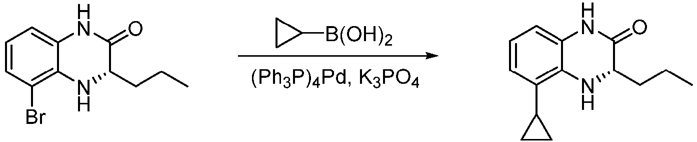

**Step 3: (*S*)-5-Cyclopropyl-3-propyl-3,4-dihydroquinoxalin-2(1*H*)-one.** To a mixture of (*S*)-5-bromo-3-propyl-3,4-dihydroquinoxalin-2(1*H*)-one (265 mg, 0.99 mmol) in toluene (3 mL) and water (800 µL) was added cyclopropylboronic acid (170 mg, 1.99 mmol), potassium phosphate (420 mg, 1.98 mmol), and tetrakis(triphenylphosphine)palladium (60 mg, 0.05 mmol). This mixture was heated to 100 °C and stirred overnight, then cooled to room temperature and diluted with water. Ethyl acetate was added, and the organic layer was separated, dried over MgSO₄, concentrated, and purified using amino silica gel chromatography (0-50% EtOAc/hexane) and silica gel (0-50% EtOAc/hexane) to give (*S*)-5-cyclopropyl-3-propyl-3,4-dihydroquinoxalin-2(1*H*)-one (161 mg, 71%) as a yellow oil.

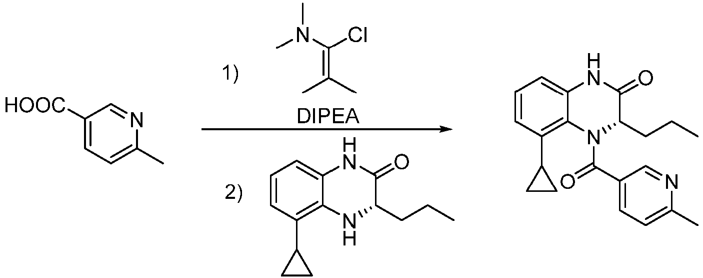

**Step 4: (*S*)-5-Cyclopropyl-4-(6-methylnicotinoyl)-3-propyl-3,4-dihydroquinoxalin-2(1*H*)-one.** To a solution of 6-methylnicotinic acid (188 mg, 1.4 mmol) in dichloroethane (1.6 mL) was added 1-chloro-*N*,*N*,2-trimethylprop-1-en-1-amine (182 µL, 1.4 mmol). The reaction mixture was stirred for 1 h, and a solution of (*S*)-5-cyclopropyl-3-propyl-3,4-dihydroquinoxalin-2(1*H*)-one (158 mg, 0.7 mmol) in DCE (1.6 mL) and DIPEA (350 µL, 2.04 mmol) was added. This mixture was stirred overnight and then diluted with water and CHCl₃. The organic layer was separated, concentrated, and purified using amino silica gel chromatography (0-50% EtOAc/hexane) and reverse phase ODS silica gel chromatography 0-50% EtOH/H2O), and then triturated with acetonitrile to give (S)-5-cyclopropyl-4-(6-methylnicotinoyl)-3-propyl-3,4-dihydroquinoxalin-2(1*H*)-one (116 mg) as a colorless solid. LRMS (ESI) m/z 350.4 [M + H]⁺.

### Example 4: Synthesis of Compound 69

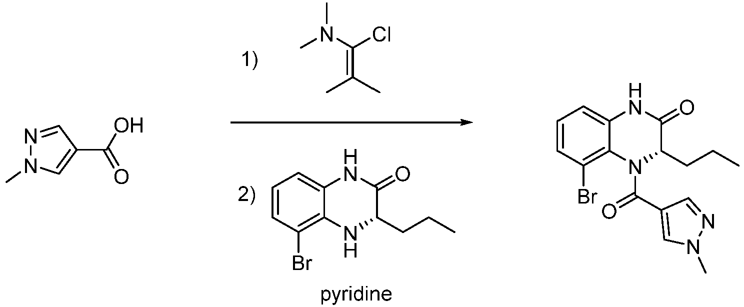

To a solution of 1-methyl-1*H*-pyrazole-4-carboxylic acid (105 mg, 0.8 mmol) in dichloroethane (2.0 mL) was added 1-chloro-*N*,*N*,2-trimethylprop-1-en-1-amine (120 µL, 0.9 mmol). The reaction mixture was stirred for 2 h, and then (*S*)-5-bromo-3-propyl-3,4-dihydroquinoxalin-2(1*H*)-one (150 mg, 0.6 mmol) was added, followed by pyridine (135 µL, 1.7 mmol). This mixture was stirred overnight at room temperature, concentrated, and purified using amino silica gel chromatography (0-5% MeOH/CHCl₃) and silica gel chromatography (0-5% MeOH/CHCl₃) to give (*S*)-5-bromo-4-(1-methyl-1*H*-pyrazole-4-carbonyl)-3-propyl-3,4-dihydroquinoxalin-2(1*H*)-one (24 mg, 11%) as a colorless solid. 1H NMR (399 MHz, DMSO-d6) δ 10.91 (s, 1H), 7.76 - 7.57 (m, 1H), 7.29 - 7.20 (m, 2H), 7.16 - 7.09 (m, 1H), 6.88 - 6.68 (m, 1H), 5.10 - 4.93 (m, 1H), 3.77 (brs, 3H), 1.53-1.19 (m, 4H), 0.90 (t, J = 7.0 Hz, 3H). LRMS (ESI) m/z 377.1 [M + H]⁺.

The following compounds were prepared by methods analogous to the method described for Compound 69 (standard protecting group strategies were employed for compounds with reactive heteroatoms):

| Compound No. | LRMS *m*/*z* [M+H] | ¹H NMR |
|---|---|---|
| 1 | 330.2 (ESI) | |
| 2 | 364.1 (ESI) | |
| 3 | 310.2 (ESI) | |
| 5 | 330.1 (ESI) | |
| 6 | 314.2 (ESI) | |
| 9 | 284.2 (ESI) | |
| 10 | 295.2 (ESI) | |
| 11 | 296.2 (ESI) | |
| 12 | 296.2 (ESI) | |
| 13 | 299.2 (ESI) | ¹H NMR (500 MHz, DMSO-d6) δ 10.75 (s, 1H), 7.79 (s, 1H), 7.20 (dt, J = 1.3, 7.7 Hz, 1H), 7.08 (s, 1H), 7.06 (dd, J = 1.3, 8.0 Hz, 1H), 7.01 (d, J = 7.3 Hz, 1H), 6.90 (dt, J = 1.3, 7.7 Hz, 1H), 4.94 (dd, J = 5.5, 9.2 Hz, 1H), 3.78 (s, 3H), 1.51 - 1.42 (m, 1H), 1.37 - 1.27 (m, 3H), 0.87 (t, J = 7.2 Hz, 3H) |
| 14 | 300.2 (ESI) | |
| 15 | 301.2 (ESI) | |
| 16 | 310.2 (ESI) | |
| 17 | 313.2 (ESI) | |
| 18 | 314.2 (ESI) | |
| 19 | 323.2 (ESI) | |
| 20 | 330.2 (ESI) | |
| 21 | 331.2 (ESI) | |
| 22 | 353.2 (ESI) | |
| 23 | 361.2 (ESI) | |
| 24 | 362.2 (ESI) | |
| 25 | 380.2 (ESI) | |
| 26 | 335.2 (ESI) | |
| 30 | 313.3 (ESI) | |
| 31 | 339.3 (ESI) | |
| 32 | 339.4 (ESI) | |
| 45 | 313.2 (ESI) | |
| 63 | 313.2 (ESI) | ¹H NMR (500 MHz, DMSO-d6) δ 10.77 (s, 1H), 7.75 (s, 1H), 7.20 (dt, J = 1.3, 7.7 Hz, 1H), 7.05 (dd, J = 1.2, 8.0 Hz, 1H), 7.03 - 6.99 (m, 2H), 6.89 (dt, J = 1.2, 7.7 Hz, 1H), 4.68 (d, J = 10.1 Hz, 1H), 3.76 (s, 3H), 1.49 - 1.40 (m, 1H), 1.38 - 1.30 (m, 1H), 1.14 - 1.05 (m, 1H), 0.89 (d, J = 6.8 Hz, 3H), 0.76 (t, J = 7.4 Hz, 3H) |
| 65 | 335.3 (ESI) | |
| 66 | 335.2 (ESI) | |
| 67 | 329.2 (ESI) | ¹H NMR (500 MHz, DMSO-d6) δ 10.74 (s, 1H), 7.74 (s, 1H), 7.20 (dt, J = 1.2, 7.7 Hz, 1H), 7.14 (s, 1H), 7.06 (dd, J = 1.2, 8.0 Hz, 1H), 7.03 (d, J = 7.9 Hz, 1H), 6.89 (dt, J = 1.1, 7.7 Hz, 1H), 4.95 (dd, J = 5.5, 9.1 Hz, 1H), 4.84 (t, J = 5.3 Hz, 1H), 4.12 - 4.02 (m, 2H), 3.65 (dd, J = 5.5, 10.9 Hz, 2H), 1.51 - 1.42 (m, 1H), 1.37 - 1.28 (m, 3H), 0.87 (t, J = 7.1 Hz, 3H) |
| 68 | 313.2 (ESI) | |
| 69 | 377.1 (ESI) | ¹H NMR (399 MHz, DMSO-d6) δ 10.91 (s, 1H), 7.76 - 7.57 (m, 1H), 7.29 - 7.20 (m, 2H), 7.16 - 7.09 (m, 1H), 6.88 - 6.68 (m, 1H), 5.10 - 4.93 (m, 1H), 3.77 (brs, 3H), 1.52 - 1.39 (m, 3H), 1.31 - 1.20 (m, 1H), 0.90 (t, J = 7.0 Hz, 3H) |
| 70 | 335.3 (ESI) | ¹H NMR (399 MHz, DMSO-d6) δ 10.73 (s, 1H), 8.91 (s, 1H), 7.86 (s, 1H), 7.75 (d, J = 2.4 Hz, 1H), 7.11 - 7.05 (m, 1H), 7.02 - 6.97 (m, 2H), 6.91 - 6.75 (m, 2H), 6.72 (d, J = 3.8 Hz, 1H), 4.94 (t, J = 7.4 Hz, 1H), 1.56 - 1.30 (m, 4H), 0.87 (t, J = 7.2 Hz, 3H) |
| 71 | 285.3 (ESI) | ¹H NMR (399 MHz, DMSO-d6) δ 13.15 (brs, 1H), 10.74 (s, 1H), 7.95 - 7.04 (m, 2H), 7.19 (dt, J = 1.3, 7.6 Hz, 1H), 7.06 (dd, J = 1.2, 7.9 Hz, 1H), 6.98 (d, J = 7.6 Hz, 1H), 6.88 (dt, J = 1.4, 7.7 Hz, 1H), 4.96 (dd, J = 5.4, 9.2 Hz, 1H), 1.53 - 1.41 (m, 1H), 1.38 - 1.27 (m, 3H), 0.87 (t, J = 7.1 Hz, 3H) |
| 72 | 299.2 (ESI) | ¹H NMR (399 MHz, DMSO-d6) δ 13.13 (brs, 1H), 10.76 (s, 1H), 7.69 (brs, 1H), 7.20 (dt, J = 1.3, 7.7 Hz, 1H), 7.15 (brs, 1H), 7.06 (dd, J = 1.2, 7.9 Hz, 1H), 6.98 (d, J = 7.8 Hz, 1H), 6.88 (dt, J = 1.1, 7.7 Hz, 1H), 4.70 (d, J = 10.1 Hz, 1H), 1.51 - 1.39 (m, 1H), 1.39 - 1.29 (m, 1H), 1.16 - 1.04 (m, 1H), 0.90 (d, J = 6.7 Hz, 3H), 0.77 (t, J = 7.4 Hz, 3H) |
| 73 | 336.2 (ESI) | |
| 74 | 325.2 (ESI) | |
| 75 | 326.1 (ESI) | |
| 76 | 325.2 (ESI) | |
| 77 | 299.2 (ESI) | ¹H NMR (399 MHz, DMSO-d6) δ 10.76 (s, 1H), 7.78 (s, 1H), 7.20 (dt, J = 1.3, 7.6 Hz, 1H), 7.07 - 7.01 (m, 3H), 6.89 (dt, J = 1.3, 7.7 Hz, 1H), 4.59 (d, J = 10.1 Hz, 1H), 3.77 (s, 3H), 1.58 - 1.49 (m, 1H), 0.96 (d, J = 6.7 Hz, 3H), 0.84 (d, J = 6.7 Hz, 3H) |
| 78 | 343.2 (ESI) | ¹H NMR (500 MHz, DMSO-d6) δ 10.76 (s, 1H), 7.71 (s, 1H), 7.21 (dt, J = 1.3, 7.7 Hz, 1H), 7.08 - 7.04 (m, 2H), 7.03 (d, J = 8.0 Hz, 1H), 6.89 (dt, J = 1.3, 7.7 Hz, 1H), 4.83 (t, J = 5.3 Hz, 1H), 4.69 (d, J = 10.1 Hz, 1H), 4.11 - 4.01 (m, 2H), 3.64 (dd, J = 5.5, 10.9 Hz, 2H), 1.49 - 1.40 (m, 1H), 1.39 - 1.30 (m, 1H), 1.15 - 1.05 (m, 1H), 0.89 (d, J = 6.7 Hz, 3H), 0.77 (t, J = 7.4 Hz, 3H) |
| 79 | 299.2 (ESI) | ¹H NMR (500 MHz, DMSO-d6) δ 13.21 (brs, 1H), 10.72 (s, 1H), 7.78 (brs, 1H), 7.20 - 6.77 (m, 4H), 6.46 (brs, 1H), 5.08 (brs, 1H), 1.52 - 1.42 (m, 1H), 1.42 - 1.33 (m, 1H), 1.17 - 1.07 (m, 1H), 0.88 (d, J = 6.7 Hz, 3H), 0.74 (t, J = 7.3 Hz, 3H) |
| 80 | 356.2 (ESI) | |
| 81 | 326.1 (ESI) | ¹H NMR (500 MHz, DMSO-d6) δ 10.79 (s, 1H), 8.10 (d, J = 2.5 Hz, 1H), 7.14 (dt, J = 1.5, 7.5 Hz, 1H), 7.03 (dd, J = 1.2, 8.0 Hz, 1H), 6.91 (dd, J = 1.0, 7.9 Hz, 1H), 6.87 (dd, J = 1.3, 7.2 Hz, 1H), 6.84 (dd, J = 2.6, 9.5 Hz, 1H), 6.11 (d, J = 9.5 Hz, 1H), 4.56 (d, J = 10.0 Hz, 1H), 3.41 (s, 3H), 1.60 - 1.52 (m, 1H), 0.96 (d, J = 6.7 Hz, 3H), 0.85 (d, J = 6.7 Hz, 3H) |
| 82 | 311.2 (ESI) | |
| 83 | 336.1 (ESI) | |
| 84 | 325.2 (ESI) | |
| 85 | 312.1 (ESI) | ¹H NMR (399 MHz, DMSO-d6) δ 11.80 (brs, 1H), 10.77 (s, 1H), 7.40 (d, J = 2.5 Hz, 1H), 7.14 (dt, J = 1.6, 7.5 Hz, 1H), 7.09 (dd, J = 2.6, 9.5 Hz, 1H), 7.02 (dd, J = 1.1, 8.0 Hz, 1H), 6.94 - 6.85 (m, 2H), 6.14 (d, J = 9.6 Hz, 1H), 4.54 (d, J = 10.0 Hz, 1H), 1.61 - 1.52 (m, 1H), 0.95 (d, J = 6.7 Hz, 3H), 0.85 (d, J = 6.7 Hz, 3H) |
| 86 | 285.2 (ESI) | |
| 109 | 263.4 (ESI) | |
| 110 | 277.3 (ESI) | ¹H NMR (500 MHz, DMSO-d6) δ 10.65 (s, 1H), 7.53 (d, J = 7.9 Hz, 1H), 7.21 (t, J = 7.5 Hz, 1H), 7.05 (t, J = 7.4 Hz, 1H), 7.00 (d, J = 7.8 Hz, 1H), 4.84 (d, J = 9.8 Hz, 1H), 4.59 (t, J = 5.1 Hz, 1H), 3.69 - 3.61 (m, 2H), 2.91 - 2.82 (m, 1H), 2.47 - 2.39 (m, 1H), 1.41 - 1.31 (m, 1H), 1.31 - 1.21 (m, 1H), 1.06 - 0.95 (m, 1H), 0.86 (d, J = 6.7 Hz, 3H), 0.73 (t, J = 7.5 Hz, 3H) |

### Example 5: Synthesis of Compound 77

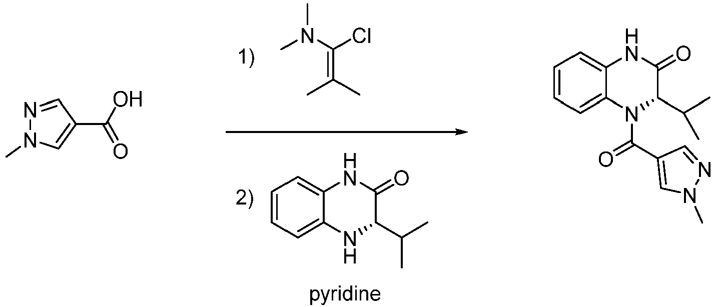

To a solution of 1-methyl-1H-pyrazole-4-carboxylic acid (150 mg, 1.2 mmol) in dichloroethane (2.0 mL) was added 1-chloro-*N*,*N*,2-trimethylprop-1-en-1-amine (170 µL, 1.3 mmol). The reaction mixture was stirred for 1 h, and (*S*)-3-isopropyl-3,4-dihydroquinoxalin-2(1*H*)-one (150 mg, 0.8 mmol) was added, followed by pyridine (190 µL, 2.4 mmol). This mixture was stirred overnight and then diluted with water and 20% MeOH/CHCl₃. The organic layer was separated, concentrated, and purified using amino silica gel chromatography (0-5% MeOH/CHCl₃) and silica gel chromatography (0-10% MeOH/CHCl₃) to give (*S*)-3-isopropyl-4-(1-methyl-1*H*-pyrazole-4-carbonyl)-3,4-dihydroquinoxalin-2(1*H*)-one (29 mg, 12%) as a colorless solid. 1H NMR (399 MHz, DMSO-d6) δ 10.76 (s, 1H), 7.78 (s, 1H), 7.20 (dt, J = 1.3, 7.6 Hz, 1H), 7.07 - 7.01 (m, 3H), 6.89 (dt, J = 1.3, 7.7 Hz, 1H), 4.59 (d, J = 10.1 Hz, 1H), 3.77 (s, 3H), 1.58 - 1.49 (m, 1H), 0.96 (d, J = 6.7 Hz, 3H), 0.84 (d, J = 6.7 Hz, 3H). LRMS (ESI) m/z 299.2 [M + H]⁺.

### Example 6: Synthesis of Compound 63

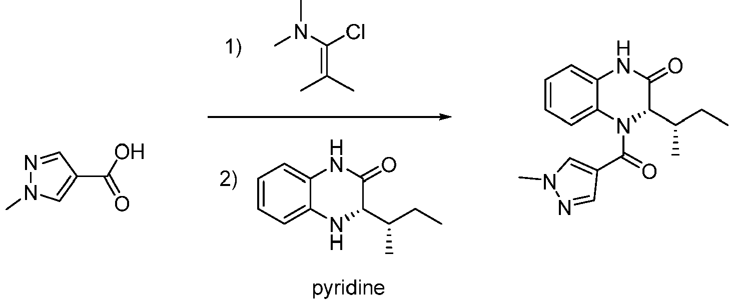

To a solution of 1-methyl-1*H*-pyrazole-4-carboxylic acid (140 mg, 1.1 mmol) in dichloroethane (2 mL) was added 1-chloro-*N*,*N*,2-trimethylprop-1-en-1-amine (160 µL, 1.2 mmol). The reaction mixture was stirred for 2 h at room temperature, and a solution of (*S*)-3-((*S*)-*sec*-butyl)-3,4-dihydroquinoxalin-2(1*H*)-one (150 mg, 0.7 mmol) in CH₂Cl₂ (2.0 mL) and pyridine (178 µL, 2.2 mmol) was added. This mixture was stirred at room temperature overnight and then diluted with water and CHCl₃. The organic layer was separated, concentrated, and purified using amino silica gel chromatography (0-5% MeOH/CHCl₃), and triturated with isopropyl ether/hexanes to give (S)-3-((S)-sec-butyl)-4-(1-methyl-1*H*-pyrazole-4-carbonyl)-3,4-dihydroquinoxalin-2(1*H*)-one (130 mg, 57%) as a colorless solid. ¹H NMR (500 MHz, DMSO-d6) δ 10.77 (s, 1H), 7.75 (s, 1H), 7.20 (dt, J = 1.3, 7.7 Hz, 1H), 7.05 (dd, J = 1.2, 8.0 Hz, 1H), 7.03 - 6.99 (m, 2H), 6.89 (dt, J = 1.2, 7.7 Hz, 1H), 4.68 (d, J = 10.1 Hz, 1H), 3.76 (s, 3H), 1.49 - 1.40 (m, 1H), 1.38 - 1.30 (m, 1H), 1.14 - 1.05 (m, 1H), 0.89 (d, J = 6.8 Hz, 3H), 0.76 (t, J = 7.4 Hz, 3H). LRMS (ESI) m/z 313.2 [M + H]⁺.

### Example 7: Synthesis of Compound 81

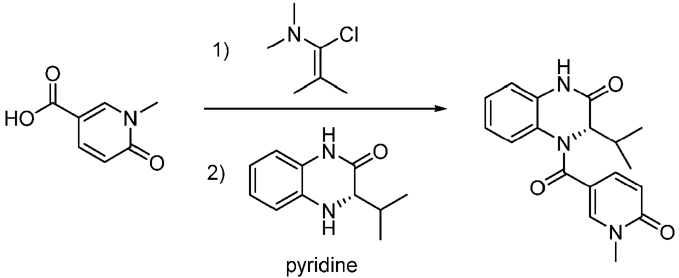

To a solution of 1-methyl-6-oxo-1,6-dihydropyridine-3-carboxylic acid (180 mg, 1.2 mmol) in dichloroethane (3 mL) was added 1-chloro-*N*,*N*,2-trimethylprop-1-en-1-amine (250 µL, 1.9 mmol). The reaction mixture was stirred for 1 h at room temperature, and then (*S*)-3-propyl-3,4-dihydroquinoxalin-2(1*H*)-one (200 mg, 1.1 mmol) and pyridine (260 µL, 3.2 mmol) were added. This mixture was stirred for 3 h, concentrated, and purified using silica gel chromatography (0-10% MeOH/CHCl₃), purified again using silica gel chromatography (0-75% EtOAc/CHCl₃), and triturated using isopropyl ether to give (S)-3-isopropyl-4-(1-methyl-6-oxo-1,6-dihydropyridine-3-carbonyl)-3,4-dihydroquinoxalin-2(1*H*)-one (281 mg, 82%) as a colorless solid. 1H NMR (500 MHz, DMSO-d6) δ 10.79 (s, 1H), 8.10 (d, J = 2.5 Hz, 1H), 7.14 (dt, J = 1.5, 7.5 Hz, 1H), 7.03 (dd, J = 1.2, 8.0 Hz, 1H), 6.91 (dd, J = 1.0, 7.9 Hz, 1H), 6.87 (dd, J = 1.3, 7.2 Hz, 1H), 6.84 (dd, J = 2.6, 9.5 Hz, 1H), 6.11 (d, J = 9.5 Hz, 1H), 4.56 (d, J = 10.0 Hz, 1H), 3.31 (s, 3H), 1.60 - 1.52 (m, 1H), 0.96 (d, J = 6.7 Hz, 3H), 0.85 (d, J = 6.7 Hz, 3H). LRMS (ESI) m/z 326.1 [M + H]⁺.

### Example 8: Synthesis of Compound 99

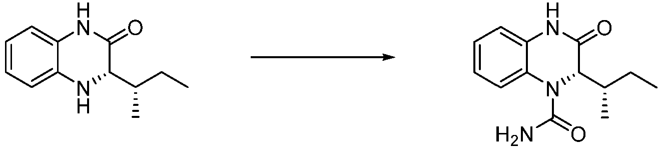

To an ice-cooled solution of (*S*)-3-((*S*)-*sec*-butyl)-3,4-dihydroquinoxalin-2(1*H*)-one (685 mg, 3.4 mmol) in THF (7 mL) was added triphosgene (400 mg, 1.4 mmol). After stirring for 30 min, 7 M NH₃/MeOH (2 mL, 14 mmol) was added to the reaction mixture. The reaction mixture stirred for 30 min at ice-bath temperature, and was then quenched with water, and extracted with chloroform. The organic layer was separated, dried over MgSO₄, concentrated, and purified using silica gel chromatography (0-10% MeOH/CHCl₃) to give (*S*)-2-((*S*)*-sec-*butyl)-3-oxo-3,4-dihydroquinoxaline-1(2*H*)-carboxamide (549 mg, 66%) as a colorless solid. 1H NMR (500 MHz, DMSO-d6) δ 10.56 (s, 1H), 7.40 (dd, J = 1.2, 8.0 Hz, 1H), 7.10 (dt, J = 1.4, 7.7 Hz, 1H), 7.01 (dt, J = 1.5, 7.7 Hz, 1H), 6.94 (dd, J = 1.4, 7.8 Hz, 1H), 6.35 (s, 2H), 4.50 (d, J = 10.1 Hz, 1H), 1.44 - 1.36 (m, 1H), 1.24 - 1.15 (m, 1H), 1.09 - 1.00 (m, 1H), 0.85 (d, J = 6.7 Hz, 3H), 0.73 (t, J = 7.5 Hz, 3H). LRMS (ESI) m/z 248.2 [M + H]⁺.

### Example 9: Synthesis of Compound 132

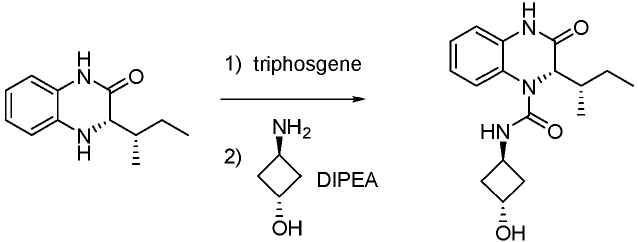

To a solution of (S)-3-((S)-sec-butyl)-3,4-dihydroquinoxalin-2(1*H*)-one (250 mg, 1.2 mmol) in THF (3 mL) was added DIPEA (0.2 mL, 1.2 mmol) and triphosgene (145 mg, 0.5 mmol). After stirring at room temperature for 30 min, this solution was added to a mixture of (1r,3r)-3-aminocyclobutan-1-ol (225 mg, 1.82 mmol), DIPEA (0.42 mL, 0.25 mmol), and THF (3 mL). The reaction mixture was stirred at room temperature overnight, quenched with water, and extracted with chloroform. The organic layer was separated, concentrated, and purified using silica gel chromatography (0-10% MeOH/CHCl₃) to give the crude product. The crude product was triturated with IPA and collected to yield pure (*S*)-2-((*S*)-*sec*-butyl)-*N-*((1*R*,3*S*)-3-hydroxycyclobutyl)-3-oxo-3,4-dihydroquinoxaline-1(2*H*)-carboxamide (211 mg, 54%) as a colorless solid. 1H NMR (500 MHz, DMSO-d6) δ 10.56 (s, 1H), 7.36 (dd, J = 1.1, 8.0 Hz, 1H), 7.09 (dt, J = 1.4, 7.6 Hz, 1H), 7.02 (dt, J = 1.5, 7.7 Hz, 1H), 6.95 (dd, J = 1.5, 7.8 Hz, 1H), 6.75 (d, J = 6.7 Hz, 1H), 4.88 (d, J = 5.2 Hz, 1H), 4.46 (d, J = 10.0 Hz, 1H), 4.27 - 4.15 (m, 2H), 2.24 - 2.18 (m, 1H), 2.15 - 2.08 (m, 1H), 2.08 - 1.99 (m, 2H), 1.43 - 1.34 (m, 1H), 1.24 - 1.15 (m, 1H), 1.08 - 0.99 (m, 1H), 0.84 (d, J = 6.7 Hz, 3H), 0.72 (t, J = 7.4 Hz, 3H). LRMS (ESI) m/z 318.3 [M + H]⁺.

The following compounds were prepared by methods analogous to the method described for Compound 132:

| Compound No. | LRMS *m*/*z* [M+H] | ¹H NMR |
|---|---|---|
| 8 | 325.3 (ESI) | |
| 46 | 316.3 (ESI) | |
| 47 | 311.2 (ESI) | |
| 48 | 311.2 (ESI) | |
| 49 | 324.2 (ESI) | |
| 50 | 338.3 (ESI) | |
| 51 | 330.3 (ESI) | |
| 52 | 336.2 (ESI) | |
| 53 | 340.2 (ESI) | |
| 54 | 340.2 (ESI) | |
| 55 | 340.2 (ESI) | |
| 56 | 328.2 (ESI) | |
| 57 | 328.2 (ESI) | |
| 58 | 328.2 (ESI) | |
| 59 | 314.2 (ESI) | |
| 60 | 324.2 (ESI) | |
| 61 | 324.2 (ESI) | |
| 62 | 324.2 (ESI) | |
| 101 | 304.2 (ESI) | |
| 102 | 262.4 (ESI) | ¹H NMR (500 MHz, DMSO-d6) δ 10.56 (s, 1H), 7.36 (dd, J = 1.2, 8.0 Hz, 1H), 7.11 (dt, J = 1.4, 7.7 Hz, 1H), 7.02 (dt, J = 1.5, 7.7 Hz, 1H), 6.95 (dd, J = 1.4, 7.9 Hz, 1H), 6.51 (q, J = 4.3 Hz, 1H), 4.50 (d, J = 10.1 Hz, 1H), 2.61 (d, J = 4.4 Hz, 3H), 1.44 - 1.35 (m, 1H), 1.24 - 1.15 (m, 1H), 1.09 - 1.00 (m, 1H), 0.84 (d, J = 6.8 Hz, 3H), 0.73 (t, J = 7.4 Hz, 3H) |
| 103 | 276.4 (ESI) | |
| 104 | 302.4 (ESI) | |
| 105 | 292.4 (ESI) | |
| 106 | 318.4 (ESI) | |
| 107 | 332.4 (ESI) | |
| 108 | 332.3 (ESI) | |
| 111 | 318.4 (ESI) | |
| 112 | 306.3 (ESI) | |
| 114 | 332.4 (ESI) | |
| 115 | 320.4 (ESI) | |
| 116 | 346.4 (ESI) | |
| 118 | 324.4 (ESI) | |
| 119 | 332.4 (ESI) | |
| 120 | 345.4 (ESI) | |
| 121 | 320.4 (ESI) | |
| 122 | 318.4 (ESI) | |
| 123 | 304.4 (ESI) | |
| 124 | 312.3 (ESI) | |
| 125 | 326.4 (ESI) | |
| 127 | 317.4 (ESI) | |
| 128 | 361.4 (ESI) | |
| 129 | 344.4 (ESI) | |
| 129 | 331.4 (ESI) | |
| 130 | 375.5 (ESI) | |
| 131 | 306.4 (ESI) | |
| 133 | 318.2 (ESI) | |

### Example 10: Synthesis of Compound 92

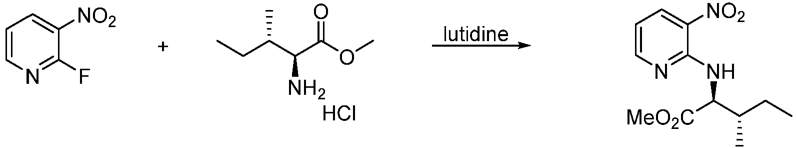

**Step 1: Methyl (3-nitropyridin-2-yl)-*L*-isoleucinate.** To a 100-mL round bottom flask was added 2-fluoro-3-nitropyridine (5.0 g, 35.2 mmol), 2,6-lutidine (10.2 mL, 88.0 mmol), methyl L-isoleucinate (12.8 g, 70.4 mmol) and DMF (50 mL). The reaction mixture was stirred for 4 d, followed by dilution with water (300 mL) and extracted with Et₂O (4x100 mL). The organic layers were combined, dried over sodium sulfate, filtered through a pad of celite, and concentrated to give 9.5 g of methyl (3-nitropyridin-2-yl)-L-isoleucinate as a bright yellow oil.

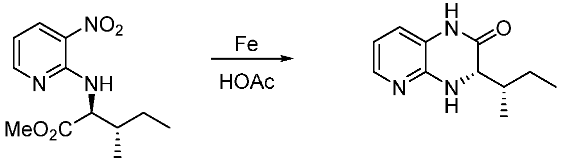

**Step 2: (*S*)-3-((*S*)-*sec*-Butyl)-3,4-dihydropyrido[2,3-b]pyrazin-2(1*H*)-one.** Iron powder (9.4 g) was added to a stirring solution of methyl (3-fluoro-2-nitrophenyl)-L-isoleucinate (9.00 g, 33.6 mmol) in AcOH (100 mL) at room temperature. After 2h, the reaction was filtered through a pad of celite, concentrated, and azeotroped with cyclohexane (100 mL) and toluene (100 mL) to give (*S*)-3-((*S*)-*sec*-butyl)-3,4-dihydropyrido[2,3-b]pyrazin-2(1*H*)-one as a red solid.

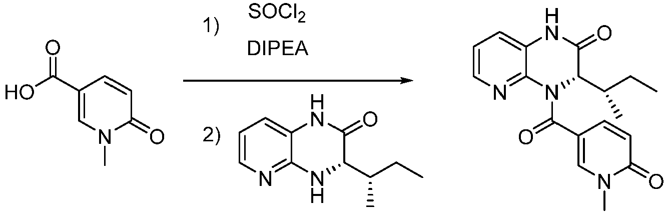

**Step 3: (*S*)-3-((*S*)-*sec*-Butyl)-4-(1-methyl-6-oxo-1,6-dihydropyridine-3-carbonyl)-3,4-dihydropyrido[2,3-b]pyrazin-2(1*H*)-one.** Thionyl chloride (0.87 g, 7.3 mmol) was added to a stirring solution of 1-methyl-6-oxo-1,6-dihydropyridine-3-carboxylic acid (560 mg, 3.7 mmol), DMF (1 drop), and CH₂Cl₂ (10 mL) at room temperature. After 2 h, the reaction was concentrated, followed by the sequential addition of NMP (5 mL), (*S*)-3-((*S*)-*sec*-butyl)-3,4-dihydropyrido[2,3-b]pyrazin-2(1*H*)-one (250 mg, 1.2 mmol), and DIPEA (0.48 g, 3.7 mmol). The reaction stirred for 2 h, quenched with MeOH (10 mL), and stirred for 5 min. The mixture was concentrated, filtered through a 0.4 µm syringe filter, and purified using reverse phase HPLC (0-30% MeCN/H₂O w/ 0.1% formic acid) to give 115 mg of (*S*)-3-((*S*)-*sec*-butyl)-4-(1-methyl-6-oxo-1,6-dihydropyridine-3-carbonyl)-3,4-dihydropyrido[2,3-b]pyrazin-2(1*H*)-one as a white solid. 1H NMR (400 MHz, Methanol-d4) δ 7.90 (d, J = 2.6 Hz, 1H), 7.76 (dd, J = 4.9, 1.6 Hz, 1H), 7.33 (dd, J = 7.9, 1.6 Hz, 1H), 7.12 (dd, J = 8.0, 4.9 Hz, 1H), 6.86 (dd, J = 9.4, 2.6 Hz, 1H), 6.13 (d, J = 9.5 Hz, 1H), 4.78 (s, 1H), 3.44 (s, 3H), 1.58 - 1.32 (m, 2H), 1.31 - 1.02 (m, 2H), 0.86 (d, J = 6.7 Hz, 3H), 0.75 (t, J = 7.4 Hz, 3H). LRMS (APCI) m/z 341.0 [M + H]⁺.

The following compounds were prepared by methods analogous to the method described for Compound 92:

| Compound No. | LRMS *m*/*z* [M+H] |
|---|---|
| 88 | 300 (APCI) |
| 89 | 314 (APCI) |
| 90 | 327 (APCI) |
| 91 | 341 (APCI) |
| 92 | 341 (APCI) |
| 93 | 314 (APCI) |
| 96 | 314 (APCI) |
| 97 | 310 (APCI) |
| 98 | 324 (APCI) |

### Example 11: Synthesis of Compound 94

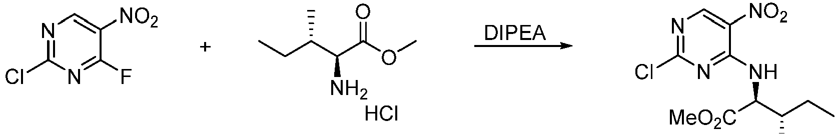

**Step 1: Methyl (2-chloro-5-nitropyrimidin-4-yl)-*L*-isoleucinate.** Methyl *L-*isoleucinate (5.8 g, 32.2 mmol) was slowly added to a stirring solution of 2-chloro-4-fluoro-5-nitropyrimidine (5.0 g, 25.8 mmol), DIPEA (13.5 mL, 77.3 mmol), and DMF (50 mL). After 30 min, the reaction was poured into water (300 mL) and extracted with Et₂O (4x100 mL). The organic layers were combined, dried over sodium sulfate, concentrated, and purified using silica gel chromatography (0-15% EtOAc/hexanes) to give 2.75 g of methyl (2-chloro-5-nitropyrimidin-4-yl)-*L*-isoleucinate as a yellow oil.

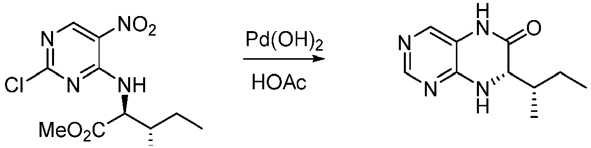

**Step 2: (*S*)-7-((*S*)-*sec*-butyl)-7,8-dihydropteridin-6(5*H*)-one.** Methyl (2-chloro-5-nitropyrimidin-4-yl)-*L*-isoleucinate, Pearlman's catalyst, and MeOH (50 mL) were mixed and stirred under a hydrogen atmosphere (551.58 kPa (80 psi)) at room temperature for 4 h. The reaction was then filtered through a pad of celite and concentrated. Acetic acid was added, and the mixture was heated to 100 °C and stirred for 2 h. The reaction was then cooled to room temperature, concentrated, suspended in 10% MeOH/CH₂Cl₂, and filtered through a pad of silica to give 1.5 g of (*S*)-7-((*S*)-*sec*-butyl)-7,8-dihydropteridin-6(5*H*)-one.

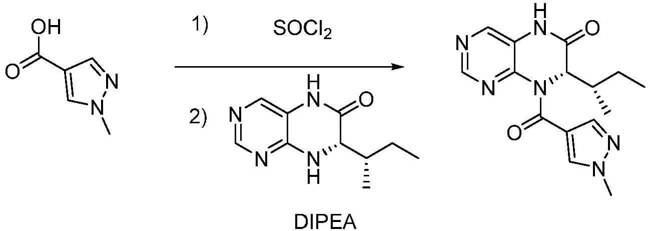

**Step 3: (*S*)-7-((*S*)-*sec*-butyl)-8-(1-methyl-1H-pyrazole-4-carbonyl)-7,8-dihydropteridin-6(5*H*)-one.** Thionyl chloride (0.58 g, 4.8 mmol) was added to a stirring solution of 1-methyl-1*H*-pyrazole-4-carboxylic acid (0.46 g, 3.6 mmol), DMF (1 drop), and CH₂Cl₂ (10 mL) at room temperature. After 2 h, the reaction was concentrated, followed by the sequential addition of NMP (2 mL), (*S*)-7-((*S*)-*sec*-butyl)-7,8-dihydropteridin-6(5*H*)-one (250 mg, 1.2 mmol), and DIPEA (0.8 g, 6.1 mmol). The reaction stirred for 4 h, quenched with a methylamine/MeOH solution, and stirred for 5 min. The mixture was concentrated, filtered through a 0.4 µm syringe filter, and purified using reverse phase HPLC (0-30% MeCN/H₂O w/ 0.1% formic acid) to give 55 mg of (*S*)-7-((*S*)-*sec*-butyl)-8-(1-methyl-1H-pyrazole-4-carbonyl)-7,8-dihydropteridin-6(5*H*)-one as a white solid. 1H NMR (400 MHz, Methanol-d4) δ 8.35 (s, 1H), 8.26 (s, 1H), 7.72 (s, 1H), 7.25 (s, 1H), 4.73 (s, 1H), 3.74 (s, 3H), 1.50 (tq, J = 10.3, 4.2, 2.9 Hz, 2H), 1.14 (ddt, J = 15.9, 8.5, 4.3 Hz, 1H), 0.83 (d, J = 6.5 Hz, 3H), 0.78 (t, J = 7.2 Hz, 3H). LRMS (APCI) m/z 315.0 [M + H]⁺.

Compound 125 was prepared by using an analogous method:

| Compound No. | LRMS *m*/*z* [M+H] |
|---|---|
| 125 | 263.3 (ESI) |

### Example 12: Synthesis of Compound 95

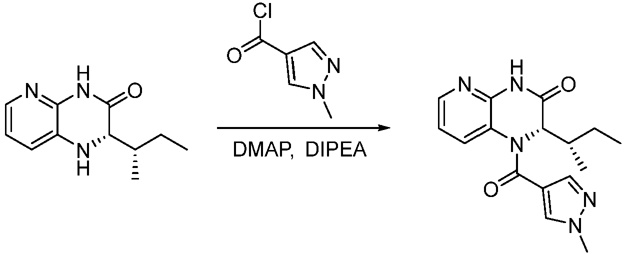

To a stirring solution of 1-methyl-1*H*-pyrazole-4-carbonyl chloride (0.63 g, 4.4 mmol) was added a mixture of (*S*)-2-((*S*)-*sec*-butyl)-1,4-dihydropyrido[2,3-b]pyrazin-3(2H)-one (300 mg, 1.5 mmol), DIPEA (0.8 mL, 5.8 mmol), DMAP (18 mg, 0.1 mmol), and NMP (5 mL). The reaction was stirred for 2 h at room temperature, quenched with methylamine/MeOH, and stirred for 5 min. The mixture was concentrated, filtered through a 0.4 µm syringe filter, and purified using reverse phase HPLC (0-30% MeCN/H₂O w/ 0.1% formic acid) to give (*S*)-2-((*S*)-*sec*-butyl)-1-(1-methyl-1*H*-pyrazole-4-carbonyl)-1,4-dihydropyrido[2,3-b]pyrazin-3(2*H*)-one as a white solid. 1H NMR (400 MHz, Methanol-d4) δ 8.09 (dd, J = 4.9, 1.4 Hz, 1H), 7.64 (s, 1H), 7.40 (d, J = 7.9 Hz, 1H), 7.19 (s, 1H), 6.87 (dd, J = 8.0, 4.9 Hz, 1H), 4.79 (d, J = 9.6 Hz, 1H), 3.74 (s, 3H), 1.46 (dqd, J = 15.0, 7.5, 3.7 Hz, 1H), 1.35 (tdd, J = 9.7, 6.4, 3.2 Hz, 1H), 1.19 - 1.00 (m, 1H), 0.89 (d, J = 6.7 Hz, 3H), 0.75 (t, J = 7.4 Hz, 3H). LRMS (APCI) m/z 314.0 [M + H]⁺.

### Example 13: Synthesis of Compounds 27-29 and 33-44

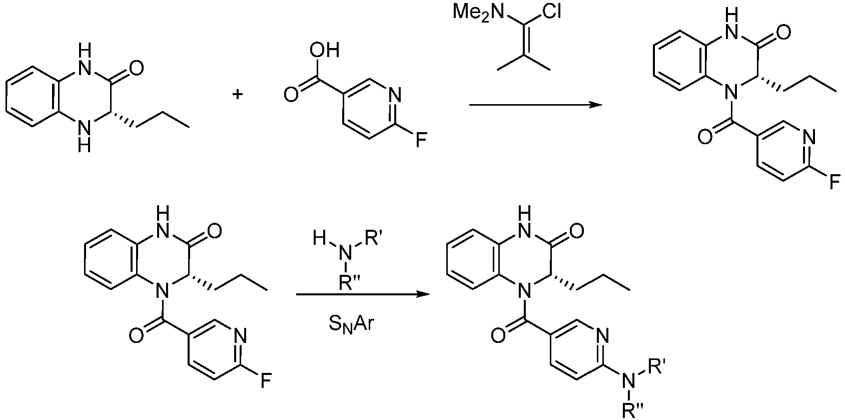

The following compounds were prepared by nucleophilic aromatic substitution (S_{N}Ar) by reacting (*S*)-4-(6-fluoronicotinoyl)-3-propyl-3,4-dihydroquinoxalin-2(1*H*)-one with the appropriate amine:

| Compound No. | LRMS *m*/*z* [M+H] | Amine 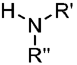 |
|---|---|---|
| 27 | 311.2 (ESI) | NH₃ |
| 28 | 339.3 (ESI) | 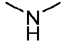 |
| 29 | 379.3 (ESI) | 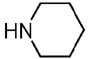 |
| 33 | 353.3 (ESI) | 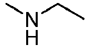 |
| 34 | 393.2 (ESI) | 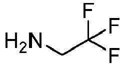 |
| 35 | 393.3 (ESI) | 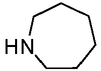 |
| 36 | 401.3 (ESI) | 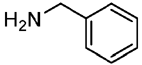 |
| 37 | 365.3 (ESI) | 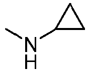 |
| 38 | 369.3 (ESI) | 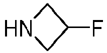 |
| 39 | 415.3 (ESI) | 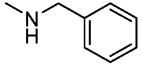 |
| 40 | 381.3 (ESI) | 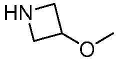 |
| 41 | 383.3 (ESI) | 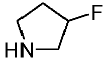 |
| 42 | 387.3 (ESI) | 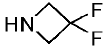 |
| 43 | 401.3 (ESI) | 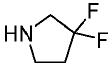 |
| 44 | 421.3 (ESI) | 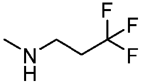 |

Additional compounds can be prepared by nucleophilic aromatic substitution (S_{N}Ar) by reacting the appropriate fluoroaryl or fluoroheteroaryl intermediate with the appropriate amine. The reaction can be conducted in the presence of quantitative or excess amine at elevated temperatures, for example >100 °C, using a polar aprotic solvent such as NMP, DMSO, DMF, dioxanes, THF, or no solvent at all.

### Biological Example 1

### Assay Example 1: Preparation and assay of fast skeletal myofibrils

Preparation of fast skeletal myofibrils: Rabbit skeletal myofibrils were prepared based upon the method of Herrmann et al. (Biochem. 32(28):7255-7263(1993). Myofibrils were prepared from rabbit psoas muscle purchased from Pel-Freez Biologicals (Arkansas) within 2 days of ordering, stored on ice. Minced muscle was homogenized in 10 volumes of ice-cold "standard" buffer (50 mM Tris, pH 7.4, 0.1 M KOAc, 5 mM KCl, 2 mM dithiothreitol (DTT), 0.2 mM phenylmethylsulfonyl fluoride (PMSF), 10 µM leupeptin, 5 µM pepstatin, and 0.5 mM sodium azide) containing 5 mM ethylenediaminetetraacetic acid (EDTA) and 0.5% Triton X-100 using an Omni-Macro homogenizer. Myofibrils were recovered by low speed centrifugation (3000 rpm for 10 minutes) and washed 2 times in the Triton X-100 containing buffer to ensure removal of cellular membrane. Following the Triton washes, myofibrils were washed 3 times in "standard" buffer containing 2 mM magnesium acetate. A final wash in assay buffer (12 mM piperazine-1,4-bis(2-ethanesulfonic acid) (PIPES), pH 6.8, 60 mM KCl, 1 mM DTT) was performed and brought to 10% sucrose for flash freezing in liquid nitrogen and storage at -80°C.

Activation of Fast Skeletal Myofibrils: Fast fiber activators were identified by measuring the enzymatic activity of muscle myofibril preparations using the proprietary PUMA (trademark) (see, e.g., U.S. Patent Nos. 6,410,254, 6,743,599, 7,202,051, and 7,378,254) assay system. Myofibril preparations consisted of rabbit skeletal muscle (approximately 90% fast fibers) that had been mechanically homogenized and washed with a detergent (Triton X-100) to remove cellular membranes. This preparation retained all of the sarcomeric components in a native conformation and the enzymatic activity was still regulated by calcium. Compounds were tested using a myofibril suspension and a level of calcium sufficient to increase enzymatic activity of the myofibrils to 25% of their maximal rate (termed pCa25). Enzymatic activity was tracked via a pyruvate kinase and lactate dehydrogenase-coupled enzyme system. This assay regenerates myosin-produced ADP into ATP by oxidizing NADH, producing an absorbance change at 340 nm. The buffering system was 12 mM PIPES, 2 mM MgCl₂, 1 mM DTT at pH 6.8 (PM12 buffer). Data were reported as AC1.4, which is the concentration at which the compound increased the enzymatic activity by 40%. In Table 3, "Example Compound" refers to the compound labeled with the same number in Table 2.

**Table 3**

| Example Compound | AC1.4 (µM) | Example Compound | AC1.4 (µM) |
|---|---|---|---|
| 1 | 0.45 | 2 | 0.71 |
| 3 | 0.77 | 4 | 0.72 |
| 5 | 0.38 | 6 | 2.19 |
| 7 | 0.96 | 8 | 2.73 |
| 9 | 0.74 | 10 | 0.33 |
| 11 | 0.97 | 12 | 1.55 |
| 13 | 0.13 | 14 | 1.66 |
| 15 | 0.17 | 16 | 1.41 |
| 17 | 0.17 | 18 | 2.97 |
| 19 | 1.46 | 20 | 1.52 |
| 21 | 0.07 | 22 | 2.76 |
| 23 | 1.33 | 24 | 0.4 |
| 25 | 2.34 | 26 | 0.56 |
| 27 | 0.76 | 28 | 1.42 |
| 29 | 2.7 | 30 | 0.42 |
| 31 | 0.41 | 32 | 0.71 |
| 33 | 0.95 | 34 | 2.59 |
| 35 | 1.32 | 36 | 0.65 |
| 37 | 0.58 | 38 | 1.58 |
| 39 | 0.27 | 40 | 2.37 |
| 41 | 1.63 | 42 | 2.34 |
| 43 | 2.78 | 44 | 2.61 |
| 45 | 0.2 | 46 | 0.47 |
| 47 | 1.51 | 48 | 1.08 |
| 49 | 2.08 | 50 | 2.36 |
| 51 | 0.48 | 52 | 0.58 |
| 53 | 0.55 | 54 | 0.31 |
| 55 | 1.88 | 56 | 0.42 |
| 57 | 0.24 | 58 | 0.26 |
| 59 | 1.96 | 60 | 0.82 |
| 61 | 0.43 | 62 | 1.39 |
| 63 | 0.04 | 64 | 0.24 |
| 65 | 0.39 | 66 | 0.17 |
| 67 | 1.31 | 68 | 0.16 |
| 69 | 1.52 | 70 | 0.46 |
| 71 | 0.54 | 72 | 0.07 |
| 73 | 0.11 | 74 | 0.08 |
| 75 | 0.05 | 76 | 0.47 |
| 77 | 0.08 | 78 | 0.16 |
| 79 | 0.88 | 80 | 0.63 |
| 81 | 0.22 | 82 | 0.26 |
| 83 | 0.06 | 84 | 0.29 |
| 85 | 0.15 | 86 | 0.2 |
| 87 | 2.15 | 88 | 0.85 |
| 89 | 0.23 | 90 | 0.71 |
| 91 | 0.72 | 92 | 1.03 |
| 93 | 1.73 | 94 | 1.66 |
| 95 | 0.84 | 96 | 0.6 |
| 97 | 0.28 | 98 | 0.13 |
| 99 | 0.87 | 100 | 1.58 |
| 101 | 2.1 | 102 | 0.94 |
| 103 | 0.78 | 104 | 1.31 |
| 105 | 2.23 | 106 | 1.77 |
| 107 | 0.44 | 108 | 2.77 |
| 109 | 1.51 | 110 | 1.18 |
| 111 | 2.06 | 112 | 1.01 |
| 113 | 2.01 | 114 | 1.69 |
| 115 | 2.29 | 116 | 0.83 |
| 117 | 1.24 | 118 | 1.01 |
| 119 | 0.89 | 120 | 1.5 |
| 121 | 2.44 | 122 | 1.37 |
| 123 | 1.2 | 124 | 1.09 |
| 125 | 1.25 | 126 | 1.53 |
| 127 | 1.52 | 128 | 0.84 |
| 129 | 1.3 | 130 | 2.13 |
| 131 | 2.15 | 132 | 0.64 |
| 133 | 1.68 | | |

### Assay Example 2: Preparation and assay of rat isometric ankle plantarflexor muscle force

Female Sprague Dawley rats were placed under a stable anesthesic plane with inhaled isoflurane (1-5%). One incision was made on the mid-thigh region of the right leg to expose the sciatic nerve. To prevent co-contraction of the ankle dorsiflexors, an additional incision was made lateral to the patella to isolate and sever the peroneal nerve. Rats were then placed on a temperature-maintained *in situ* muscle analysis rig (Aurora Scientific, Model 806C). The knee was immobilized in a clamp between two sharpened screws and the foot was taped to a footplate attached to a force transducer (Aurora Scientific, Ontario, Canada). Stainless steel needle electrodes (0.10 mm) were hooked around the exposed sciatic nerve. Isometric ankle plantarflexor muscle contractile force was assessed with the ankle joint at 90° flexion. A 30 Hz electrical stimulation (under supramaximal voltage conditions) was applied to the nerve and the resulting muscle force was recorded via a servomotor. A pre-dose 30Hz force response was established as the baseline force. A pre-dose 150 Hz force response was established as the maximum isometric force. Compounds were formulated in 50% polyethylene glycol (PEG): 16% Cavitron: 10% dimethylacetamide (DMA) and administered by continuous intravenous infusion over a sixty minute period. The muscle force response to compound was measured every two minutes over the dosing period. Data were reported as an estimated EC₅₀ value, which is the concentration at which muscle force is 50% of the pre-dose maximum tension. The EC₅₀ results are summarized in Table 4 below. In Table 4, "Example Compound" refers to the compound labeled with the same number in Table 2.

**Table 4**

| Example Compound | PLANTARFLEXOR EC₅₀ (µM) |
|---|---|
| 13 | 15.7 |
| 88 | 20.1 |
| 89 | 23.9 |

## Claims

1. A compound of formula (I): 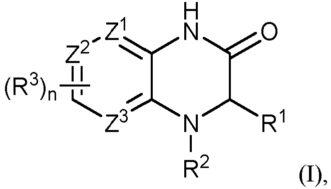 or a pharmaceutically acceptable salt thereof, wherein:
Z¹, Z², and Z³ are, independently of each other, CH, CR³, or N, provided that at least one of Z¹, Z², and Z³ is CH or CR³;
R¹ is unsubstituted C₃-C₄ alkyl;
R² is -C(=O)R⁴, -C(=O)-(CH₂)ₚ-R⁵, -C(=O)NR⁶R⁷, or -C(=O)OCH₃;
each R³ is independently halogen, C₁-C₆-alkyl, C₁-C₆-haloakyl, C₁-C₆-alkoxy, C₁-C₆-haloakoxy, C₃-C₁₂-cycloalkyl, or 3-12 membered heterocycloalkyl;
R⁴ is C₆-C₁₂-aryl optionally substituted with one to five R^{4A}, 5-12 membered heteroaryl optionally substituted with one to five R^{4B}, C₃-C₁₂ cycloalkyl optionally substituted with one to five R^{4C}, 4-12 membered heterocycloalkyl optionally substituted with one to five R^{4D}, or 4-12 membered heterocycloalkenyl optionally substituted with one to five R^{4E};
each R^{4A} is independently selected from the group consisting of halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), C₆-C₁₂-aryl, and 5-12 membered heteroaryl;
each R^{4B} is independently selected from the group consisting of halo, C₁-C₆-alkyl, C₁-C₆-haloalkyl, OH, C₁-C₆-alkoxy, C₁-C₆-haloalkoxy, NH₂, NH(C₁-C₆-alkyl), N(C₁-C₆-alkyl)₂, NH(C₁-C₆-haloalkyl), N(C₁-C₆-alkyl)(C₁-C₆-haloalkyl), N(C₁-C₆-haloalkyl)₂, NH(C₁-C₆-alkylene-(C₆-C₁₂-aryl)), N(C₁-C₆-alkyl)(C₁-C₆-alkylene-(C₆-C₁₂-aryl)), N(C₁-C₆-alkylene-(C₆-C₁₂-aryl))₂, NH(C₃-C₁₂ cycloalkyl), N(C₁-C₆-alkyl)(C₃-C₁₂ cycloalkyl), N(C₃-C₁₂ cycloalkyl)₂, NHC(O)-C₁-C₆-alkyl, C₃-C₁₂ cycloalkyl optionally substituted with one to five R^{4B1}, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), C₁-C₆-alkylene-(4-12 membered heterocycloalkyl), and 5-12 membered heteroaryl;
each R^{4B1} is independently selected from the group consisting of halo, C₁-C₆-alkyl, C₁-C₆-haloalkyl, OH, C₁-C₆-alkoxy, C₁-C₆-haloalkoxy, and C₁-C₆-alkylene-OH;
each R^{4C} is independently selected from the group consisting of halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
each R^{4D} is independently selected from the group consisting of halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
each R^{4E} is independently selected from the group consisting of oxo, halo, C₁-C₆-alkyl, C₁-C₆-haloalkyl, OH, C₁-C₆-alkoxy, C₁-C₆-haloalkoxy, NHC(O)-C₁-C₆-alkyl, and C₁-C₆-alkylene-OH;
R⁵ is OH, C₁-C₆-alkoxy, C₁-C₆-haloalkoxy, C₃-C₁₂ cycloalkyl, 3-12 membered heterocycloalkyl, or C₆-C₁₂-aryl;
R⁶ and R⁷, independently of each other, are selected from the group consisting of H, C₁-C₆-alkyl, C₁-C₆-haloalkyl, C₃-C₁₂ cycloalkyl optionally substituted with one to five R⁸, 4-12 membered heterocycloalkyl optionally substituted with one to five R⁸, C₆-C₁₂-aryl optionally substituted with one to five R⁸, 5-12 membered heteroaryl optionally substituted with one to five R⁸, C₁-C₆-alkylene-OH, C₁-C₆-alkylene-(C₃-C₁₂ cycloalkyl), C₁-C₆-alkylene-(4-12 membered heterocycloalkyl), C₁-C₆-alkylene-(C₆-C₁₂-aryl), and C₁-C₆-alkylene-(5-12 membered heteroaryl);
each R⁸ is independently selected from the group consisting of halo, C₁-C₆-alkyl, C₁-C₆-haloalkyl, OH, C₁-C₆-alkoxy, C₁-C₆-alkylene-OH, and C₁-C₆-haloalkoxy;
n is 0, 1, 2, 3, or 4;
p is 1, 2, or 3; and
provided that the compound of Formula (I) is not a compound selected from the group consisting of:
4-(3-methylbenzoy1)-3-propy1-3,4-dihydroquinoxalin-2(1H)-one,
4-(2-chlorobenzoyl)-3-isobutyl-3,4-dihydroquinoxalin-2(1H)-one, and
4-(4-(tert-butyl)benzoy1)-3-propyl-3,4-dihydroquinoxalin-2(1H)-one.

2. The compound of claim 1, wherein the compound of formula (I) is a compound of formula (I-a), (I-b), (I-c), (I-d), or (I-e): 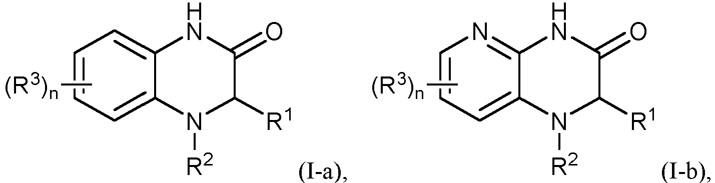 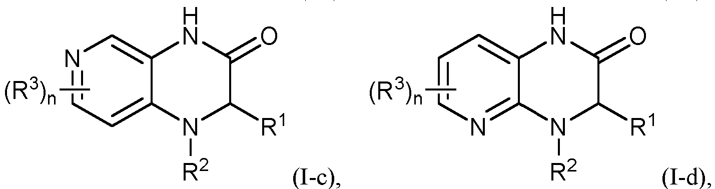 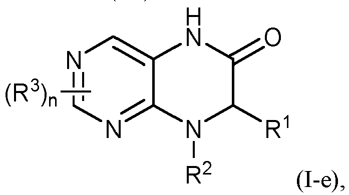 or a pharmaceutically acceptable salt thereof.

3. The compound of claim 1, wherein the compound of formula (I) is a compound of formula (II): 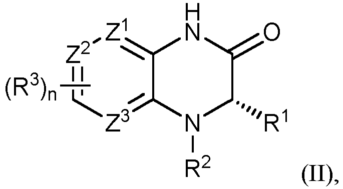 or a pharmaceutically acceptable salt thereof.

4. The compound of claim 3, wherein the compound of formula (II) is a compound of formula (II-a), (II-b), (II-c), (II-d), or (II-e): 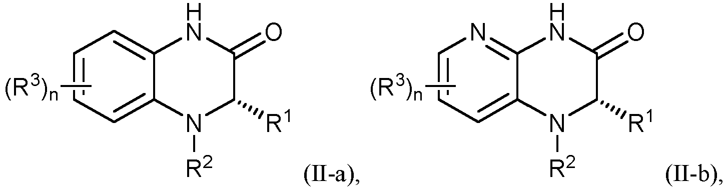 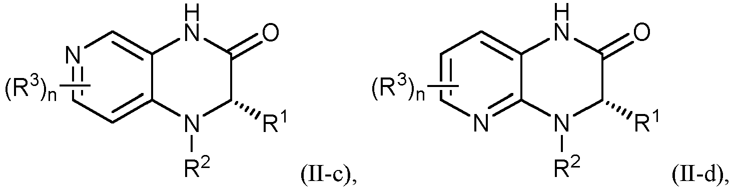 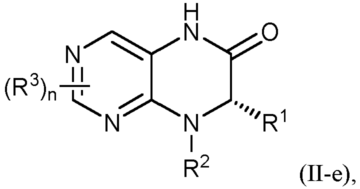 or a pharmaceutically acceptable salt thereof,
and/or wherein the compound of formula (II) is a compound of formula (II-a): 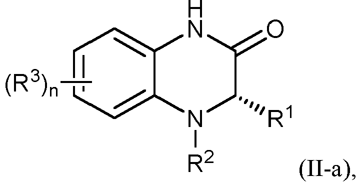
or a pharmaceutically acceptable salt thereof.

5. The compound of any one of claims 1-4, or a pharmaceutically acceptable salt thereof, wherein R¹ is selected from the group consisting of prop-1-yl, prop-2-yl, but-2-yl, and 2-methylprop-1-yl.

6. The compound of any one of claims 1-5, or a pharmaceutically acceptable salt thereof, wherein:
(a) R³ is halogen or C₃-C₁₂-cycloalkyl;
(b) R³ is halogen; or
(c) R³ is C₃-C₆-cycloalkyl.

7. The compound of any one of claims 1-6, or a pharmaceutically acceptable salt thereof, wherein n is 0 or 1.

8. The compound of any one of claims 1-7, or a pharmaceutically acceptable salt thereof, wherein:
(a) R² is -C(=O)R⁴, optionally wherein:
(a)(i) R⁴ is C₆-C₁₂-aryl optionally substituted with one to five R^{4A}, 5-12 membered heteroaryl optionally substituted with one to five R^{4B}, C₃-C₁₂ cycloalkyl, 4-12 membered heterocycloalkyl optionally substituted with one to five R^{4D}, or 4-12 membered heterocycloalkenyl optionally substituted with one to five R^{4E};
(a)(ii) R⁴ is C₆-C₁₂-aryl substituted with one to five R^{4A} and R^{4A} is halo or C₁-C₆-alkyl;
(a)(iii) R⁴ is 5-12 membered heteroaryl substituted with one to five R^{4B} and R^{4B} is selected from the group consisting of halo, C₁-C₆-alkyl, C₁-C₆-haloalkoxy, NH₂, N(C₁-C₆-alkyl)₂, NH(C₁-C₆-haloalkyl), N(C₁-C₆-alkyl)(C₁-C₆-haloalkyl), NH(C₁-C₆-alkylene-(C₆-C₁₂-aryl)), N(C₁-C₆-alkyl)(C₃-C₁₂ cycloalkyl), NHC(O)-C₁-C₆-alkyl, C₃-C₁₂ cycloalkyl, 4-12 membered heterocycloalkyl optionally substituted with one to five R^{4B1}, C₁-C₆-alkylene-OH, C₁-C₆-alkylene-CONH₂, C₁-C₆-alkylene-(C₃-C₁₂ cycloalkyl), and 5-12 membered heteroaryl, optionally wherein R^{4B} is 4-12 membered heterocycloalkyl substituted with one to five R^{4B1} and R^{4B1} is halo or C₁-C₆-alkoxy;
(a)(iv) R⁴ is 4-12 membered heterocycloalkyl substituted with one to five R^{4D} and R^{4D} is selected from the group consisting of halo, C₁-C₆-alkyl, OH, NHC(O)-C₁-C₆-alkyl, and C₁-C₆-alkylene-OH; or
(a)(v) R⁴ is 4-12 membered heterocycloalkenyl substituted with one to five R^{4E} and R^{4E} is oxo or C₁-C₆-alkyl; or
(b) R² is -C(=O)-(CH₂)ₚ-R⁵, optionally wherein:
(b)(i) R⁵ is OH or C₆-C₁₂-aryl; and/or
(b)(ii) p is 1 or 2; or
(c) R² is -C(=O)NR⁶R⁷, optionally wherein:
(c)(i) R⁶ and R⁷, independently of each other, are selected from the group consisting of H, C₁-C₆-alkyl, C₁-C₆-haloalkyl, C₃-C₁₂ cycloalkyl, C₆-C₁₂-aryl optionally substituted with one to five R⁸, 5-12 membered heteroaryl optionally substituted with one to five R⁸, C₁-C₆-alkylene-OH, C₁-C₆-alkylene-(C₃-C₁₂ cycloalkyl), C₁-C₆-alkylene-(C₆-C₁₂-aryl), and C₁-C₆-alkylene-(5-12 membered heteroaryl), optionally wherein each R⁸ is independently selected from the group consisting of halo, C₁-C₆-alkyl, C₁-C₆-alkylene-OH, and C₁-C₆-alkoxy; or
(d) R² is -C(=O)OCH₃.

9. A compound of claim 1 selected from the group consisting of compounds 1-133:
| Compound No. | Compound Structure | Compound Name |
|---|---|---|
| 1 | 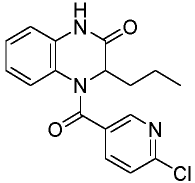 | 4-(6-chloronicotinoyl)-3-propyl-3,4-dihydroquinoxalin-2(1H)-one |
| 2 | 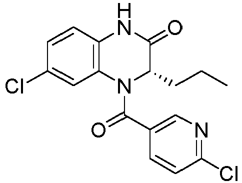 | (S)-6-chloro-4-(6-chloronicotinoyl)-3-propyl-3,4-dihydroquinoxalin-2(1H)-one |
| 3 | 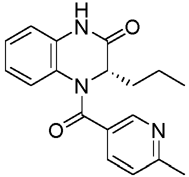 | (S)-4-(6-methylnicotinoyl)-3-propyl-3,4-dihydroquinoxalin-2(1H)-one |
| 4 | 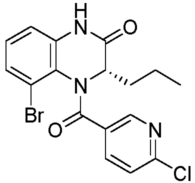 | (S)-5-bromo-4-(6-chloronicotinoyl)-3-propyl-3,4-dihydroquinoxalin-2(1H)-one |
| 5 | 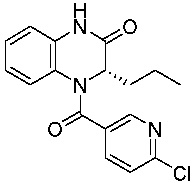 | (S)-4-(6-chloronicotinoyl)-3-propyl-3,4-dihydroquinoxalin-2(1H)-one |
| 6 | 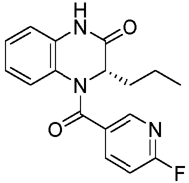 | (S)-4-(6-fluoronicotinoyl)-3-propyl-3,4-dihydroquinoxalin-2(1H)-one |
| 7 | 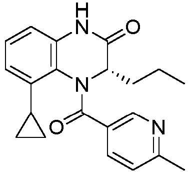 | (S)-5-cyclopropyl-4-(6-methylnicotinoyl)-3-propyl-3,4-dihydroquinoxalin-2(1H)-one |
| 8 | 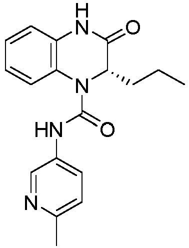 | (S)-N-(6-methylpyridin-3-yl)-3-oxo-2-propyl-3,4-dihydroquinoxaline-1 (2H)-carboxamide |
| 9 | 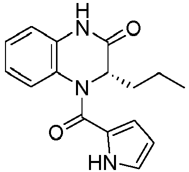 | (S)-3-propyl-4-(1H-pyrrole-2-carbonyl)-3,4-dihydroquinoxalin-2(1H)-one |
| 10 | 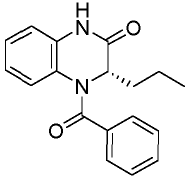 | (S)-4-benzoyl-3-propyl-3,4-dihydroquinoxalin-2(1H)-one |
| 11 | 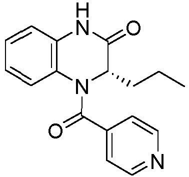 | (S)-4-isonicotinoyl-3-propyl-3,4-dihydroquinoxalin-2(1H)-one |
| 12 | 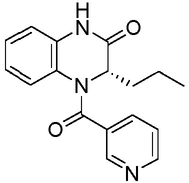 | (S)-4-nicotinoyl-3-propyl-3,4-dihydroquinoxalin-2(1H)-one |
| 13 | 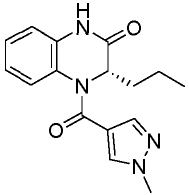 | (S)-4-(1-methyl-1H-pyrazole-4-carbonyl)-3-propyl-3,4-dihydroquinoxalin-2(1H)-one |
| 14 | 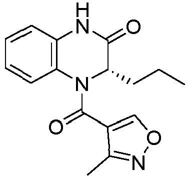 | (S)-4-(3-methylisoxazole-4-carbonyl)-3-propyl-3,4-dihydroquinoxalin-2(1H)-one |
| 15 | 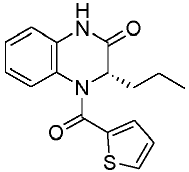 | (S)-3-propyl-4-(thiophene-2-carbonyl)-3,4-dihydroquinoxalin-2(1H)-one |
| 16 | 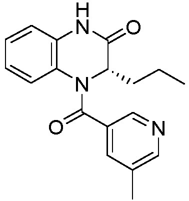 | (S)-4-(5-methylnicotinoyl)-3-propyl-3,4-dihydroquinoxalin-2(1H)-one |
| 17 | 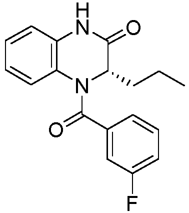 | (S)-4-(3-fluorobenzoyl)-3-propyl-3,4-dihydroquinoxalin-2(1H)-one |
| 18 | 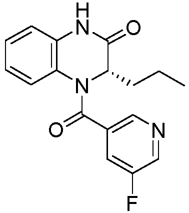 | (S)-4-(5-fluoronicotinoyl)-3-propyl-3,4-dihydroquinoxalin-2(1H)-one |
| 19 | 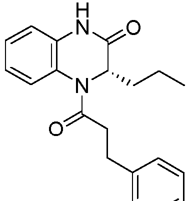 | (S)-4-(3-phenylpropanoyl)-3-propyl-3,4-dihydroquinoxalin-2(1H)-one |
| 20 | 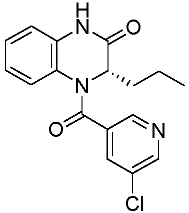 | (S)-4-(5-chloronicotinoyl)-3-propyl-3,4-dihydroquinoxalin-2(1H)-one |
| 21 | 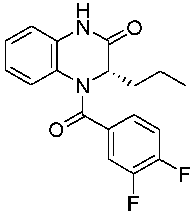 | (S)-4-(3,4-difluorobenzoyl)-3-propyl-3,4-dihydroquinoxalin-2(1H)-one |
| 22 | 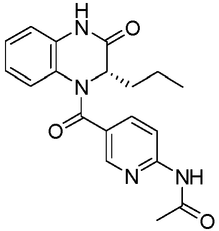 | (S)-N-(5-(3-oxo-2-propyl-1,2,3,4-tetrahydroquinoxaline-1-carbonyl)pyridin-2-yl)acetamide |
| 23 | 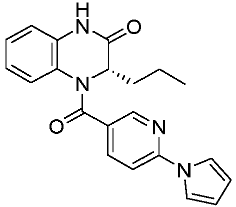 | (S)-4-(6-(1H-pyrrol-1-yl)nicotinoyl)-3-propyl-3,4-dihydroquinoxalin-2(1H)-one |
| 24 | 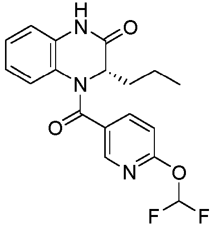 | (S)-4-(6-(difluoromethoxy)nicotinoyl)-3-propyl-3,4-dihydroquinoxalin-2(1H)-one |
| 25 | 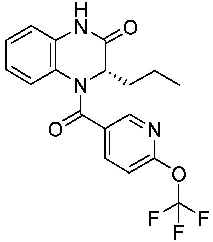 | (S)-3-propyl-4-(6-(trifluoromethoxy)nicotinoyl)-3,4-dihydroquinoxalin-2(1H)-one |
| 26 | 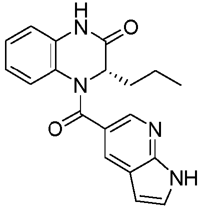 | (S)-3-propyl-4-(1H-pyrrolo[2,3-b]pyridine-5-carbonyl)-3,4-dihydroquinoxalin-2(1H)-one |
| 27 | 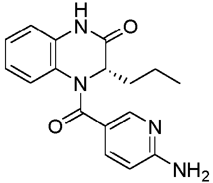 | (S)-4-(6-aminonicotinoyl)-3-propyl-3,4-dihydroquinoxalin-2(1H)-one |
| 28 | 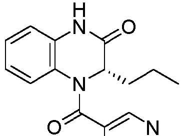 | (S)-4-(6-(dimethylamino)nicotinoyl)-3-propyl-3,4-dihydroquinoxalin-2(1H)-one |
| 29 | 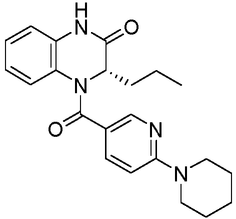 | (S)-4-(6-(piperidin-1-yl)nicotinoyl)-3-propyl-3,4-dihydroquinoxalin-2(1H)-one |
| 30 | 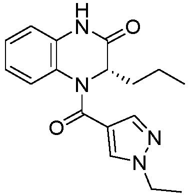 | (S)-4-(1-ethyl-1H-pyrazole-4-carbonyl)-3-propyl-3,4-dihydroquinoxalin-2(1H)-one |
| 31 | 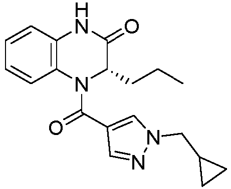 | (S)-4-(1-(cyclopropylmethyl)-1H-pyrazole-4-carbonyl)-3-propyl-3,4-dihydroquinoxalin-2(1H)-one |
| 32 | 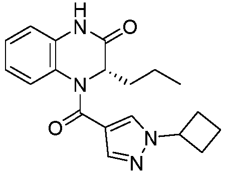 | (S)-4-(1-cyclobutyl-1H-pyrazole-4-carbonyl)-3-propyl-3,4-dihydroquinoxalin-2(1H)-one |
| 33 | 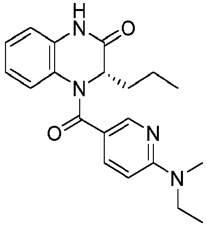 | (S)-4-(6-(ethyl(methyl)amino)nicotinoyl)-3-propyl-3,4-dihydroquinoxalin-2(1H)-one |
| 34 | 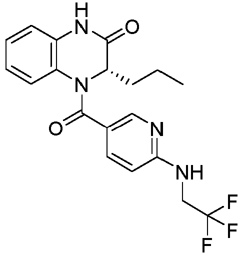 | (S)-3-propyl-4-(6-((2,2,2-trifluoroethyl)amino)nicotinoyl)-3,4-dihydroquinoxalin-2(1H)-one |
| 35 | 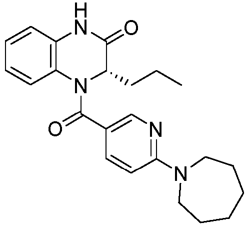 | (S)-4-(6-(azepan-1-yl)nicotinoyl)-3-propyl-3,4-dihydroquinoxalin-2(1H)-one |
| 36 | 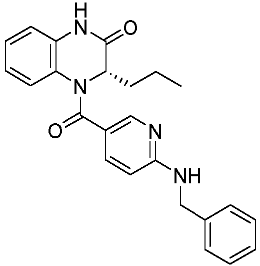 | (S)-4-(6-(benzylamino)nicotinoyl)-3-propyl-3,4-dihydroquinoxalin-2(1H)-one |
| 37 | 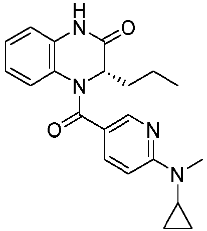 | (S)-4-(6-(cyclopropyl(methyl)amino)nicotinoyl)-3-propyl-3,4-dihydroquinoxalin-2(1H)-one |
| 38 | 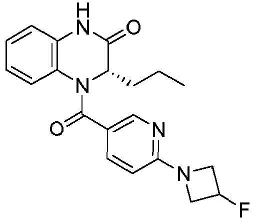 | (S)-4-(6-(3-fluoroazetidin-1-yl)nicotinoyl)-3-propyl-3,4-dihydroquinoxalin-2(1H)-one |
| 39 | 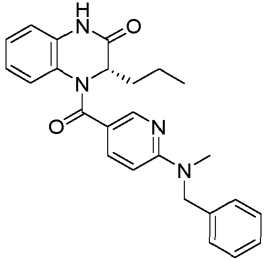 | (S)-4-(6-(benzyl(methyl)amino)nicotinoyl)-3-propyl-3,4-dihydroquinoxalin-2(1H)-one |
| 40 | 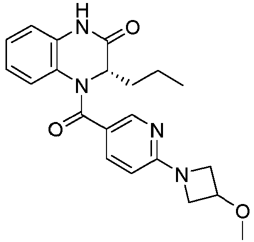 | (S)-4-(6-(3-methoxyazetidin-1-yl)nicotinoyl)-3-propyl-3,4-dihydroquinoxalin-2(1H)-one |
| 41 | 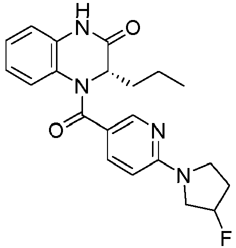 | (3S)-4-(6-(3-fluoropyrrolidin-1-yl)nicotinoyl)-3-propyl-3,4-dihydroquinoxalin-2(1H)-one |
| 42 | 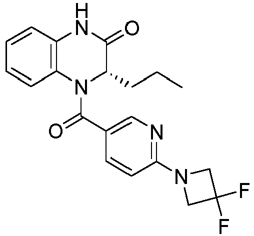 | (S)-4-(6-(3,3-difluoroazetidin-1-yl)nicotinoyl)-3-propyl-3,4-dihydroquinoxalin-2(1H)-one |
| 43 | 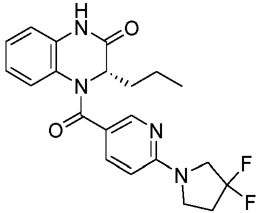 | (S)-4-(6-(3,3-difluoropyrrolidin-1-yl)nicotinoyl)-3-propyl-3,4-dihydroquinoxalin-2(1H)-one |
| 44 | 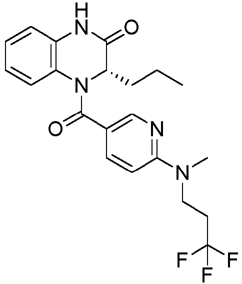 | (S)-4-(6-(methyl(3,3,3-trifluoropropyl)amino)nicotinoyl)-3-propyl-3,4-dihydroquinoxalin-2(1H)-one |
| 45 | 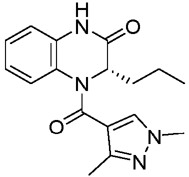 | (S)-4-(1,3-dimethyl-1H-pyrazole-4-carbonyl)-3-propyl-3,4-dihydroquinoxalin-2(1H)-one |
| 46 | 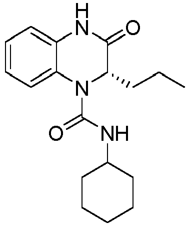 | (S)-N-cyclohexyl-3 -oxo-2-propyl-3,4-dihydroquinoxaline-1(2H)-carboxamide |
| 47 | 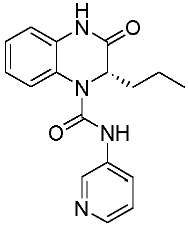 | (S)-3-oxo-2-propyl-N-(pyridin-3-yl)-3,4-dihydroquinoxaline-1(2H)-carboxamide |
| 48 | 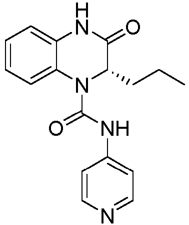 | (S)-3-oxo-2-propyl-N-(pyridin-4-yl)-3,4-dihydroquinoxaline-1(2H)-carboxamide |
| 49 | 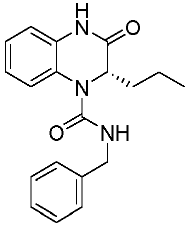 | (S)-N-benzyl-3-oxo-2-propyl-3,4-dihydroquinoxaline-1(2H)-carboxamide |
| 50 | 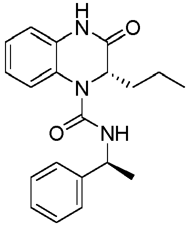 | (S)-3-oxo-N-((S)-1-phenylethyl)-2-propyl-3,4-dihydroquinoxaline-1(2H)-carboxamide |
| 51 | 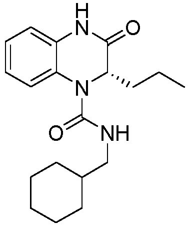 | (S)-N-(cyclohexylmethyl)-3-oxo-2-propyl-3,4-dihydroquinoxaline-1(2H)-carboxamide |
| 52 | 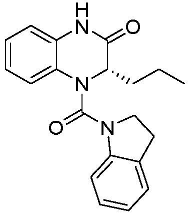 | (S)-4-(indoline-1-carbonyl)-3-propyl-3,4-dihydroquinoxalin-2(1H)-one |
| 53 | 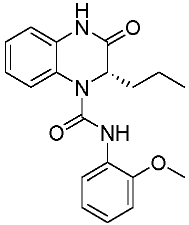 | (S)-N-(2-methoxyphenyl)-3-oxo-2-propyl-3,4-dihydroquinoxaline-1(2H)-carboxamide |
| 54 | 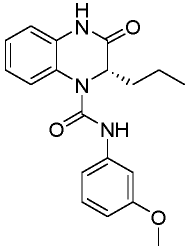 | (S)-N-(3-methoxyphenyl)-3-oxo-2-propyl-3,4-dihydroquinoxaline-1(2H)-carboxamide |
| 55 | 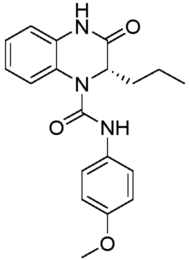 | (S)-N-(4-methoxyphenyl)-3-oxo-2-propyl-3,4-dihydroquinoxaline-1(2H)-carboxamide |
| 56 | 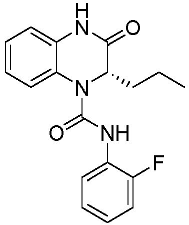 | (S)-N-(2-fluorophenyl)-3-oxo-2-propyl-3,4-dihydroquinoxaline-1(2H)-carboxamide |
| 57 | 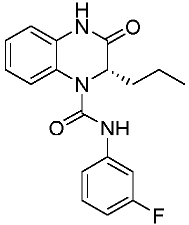 | (S)-N-(3-fluorophenyl)-3-oxo-2-propyl-3,4-dihydroquinoxaline-1(2H)-carboxamide |
| 58 | 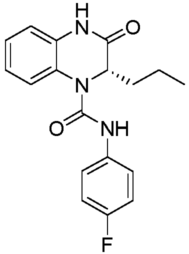 | (S)-N-(4-fluorophenyl)-3-oxo-2-propyl-3,4-dihydroquinoxaline-1(2H)-carboxamide |
| 59 | 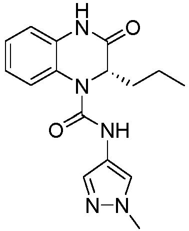 | (S)-N-(1-methyl-1H-pyrazol-4-yl)-3-oxo-2-propyl-3,4-dihydroquinoxaline-1(2H)-carboxamide |
| 60 | 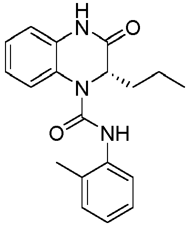 | (S)-3-oxo-2-propyl-N-(o-tolyl)-3,4-dihydroquinoxaline-1(2H)-carboxamide |
| 61 | 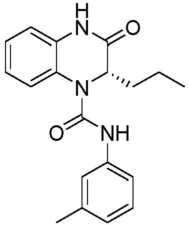 | (S)-3-oxo-2-propyl-N-(m-tolyl)-3,4-dihydroquinoxaline-1(2H)-carboxamide |
| 62 | 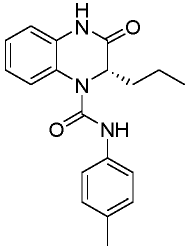 | (S)-3-oxo-2-propyl-N-(p-tolyl)-3,4-dihydroquinoxaline-1(2H)-carboxamide |
| 63 | 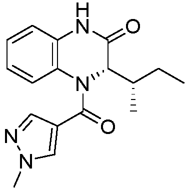 | (S)-3-((S)-sec-butyl)-4-(1-methyl-1H-pyrazole-4-carbonyl)-3,4-dihydroquinoxalin-2(1H)-one |
| 64 | 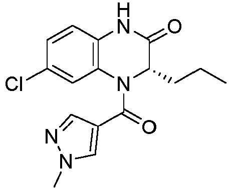 | (S)-6-chloro-4-(1-methyl-1H-pyrazole-4-carbonyl)-3-propyl-3,4-dihydroquinoxalin-2(1H)-one |
| 65 | 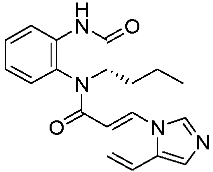 | (S)-4-(imidazo[1,5-a]pyridine-6-carbonyl)-3-propyl-3,4-dihydroquinoxalin-2(1H)-one |
| 66 | 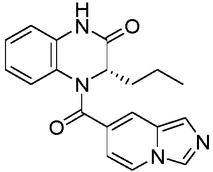 | (S)-4-(imidazo[1,5-a]pyridine-7-carbonyl)-3-propyl-3,4-dihydroquinoxalin-2(1H)-one |
| 67 | 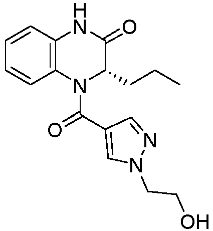 | (S)-4-(1-(2-hydroxyethyl)-1H-pyrazole-4-carbonyl)-3-propyl-3,4-dihydroquinoxalin-2(1H)-one |
| 68 | 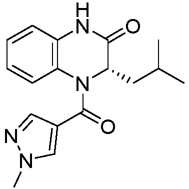 | (S)-3-isobutyl-4-(1-methyl-1H-pyrazole-4-carbonyl)-3,4-dihydroquinoxalin-2(1H)-one |
| 69 | 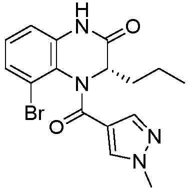 | (S)-5-bromo-4-(1-methyl-1H-pyrazole-4-carbonyl)-3-propyl-3,4-dihydroquinoxalin-2(1H)-one |
| 70 | 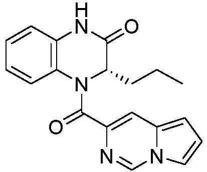 | (S)-3-propyl-4-(pyrrolo[1,2-c]pyrimidine-3-carbonyl)-3,4-dihydroquinoxalin-2(1H)-one |
| 71 | 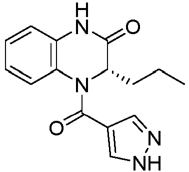 | (S)-3-propyl-4-(1H-pyrazole-4-carbonyl)-3,4-dihydroquinoxalin-2(1H)-one |
| 72 | 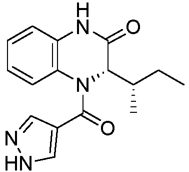 | (S)-3-((S)-sec-butyl)-4-(1H-pyrazole-4-carbonyl)-3,4-dihydroquinoxalin-2(1H)-one |
| 73 | 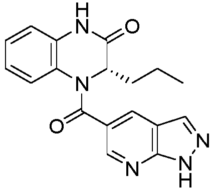 | (S)-3-propyl-4-(1H-pyrazolo[3,4-b]pyridine-5-carbonyl)-3,4-dihydroquinoxalin-2(1H)-one |
| 74 | 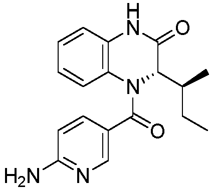 | (S)-4-(6-aminonicotinoyl)-3-((S)-sec-butyl)-3,4-dihydroquinoxalin-2(1H)-one |
| 75 | 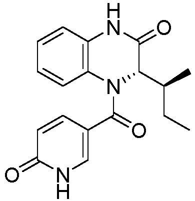 | (S)-3-((S)-sec-butyl)-4-(6-oxo-1,6-dihydropyridine-3-carbonyl)-3,4-dihydroquinoxalin-2(1H)-one |
| 76 | 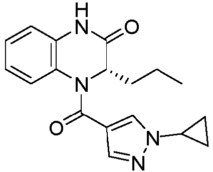 | (S)-4-(1-cyclopropyl-1H-pyrazole-4-carbonyl)-3-propyl-3,4-dihydroquinoxalin-2(1H)-one |
| 77 | 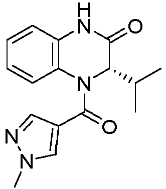 | (S)-3-isopropyl-4-(1-methyl-1H-pyrazole-4-carbonyl)-3,4-dihydroquinoxalin-2(1H)-one |
| 78 | 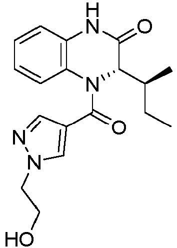 | (S)-3-((S)-sec-butyl)-4-(1-(2-hydroxyethyl)-1H-pyrazole-4-carbonyl)-3,4-dihydroquinoxalin-2(1H)-one |
| 79 | 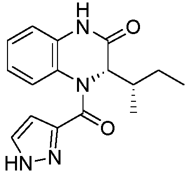 | (S)-3-((S)-sec-butyl)-4-(1H-pyrazole-3-carbonyl)-3,4-dihydroquinoxalin-2(1H)-one |
| 80 | 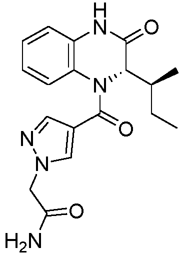 | 2-(4-((S)-2-((S)-sec-butyl)-3-oxo-1,2,3,4-tetrahydroquinoxaline-1-carbonyl)-1H-pyrazol-1-yl)acetamide |
| 81 | 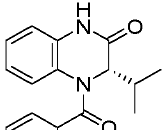 | (S)-3-isopropyl-4-(1-methyl-6-oxo-1,6-dihydropyridine-3-carbonyl)-3,4-dihydroquinoxalin-2(1H)-one |
| 82 | 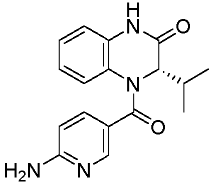 | (S)-4-(6-aminonicotinoyl)-3-isopropyl-3,4-dihydroquinoxalin-2(1H)-one |
| 83 | 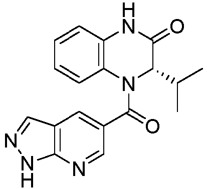 | (S)-3-isopropyl-4-(1H-pyrazolo[3,4-b]pyridine-5-carbonyl)-3,4-dihydroquinoxalin-2(1H)-one |
| 84 | 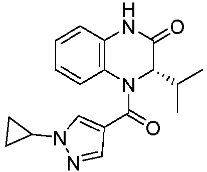 | (S)-4-(1-cyclopropyl-1H-pyrazole-4-carbonyl)-3-isopropyl-3,4-dihydroquinoxalin-2(1H)-one |
| 85 | 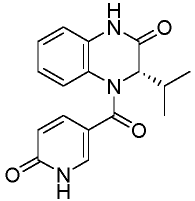 | (S)-3-isopropyl-4-(6-oxo-1,6-dihydropyridine-3-carbonyl)-3,4-dihydroquinoxalin-2(1H)-one |
| 86 | 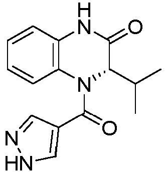 | (S)-3-isopropyl-4-(1H-pyrazole-4-carbonyl)-3,4-dihydroquinoxalin-2(1H)-one |
| 87 | 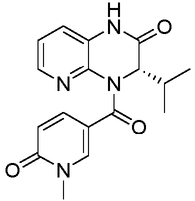 | (S)-3-isopropyl-4-(1-methyl-6-oxo-1,6-dihydropyridine-3-carbonyl)-3,4-dihydropyrido[2,3-b]pyrazin-2(1H)-one |
| 88 | 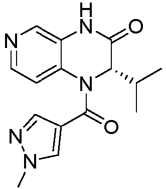 | (S)-2-isopropyl-1-(1-methyl-1H-pyrazole-4-carbonyl)-1,4-dihydropyrido[3,4-b]pyrazin-3(2H)-one |
| 89 | 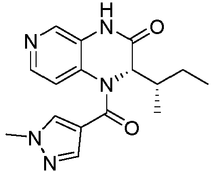 | (S)-2-((S)-sec-butyl)-1-(1-methyl-1H-pyrazole-4-carbonyl)-1,4-dihydropyrido[3,4-b]pyrazin-3(2H)-one |
| 90 | 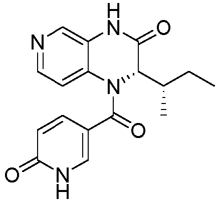 | (S)-2-((S)-sec-butyl)-1-(6-oxo-1,6-dihydropyridine-3-carbonyl)-1,4-dihydropyrido[3,4-b]pyrazin-3(2H)-one |
| 91 | 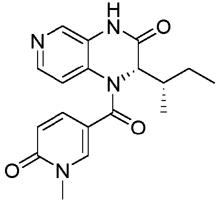 | (S)-2-((S)-sec-butyl)-1-(1-methyl-6-oxo-1,6-dihydropyridine-3-carbonyl)-1,4-dihydropyrido[3,4-b]pyrazin-3(2H)-one |
| 92 | 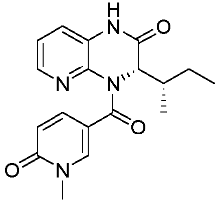 | (S)-3-((S)-sec-butyl)-4-(1-methyl-6-oxo-1,6-dihydropyridine-3-carbonyl)-3,4-dihydropyrido[2,3-b]pyrazin-2(1H)-one |
| 93 | 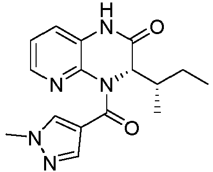 | (S)-3-((S)-sec-butyl)-4-(1-methyl-1H-pyrazole-4-carbonyl)-3,4-dihydropyrido[2,3-b]pyrazin-2(1H)-one |
| 94 | 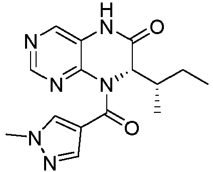 | (S)-7-((S)-sec-butyl)-8-(1-methyl-1H-pyrazole-4-carbonyl)-7,8-dihydropteridin-6(5H)-one |
| 95 | 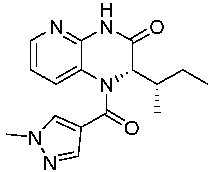 | (S)-2-((S)-sec-butyl)-1-(1-methyl-1H-pyrazole-4-carbonyl)-1,4-dihydropyrido[2,3-b]pyrazin-3(2H)-one |
| 96 | 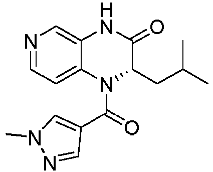 | (S)-2-isobutyl-1-(1-methyl-1H-pyrazole-4-carbonyl)-1,4-dihydropyrido[3,4-b]pyrazin-3(2H)-one |
| 97 | 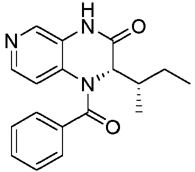 | (S)-1-benzoyl-2-((S)-sec-butyl)-1,4-dihydropyrido[3,4-b]pyrazin-3(2H)-one |
| 98 | 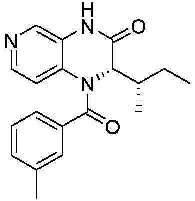 | (S)-2-((S)-sec-butyl)-1-(3-methylbenzoyl)-1,4-dihydropyrido[3,4-b]pyrazin-3(2H)-one |
| 99 | 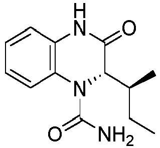 | (S)-2-((S)-sec-butyl)-3-oxo-3,4-dihydroquinoxaline-1 (2H)-carboxamide |
| 100 | 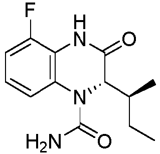 | (S)-2-((S)-sec-butyl)-5-fluoro-3-oxo-3,4-dihydroquinoxaline-1 (2H)-carboxamide |
| 101 | 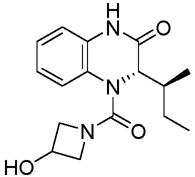 | (S)-3-((S)-sec-butyl)-4-(3-hydroxyazetidine-1-carbonyl)-3,4-dihydroquinoxalin-2(1H)-one |
| 102 | 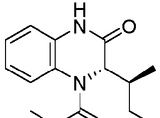 | (S)-2-((S)-sec-butyl)-N-methyl-3-oxo-3,4-dihydroquinoxaline-1 (2H)-carboxamide |
| 103 | 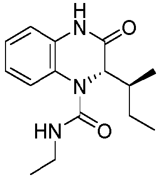 | (S)-2-((S)-sec-butyl)-N-ethyl-3-oxo-3,4-dihydroquinoxaline-1 (2H)-carboxamide |
| 104 | 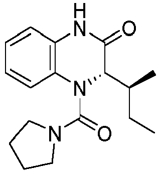 | (S)-3-((S)-sec-butyl)-4-(pyrrolidine-1-carbonyl)-3,4-dihydroquinoxalin-2(1H)-one |
| 105 | 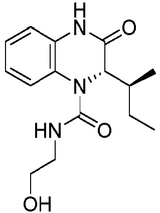 | (S)-2-((S)-sec-butyl)-N-(2-hydroxyethyl)-3-oxo-3,4-dihydroquinoxaline-1(2H)-carboxamide |
| 106 | 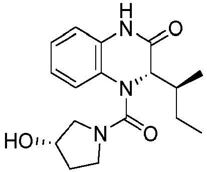 | (S)-3-((S)-sec-butyl)-4-((S)-3-hydroxypyrrolidine-1-carbonyl)-3,4-dihydroquinoxalin-2(1H)-one |
| 107 | 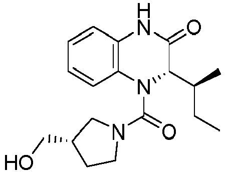 | (S)-3-((S)-sec-butyl)-4-((S)-3-(hydroxymethyl)pyrrolidine-1-carbonyl)-3,4-dihydroquinoxalin-2(1H)-one |
| 108 | 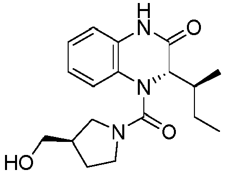 | (S)-3-((S)-sec-butyl)-4-((R)-3-(hydroxymethyl)pyrrolidine-1-carbonyl)-3,4-dihydroquinoxalin-2(1H)-one |
| 109 | 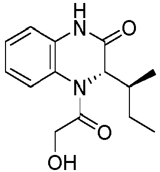 | (S)-3-((S)-sec-butyl)-4-(2-hydroxyacetyl)-3,4-dihydroquinoxalin-2(1H)-one |
| 110 | 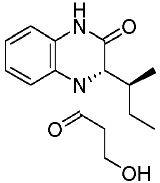 | (S)-3-((S)-sec-butyl)-4-(3-hydroxypropanoyl)-3,4-dihydroquinoxalin-2(1H)-one |
| 111 | 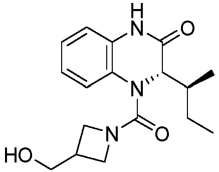 | (S)-3-((S)-sec-butyl)-4-(3-(hydroxymethyl)azetidine-1-carbonyl)-3,4-dihydroquinoxalin-2(1H)-one |
| 112 | 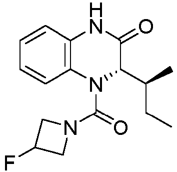 | (S)-3-((S)-sec-butyl)-4-(3-fluoroazetidine-1-carbonyl)-3,4-dihydroquinoxalin-2(1H)-one |
| 113 | 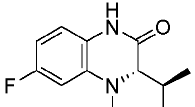 | (S)-2-((S)-sec-butyl)-7-fluoro-3-oxo-3,4-dihydroquinoxaline-1 (2H)-carboxamide |
| 114 | 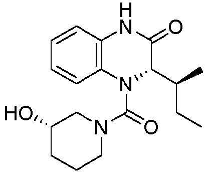 | (S)-3-((S)-sec-butyl)-4-((S)-3-hydroxypiperidine-1-carbonyl)-3,4-dihydroquinoxalin-2(1H)-one |
| 115 | 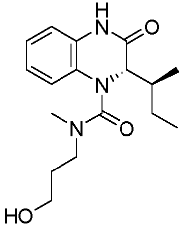 | (S)-2-((S)-sec-butyl)-N-(3-hydroxypropyl)-N-methyl-3-oxo-3,4-dihydroquinoxaline-1 (2H)-carboxamide |
| 116 | 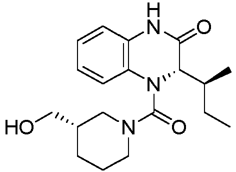 | (S)-3-((S)-sec-butyl)-4-((S)-3-(hydroxymethyl)piperidine-1-carbonyl)-3,4-dihydroquinoxalin-2(1H)-one |
| 117 | 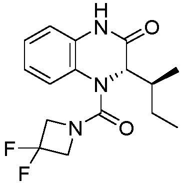 | (S)-3-((S)-sec-butyl)-4-(3,3-difluoroazetidine-1-carbonyl)-3,4-dihydroquinoxalin-2(1H)-one |
| 118 | 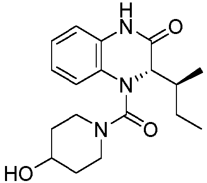 | (S)-3-((S)-sec-butyl)-4-(4-hydroxypiperidine-1-carbonyl)-3,4-dihydroquinoxalin-2(1H)-one |
| 119 | 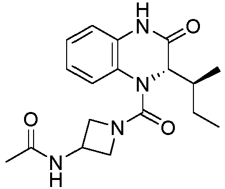 | N-(1-((S)-2-((S)-sec-butyl)-3-oxo-1,2,3,4-tetrahydroquinoxaline-1-carbonyl)azetidin-3-yl)acetamide |
| 120 | 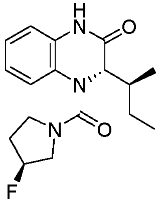 | (S)-3-((S)-sec-butyl)-4-((S)-3-fluoropyrrolidine-1-carbonyl)-3,4-dihydroquinoxalin-2(1H)-one |
| 121 | 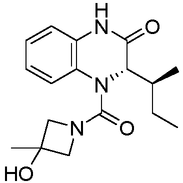 | (S)-3-((S)-sec-butyl)-4-(3-hydroxy-3-methylazetidine-1-carbonyl)-3,4-dihydroquinoxalin-2(1H)-one |
| 122 | 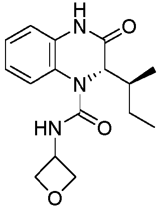 | (S)-2-((S)-sec-butyl)-N-(oxetan-3-yl)-3-oxo-3,4-dihydroquinoxaline-1 (2H)-carboxamide |
| 123 | 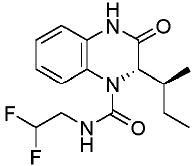 | (S)-2-((S)-sec-butyl)-N-(2,2-difluoroethyl)-3-oxo-3,4-dihydroquinoxaline-1(2H)-carboxamide |
| 124 | 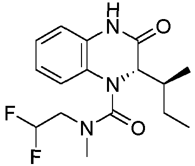 | (S)-2-((S)-sec-butyl)-N-(2,2-difluoroethyl)-N-methyl-3-oxo-3,4-dihydroquinoxaline-1 (2H)-carboxamide |
| 125 | 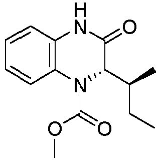 | methyl (S)-2-((S)-sec-butyl)-3-oxo-3,4-dihydroquinoxaline-1(2H)-carboxylate |
| 126 | 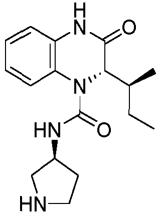 | (S)-2-((S)-sec-butyl)-3-oxo-N-((S)-pyrrolidin-3-yl)-3,4-dihydroquinoxaline-1 (2H)-carboxamide |
| 127 | 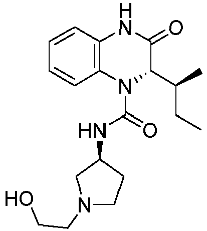 | (S)-2-((S)-sec-butyl)-N-((S)-1-(2-hydroxyethyl)pyrrolidin-3-yl)-3-oxo-3,4-dihydroquinoxaline-1 (2H)-carboxamide |
| 128 | 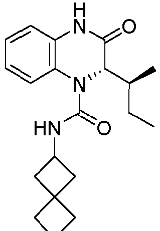 | (S)-2-((S)-sec-butyl)-3-oxo-N-(2-oxaspiro[3.3]heptan-6-yl)-3,4-dihydroquinoxaline-1(2H)-carboxamide |
| 129 | 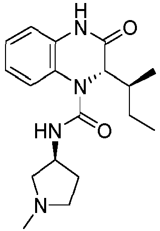 | (S)-2-((S)-sec-butyl)-N-((S)-1-methylpyrrolidin-3-yl)-3-oxo-3,4-dihydroquinoxaline-1(2H)-carboxamide |
| 130 | 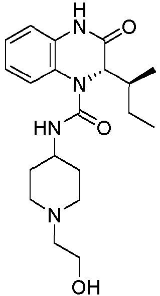 | (S)-2-((S)-sec-butyl)-N-(1-(2-hydroxyethyl)piperidin-4-yl)-3-oxo-3,4-dihydroquinoxaline-1 (2H)-carboxamide |
| 131 | 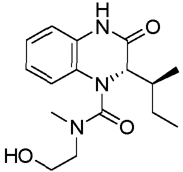 | (S)-2-((S)-sec-butyl)-N-(2-hydroxyethyl)-N-methyl-3-oxo-3,4-dihydroquinoxaline-1 (2H)-carboxamide |
| 132 | 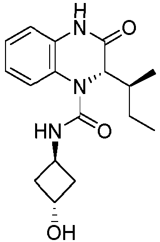 | (S)-2-((S)-sec-butyl)-N-((1r,3S)-3-hydroxycyclobutyl)-3-oxo-3,4-dihydroquinoxaline-1(2H)-carboxamide |
| 133 | 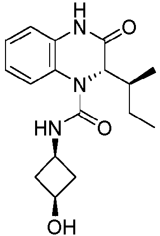 | (S)-2-((S)-sec-butyl)-N-((1s,3R)-3-hydroxycyclobutyl)-3-oxo-3,4-dihydroquinoxaline-1(2H)-carboxamide |
or a pharmaceutically acceptable salt thereof.

10. A compound of claim 1 of the formula: 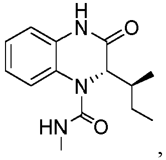 or a pharmaceutically acceptable salt thereof.

11. A compound of claim 1 of the formula: 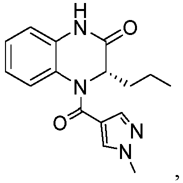 or a pharmaceutically acceptable salt thereof.

12. A compound of claim 1 of the formula: 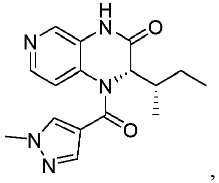 or a pharmaceutically acceptable salt thereof.

13. A pharmaceutical composition comprising a compound of any one of claims 1-12, or a pharmaceutically acceptable salt thereof, and a pharmaceutical carrier.

14. The pharmaceutical composition of claim 13, wherein the pharmaceutical composition is formulated for oral, sublingual, subcutaneous, parenteral, intravenous, intranasal, topical, transdermal, intraperitoneal, intramuscular, intrapulmonary, vaginal, rectal, or intraocular administration.

15. The compound of any one of claims 1-12, or a pharmaceutically acceptable salt thereof, or the pharmaceutical composition of claim 13 or 14, for use in treating a disease or condition selected from the group consisting of:
(a) peripheral vascular disease, peripheral arterial disease, rehabilitation-related deficits, metabolic syndrome, obesity, ventilator-induced muscle weakness, chronic fatigue syndrome, neuromuscular disorders, conditions of muscle wasting, muscular myopathies, muscle atrophy, muscle fatigue, and frailty in a subject;
(b) amyotrophic lateral sclerosis (ALS), spinal muscular atrophy (SMA), myasthenia gravis, and muscular myopathies in a subject; or
(c) stress urinary incontinence (SUI), mixed urinary incontinence (MUI), fecal incontinence, frailty, sarcopenia, chronic obstructive pulmonary disease (COPD), cachexia syndrome, muscle wasting caused by heart failure, cancer, or chronic kidney disease/dialysis, post-spinal cord injury (SCI) muscle dysfunction, and post-stroke muscle dysfunction in a subject.

## Patentansprüche

1. Verbindung der Formel (I): 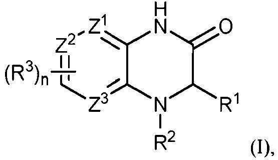 oder ein pharmazeutisch annehmbares Salz davon, wobei:
Z¹, Z² und Z³ unabhängig voneinander CH, CR³ oder N sind, mit der Maßgabe, dass mindestens eines von Z¹, Z² und Z³ CH oder CR³ ist;
R¹ substituiertes C₃-C₄-Alkyl ist;
R² -C(=O)R⁴, -C(=O)-(CH₂)ₚ-R⁵, -C(=O)NR⁶R⁷ oder -C(=O)OCH₃ ist;
jedes R³ unabhängig Halogen, C₁-C₆-Alkyl, C₁-C₆-Halogenakyl, C₁-C₆-Alkoxy, C₁-C₆-Halogenakoxy, C₃-C₁₂-Cycloalkyl oder 3-12-gliedriges Heterocycloalkyl ist;
R⁴ C₆-C₁₂-Aryl ist, gegebenenfalls substituiert mit einem bis fünf R^{4A}, 5-12-gliedriges Heteroaryl, gegebenenfalls substituiert mit einem bis fünf R^{4B}, C₃-C₁₂-Cycloalkyl, gegebenenfalls substituiert mit einem bis fünf R^{4C}, 4-12-gliedriges Heterocycloalkyl, gegebenenfalls substituiert mit einem bis fünf R^{4D}, oder 4-12-gliedriges Heterocycloalkenyl, gegebenenfalls substituiert mit einem bis fünf R^{4E};
jedes R^{4A} unabhängig ausgewählt ist aus der Gruppe bestehend aus Halogen, C₁-C₆-Alkyl, C₁-C₆-Halogenalkyl, OH, C₁-C₆-Alkoxy, C₁-C₆-Halogenalkoxy, NH₂, NH(C₁-C₆-Alkyl), N(C₁-C₆-Alkyl)₂, NH(C₁-C₆-Halogenalkyl), n(C₁-C₆-Alkyl) (C₁-C₆-halogenalkyl), N (C₁-C₆-Halogenalkyl)₂, NH(C₁-C₆-Alkylen-(C₆-C₁₂-aryl)), N (C₁-C₆-Alkyl)(C₁-C₆-alkylen-(C₆-C₁₂-aryl)), N(C₁-C₆-Alkylen-(C₆-C₁₂-aryl))₂, NH(C₃-C₁₂-Cycloalkyl), N(C₁-C₆-Alkyl)(C₃-C₁₂-cycloalkyl), N(C₃-C₁₂-Cycloalkyl)₂, NHC(O)-C₁-C₆-Alkyl, C₃-C₁₂-Cycloalkyl, gegebenenfalls substituiert mit einem bis fünf R^{4B1}, 4-12-gliedrigem Heterocycloalkyl, gegebenenfalls substituiert mit ein bis fünf R^{4B1}, C₁-C₆-Alkylen-OH, C₁-C₆-Alkylen-CONH₂, C₁-C₆-Alkylen-(C₃-C₁₂-cycloalkyl), C₁-C₆-Alkylen(4-12-gliedriges Heterocycloalkyl), C₆-C₁₂-Aryl und 5-12-gliedrigem Heteroaryl;
jedes R^{4B} unabhängig ausgewählt ist aus der Gruppe bestehend aus Halogen, C₁-C₆-Alkyl, C₁-C₆-Halogenalkyl, OH, C₁-C₆-Alkoxy, C₁-C₆-Halogenalkoxy, NH₂, NH (C₁-C₆-Alkyl), N(C₁-C₆-Alkyl)₂, NH(C₁-C₆-Halogenalkyl), N(C₁-C₆-Alkyl) (C₁-C₆-halogenalkyl), N(C₁-C₆-Halogenalkyl)₂, NH(C₁-C₆-Alkylen-(C₆-C₁₂-aryl)), N (C₁-C₆-Alkyl)(C₁-C₆-alkylen-(C₆-C₁₂-aryl)), N(C₁-C₆-Alkylen-(C₆-C₁₂-aryl)) 2, NH(C₃-C₁₂-Cycloalkyl), N(C₁-C₆-Alkyl)(C₃-C₁₂-cycloalkyl), N(C₃-C₁₂-Cycloalkyl)₂, NHC(O)-C₁-C₆-Alkyl, C₃-C₁₂-Cycloalkyl, gegebenenfalls substituiert mit einem bis fünf R^{4B1}, 4-12-gliedrigem Heterocycloalkyl, gegebenenfalls substituiert mit einem bis fünf R^{4B1}, C₁-C₆-Alkylen-OH, C₁-C₆-Alkylen-CONH₂, C₁-C₆-Alkylen-(C₃-C₁₂-cycloalkyl), C₁-C₆-Alkylen-(4-12-gliedriges Heterocycloalkyl) und 5-12-gliedrigem Heteroaryl; jedes R^{4B1} unabhängig ausgewählt ist aus der Gruppe bestehend aus Halogen, C₁-C₆-Alkyl, C₁-C₆-Halogenalkyl, OH, C₁-C₆-Alkoxy, C₁-C₆-Halogenalkoxy und C₁-C₆-Alkylen-OH;
jedes R^{4C} unabhängig ausgewählt ist aus der Gruppe bestehend aus Halogen, C₁-C₆-Alkyl, C₁-C₆-Halogenalkyl, OH, C₁-C₆-Alkoxy, C₁-C₆-Halogenalkoxy, NHC(O)-C₁-C₆-Alkyl und C₁-C₆-Alkylen-OH;
jedes R^{4D} unabhängig ausgewählt ist aus der Gruppe bestehend aus Halogen, C₁-C₆-Alkyl, C₁-C₆-Halogenalkyl, OH, C₁-C₆-Alkoxy, C₁-C₆-Halogenalkoxy, NHC(O)-C₁-C₆-Alkyl und C₁-C₆-Alkylen-OH;
jedes R^{4E} unabhängig ausgewählt ist aus der Gruppe bestehend aus Oxo, Halogen, C₁-C₆-Alkyl, C₁-C₆-Halogenalkyl, OH, C₁-C₆-Alkoxy, C₁-C₆-Halogenalkoxy, NHC (O) -C₁-C₆-Alkyl und C₁-C₆-Alkylen-OH;
R⁵ OH, C₁-C₆-Alkoxy, C₁-C₆-Halogenalkoxy, C₃-C₁₂-Cycloalkyl, 3-12-gliedriges Heterocycloalkyl oder C₆-C₁₂-Aryl ist;
R⁶ und R⁷ unabhängig voneinander ausgewählt sind aus der Gruppe bestehend aus H, C₁-C₆-Alkyl, C₁-C₆-Halogenalkyl, C₃-C₁₂-Cycloalkyl, gegebenenfalls substituiert mit einem bis fünf R⁸, 4-12-gliedrigem Heterocycloalkyl, gegebenenfalls substituiert mit einem bis fünf R⁸, C₆-C₁₂-Aryl, gegebenenfalls substituiert mit einem bis fünf R⁸, 5-12-gliedrigem Heteroaryl, gegebenenfalls substituiert mit einem bis fünf R⁸, C₁-C₆-Alkylen-OH, C₁-C₆-Alkylen-(C₃-C₁₂-cycloalkyl), C₁-C₆-Alkylen-(4-12-gliedriges Heterocycloalkyl), C₁-C₆-Alkylen-(C₆-C₁₂-aryl) und C₁-C₆-Alkylen-(5-12-gliedriges Heteroaryl);
jedes R⁸ unabhängig ausgewählt ist aus der Gruppe bestehend aus Halogen, C₁-C₆-Alkyl, C₁-C₆-Halogenalkyl, OH, C₁-C₆-Alkoxy, C₁-C₆-Alkylen-OH und C₁-C₆-Halogenalkoxy;
n 0, 1, 2, 3 oder 4 ist;
p 1, 2 oder 3 ist; und
mit der Maßgabe, dass die Verbindung der Formel (I) keine Verbindung ist, die ausgewählt ist aus der Gruppe bestehend aus:
4-(3-Methylbenzoyl)-3-propyl-3,4-dihydrochinoxalin-2(1H)-on,
4-(2-Chlorbenzoyl)-3-isobutyl-3,4-dihydrochinoxalin-2(1H)-on und
4-(4-(tert-Butyl)benzoyl)-3-propyl-3,4-dihydrochinoxalin-2(1H)-on.

2. Verbindung nach Anspruch 1, wobei die Verbindung der Formel (I) eine Verbindung der Formel (I-a), (I-b), (I-c), (I-d) oder (I-e) ist: 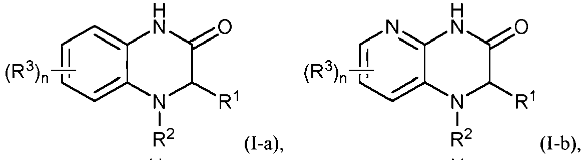 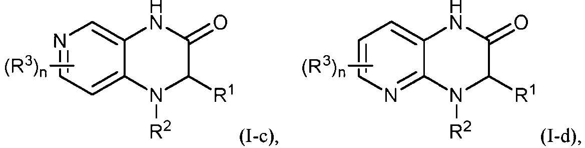 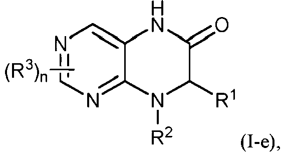 oder ein pharmazeutisch annehmbares Salz davon.

3. Verbindung nach Anspruch 1, wobei die Verbindung der Formel (I) eine Verbindung der Formel (II) ist: 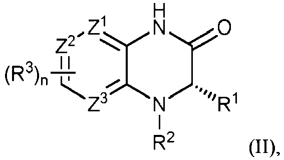 oder ein pharmazeutisch annehmbares Salz davon.

4. Verbindung nach Anspruch 3, wobei die Verbindung der Formel (II) eine Verbindung der Formel (II-a), (II-b), (II-c), (II-d) oder (II-e) ist: 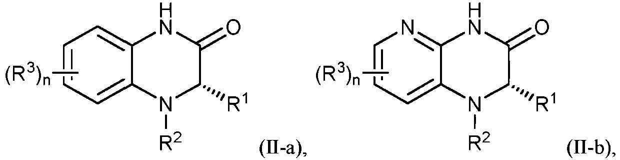 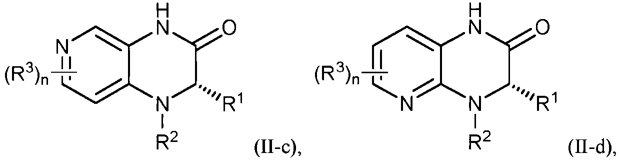 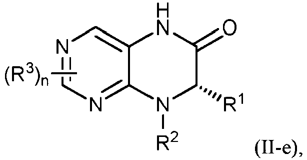 oder ein pharmazeutisch annehmbares Salz davon,
und/oder wobei die Verbindung der Formel (II) eine Verbindung der Formel (II-a) ist: 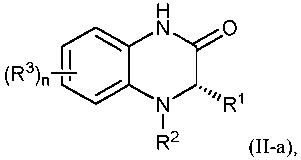
oder ein pharmazeutisch annehmbares Salz davon.

5. Verbindung nach einem der Ansprüche 1-4 oder ein pharmazeutisch annehmbares Salz davon, wobei R¹ ausgewählt ist aus der Gruppe bestehend aus Prop-1-yl, Prop-2-yl, But-2-yl und 2-Methylprop-1-yl.

6. Verbindung nach einem der Ansprüche 1-5 oder ein pharmazeutisch annehmbares Salz davon, wobei:
(a) R³ Halogen oder C₃-C₁₂-Cycloalkyl ist;
(b) R³ Halogen ist; oder
(c) R³ C₃-C₆-Cycloalkyl ist.

7. Verbindung nach einem der Ansprüche 1-6 oder pharmazeutisch annehmbares Salz davon, wobei n 0 oder 1 ist.

8. Verbindung nach einem der Ansprüche 1-7 oder ein pharmazeutisch annehmbares Salz davon, wobei:
(a) R² -C(=O)R⁴ ist, gegebenenfalls wobei:
(a) (i) R⁴ C₆-C₁₂-Aryl ist, gegebenenfalls substituiert mit einem bis fünf R^{4A}, 5-12-gliedriges Heteroaryl, gegebenenfalls substituiert mit einem bis fünf R^{4B}, C₃-C₁₂-Cycloalkyl, 4-12-gliedriges Heterocycloalkyl, gegebenenfalls substituiert mit einem bis fünf R^{4D}, oder 4-12-gliedriges Heterocycloalkenyl, gegebenenfalls substituiert mit einem bis fünf R^{4E};
(a) (ii) R⁴ C₆-C₁₂-Aryl ist, substituiert mit einem bis fünf R^{4A}, und R^{4A} Halogen oder C₁-C₆-Alkyl ist;
(a) (iii) R⁴ 5-12-gliedriges Heteroaryl ist, substituiert mit einem bis fünf R^{4B}, und R^{4B} ausgewählt ist aus der Gruppe bestehend aus Halogen, C₁-C₆-Alkyl, C₁-C₆-Halogenalkoxy, NH₂, N(C₁-C₆-Alkyl)₂, NH(C₁-C₆-Halogenalkyl), N(C₁-C₆-Alkyl)(C₁-C₆-halogenalkyl), NH(C₁-C₆-Alkylen-(C₆-C₁₂-aryl)), N(C₁-C₆-Alkyl)(C₃-C₁₂-cycloalkyl), NHC(O)-C₁-C₆-Alkyl, C₃-C₁₂-Cycloalkyl, 4-12-gliedrigem Heterocycloalkyl, gegebenenfalls substituiert mit einem bis fünf R^{4B1}, C₁-C₆-Alkylen-OH, C₁-C₆-Alkylen-CONH₂, C₁-C₆-Alkylen-(C₃-C₁₂-cycloalkyl)und 5-12-gliedrigem Heteroaryl, gegebenenfalls wobei R^{4B} 4-12-gliedriges Heterocycloalkyl ist, substituiert mit einem bis fünf R^{4B1}, und R^{4B1} Halogen oder C₁-C₆-Alkoxy ist;
(a) (iv) R⁴ 4-12-gliedriges Heterocycloalkyl ist, substituiert mit einem bis fünf R^{4D}, und R^{4D} ausgewählt ist aus der Gruppe bestehend aus Halogen, C₁-C₆-Alkyl, OH, NHC(O)-C₁-C₆-Alkyl und C₁-C₆-Alkylen-OH; oder
(a) (v) R⁴ 4-12-gliedriges Heterocycloalkenyl ist, substituiert mit einem bis fünf R^{4E}, und R^{4E} Oxo oder C₁-C₆-Alkyl ist; oder
(b) R² -C(=O)-(CH₂)ₚ-R⁵ ist, gegebenenfalls wobei:
(b) (i) R⁵ OH oder C₆-C₁₂-Aryl ist; und/oder
(b) (ii) p 1 oder 2 ist; oder
(c) R² -C(=O)NR⁶R⁷ ist, wobei gegebenenfalls:
(c) (i) R⁶ und R⁷ unabhängig voneinander ausgewählt sind aus der Gruppe bestehend aus H, C₁-C₆-Alkyl, C₁-C₆-Halogenalkyl, C₃-C₁₂-Cycloalkyl, C₆-C₁₂-Aryl, gegebenenfalls substituiert mit einem bis fünf R⁸, 5-12-gliedrigem Heteroaryl, gegebenenfalls substituiert mit einem bis fünf R⁸, C₁-C₆-Alkylen-OH, C₁-C₆-Alkylen-(C₃-C₁₂-cycloalkyl), C₁-C₆-Alkylen-(C₆-C₁₂-aryl)und C₁-C₆-Alkylen-(5-12-gliedriges Heteroaryl), gegebenenfalls wobei jedes R⁸ unabhängig ausgewählt ist aus der Gruppe bestehend aus Halogen, C₁-C₆-Alkyl, C₁-C₆-Alkylen-OH und C₁-C₆-Alkoxy; oder
(d) R² -C(=O)OCH₃ ist.

9. Verbindung nach Anspruch 1, ausgewählt aus der Gruppe bestehend aus Verbindungen 1-133:
| Verbindu ng Nr. | Verbindungsstrukt ur | Verbindungsname |
|---|---|---|
| 1 | 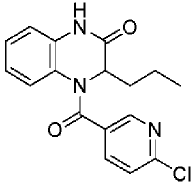 | 4-(6-Chlornicotinoyl)-3-propyl-3,4-dihydrochinoxalin-2(1H)-on |
| 2 | 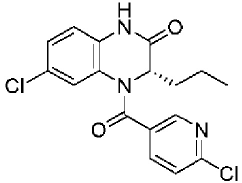 | (S)-6-Chlor-4-(6-chlornicotinoyl)-3-propyl-3,4-dihydrochinoxalin-2(1H)-on |
| 3 | 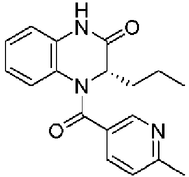 | (S)-4-(6-Methylnicotinoyl)-3-propyl-3,4-dihydrochinoxalin-2(1H)-on |
| 4 | 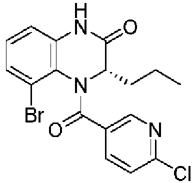 | (S)-5-Brom-4-(6-chlornicotinoyl)-3-propyl-3,4-dihydrochinoxalin-2(1H)-on |
| 5 | 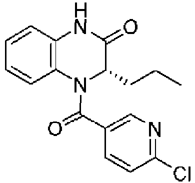 | (S)-4-(6-Chlornicotinoyl)-3-propyl-3,4-dihydrochinoxalin-2(1H)-on |
| 6 | 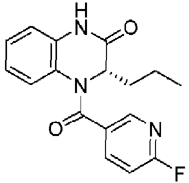 | (S)-4-(6-Fluoronicotinoyl)-3-propyl-3,4-dihydrochinoxalin-2(1H)-on |
| 7 | 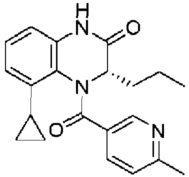 | (S)-5-Cyclopropyl-4-(6-methylnicotinoyl)-3-propyl-3,4-dihydrochinoxalin-2(1H)-on |
| 8 | 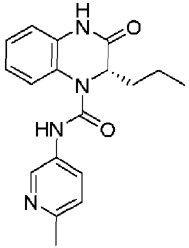 | (S)-N-(6-Methylpyridin-3-yl)-3-oxo-2-propyl-3,4-dihydrochinoxalin-1(2H)-carboxamid |
| 9 | 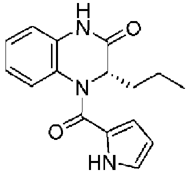 | (S)-3-Propyl-4-(1H-pyrrol-2-carbonyl)-3,4-dihydrochinoxalin-2(1H)-on |
| 10 | 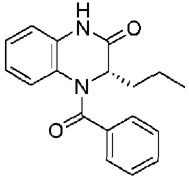 | (S)-4-Benzoyl-3-propyl-3,4-dihydrochinoxalin-2(1H)-on |
| 11 | 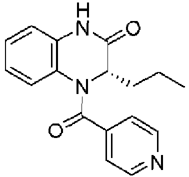 | (S)-4-Isonicotinoyl-3-propyl-3,4-dihydrochinoxalin-2(1H)-on |
| 12 | 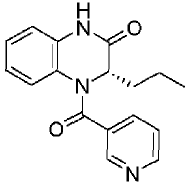 | (S)-4-Nicotinoyl-3-propyl-3,4-dihydrochinoxalin-2(1H)-on |
| 13 | 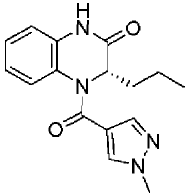 | (S)-4-(1-Methyl-1H-pyrazol-4-carbonyl)-3-propyl-3,4-dihydrochinoxalin-2(1H)-on |
| 14 | 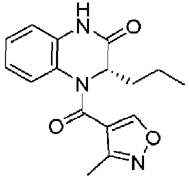 | (S)-4-(3-Methylisoxazol-4-carbonyl)-3-propyl-3,4-dihydrochinoxalin-2(1H)-on |
| 15 | 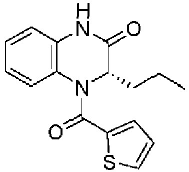 | (S)-3-Propyl-4-(thiophen-2-carbonyl)-3,4-dihydrochinoxalin-2(1H)-on |
| 16 | 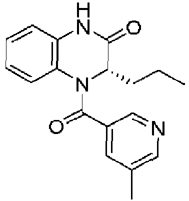 | (S)-4-(5-Methylnicotinoyl)-3-propyl-3,4-dihydrochinoxalin-2(1H)-on |
| 17 | 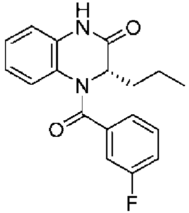 | (S)-4-(3-Fluorbenzoyl)-3-propyl-3,4-dihydrochinoxalin-2(1H)-on |
| 18 | 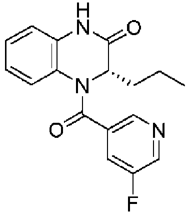 | (S)-4-(5-Fluoronicotinoyl)-3-propyl-3,4-dihydrochinoxalin-2(1H)-on |
| 19 | 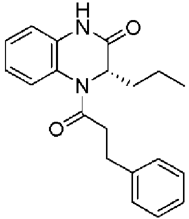 | (S)-4-(3-Phenylpropanoyl)-3-propyl-3,4-dihydrochinoxalin-2(1H)-on |
| 20 | 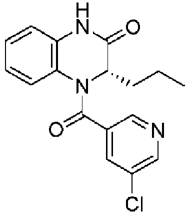 | (S)-4-(5-Chlornicotinoyl)-3-propyl-3,4-dihydrochinoxalin-2(1H)-on |
| 21 | 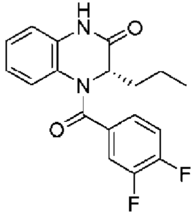 | (S)-4-(3,4-Difluorbenzoyl)-3-propyl-3,4-dihydrochinoxalin-2(1H)-on |
| 22 | 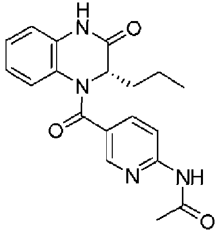 | (S)-N-(5-(3-Oxo-2-propyl-1,2,3,4-tetrahydrochinoxalin-1-carbonyl)pyridin-2-yl)acetamid |
| 23 | 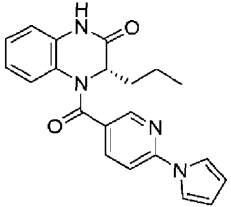 | (S)-4-(6-(1H-Pyrrol-1-yl)nicotinoyl)-3-propyl-3,4-dihydrochinoxalin-2(1H)-on |
| 24 | 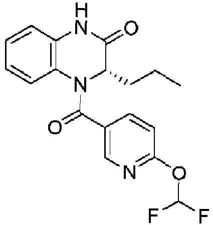 | (S)-4-(6-(Difluormethoxy)nicotinoy l)-3-propyl-3,4-dihydrochinoxalin-2(1H)-on |
| 25 | 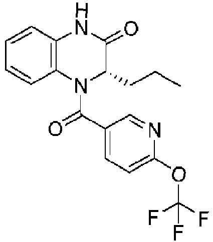 | (S)-3-Propyl-4-(6-(trifluormethoxy)nicotino yl)-3,4-dihydrochinoxalin-2(1H)-on |
| 26 | 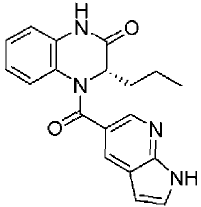 | (S)-3-Propyl-4-(1H-pyrrolo[2,3-b]pyridin-5-carbonyl)-3,4-dihydrochinoxalin-2(1H)-on |
| 27 | 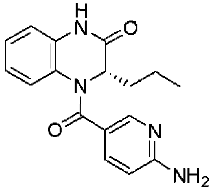 | (S)-4-(6-Aminonicotinoyl)-3-propyl-3,4-dihydrochinoxalin-2(1H)-on |
| 28 | 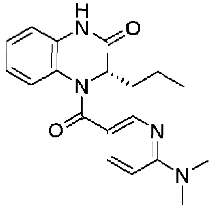 | (S)-4-(6-(Dimethylamino)nicotinoyl )-3-propyl-3,4-dihydrochinoxalin-2(1H)-on |
| 29 | 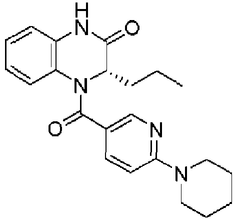 | (S)-4-(6-(Piperidin-1-yl)nicotinoyl)-3-propyl-3,4-dihydrochinoxalin-2(1H)-on |
| 30 | 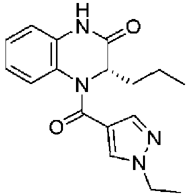 | (S)-4-(1-Ethyl-1H-pyrazol-4-carbonyl)-3-propyl-3,4-dihydrochinoxalin-2(1H)-on |
| 31 | 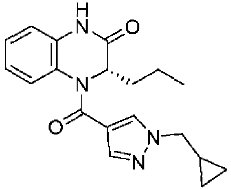 | (S)-4-(1-(Cyclopropylmethyl)-1H-pyrazol-4-carbonyl)-3-propyl-3,4-dihydrochinoxalin-2(1H)-on |
| 32 | 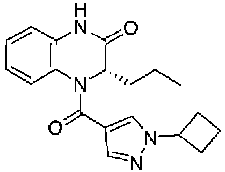 | (S)-4-(1-Cyclobutyl-1H-pyrazol-4-carbonyl)-3-propyl-3,4-dihydrochinoxalin-2(1H)-on |
| 33 | 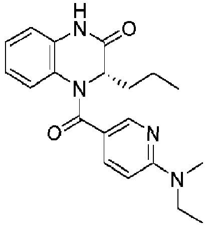 | (S)-4-(6-(Ethyl(methyl)amino)nicot inoyl)-3-propyl-3,4-dihydrochinoxalin-2(1H)-on |
| 34 | 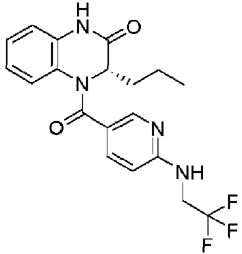 | (S)-3-Propyl-4-(6-((2,2,2-trifluorethyl)amino)nicot inoyl)-3,4-dihydrochinoxalin-2(1H)-on |
| 35 | 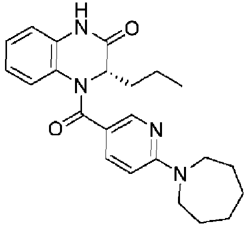 | (S)-4-(6-(Azepan-1-yl)nicotinoyl)-3-propyl-3,4-dihydrochinoxalin-2(1H)-on |
| 36 | 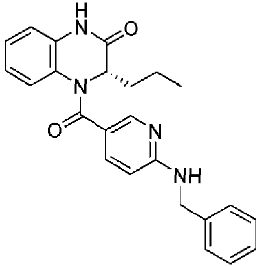 | (S)-4-(6-(Benzylamino)nicotinoyl)-3-propyl-3,4-dihydrochinoxalin-2(1H)-on |
| 37 | 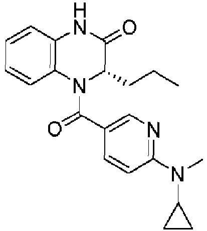 | (S)-4-(6-(Cyclopropyl(methyl)amino )nicotinoyl)-3-propyl-3,4-dihydrochinoxalin-2(1H)-on |
| 38 | 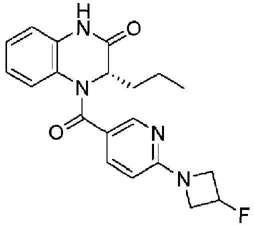 | (S)-4-(6-(3-Fluorazetidin-1-yl)nicotinoyl)-3-propyl-3,4-dihydrochinoxalin-2(1H)-on |
| 39 | 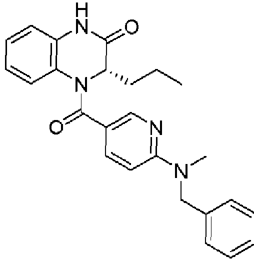 | (S)-4-(6-(Benzyl(methyl)amino)nico tinoyl)-3-propyl-3,4-dihydrochinoxalin-2(1H)-on |
| 40 | 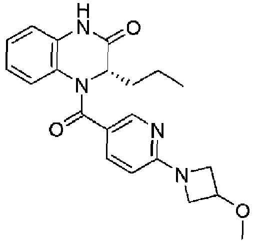 | (S)-4-(6-(3-Methoxyazetidin-1-yl)nicotinoyl)-3-propyl-3,4-dihydrochinoxalin-2(1H)-on |
| 41 | 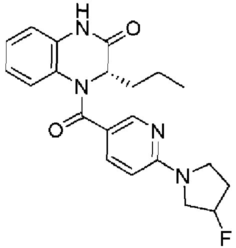 | (3S)-4-(6-(3-Fluorpyrrolidin-1-yl)nicotinoyl)-3-propyl-3,4-dihydrochinoxalin-2(1H)-on |
| 42 | 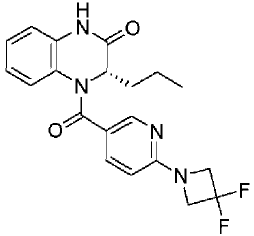 | (S)-4-(6-(3,3-Difluorazetidin-1-yl)nicotinoyl)-3-propyl-3,4-dihydrochinoxalin-2(1H)-on |
| 43 | 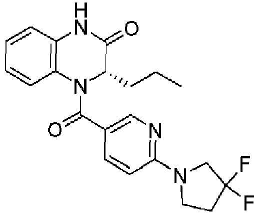 | (S)-4-(6-(3,3-Difluorpyrrolidin-1-yl)nicotinoyl)-3-propyl-3,4-dihydrochinoxalin-2(1H)-on |
| 44 | 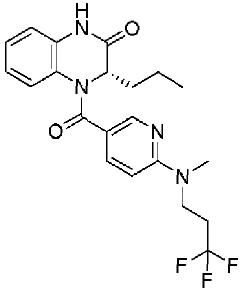 | (S)-4-(6-(Methyl(3,3,3-trifluorpropyl)amino)nico tinoyl)-3-propyl-3,4-dihydrochinoxalin-2(1H)-on |
| 45 | 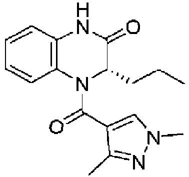 | (S)-4-(1,3-Dimethyl-1H-pyrazol-4-carbonyl)-3-propyl-3,4-dihydrochinoxalin-2(1H)-on |
| 46 | 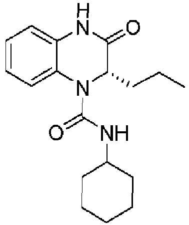 | (S)-N-Cyclohexyl-3-oxo-2-propyl-3,4-dihydrochinoxalin-1(2H)-carboxamid |
| 47 | 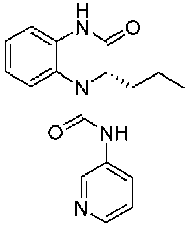 | (S)-3-Oxo-2-propyl-N-(pyridin-3-yl)-3,4-dihydrochinoxalin-1(2H)-carboxamid |
| 48 | 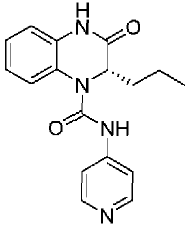 | (S)-3-Oxo-2-propyl-N-(pyridin-4-yl)-3,4-dihydrochinoxalin-1(2H)-carboxamid |
| 49 | 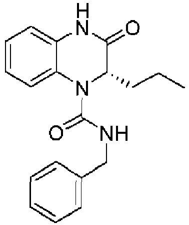 | (S)-N-Benzyl-3-oxo-2-propyl-3,4-dihydrochinoxalin-1(2H)-carboxamid |
| 50 | 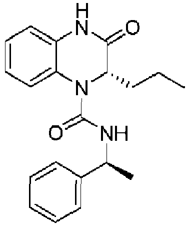 | (S)-3-Oxo-N-((S)-1-phenylethyl)-2-propyl-3,4-dihydrochinoxalin-1(2H)-carboxamid |
| 51 | 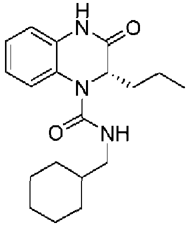 | (S)-N-(Cyclohexylmethyl)-3-oxo-2-propyl-3,4-dihydrochinoxalin-1(2H)-carboxamid |
| 52 | 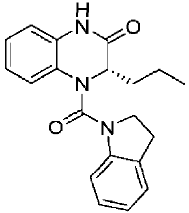 | (S)-4-(Indolin-1-carbonyl)-3-propyl-3,4-dihydrochinoxalin-2(1H)-on |
| 53 | 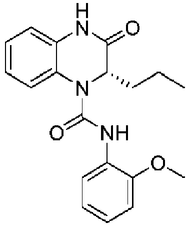 | (S)-N-(2-Methoxyphenyl)-3-oxo-2-propyl-3,4-dihydrochinoxalin-1(2H)-carboxamid |
| 54 | 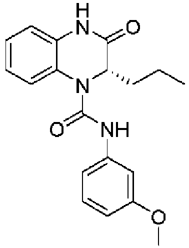 | (S)-N-(3-Methoxyphenyl)-3-oxo-2-propyl-3,4-dihydrochinoxalin-1(2H)-carboxamid |
| 55 | 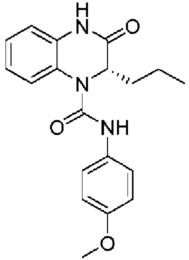 | (S)-N-(4-Methoxyphenyl)-3-oxo-2-propyl-3,4-dihydrochinoxalin-1(2H)-carboxamid |
| 56 | 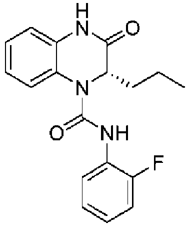 | (S)-N-(2-Fluorphenyl)-3-oxo-2-propyl-3,4-dihydrochinoxalin-1(2H)-carboxamid |
| 57 | 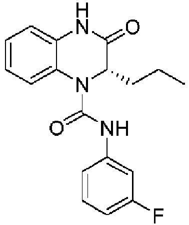 | (S)-N-(3-Fluorphenyl)-3-oxo-2-propyl-3,4-dihydrochinoxalin-1(2H)-carboxamid |
| 58 | 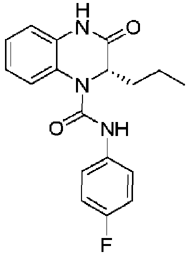 | (S)-N-(4-Fluorphenyl)-3-oxo-2-propyl-3,4-dihydrochinoxalin-1(2H)-carboxamid |
| 59 | 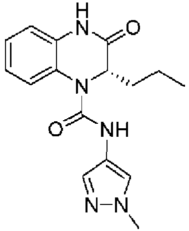 | (S)-N-(1-Methyl-1H-pyrazol-4-yl)-3-oxo-2-propyl-3,4-dihydrochinoxalin-1(2H)-carboxamid |
| 60 | 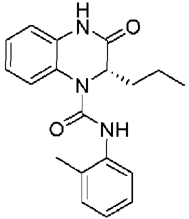 | (S)-3-Oxo-2-propyl-N-(o-tolyl)-3,4-dihydrochinoxalin-1(2H)-carboxamid |
| 61 | 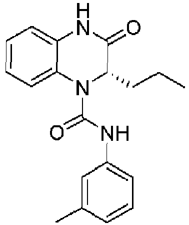 | (S)-3-Oxo-2-propyl-N-(m-tolyl)-3,4-dihydrochinoxalin-1(2H)-carboxamid |
| 62 | 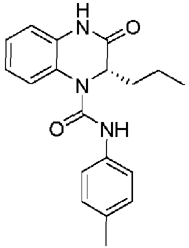 | (S)-3-Oxo-2-propyl-N-(p-tolyl)-3,4-dihydrochinoxalin-1(2H)-carboxamid |
| 63 | 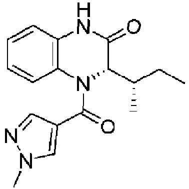 | (S)-3-((S)-sec-Butyl)-4-(1-methyl-1H-pyrazol-4-carbonyl)-3,4-dihydrochinoxalin-2(1H)-on |
| 64 | 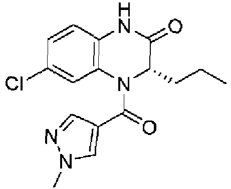 | (S)-6-Chlor-4-(1-methyl-1H-pyrazol-4-carbonyl)-3-propyl-3,4-dihydrochinoxalin-2(1H)-on |
| 65 | 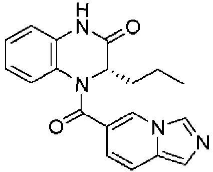 | (S)-4-(Imidazo[1,5-a]pyridin-6-carbonyl)-3-propyl-3,4-dihydrochinoxalin-2(1H)-on |
| 66 | 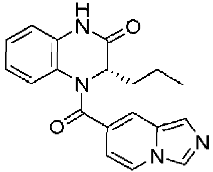 | (S)-4-(Imidazo[1,5-a]pyridin-7-carbonyl)-3-propyl-3,4-dihydrochinoxalin-2(1H)-on |
| 67 | 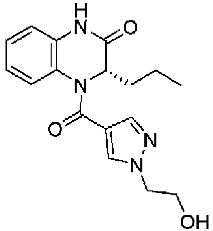 | (S)-4-(1-(2-Hydroxyethyl)-1H-pyrazol-4-carbonyl)-3-propyl-3,4-dihydrochinoxalin-2(1H)-on |
| 68 | 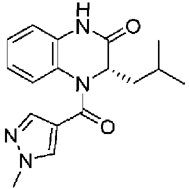 | (S)-3-Isobutyl-4-(1-methyl-1H-pyrazol-4-carbonyl)-3,4-dihydrochinoxalin-2(1H)-on |
| 69 | 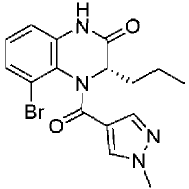 | (S)-5-Brom-4-(1-methyl-1H-pyrazol-4-carbonyl)-3-propyl-3,4-dihydrochinoxalin-2(1H)-on |
| 70 | 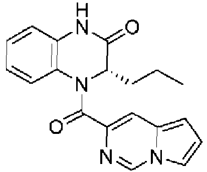 | (S)-3-Propyl-4-(pyrrolo[1,2-c]pyrimidin-3-carbonyl)-3,4-dihydrochinoxalin-2(1H)-on |
| 71 | 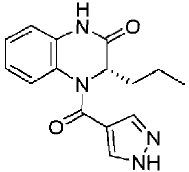 | (S)-3-Propyl-4-(1H-pyrazol-4-carbonyl)-3,4-dihydrochinoxalin-2(1H)-on |
| 72 | 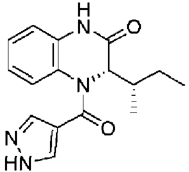 | (S)-3-((S)-sec-Butyl)-4-(lH-pyrazol-4-carbonyl)-3,4-dihydrochinoxalin-2(1H)-on |
| 73 | 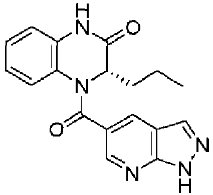 | (S)-3-Propyl-4-(1H-pyrazolo[3,4-b]pyridin-5-carbonyl)-3,4-dihydrochinoxalin-2(1H)-on |
| 74 | 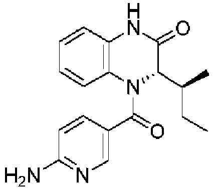 | (S)-4-(6-Aminonicotinoyl)-3-((S)-sec-butyl)-3,4-dihydrochinoxalin-2(1H)-on |
| 75 | 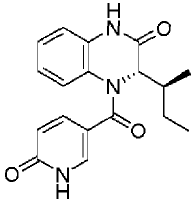 | (S)-3-((S)-sec-Butyl)-4-(6-oxo-1,6-dihydropyridin-3-carbonyl)-3,4-dihydrochinoxalin-2(1H)-on |
| 76 | 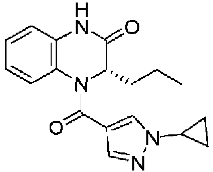 | (S)-4-(1-Cyclopropyl-1H-pyrazol-4-carbonyl)-3-propyl-3,4-dihydrochinoxalin-2(1H)-on |
| 77 | 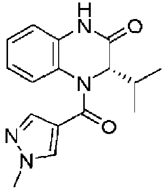 | (S)-3-Isopropyl-4-(1-methyl-1H-pyrazol-4-carbonyl)-3,4-dihydrochinoxalin-2(1H)-on |
| 78 | 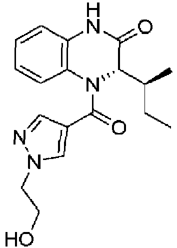 | (S)-3-((S)-sec-Butyl)-4-(1-(2-hydroxyethyl)-1H-pyrazol-4-carbonyl)-3,4-dihydrochinoxalin-2(1H)-on |
| 79 | 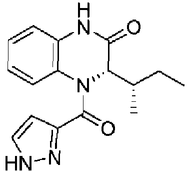 | (S)-3-((S)-sec-Butyl)-4-(1H-pyrazol-3-carbonyl)-3,4-dihydrochinoxalin-2(1H)-on |
| 80 | 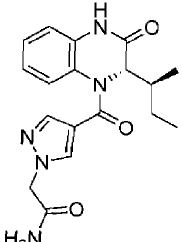 | 2-(4-((S)-2-((S)-sec-Butyl)-3-oxo-1,2,3,4-tetrahydrochinoxalin-1-carbonyl)-1H-pyrazol-1-yl)acetamid |
| 81 | 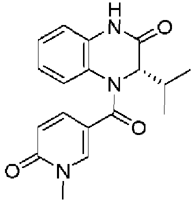 | (S)-3-Isopropyl-4-(1-methyl-6-oxo-1,6-dihydropyridin-3-carbonyl)-3,4-dihydrochinoxalin-2(1H)-on |
| 82 | 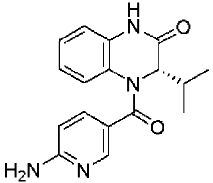 | (S)-4-(6-Aminonicotinoyl)-3-isopropyl-3,4-dihydrochinoxalin-2(1H)-on |
| 83 | 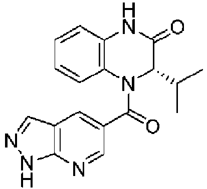 | (S)-3-Isopropyl-4-(1H-pyrazolo[3,4-b]pyridin-5-carbonyl)-3,4-dihydrochinoxalin-2(1H)-on |
| 84 | 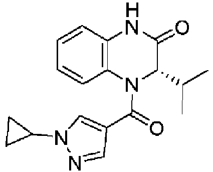 | (S)-4-(1-Cyclopropyl-1H-pyrazol-4-carbonyl)-3-isopropyl-3,4-dihydrochinoxalin-2(1H)-on |
| 85 | 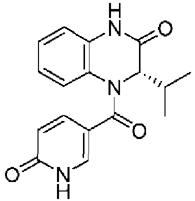 | (S)-3-Isopropyl-4-(6-oxo-1,6-dihydropyridin-3-carbonyl)-3,4-dihydrochinoxalin-2(1H)-on |
| 86 | 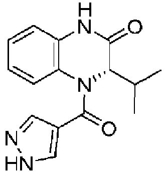 | (S)-3-Isopropyl-4-(1H-pyrazol-4-carbonyl)-3,4-dihydrochinoxalin-2(1H)-on |
| 87 | 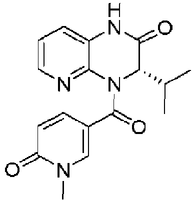 | (S)-3-Isopropyl-4-(1-methyl-6-oxo-1,6-dihydropyridin-3-carbonyl)-3,4-dihydropyrido[2,3-b]pyrazin-2(lH)-on |
| 88 | 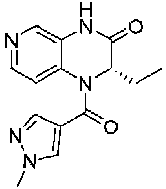 | (S)-2-Isopropyl-1-(1-methyl-1H-pyrazol-4-carbonyl)-1,4-dihydropyrido[3,4-b]pyrazin-3(2H)-on |
| 89 | 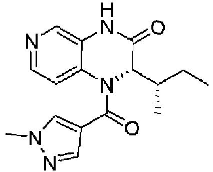 | (S)-2-((S)-sec-Butyl)-1-(1-methyl-1H-pyrazol-4-carbonyl)-1,4-dihydropyrido[3,4-b]pyrazin-3(2H)-on |
| 90 | 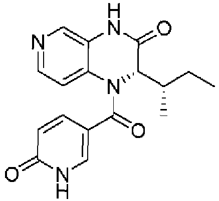 | (S)-2-((S)-sec-Butyl)-1-(6-oxo-1,6-dihydropyridin-3-carbonyl)-1,4-dihydropyrido[3,4-b]pyrazin-3(2H)-on |
| 91 | 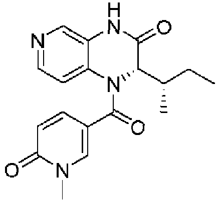 | (S)-2-((S)-sec-Butyl)-1-(1-methyl-6-oxo-1,6-dihydropyridin-3-carbonyl)-1,4-dihydropyrido[3,4-b]pyrazin-3(2H)-on |
| 92 | 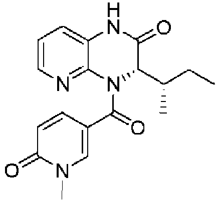 | (S)-3-((S)-sec-Butyl)-4-(1-methyl-6-oxo-1,6-dihydropyridin-3-carbonyl)-3,4-dihydropyrido[2,3-b]pyrazin-2(1H)-on |
| 93 | 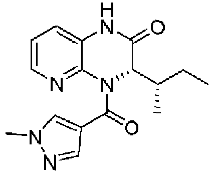 | (S)-3-((S)-sec-Butyl)-4-(1-methyl-1H-pyrazol-4-carbonyl) -3,4-dihydropyrido[2,3-b]pyrazin-2(1H)-on |
| 94 | 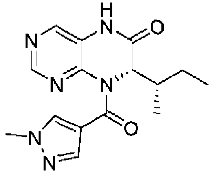 | (S)-7-((S)-sec-Butyl)-8-(1-methyl-1H-pyrazol-4-carbonyl)-7,8-dihydropteridin-6(5H)-on |
| 95 | 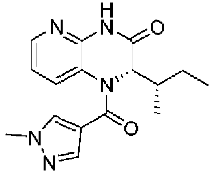 | (S)-2-((S)-sec-Butyl)-1-(1-methyl-1H-pyrazol-4-carbonyl)-1,4-dihydropyrido[2,3-b]pyrazin-3(2H)-on |
| 96 | 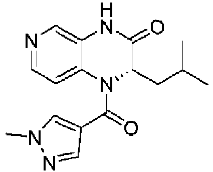 | (S)-2-Isobutyl-1-(1-methyl-1H-pyrazol-4-carbonyl)-1,4-dihydropyrido[3,4-b]pyrazin-3(2H)-on |
| 97 | 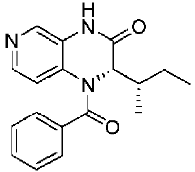 | (S)-1-Benzoyl-2-((S)-sec-butyl)-1,4-dihydropyrido[3,4-b]pyrazin-3(2H)-on |
| 98 | 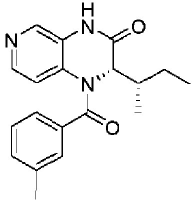 | (S)-2-((S)-sec-Butyl)-1-(3-methylbenzoyl)-1,4-dihydropyrido[3,4-b]pyrazin-3(2H)-on |
| 99 | 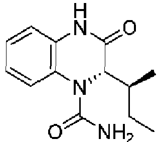 | (S)-2-((S)-sec-Butyl)-3-oxo-3,4-dihydrochinoxalin-1(2H)-carboxamid |
| 100 | 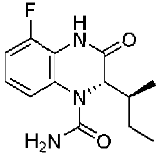 | (S)-2-((S)-sec-Butyl)-5-fluor-3-oxo-3,4-dihydrochinoxalin-1(2H)-carboxamid |
| 101 | 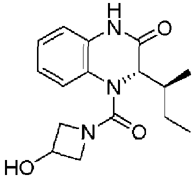 | (S)-3-((3)-sec-Butyl)-4-(3-hydroxyazetidin-1-carbonyl)-3,4-dihydrochinoxalin-2(1H)-on |
| 102 | 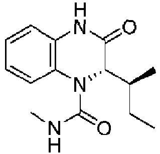 | (S)-2-((S)-sec-Butyl)-N-methyl-3-oxo-3,4-dihydrochinoxalin-1(2H)-carboxamid |
| 103 | 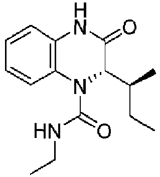 | (S)-2-((S)-sec-Butyl)-N-ethyl-3-oxo-3,4-dihydrochinoxalin-1(2H)-carboxamid |
| 104 | 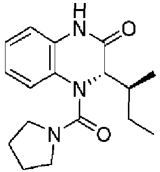 | (S)-3-((S)-sec-Butyl)-4-(pyrrolidin-1-carbonyl)-3,4-dihydrochinoxalin-2(1H)-on |
| 105 | 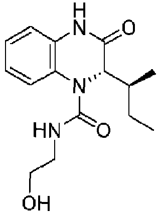 | (S)-2-((S)-sec-Butyl)-N-(2-hydroxyethyl)-3-oxo-3,4-dihydrochinoxalin-1(2H)-carboxamid |
| 106 | 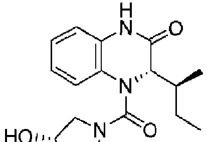 | (S)-3-((S)-sec-Butyl)-4-((S)-3-hydroxypyrrolidin-1-carbonyl)-3,4-dihydrochinoxalin-2(1H)-on |
| 107 | 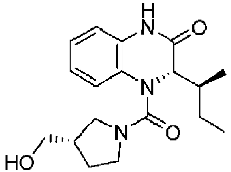 | (S)-3-((S)-sec-Butyl)-4-((S)-3-(hydroxymethyl)pyrrolidin -1-carbonyl)-3,4-dihydrochinoxalin-2(1H)-on |
| 108 | 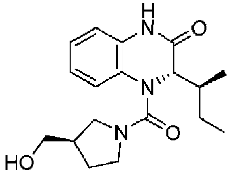 | (S)-3-((S)-sec-Butyl)-4-((R)-3-(hydroxymethyl)pyrrolidin -1-carbonyl)-3,4-dihydrochinoxalin-2(1H)-on |
| 109 | 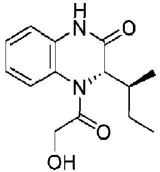 | (S)-3-((S)-sec-Butyl)-4-(2-hydroxyacetyl)-3,4-dihydrochinoxalin-2(1H)-on |
| 110 | 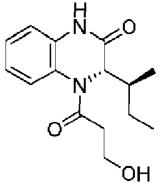 | (S)-3-((S)-sec-Butyl)-4-(3-hydroxypropanoyl)-3,4-dihydrochinoxalin-2(1H)-on |
| 111 | 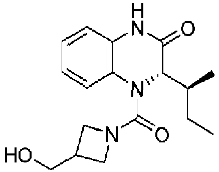 | (S)-3-((S)-sec-Butyl)-4-(3-(hydroxymethyl)azetidin-1-carbonyl)-3,4-dihydrochinoxalin-2(1H)-on |
| 112 | 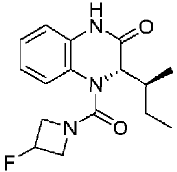 | (S)-3-((S)-sec-Butyl)-4-(3-fluorazetidin-1-carbonyl)-3,4-dihydrochinoxalin-2(1H)-on |
| 113 | 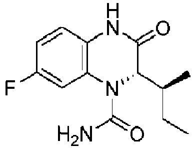 | (S)-2-((S)-sec-Butyl)-7-fluor-3-oxo-3,4-dihydrochinoxalin-1(2H)-carboxamid |
| 114 | 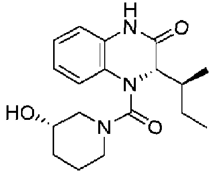 | (S)-3-((S)-sec-Butyl)-4-((S)-3-hydroxypiperidin-1-carbonyl)-3,4-dihydrochinoxalin-2(1H)-on |
| 115 | 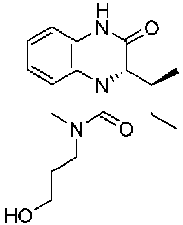 | (S)-2-((S)-sec-Butyl)-N-(3-hydroxypropyl)-N-methyl-3-oxo-3,4-dihydrochinoxalin-1(2H)-carboxamid |
| 116 | 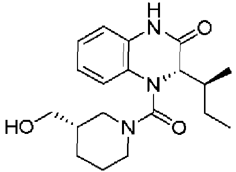 | (S)-3-((S)-sec-Butyl)-4-((S)-3-(hydroxymethyl)piperidin-1-carbonyl)-3,4-dihydrochinoxalin-2(1H)-on |
| 117 | 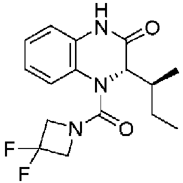 | (S)-3-((S)-sec-Butyl)-4-(3,3-difluorazetidin-1-carbonyl)-3,4-dihydrochinoxalin-2(1H)-on |
| 118 | 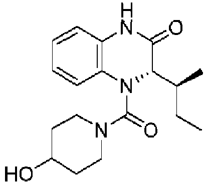 | (S)-3-((S)-sec-Butyl)-4-(4-hydroxypiperidin-1-carbonyl)-3,4-dihydrochinoxalin-2(1H)-on |
| 119 | 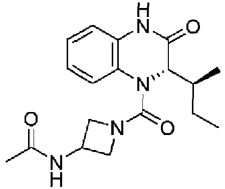 | N-(1-((S)-2-((S)-sec-Butyl)-3-oxo-1,2,3,4-tetrahydrochinoxalin-1-carbonyl)azetidin-3-yl)acetamid |
| 120 | 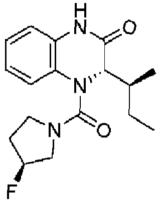 | (S)-3-((S)-sec-Butyl)-4-((S)-3-fluorpyrrolidin-1-carbonyl)-3,4-dihydrochinoxalin-2(1H)-on |
| 121 | 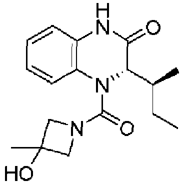 | (S)-3-((S)-sec-Butyl)-4-(3-hydroxy-3-methylazetidin-1-carbonyl)-3,4-dihydrochinoxalin-2(1H)-on |
| 122 | 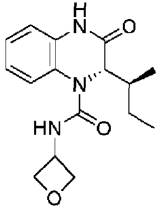 | (S)-2-((S)-sec-Butyl)-N-(oxetan-3-yl)-3-oxo-3,4-dihydrochinoxalin-1(2H)-carboxamid |
| 123 | 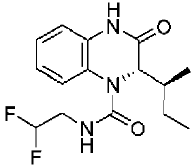 | (S)-2-((S)-sec-Butyl)-N-(2,2-difluorethyl)-3-oxo-3,4-dihydrochinoxalin-1(2H)-carboxamid |
| 124 | 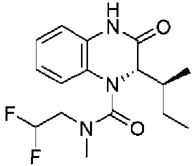 | (S)-2-((S)-sec-Butyl)-N-(2,2-difluorethyl)-N-methyl-3-oxo-3,4-dihydrochinoxalin-1(2H)-carboxamid |
| 125 | 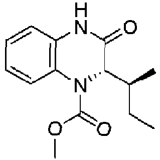 | Methyl-(S)-2-((S)-sec-butyl)-3-oxo-3,4-dihydrochinoxalin-1(2H)-carboxylat |
| 126 | 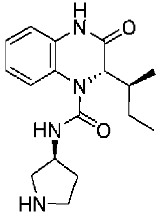 | (S)-2-((S)-sec-Butyl)-3-oxo-N-((S)-pyrrolidin-3-yl)-3,4-dihydrochinoxalin-1(2H)-carboxamid |
| 127 | 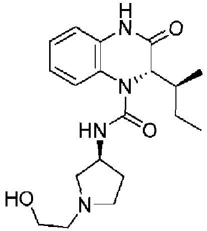 | (S)-2-((S)-sec-Butyl)-N-((S)-1-(2-hydroxyethyl)pyrrolidin-3-yl)-3-oxo-3,4-dihydrochinoxalin-1(2H)-carboxamid |
| 128 | 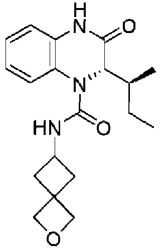 | (S)-2-((S)-sec-Butyl)-3-oxo-N-(2-oxaspiro[3.3]heptan-6-yl)-3,4-dihydrochinoxalin-1(2H)-carboxamid |
| 129 | 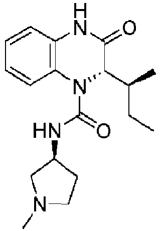 | (S)-2-((S)-sec-Butyl)-N-((S)-1-methylpyrrolidin-3-yl)-3-oxo-3,4-dihydrochinoxalin-1(2H)-carboxamid |
| 130 | 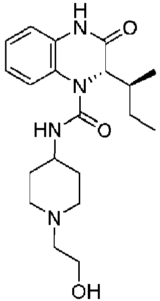 | (S)-2-((S)-sec-Butyl)-N-(1-(2-hydroxyethyl)piperidin-4-yl)-3-oxo-3,4-dihydrochinoxalin-1(2H)-carboxamid |
| 131 | 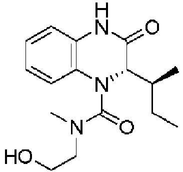 | (S)-2-((S)-sec-Butyl)-N-(2-hydroxyethyl)-N-methyl-3-oxo-3,4-dihydrochinoxalin-1(2H)-carboxamid |
| 132 | 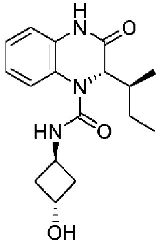 | (S)-2-((S)-sec-Butyl)-N-((1r,3S)-3-hydroxycyclobutyl)-3-oxo-3,4-dihydrochinoxalin-1(2H)-carboxamid |
| 133 | 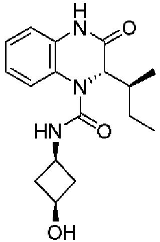 | (S)-2-((S)-sec-Butyl)-N-((1s,3R)-3-hydroxycyclobutyl)-3-oxo-3,4-dihydrochinoxalin-1(2H)-carboxamid |
oder ein pharmazeutisch annehmbares Salz davon.

10. Verbindung nach Anspruch 1 der Formel: 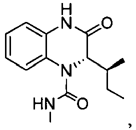 oder ein pharmazeutisch annehmbares Salz davon.

11. Verbindung nach Anspruch 1 der Formel: 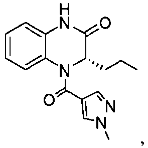 oder ein pharmazeutisch annehmbares Salz davon.

12. Verbindung nach Anspruch 1 der Formel: 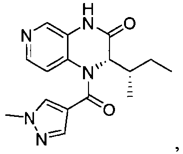 oder ein pharmazeutisch annehmbares Salz davon.

13. Pharmazeutische Zusammensetzung umfassend eine Verbindung nach einem der Ansprüche 1-12 oder ein pharmazeutisch annehmbares Salz davon und einen pharmazeutischen Träger.

14. Pharmazeutische Zusammensetzung nach Anspruch 13, wobei die pharmazeutische Zusammensetzung für orale, sublinguale, subkutane, parenterale, intravenöse, intranasale, topische, transdermale, intraperitoneale, intramuskuläre, intrapulmonale, vaginale, rektale oder intraokulare Verabreichung formuliert ist.

15. Verbindung nach einem der Ansprüche 1-12 oder ein pharmazeutisch annehmbares Salz davon oder pharmazeutische Zusammensetzung nach Anspruch 13 oder 14 zur Verwendung bei der Behandlung einer Krankheit oder eines Leidens ausgewählt aus der Gruppe bestehend aus:
(a) periphere Gefäßerkrankung, periphere arterielle Erkrankung, Rehabilitationsdefizite, metabolisches Syndrom, Adipositas, beatmungsinduzierte Muskelschwäche, chronisches Ermüdungssyndrom, neuromuskuläre Störungen, Zustände von Muskelschwund, Muskelmyopathien, Muskelatrophie, Muskelermüdung und Gebrechlichkeit bei einem Subjekt;
(b) amyotrophe Lateralsklerose (ALS), spinale Muskelatrophie (SMA), Myasthenia gravis und muskuläre Myopathien bei einem Subjekt; oder
(c) Stress-Harninkontinenz (SUI), gemischte Harninkontinenz (MUI), fäkale Inkontinenz, Gebrechlichkeit, Sarkopenie, chronisch obstruktive Lungenerkrankung (COPD), Kachexie-Syndrom, Muskelschwund durch Herzinsuffizienz, Krebs oder chronische Nierenerkrankung/Dialyse, Muskeldysfunktion nach Rückenmarksverletzung (SCI) und Muskeldysfunktion nach Schlaganfall bei einem Subjekt.

## Revendications

1. Composé de formule (I) : 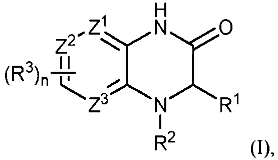
ou sel pharmaceutiquement acceptable correspondant,
Z¹, Z², et Z³ étant, indépendamment les uns des autres, CH, CR³, ou N, à condition qu'au moins l'un parmi Z¹, Z², et Z³ soit CH ou CR³ ;
R¹ étant C₃-C₄ alkyle non substitué ;
R² étant -C(=O)R⁴, -C (=O)- (CH₂)ₚ-R⁵, -C (=O) NR⁶R⁷, ou - C (=O) OCH₃ ;
chaque R³ étant indépendamment halogène, C₁-C₆-alkyle, C₁-C₆-halogénoalkyle, C₁-C₆-alcoxy, C₁-C₆-halogénoalcoxy, C₃-C₁₂-cycloalkyle, ou hétérocycloalkyle à 3 à 12 chaînons ; R⁴ étant C₆-C₁₂-aryle éventuellement substitué par un à cinq R^{4A}, hétéroaryle à 5 à 12 chaînons éventuellement substitué par un à cinq R^{4B}, C₃-C₁₂ cycloalkyle éventuellement substitué par un à cinq R^{4C}, hétérocycloalkyle à 4 à 12 chaînons éventuellement substitué par un à cinq R^{4D}, ou hétérocycloalcényle à 4 à 12 chaînons éventuellement substitué par un à cinq R^{4E} ; chaque R^{4A} étant indépendamment choisi dans le groupe constitué par halogéno, C₁-C₆-alkyle, C₁-C₆-halogénoalkyle, OH, C₁-C₆-alcoxy, C₁-C₆-halogénoalcoxy, NH₂, NH (C₁-C₆-alkyle), N (C₁-C₆-alkyle)₂, NH (C₁-C₆-halogénoalkyle), N (C₁-C₆-alkyl)(C₁-C₆-halogénoalkyle), N(C₁-C₆-halogénoalkyle)₂, NH(C₁-C₆-alkylène-(C₆-C₁₂-aryle)), N(C₁-C₆-alkyl)(C₁-C₆-alkylène-(C₆-C₁₂-aryle)), N(C₁-C₆-alkylène-(C₆-C₁₂-aryle))₂, NH (C₃-C₁₂ cycloalkyle), N(C₁-C₆-alkyl)(C₃-C₁₂ cycloalkyle), N (C₃-C₁₂ cycloalkyle)₂, NHC (O) -C₁-C₆-alkyle, C₃-C₁₂ cycloalkyle éventuellement substitué par un à cinq R^{4B1}, hétérocycloalkyle à 4 à 12 chaînons éventuellement substitué par un à cinq R^{4B1}, C₁-C₆-alkylène-OH, C₁-C₆-alkylène-CONH₂, C₁-C₆-alkylène- (C₃-C₁₂ cycloalkyle), C₁-C₆-alkylène- (hétérocycloalkyle à 4 à 12 chaînons), C₆-C₁₂-aryle, et hétéroaryle à 5 à 12 chaînons ;
chaque R^{4B} étant indépendamment choisi dans le groupe constitué par halogéno, C₁-C₆-alkyle, C₁-C₆-halogénoalkyle, OH, C₁-C₆-alcoxy, C₁-C₆-halogénoalcoxy, NH₂, NH (C₁-C₆-alkyle), N (C₁-C₆-alkyl)₂, NH (C₁-C₆-halogénoalkyle), N(C₁-C₆-alkyl)(C₁-C₆-halogénoalkyle), N(C₁-C₆-halogénoalkyl)₂, NH(C₁-C₆-alkylène-(C₆-C₁₂-aryle)), N(C₁-C₆-alkyl)(C₁-C₆-alkylène-(C₆-C₁₂-aryle)), N(C₁-C₆-alkylène-(C₆-C₁₂-aryl)) 2, NH (C₃-C₁₂ cycloalkyle), N(C₁-C₆-alkyl)(C₃-C₁₂ cycloalkyle), N (C₃-C₁₂ cycloalkyl) ₂, NHC (O) -C₁-C₆-alkyle, C₃-C₁₂ cycloalkyle éventuellement substitué par un à cinq R^{4B1}, hétérocycloalkyle à 4 à 12 chaînons éventuellement substitué par un à cinq R^{4B1}, C₁-C₆-alkylène-OH, C₁-C₆-alkylène-CONH₂, C₁-C₆-alkylène- (C₃-C₁₂ cycloalkyle), C₁-C₆-alkylène- (hétérocycloalkyle à 4 à 12 chaînons), et hétéroaryle à 5 à 12 chaînons ; chaque R^{4B1} étant indépendamment choisi dans le groupe constitué par halogéno, C₁-C₆-alkyle, C₁-C₆-halogénoalkyle, OH, C₁-C₆-alcoxy, C₁-C₆-halogénoalcoxy, et C₁-C₆-alkylène-OH ;
chaque R^{4C} étant indépendamment choisi dans le groupe constitué par halogéno, C₁-C₆-alkyle, C₁-C₆-halogénoalkyle, OH, C₁-C₆-alcoxy, C₁-C₆-halogénoalcoxy, NHC (O) -C₁-C₆-alkyle, et C₁-C₆-alkylène-OH ;
chaque R^{4D} étant indépendamment choisi dans le groupe constitué par halogéno, C₁-C₆-alkyle, C₁-C₆-halogénoalkyle, OH, C₁-C₆-alcoxy, C₁-C₆-halogénoalcoxy, NHC (O) -C₁-C₆-alkyle, et C₁-C₆-alkylène-OH ;
chaque R^{4E} étant indépendamment choisi dans le groupe constitué par oxo, halogéno, C₁-C₆-alkyle, C₁-C₆-halogénoalkyle, OH, C₁-C₆-alcoxy, C₁-C₆-halogénoalcoxy, NHC(O)-C₁-C₆-alkyle, et C₁-C₆-alkylène-OH ;
R⁵ étant OH, C₁-C₆-alcoxy, C₁-C₆-halogénoalcoxy, C₃-C₁₂ cycloalkyle, hétérocycloalkyle à 3 à 12 chaînons, ou C₆-C₁₂-aryle ;
R⁶ et R⁷, indépendamment l'un de l'autre, étant choisis dans le groupe constitué par H, C₁-C₆-alkyle, C₁-C₆-halogénoalkyle, C₃-C₁₂ cycloalkyle éventuellement substitué par un à cinq R⁸, hétérocycloalkyle à 4 à 12 chaînons éventuellement substitué par un à cinq R⁸, C₆-C₁₂-aryle éventuellement substitué par un à cinq R⁸, hétéroaryle à 5 à 12 chaînons éventuellement substitué par un à cinq R⁸, C₁-C₆-alkylène-OH, C₁-C₆-alkylène-(C₃-C₁₂ cycloalkyle), C₁-C₆-alkylène-(hétérocycloalkyle à 4 à 12 chaînons), C₁-C₆-alkylène-(C₆-C₁₂-aryle), et C₁-C₆-alkylène-(hétéroaryle à 5 à 12 chaînons) ;
chaque R⁸ étant indépendamment choisi dans le groupe constitué par halogéno, C₁-C₆-alkyle, C₁-C₆-halogénoalkyle, OH, C₁-C₆-alcoxy, C₁-C₆-alkylène-OH, et C₁-C₆-halogénoalcoxy ;
n étant 0, 1, 2, 3, ou 4 ;
p étant 1, 2, ou 3 ; et
à condition que le composé de formule (I) ne soit pas un composé choisi dans le groupe constitué par :
4-(3-méthylbenzoyl)-3-propyl-3,4-dihydroquinoxalin-2(1H)-one,
4-(2-chlorobenzoyl)-3-isobutyl-3,4-dihydroquinoxalin-2(1H)-one, et
4-(4-(tert-butyl)benzoyl)-3-propyl-3,4-dihydroquinoxalin-2(1H)-one.

2. Composé selon la revendication 1, le composé de formule (I) étant un composé de formule (I-a), (I-b), (I-c), (I-d), ou (I-e) : 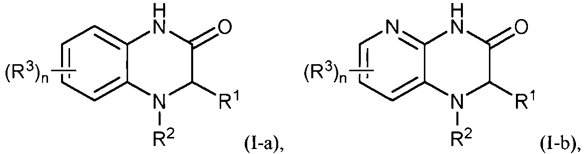 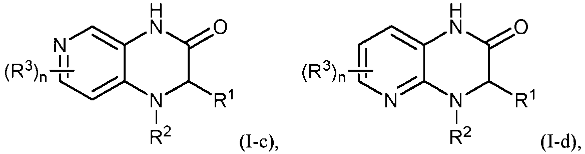 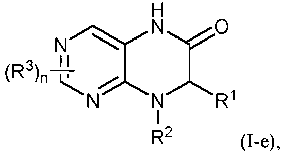 ou sel pharmaceutiquement acceptable correspondant.

3. Composé selon la revendication 1, le composé de formule (I) étant un composé de formule (II) : 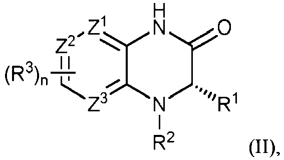 ou sel pharmaceutiquement acceptable correspondant.

4. Composé selon la revendication 3, le composé de formule (II) étant un composé de formule (II-a), (II-b), (II-c), (II-d), ou (II-e) : 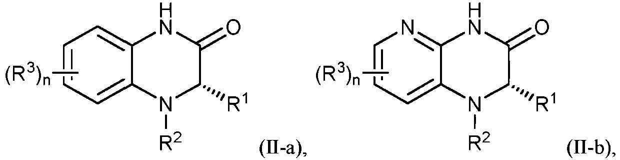 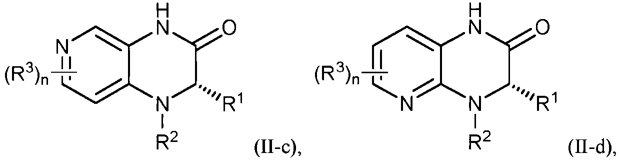 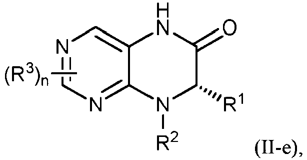 ou sel pharmaceutiquement acceptable correspondant,
et/ou le composé de formule (II) étant un composé de formule (II-a) : 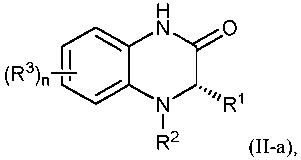
ou sel pharmaceutiquement acceptable correspondant.

5. Composé selon l'une quelconque des revendications 1-4, ou sel pharmaceutiquement acceptable correspondant, R¹ étant choisi dans le groupe constitué par prop-1-yle, prop-2-yle, but-2-yle, et 2-méthylprop-1-yle.

6. Composé selon l'une quelconque des revendications 1-5, ou sel pharmaceutiquement acceptable correspondant,
(a) R³ étant halogène ou C₃-C₁₂-cycloalkyle ;
(b) R³ étant halogène ; ou
(c) R³ étant C₃-C₆-cycloalkyle.

7. Composé selon l'une quelconque des revendications 1-6, ou sel pharmaceutiquement acceptable correspondant, n étant 0 ou 1.

8. Composé selon l'une quelconque des revendications 1-7, ou sel pharmaceutiquement acceptable correspondant,
(a) R² étant -C(=O)R⁴, éventuellement :
(a) (i) R⁴ étant C₆-C₁₂-aryle éventuellement substitué par un à cinq R^{4A}, hétéroaryle à 5 à 12 chaînons éventuellement substitué par un à cinq R^{4B}, C₃-C₁₂ cycloalkyle, hétérocycloalkyle à 4 à 12 chaînons éventuellement substitué par un à cinq R^{4D}, ou hétérocycloalcényle à 4 à 12 chaînons éventuellement substitué par un à cinq R^{4E} ;
(a) (ii) R⁴ étant C₆-C₁₂-aryle substitué par un à cinq R^{4A} et R^{4A} étant halogéno ou C₁-C₆-alkyle ;
(a) (iii) R⁴ étant hétéroaryle à 5 à 12 chaînons substitué par un à cinq R^{4B} et R^{4B} étant choisi dans le groupe constitué par halogéno, C₁-C₆-alkyle, C₁-C₆-halogénoalcoxy, NH₂, N(C₁-C₆-alkyl)₂, NH(C₁-C₆-halogénoalkyle), N(C₁-C₆-alkyl)(C₁-C₆-halogénoalkyle), NH(C₁-C₆-alkylène-(C₆-C₁₂-aryle)), N(C₁-C₆-alkyl)(C₃-C₁₂ cycloalkyle), NHC(O)-C₁-C₆-alkyle, C₃-C₁₂ cycloalkyle, hétérocycloalkyle à 4 à 12 chaînons éventuellement substitué par un à cinq R^{4B1}, C₁-C₆-alkylène-OH, C₁-C₆-alkylène-CONH₂, C₁-C₆-alkylène-(C₃-C₁₂ cycloalkyle), et hétéroaryle à 5 à 12 chaînons, éventuellement, R^{4B} étant hétérocycloalkyle à 4 à 12 chaînons substitué par un à cinq R^{4B1} et R^{4B1} étant halogéno ou C₁-C₆-alcoxy ;
(a) (iv) R⁴ étant hétérocycloalkyle à 4 à 12 chaînons substitué par un à cinq R^{4D} et R^{4D} étant choisi dans le groupe constitué par halogéno, C₁-C₆-alkyle, OH, NHC(O)-C₁-C₆-alkyle, et C₁-C₆-alkylène-OH ; ou
(a) (v) R⁴ étant hétérocycloalcényle à 4 à 12 chaînons substitué par un à cinq R^{4E} et R^{4E} étant oxo ou C₁-C₆-alkyle ; ou
(b) R² étant -C(=O)-(CH₂)ₚ-R⁵, éventuellement :
(b) (i) R⁵ étant OH ou C₆-C₁₂-aryle ; et/ou
(b) (ii) p étant 1 ou 2 ; ou
(c) R² étant -C(=O)NR⁶R⁷, éventuellement :
(c) (i) R⁶ et R⁷, indépendamment l'un de l'autre, étant choisis dans le groupe constitué par H, C₁-C₆-alkyle, C₁-C₆-halogénoalkyle, C₃-C₁₂ cycloalkyle, C₆-C₁₂-aryle éventuellement substitué par un à cinq R⁸, hétéroaryle à 5 à 12 chaînons éventuellement substitué par un à cinq R⁸, C₁-C₆-alkylène-OH, C₁-C₆-alkylène-(C₃-C₁₂ cycloalkyle), C₁-C₆-alkylène-(C₆-C₁₂-aryle), et C₁-C₆-alkylène-(hétéroaryle à 5 à 12 chaînons), éventuellement, chaque R⁸ étant indépendamment choisi dans le groupe constitué par halogéno, C₁-C₆-alkyle, C₁-C₆-alkylène-OH, et C₁-C₆-alcoxy ; ou
(d) R² étant -C(=O)OCH₃.

9. Composé selon la revendication 1 choisi dans le groupe constitué par les composés 1-133:
| composé n° | structure du composé | nom du composé |
|---|---|---|
| 1 | 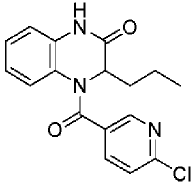 | 4-(6-chloronicotinoyl)-3-propyl-3,4-dihydroquinoxalin-2(1H)-one |
| 2 | 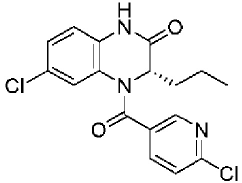 | (S)-6-chloro-4-(6-chloronicotinoyl)-3-propyl-3,4-dihydroquinoxalin-2(1H)-one |
| 3 | 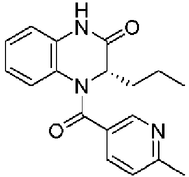 | (S)-4-(6-méthylnicotinoyl)-3-propyl-3,4-dihydroquinoxalin-2(1H)-one |
| 4 | 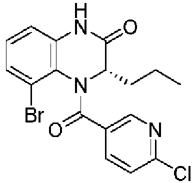 | (S)-5-bromo-4-(6-chloronicotinoyl)-3-propyl-3,4-dihydroquinoxalin-2(1H)-one |
| composé n∘ | structure du composé | nom du composé |
|---|---|---|
| 5 | 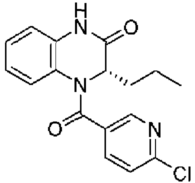 | (S)-4-(6-chloronicotinoyl)-3-propyl-3,4-dihydroquinoxalin-2(1H)-one |
| 6 | 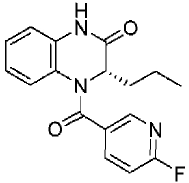 | (S)-4-(6-fluoronicotinoyl)-3-propyl-3,4-dihydroquinoxalin-2(1H)-one |
| 7 | 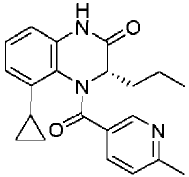 | (S)-5-cyclopropyl-4-(6-méthylnicotinoyl)-3-propyl-3,4-dihydroquinoxalin-2(1H)-one |
| 8 | 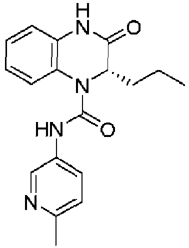 | (S)-N-(6-méthylpyridin-3-yl)-3-oxo-2-propyl-3,4-dihydroquinoxaline-1(2H)-carboxamide |
| 9 | 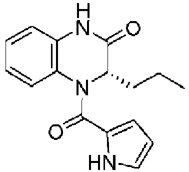 | (S)-3-propyl-4-(1H-pyrrole-2-carbonyl)-3,4-dihydroquinoxalin-2(1H)-one |
| 10 | 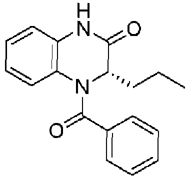 | (S)-4-benzoyl-3-propyl-3,4-dihydroquinoxalin-2(1H)-one |
| composé n° | structure du composé | nom du composé |
|---|---|---|
| 11 | 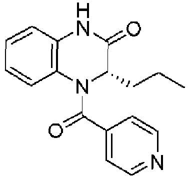 | (S)-4-isonicotinoyl-3-propyl-3,4-dihydroquinoxalin-2(1H)-one |
| 12 | 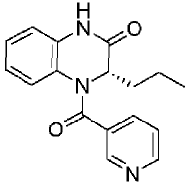 | (S)-4-nicotinoyl-3-propyl-3,4-dihydroquinoxalin-2(1H)-one |
| 13 | 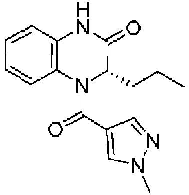 | (S)-4-(1-méthyl-1H-pyrazole-4-carbonyl)-3-propyl-3,4-dihydroquinoxalin-2(1H)-one |
| 14 | 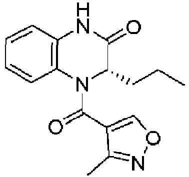 | (S)-4-(3-méthylisoxazole-4-carbonyl)-3-propyl-3,4-dihydroquinoxalin-2(1H)-one |
| 15 | 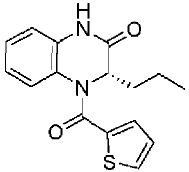 | (S)-3-propyl-4-(thiophène-2-carbonyl)-3,4-dihydroquinoxalin-2(1H)-one |
| 16 | 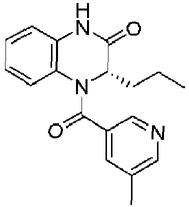 | (S)-4-(5-méthylnicotinoyl)-3-propyl-3,4-dihydroquinoxalin-2(1H)-one |
| 17 | 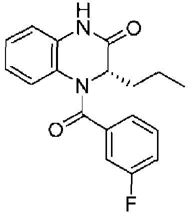 | (S)-4-(3-fluorobenzoyl)-3-propyl-3,4-dihydroquinoxalin-2(1H)-one |
| 18 | 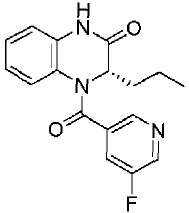 | (S)-4-(5-fluoronicotinoyl)-3-propyl-3,4-dihydroquinoxalin-2(1H)-one |
| 19 | 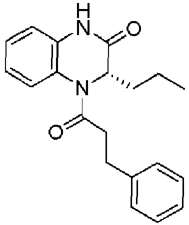 | (S)-4-(3-phénylpropanoyl)-3-propyl-3,4-dihydroquinoxalin-2(1H)-one |
| 20 | 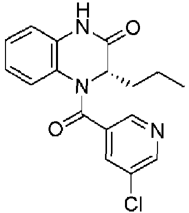 | (S)-4-(5-chloronicotinoyl)-3-propyl-3,4-dihydroquinoxalin-2(1H)-one |
| 21 | 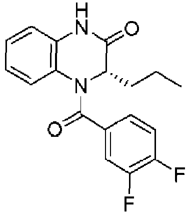 | (S)-4-(3,4-difluorobenzoyl)-3-propyl-3,4-dihydroquinoxalin-2(1H)-one |
| composé n∘ | structure du composé | nom du composé |
|---|---|---|
| 22 | 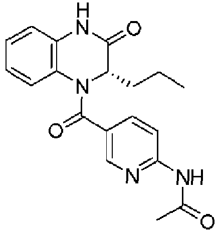 | (S)-N-(5-(3-oxo-2-propyl-1,2,3,4-tétrahydroquinoxaline-1-carbonyl)pyridin-2-yl)acétamide |
| 23 | 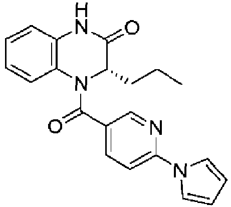 | (S)-4-(6-(1H-pyrrol-1-yl)nicotinoyl)-3-propyl-3,4-dihydroquinoxalin-2(1H)-one |
| 24 | 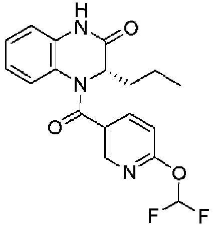 | (S)-4-(6-(difluorométhoxy)nicotino yl)-3-propyl-3,4-dihydroquinoxalin-2(1H)-one |
| 25 | 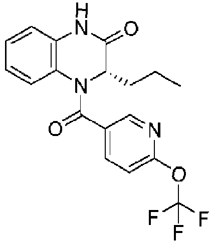 | (S)-3-propyl-4-(6-(trifluorométhoxy)nicotin oyl)-3,4-dihydroquinoxalin-2(1H)-one |
| 26 | 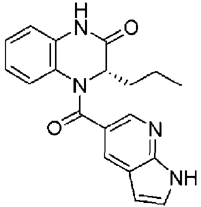 | (S)-3-propyl-4-(1H-pyrrolo[2,3-b]pyridine-5-carbonyl)-3,4-dihydroquinoxalin-2(1H)-one |
| composé n° | structure du composé | nom du composé |
|---|---|---|
| 27 | 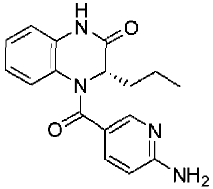 | (S)-4-(6-aminonicotinoyl)-3-propyl-3,4-dihydroquinoxalin-2(1H)-one |
| 28 | 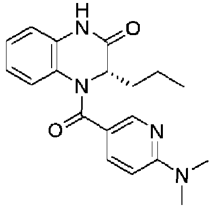 | (S)-4-(6-(diméthylamino)nicotinoyl )-3-propyl-3,4-dihydroquinoxalin-2(1H)-one |
| 29 | 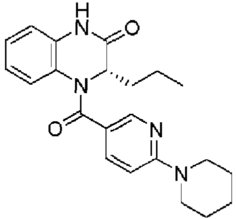 | (S)-4-(6-(pipéridin-1-yl)nicotinoyl)-3-propyl-3,4-dihydroquinoxalin-2(1H)-one |
| 30 | 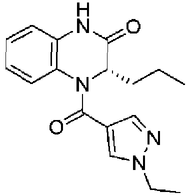 | (S)-4-(1-éthyl-1H-pyrazole-4-carbonyl)-3-propyl-3,4-dihydroquinoxalin-2(1H)-one |
| 31 | 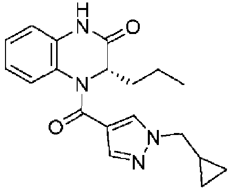 | (S)-4-(1-(cyclopropylméthyl)-1H-pyrazole-4-carbonyl)-3-propyl-3,4-dihydroquinoxalin-2(1H)-one |
| 32 | 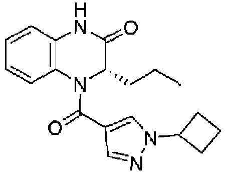 | (S)-4-(1-cyclobutyl-1H-pyrazole-4-carbonyl)-3-propyl-3,4-dihydroquinoxalin-2(1H)-one |
| composé n∘ | structure du composé | nom du composé |
|---|---|---|
| 33 | 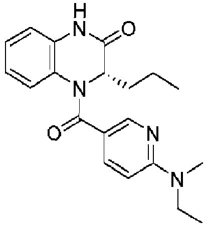 | (S)-4-(6-(éthyl(méthyl)amino)nicot inoyl)-3-propyl-3,4-dihydroquinoxalin-2(1H)-one |
| 34 | 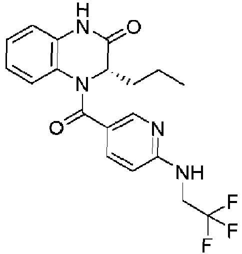 | (S)-3-propyl-4-(6-((2,2,2-trifluoroéthyl)amino)nico tinoyl)-3,4-dihydroquinoxalin-2(1H)-one |
| 35 | 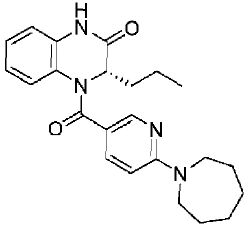 | (S)-4-(6-(azépan-1-yl)nicotinoyl)-3-propyl-3,4-dihydroquinoxalin-2(1H)-one |
| 36 | 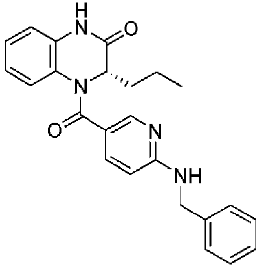 | (S)-4-(6-(benzylamino)nicotinoyl)-3-propyl-3,4-dihydroquinoxalin-2(1H)-one |
| 37 | 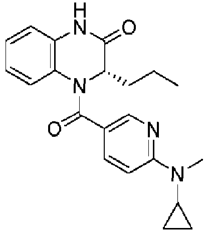 | (S)-4-(6-(cyclopropyl(méthyl)amino )nicotinoyl)-3-propyl-3,4-dihydroquinoxalin-2(1H)-one |
| composé n° | structure du composé | nom du composé |
|---|---|---|
| 38 | 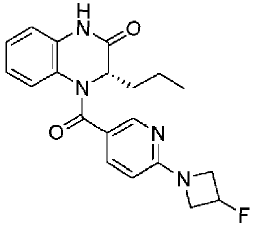 | (S)-4-(6-(3-fluoroazétidin-1-yl)nicotinoyl)-3-propyl-3,4-dihydroquinoxalin-2(1H)-one |
| 39 | 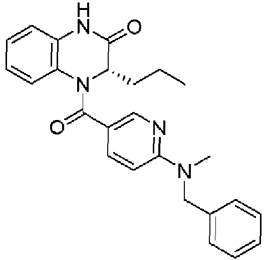 | (S)-4-(6-(benzyl(méthyl)amino)nico tinoyl)-3-propyl-3,4-dihydroquinoxalin-2(1H)-one |
| 40 | 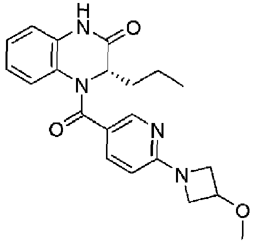 | (S)-4-(6-(3-méthoxyazétidin-1-yl)nicotinoyl)-3-propyl-3,4-dihydroquinoxalin-2(1H)-one |
| 41 | 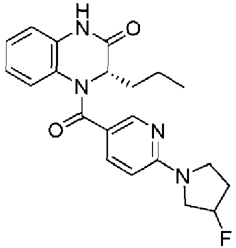 | (3S)-4-(6-(3-fluoropyrrolidin-1-yl)nicotinoyl)-3-propyl-3,4-dihydroquinoxalin-2(1H)-one |
| 42 | 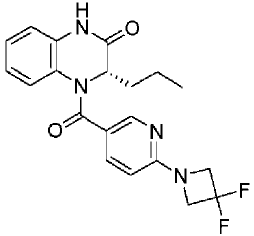 | (S)-4-(6-(3,3-difluoroazétidin-1-yl)nicotinoyl)-3-propyl-3,4-dihydroquinoxalin-2(1H)-one |
| composé n^{∘} | structure du composé | nom du composé |
|---|---|---|
| 43 | 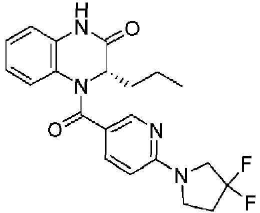 | (S)-4-(6-(3,3-difluoropyrrolidin-1-yl)nicotinoyl)-3-propyl-3,4-dihydroquinoxalin-2(1H)-one |
| 44 | 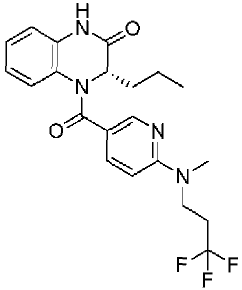 | (S)-4-(6-(méthyl(3,3,3-trifluoropropyl)amino)nic otinoyl)-3-propyl-3,4-dihydroquinoxalin-2(1H)-one |
| 45 | 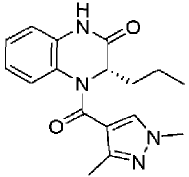 | (S)-4-(1,3-diméthyl-1H-pyrazole-4-carbonyl)-3-propyl-3,4-dihydroquinoxalin-2(1H)-one |
| 46 | 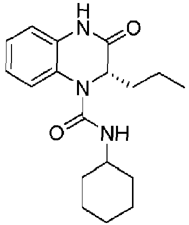 | (S)-N-cyclohexyl-3-oxo-2-propyl-3,4-dihydroquinoxaline-1(2H)-carboxamide |
| 47 | 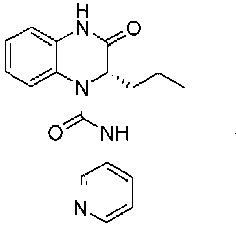 | (S)-3-oxo-2-propyl-N-(pyridin-3-yl)-3,4-dihydroquinoxaline-1(2H)-carboxamide |
| composé n | structure du composé | nom du composé |
|---|---|---|
| 48 | 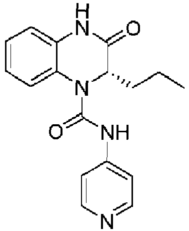 | (S)-3-oxo-2-propyl-N-(pyridin-4-yl)-3,4-dihydroquinoxaline-1(2H)-carboxamide |
| 49 | 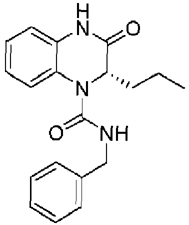 | (S)-N-benzyl-3-oxo-2-propyl-3,4-dihydroquinoxaline-1(2H)-carboxamide |
| 50 | 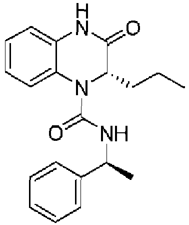 | (S)-3-oxo-N-((S)-1-phényléthyl)-2-propyl-3,4-dihydroquinoxaline-1(2H)-carboxamide |
| 51 | 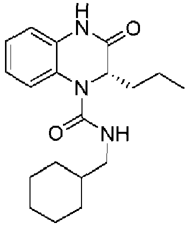 | (S)-N-(cyclohexylméthyl)-3-oxo-2-propyl-3,4-dihydroquinoxaline-1(2H)-carboxamide |
| 52 | 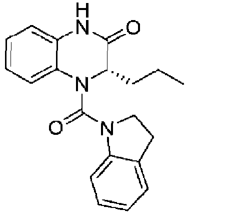 | (S)-4-(indoline-1-carbonyl)-3-propyl-3,4-dihydroquinoxalin-2(1H)-one |
| composé n° | structure du composé | nom du composé |
|---|---|---|
| 53 | 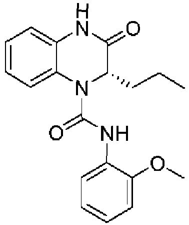 | (S)-N-(2-méthoxyphényl)-3-oxo-2-propyl-3,4-dihydroquinoxaline-1(2H)-carboxamide |
| 54 | 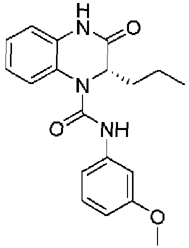 | (S)-N-(3-méthoxyphényl)-3-oxo-2-propyl-3,4-dihydroquinoxaline-1(2H)-carboxamide |
| 55 | 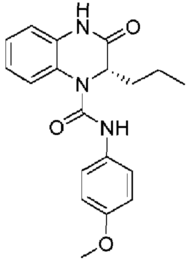 | (S)-N-(4-méthoxyphényl)-3-oxo-2-propyl-3,4-dihydroquinoxaline-1(2H)-carboxamide |
| 56 | 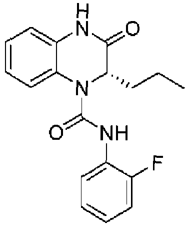 | (S)-N-(2-fluorophényl)-3-oxo-2-propyl-3,4-dihydroquinoxaline-1(2H)-carboxamide |
| 57 | 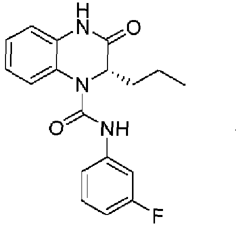 | (S)-N-(3-fluorophényl)-3-oxo-2-propyl-3,4-dihydroquinoxaline-1(2H)-carboxamide |
| 58 | 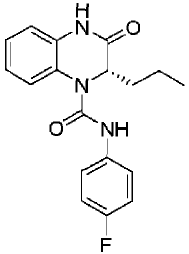 | (S)-N-(4-fluorophényl)-3-oxo-2-propyl-3,4-dihydroquinoxaline-1(2H)-carboxamide |
| 59 | 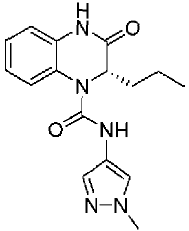 | (S)-N-(1-méthyl-1H-pyrazol-4-yl)-3-oxo-2-propyl-3,4-dihydroquinoxaline-1(2H)-carboxamide |
| 60 | 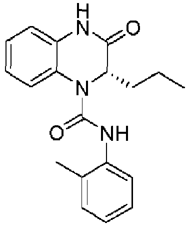 | (S)-3-oxo-2-propyl-N-(o-tolyl)-3,4-dihydroquinoxaline-1(2H)-carboxamide |
| 61 | 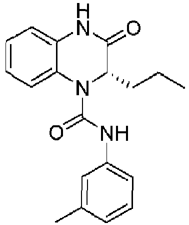 | (S)-3-oxo-2-propyl-N-(m-tolyl)-3,4-dihydroquinoxaline-1(2H)-carboxamide |
| 62 | 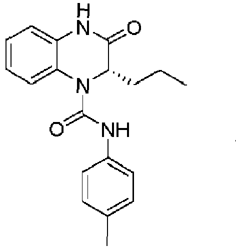 | (S)-3-oxo-2-propyl-N-(p-tolyl)-3,4-dihydroquinoxaline-1(2H)-carboxamide |
| 63 | 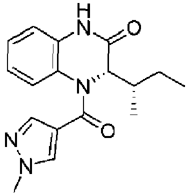 | (S)-3-((S)-sec-butyl)-4-(1-méthyl-1H-pyrazole-4-carbonyl)-3,4-dihydroquinoxalin-2(1H)-one |
| 64 | 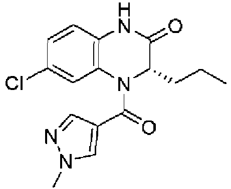 | (S)-6-chloro-4-(1-méthyl-1H-pyrazole-4-carbonyl)-3-propyl-3,4-dihydroquinoxalin-2(1H)-one |
| 65 | 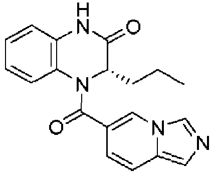 | (S)-4-(imidazo[1,5-a]pyridine-6-carbonyl)-3-propyl-3,4-dihydroquinoxalin-2(1H)-one |
| 66 | 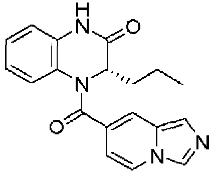 | (S)-4-(imidazo[1,5-a]pyridine-7-carbonyl)-3-propyl-3,4-dihydroquinoxalin-2(1H)-one |
| 67 | 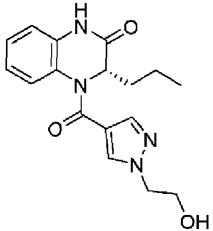 | (S)-4-(1-(2-hydroxyethyl)-1H-pyrazole-4-carbonyl)-3-propyl-3,4-dihydroquinoxalin-2(1H)-one |
| 68 | 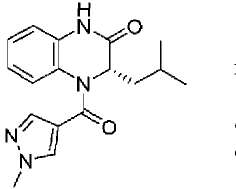 | (S)-3-isobutyl-4-(1-méthyl-1H-pyrazole-4-carbonyl)-3,4-dihydroquinoxalin-2(1H)-one |
| 69 | 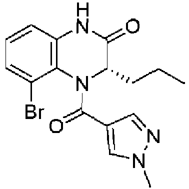 | (S)-5-bromo-4-(1-méthyl-1H-pyrazole-4-carbonyl)-3-propyl-3,4-dihydroquinoxalin-2(1H)-one |
| 70 | 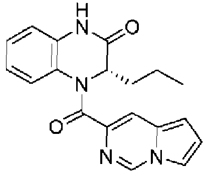 | (S)-3-propyl-4-(pyrrolo[1,2-c]pyrimidine-3-carbonyl)-3,4-dihydroquinoxalin-2(1H)-one |
| 71 | 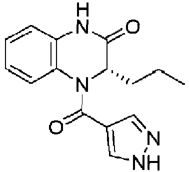 | (S)-3-propyl-4-(1H-pyrazole-4-carbonyl)-3,4-dihydroquinoxalin-2(1H)-one |
| 72 | 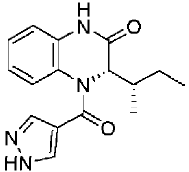 | (S)-3-((S)-sec-butyl)-4-(1H-pyrazole-4-carbonyl)-3,4-dihydroquinoxalin-2(1H)-one |
| 73 | 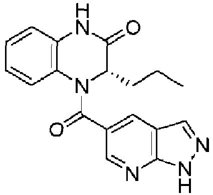 | (S)-3-propyl-4-(1H-pyrazolo[3,4-b]pyridine-5-carbonyl)-3,4-dihydroquinoxalin-2(1H)-one |
| 74 | 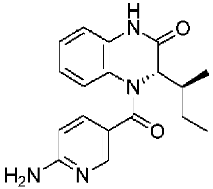 | (S)-4-(6-aminonicotinoyl)-3-((S)-sec-butyl)-3,4-dihydroquinoxalin-2(1H)-one |
| 75 | 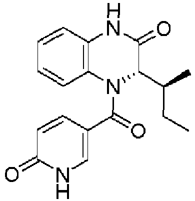 | (S)-3-((S)-sec-butyl)-4-(6-oxo-1,6-dihydropyridine-3-carbonyl)-3,4-dihydroquinoxalin-2(1H)-one |
| 76 | 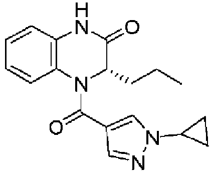 | (S)-4-(1-cyclopropyl-1H-pyrazole-4-carbonyl)-3-propyl-3,4-dihydroquinoxalin-2(1H)-one |
| 77 | 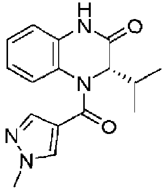 | (S)-3-isopropyl-4-(1-méthyl-1H-pyrazole-4-carbonyl)-3,4-dihydroquinoxalin-2(1H)-one |
| 78 | 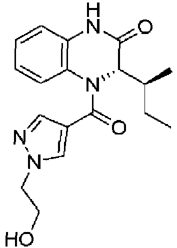 | (S)-3-((S)-sec-butyl)-4-(1-(2-hydroxyéthyl)-1H-pyrazole-4-carbonyl)-3,4-dihydroquinoxalin-2(1H)-one |
| 79 | 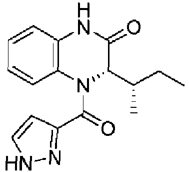 | (S)-3-((S)-sec-butyl)-4-(1H-pyrazole-3-carbonyl)-3,4-dihydroquinoxalin-2(1H)-one |
| 80 | 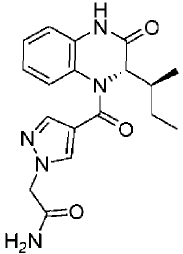 | 2-(4-((S)-2-((S)-sec-butyl)-3-oxo-1,2,3,4-tétrahydroquinoxaline-1-carbonyl)-1H-pyrazol-1-yl)acétamide |
| 81 | 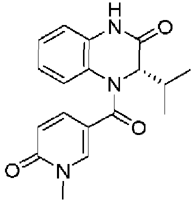 | (S)-3-isopropyl-4-(1-méthyl-6-oxo-1,6-dihydropyridine-3-carbonyl)-3,4-dihydroquinoxalin-2(1H)-one |
| 82 | 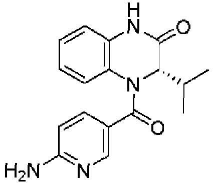 | (S)-4-(6-aminonicotinoyl)-3-isopropyl-3,4-dihydroquinoxalin-2(1H)-one |
| 83 | 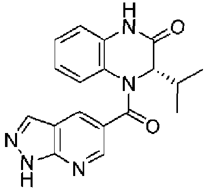 | (S)-3-isopropyl-4-(1H-pyrazolo[3,4-b]pyridine-5-carbonyl)-3,4-dihydroquinoxalin-2(1H)-one |
| 84 | 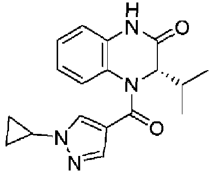 | (S)-4-(1-cyclopropyl-1H-pyrazole-4-carbonyl)-3-isopropyl-3,4-dihydroquinoxalin-2(1H)-one |
| 85 | 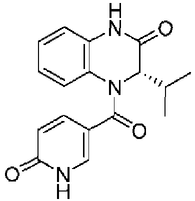 | (S)-3-isopropyl-4-(6-oxo-1,6-dihydropyridine-3-carbonyl)-3,4-dihydroquinoxalin-2(1H)-one |
| 86 | 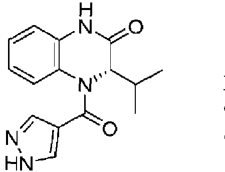 | (S)-3-isopropyl-4-(1H-pyrazole-4-carbonyl)-3,4-dihydroquinoxalin-2(1H)-one |
| 87 | 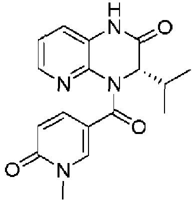 | (S)-3-isopropyl-4-(1-méthyl-6-oxo-1,6-dihydropyridine-3-carbonyl)-3,4-dihydropyrido[2,3-b]pyrazin-2(1H)-one |
| 88 | 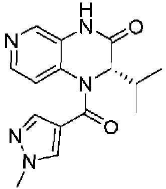 | (S)-2-isopropyl-1-(1-méthyl-1H-pyrazole-4-carbonyl)-1,4-dihydropyrido[3,4-b]pyrazin-3(2H)-one |
| 89 | 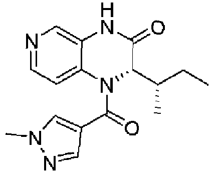 | (S)-2-((S)-sec-butyl)-1-(1-méthyl-1H-pyrazole-4-carbonyl)-1,4-dihydropyrido[3,4-b]pyrazin-3(2H)-one |
| 90 | 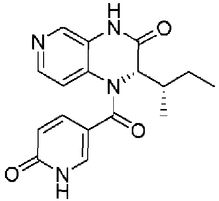 | (S)-2-((S)-sec-butyl)-1-(6-oxo-1,6-dihydropyridine-3-carbonyl)-1,4-dihydropyrido[3,4-b]pyrazin-3(2H)-one |
| 91 | 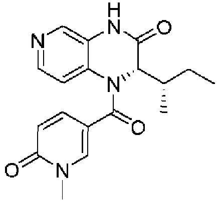 | (S)-2-((S)-sec-butyl)-1-(1-méthyl-6-oxo-1,6-dihydropyridine-3-carbonyl)-1,4-dihydropyrido[3,4-b]pyrazin-3(2H)-one |
| 92 | 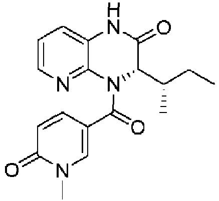 | (S)-3-((S)-sec-butyl)-4-(1-méthyl-6-oxo-1,6-dihydropyridine-3-carbonyl)-3,4-dihydropyrido[2,3-b]pyrazin-2(1H)-one |
| 93 | 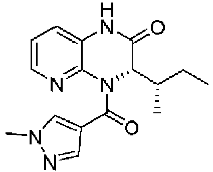 | (S)-3-((S)-sec-butyl)-4-(1-méthyl-1H-pyrazole-4-carbonyl)-3,4-dihydropyrido[2,3-b]pyrazin-2(1H)-one |
| 94 | 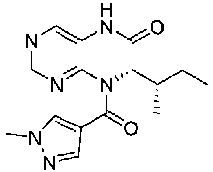 | (S)-7-((S)-sec-butyl)-8-(1-méthyl-1H-pyrazole-4-carbonyl)-7,8-dihydropteridin-6(5H)-one |
| 95 | 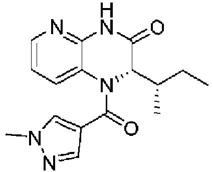 | (S)-2-((S)-sec-butyl)-1-(1-méthyl-1H-pyrazole-4-carbonyl)-1,4-dihydropyrido[2,3-b]pyrazin-3(2H)-one |
| 96 | 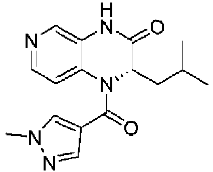 | (S)-2-isobutyl-1-(1-méthyl-1H-pyrazole-4-carbonyl)-1,4-dihydropyrido[3,4-b]pyrazin-3(2H)-one |
| 97 | 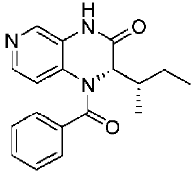 | (S)-1-benzoyl-2-((S)-sec-butyl)-1,4-dihydropyrido[3,4-b]pyrazin-3(2H)-one |
| 98 | 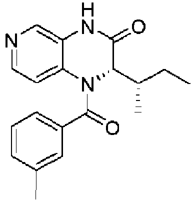 | (S)-2-((S)-sec-butyl)-1-(3-méthylbenzoyl)-1,4-dihydropyrido[3,4-b]pyrazin-3(2H)-one |
| 99 | 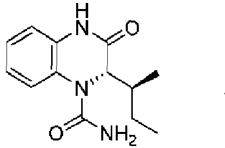 | (S)-2-((S)-sec-butyl)-3-oxo-3,4-dihydroquinoxaline-1(2H)-carboxamide |
| 100 | 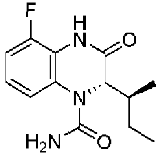 | (S)-2-((S)-sec-butyl)-5-fluoro-3-oxo-3,4-dihydroquinoxaline-1(2H)-carboxamide |
| 101 | 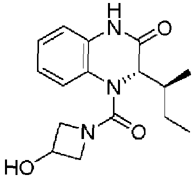 | (S)-3-((S)-sec-butyl)-4-(3-hydroxyazétidine-1-carbonyl)-3,4-dihydroquinoxalin-2(1H)-one |
| 102 | 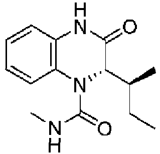 | (S)-2-((S)-sec-butyl)-N-méthyl-3-oxo-3,4-dihydroquinoxaline-1(2H)-carboxamide |
| 103 | 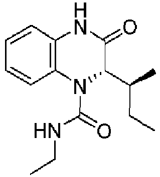 | (S)-2-((S)-sec-butyl)-N-éthyl-3-oxo-3,4-dihydroquinoxaline-1(2H)-carboxamide |
| 104 | 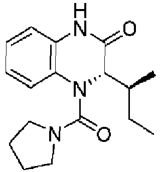 | (S)-3-((S)-sec-butyl)-4-(pyrrolidine-1-carbonyl)-3,4-dihydroquinoxalin-2(1H)-one |
| 105 | 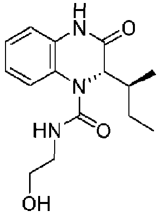 | (S)-2-((S)-sec-butyl)-N-(2-hydroxyéthyl)-3-oxo-3,4-dihydroquinoxaline-1(2H)-carboxamide |
| 106 | 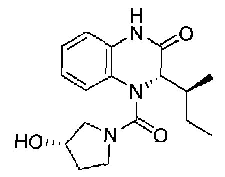 | (S)-3-((S)-sec-butyl)-4-((S)-3-hydroxypyrrolidine-1-carbonyl)-3,4-dihydroquinoxalin-2(1H)-one |
| 107 | 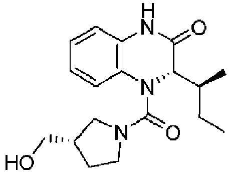 | (S)-3-((S)-sec-butyl)-4-((S)-3-(hydroxyméthyl)pyrrolidin e-1-carbonyl)-3,4-dihydroquinoxalin-2(1H)-one |
| 108 | 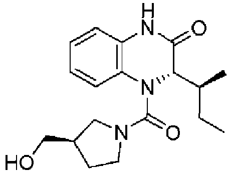 | (S)-3-((S)-sec-butyl)-4-((R)-3-(hydroxyméthyl)pyrrolidin e-1-carbonyl)-3,4-dihydroquinoxalin-2(1H)-one |
| 109 | 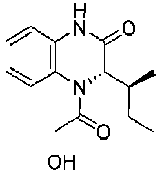 | (S)-3-((S)-sec-butyl)-4-(2-hydroxyacétyl)-3,4-dihydroquinoxalin-2(1H)-one |
| 110 | 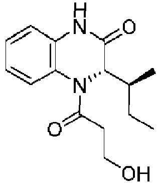 | (S)-3-((S)-sec-butyl)-4-(3-hydroxypropanoyl)-3,4-dihydroquinoxalin-2(1H)-one |
| 111 | 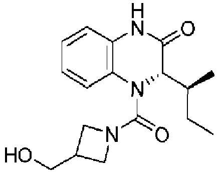 | (S)-3-((S)-sec-butyl)-4-(3-(hydroxyméthyl)azétidine-1-carbonyl)-3,4-dihydroquinoxalin-2(1H)-one |
| 112 | 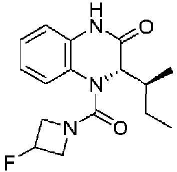 | (S)-3-((S)-sec-butyl)-4-(3-fluoroazétidine-1-carbonyl)-3,4-dihydroquinoxalin-2(1H)-one |
| 113 | 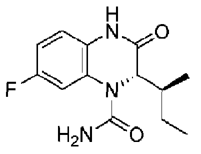 | (S)-2-((S)-sec-butyl)-7-fluoro-3-oxo-3,4-dihydroquinoxaline-1(2H)-carboxamide |
| 114 | 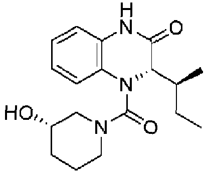 | (S)-3-((S)-sec-butyl)-4-((S)-3-hydroxypipéridine-1-carbonyl)-3,4-dihydroquinoxalin-2(1H)-one |
| 115 | 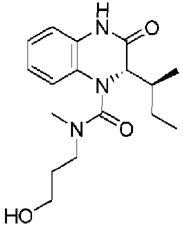 | (S)-2-((S)-sec-butyl)-N-(3-hydroxypropyl)-N-méthyl-3-oxo-3,4-dihydroquinoxaline-1(2H)-carboxamide |
| 116 | 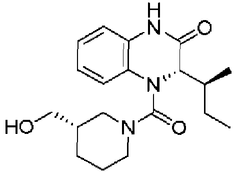 | (S)-3-((S)-sec-butyl)-4-((S)-3-(hydroxyméthyl)pipéridine -1-carbonyl)-3,4-dihydroquinoxalin-2(1H)-one |
| 117 | 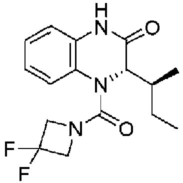 | (S)-3-((S)-sec-butyl)-4-(3,3-difluoroazétidine-1-carbonyl)-3,4-dihydroquinoxalin-2(1H)-one |
| 118 | 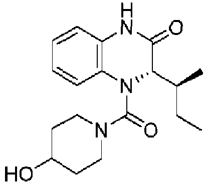 | (S)-3-((S)-sec-butyl)-4-(4-hydroxypipéridine-1-carbonyl)-3,4-dihydroquinoxalin-2(1H)-one |
| 119 | 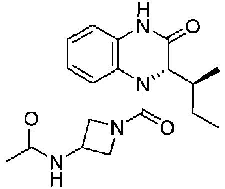 | N-(1-((S)-2-((S)-sec-butyl)-3-oxo-1,2,3,4-tétrahydroquinoxaline-1-carbonyl)azétidin-3-yl)acétamide |
| 120 | 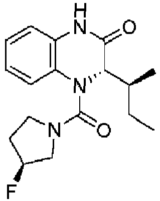 | (S)-3-((S)-sec-butyl)-4-((S)-3-fluoropyrrolidine-1-carbonyl)-3,4-dihydroquinoxalin-2(1H)-one |
| 121 | 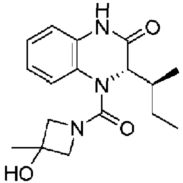 | (S)-3-((S)-sec-butyl)-4-(3-hydroxy-3-méthylazétidine-1-carbonyl)-3,4-dihydroquinoxalin-2(1H)-one |
| 122 | 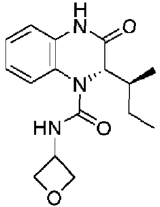 | (S)-2-((S)-sec-butyl)-N-(oxétan-3-yl)-3-oxo-3,4-dihydroquinoxaline-1(2H)-carboxamide |
| 123 | 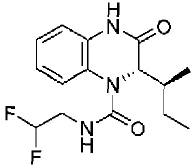 | (S)-2-((S)-sec-butyl)-N-(2,2-difluoroéthyl)-3-oxo-3,4-dihydroquinoxaline-1(2H)-carboxamide |
| 124 | 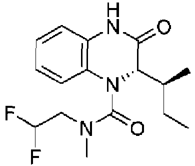 | (S)-2-((S)-sec-butyl)-N-(2,2-difluoroéthyl)-N-méthyl-3-oxo-3,4-dihydroquinoxaline-1(2H)-carboxamide |
| 125 | 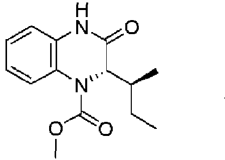 | (S)-2-((S)-sec-butyl)-3-oxo-3,4-dihydroquinoxaline-1(2H)-carboxylate de méthyle |
| 126 | 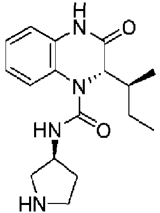 | (S)-2-((S)-sec-butyl)-3-oxo-N-((S)-pyrrolidin-3-yl)-3,4-dihydroquinoxaline-1(2H)-carboxamide |
| 127 | 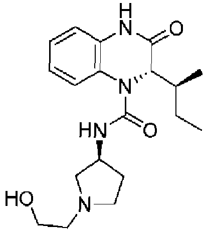 | (S)-2-((S)-sec-butyl)-N-((S)-1-(2-hydroxyéthyl)pyrrolidin-3-yl)-3-oxo-3,4-dihydroquinoxaline-1(2H)-carboxamide |
| 128 | 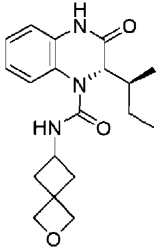 | (S)-2-((S)-sec-butyl)-3-oxo-N-(2-oxaspiro[3.3]heptan-6-yl)-3,4-dihydroquinoxaline-1(2H)-carboxamide |
| 129 | 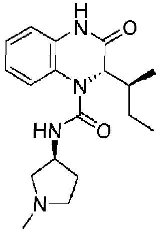 | (S)-2-((S)-sec-butyl)-N-((S)-1-methylpyrrolidin-3-yl)-3-oxo-3,4-dihydroquinoxaline-1(2H)-carboxamide |
| 130 | 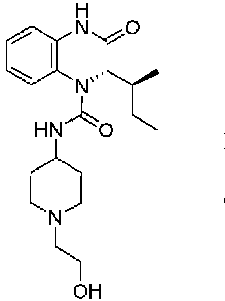 | (S)-2-((S)-sec-butyl)-N-(1-(2-hydroxyéthyl)pipéridin-4-yl)-3-oxo-3,4-dihydroquinoxaline-1(2H)-carboxamide |
| 131 | 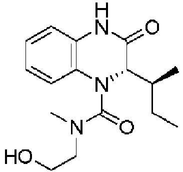 | (S)-2-((S)-sec-butyl)-N-(2-hydroxyéthyl)-N-méthyl-3-oxo-3,4-dihydroquinoxaline-1(2H)-carboxamide |
| 132 | 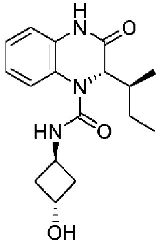 | (S)-2-((S)-sec-butyl)-N-((1r,3S)-3-hydroxycyclobutyl)-3-oxo-3,4-dihydroquinoxaline-1(2H)-carboxamide |
| 133 | 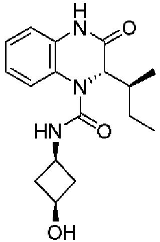 | (S)-2-((S)-sec-butyl)-N-((1s,3R)-3-hydroxycyclobutyl)-3-oxo-3,4-dihydroquinoxaline-1(2H)-carboxamide |
ou sel pharmaceutiquement acceptable correspondant.

10. Composé selon la revendication 1 de la formule : 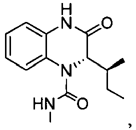 ou sel pharmaceutiquement acceptable correspondant.

11. Composé selon la revendication 1 de la formule : 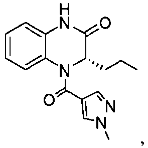 ou sel pharmaceutiquement acceptable correspondant.

12. Composé selon la revendication 1 de la formule : 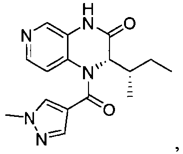 ou sel pharmaceutiquement acceptable correspondant.

13. Composition pharmaceutique comprenant un composé selon l'une quelconque des revendications 1 à 12, ou un sel pharmaceutiquement acceptable de celui-ci, et un support pharmaceutique.

14. Composition pharmaceutique de la revendication 13, dans laquelle la composition pharmaceutique est formulée pour une administration orale, sublinguale, sous-cutanée, parentérale, intraveineuse, intranasale, topique, transdermique, intrapéritonéale, intramusculaire, intrapulmonaire, vaginale, rectale ou intraoculaire.

15. Composé de l'une quelconque des revendications 1 à 12, ou sel pharmaceutiquement acceptable de celui-ci, ou composition pharmaceutique de la revendication 13 ou 14, pour utilisation dans le traitement d'une maladie ou d'une affection choisie dans le groupe constitué par :
(a) maladie vasculaire périphérique, maladie artérielle périphérique, déficits liés à la réadaptation, syndrome métabolique, obésité, faiblesse musculaire induite par le ventilateur, syndrome de fatigue chronique, troubles neuromusculaires, affections de fonte musculaire, myopathies musculaires, atrophie musculaire, fatigue musculaire et fragilité chez un sujet ;
(b) sclérose latérale amyotrophique (SLA), amyotrophie spinale (SMA), myasthénie grave et myopathies musculaires chez un sujet ; ou
(c) incontinence urinaire d'effort (IUE), incontinence urinaire mixte (IUM), incontinence fécale, fragilité, sarcopénie, bronchopneumopathie chronique obstructive (BPCO), syndrome de cachexie, fonte musculaire causée par une insuffisance cardiaque, un cancer ou une maladie rénale chronique/dialyse, dysfonctionnement musculaire post-lésion médullaire (LM) et dysfonctionnement musculaire post-AVC chez un sujet.
